# FASCICULE DE BREVET EUROPEEN

(11) **EP 2 238 126 B1**
(45) Date de publication et mention de la délivrance du brevet: **05.11.2014**
(21) Numéro de dépôt: 09720707.0
(22) Date de dépôt: 27.01.2009
(51) Int. Cl.: C07D 401/12, C07D 401/10, C07D 417/04, A61K 31/495, A61K 31/506, A61P 3/00, A61P 9/12, A61P 25/28, A61P 27/06

(54) **DÉRIVÉS D'URÉE DE TETRAHYDROQUINOXALINE, LEUR PRÉPARATION ET LEUR APPLICATION EN THÉRAPEUTIQUE**
TETRAHYDROCHINOXALIN-HARNSTOFF-DERIVATE, DEREN HERSTELLUNG UND THERAPEUTISCHE ANWENDUNG
DERIVATIVES OF TETRAHYDROQUINOXALINE UREA, PREPARATION THEREOF AND THERAPEUTIC APPLICATION THEREOF

(30) Priorité: 28.01.2008 FR 0800429; 08.08.2008 FR 0804521
(43) Date de publication de la demande: 13.10.2010
(73) Titulaire: SANOFI, 75008 Paris (FR)
(72) Inventeur: BRAUN, Alain, F-75013 Paris (FR); CRESPIN, Olivier, F-75013 Paris (FR); NAMANE, Claudie, F-75013 Paris (FR); NICOLAI, Eric, F-75013 Paris (FR); PACQUET, François, F-75013 Paris (FR); PASCAL, Cécile, F-75013 Paris (FR); PHILIPPO, Christophe, F-75013 Paris (FR); VENIER, Olivier, F-75013 Paris (FR)
(74) Mandataire: Böhm, Brigitte
(86) Numéro de dépôt international: PCT/FR2009/000083
(87) Numéro de publication internationale: WO 2009/112691

(56) Documents cités:
- WO-A-2004/056745
- WO-A-2008/000951

## Description

La présente invention se rapporte à des dérivés d'urée de tétrahydroquinoxaline, à leur préparation et à leur application en thérapeutique.

Les composés selon l'invention modulent l'activité de la 11β-hydroxystéroïde déhydrogénase type 1 (11βHSD1) et sont utiles pour le traitement des pathologies dans lesquelles une telle modulation est bénéfique, comme dans le cas du syndrome métabolique ou du diabète de type 2 non insulino dépendant.

La 11βHSD1 catalyse localement la conversion de glucocorticoïdes inactifs (cortisone chez l'Homme) en glucocorticoïdes actifs (cortisol chez l'Homme) dans différents tissus et organes, principalement le foie et le tissu adipeux, mais aussi dans les muscles, les os, le pancréas, l'endothélium, le tissu oculaire et dans certaines parties du système nerveux central. La 11βHSD1 agit comme un régulateur de l'action des glucocorticoïdes dans les tissus et organes où elle est exprimée (Tomlinson et al., Endocrine Reviews 25(5), 831-866 (2004), Davani et al., J. Biol. Chem. 275, 34841 (2000) ; Moisan et al., Endocrinology, 127, 1450 (1990)).

Les principales pathologies dans lesquelles interviennent les glucocorticoïdes et l'inhibition de la 11βHSD1 sont indiquées ci-après.

### A. Obésité, diabète de type 2 et syndrome métabolique

Le rôle de la 11βHSD1 dans l'obésité, le diabète de type 2 et le syndrome métabolique (aussi connu sous le nom de syndrome X ou syndrome de résistance à l'insuline) où les symptômes incluent l'obésité viscérale, l'intolérance au glucose, la résistance à l'insuline, l'hypertension, le diabète de type 2 et l'hyperlipidémie (Reaven Ann. Rev. Med 44, 121 (1993)) est décrit dans de nombreuses publications. Chez l'Homme, le traitement par la carbenoxolone (un inhibiteur non spécifique de la 11βHSD1) améliore la sensibilité à l'insuline chez des patients volontaires minces et chez des diabétiques de type 2 (Andrews et al., J. Clin. Endocrinol. Metab. 88, 285 (2003)). De plus, les souris dont le gène de la 11βHSD1 a été éteint sont résistantes à l'hyperglycémie induite par le stress et l'obésité, montrent une atténuation de l'induction d'enzymes hépatiques de la néoglucogenèse (PEPCK et G6P) et présentent une augmentation de la sensibilité à l'insuline dans le tissu adipeux (Kotelevstev et al., Proc. Nat Acad. Sci. 94, 14924 (1997) ; Morton et al., J. Biol. Chem. 276, 41293 (2001)). Par ailleurs, les souris transgéniques où le gène de la 11βHSD1 a été surexprimé dans les tissus adipeux présentent un phénotype similaire à celui du syndrome métabolique humain (Masuzaki et al., Science 294, 2166 (2001)). Il est à noter que le phénotype observé existe sans une augmentation du total des glucocorticoïdes circulants, mais est induit par l'augmentation spécifique de glucocorticoïdes actifs dans les dépôts adipeux.
Par ailleurs, de nouvelles classes d'inhibiteurs spécifiques de la 11βHSD1 sont apparues récemment :
- des arylsulfonamidothiazoles ont montré qu'ils amélioraient la sensibilité à l'insuline et réduisaient le niveau de glucose dans le sang de souris présentant une hyperglycémie (Barf et al., J. Med. Chem. 45, 3813 (2002)). De plus, dans une étude récente, il a été montré que ce type de composés réduisait la prise de nourriture ainsi que la prise de poids chez des souris obèses (Wang et Coll. Diabetologia 49, 1333 (2006*)*) ;
- des triazoles ont montré qu'ils amélioraient le syndrome métabolique et ralentissaient la progression de l'athérosclérose chez des souris (Hermanowski-Vosatka et al., J. Exp. Med. 202, 517 (2005)).

### A2. Complications microvasculaires du diabète

La présence des complications chroniques chez le diabétique de type 2 est souvent associée à la sévérité et à la durée du diabète. Les troubles microvasculaires fonctionnels et structuraux expliquent en grande partie le développement de certaines pathologies observées chez le diabétique telles la neuropathie, la retinopathie, et la nephropathie (Rayman, Diabetes Review 7 :261-274, 1999 ; Gärtner and Eigentler, Clin Nephrol 70 :1-9, 2008 ; Zent and Pozzi, Sem Nephrol 27 :161-171, 2007; Malecki et al., EJCI38 :925-930, 2008). L'élévation chronique de la glycémie, ou l'intolérance au glucose, représentent des facteurs de risque majeurs de ces complications microvasculaires (Robinson Singleton et al. Diabetes 52 :2867-2873, 2003 ; Lachin et al. Diabetes 57: 995-1001, 2008). En permettant un meilleur contrôle de la glycémie, grâce à une baisse de la néoglucogénèse hépatique et une augmentation de la sensibilité à l'insuline de l'organisme (voir chapitre « obésité, diabète de type 2 et syndrome métabolique »), des inhibiteurs de 11β-HSD1 peuvent éviter la progression vers les complications microvasculaires observées chez le diabétique. Cependant, le strict contrôle de la glycémie ne permet pas d'éviter entièrement le développement des complications microvasculaires, et rend nécessaire la découverte de nouveaux traitements permettant traiter plus globalement les patients diabétiques et dyslipidémiques (Girach et al. Int J Clin Pract 60(11) : 1471-1483, 2006 ; Taylor. Curr Diab Rep 8 (5) : 345-352 ; 2008). De manière intéressante, une étude de Chiodini et al. (Diabetes Care 30 : 83-88, 2007) a montré que la sécrétion de cortisol, chez les patients diabétiques, était directement associée à la présence de complications macro- ou micro-vasculaires chroniques. Par ailleurs, la réactivité microvasculaire et la fonction endothéliale sont altérées chez le patient souffrant du syndrome de Cushing, présentant un hypercortisolisme (Prazny et al. Physiol Rev 57 : 13-22, 2008).

Plus particulièrement, Bhatia et al. (Ann Ophtalmol 15 :128-130 ; 1983) ont montré une association entre les taux de cortisol plasmatiques élevés et la rétinopathie chez le diabétique.

Koh et al. ont montré que le traitement de patients atteints du syndrome de Cushing par adrenalectomie, permettant de réverser l'hypercortisolisme, améliore la fonction rénale.

Les paramètres cliniques de polyneuropathies (perception sensorielle, neuropathie autonome cardiaque) sont associés à une augmentation de la sécrétion de cortisol chez les patients diabétiques (Tsigos et al. J Clin Endocrinol Metab 76 :554-558, 1993).

Tous ces éléments montrent qu'une diminution de l'impact du cortisol par une inhibition locale de sa régénération, via des inhibiteurs de 11β-HSD1, pourrait avoir un rôle favorable dans les troubles de la microcirculation associés au diabète (polyneuropathie, rétinopathie, et la néphropathie).

### B. Cognition et démence

Les troubles cognitifs légers sont des phénomènes communs aux personnes âgées et aux diabétiques de type 1 et 2, et peuvent conduire progressivement à la dépression ou la démence (Messier et al., Neurobiol. aging 26, 26; Greenwood et al.(2005), Neurobiol. aging 26,45 (2005)). Autant dans le cas d'animaux que d'humains âgés, les différences inter-individuelles pour les fonctions cognitives générales ont été reliées aux différences d'exposition à long terme aux glucocorticoïdes (Lupien et al., Nat. Neurosci. 1, 69, (1998)). Par ailleurs, la dérégulation de l'axe HPA (hypothalamo-hypophyso-surrénalien)) résultant dans l'exposition chronique aux glucocorticoïdes de certaines sous-régions du cerveau a été proposée comme contribuant au déclin des fonctions cognitives (Mc Ewen et al., Curr. Opin. Neurobiol. 5, 205, 1995). La 11βHSD1 est abondante dans le cerveau et est exprimée dans de nombreuses sous régions incluant l'hypothalamus, le cortex frontal et le cerebellum (Sandeep et al., Proc. Natl. Acad. Sci. 101, 6734 (2004)). Les souris déficientes en 11βHSD1 sont protégées contre les dysfonctionnements de l'hypothalamus associés aux glucocorticoïdes qui sont rattachés à la vieillesse (Yau et al., Proc. Natl. Acad. Sci. 98, 4716, (2001)). De plus, dans des études chez l'Homme, il a été montré que l'administration de la carbenoxolone améliore la fluidité verbale et la mémoire verbale chez les personnes âgées (Yau et al., Proc. Natl. Acad. Sci. 98, 4716 (2001), Sandeep et al., Proc. Natl. Acad. Sci. 101, 6734 (2004)). Finalement, l'utilisation d'inhibiteurs sélectifs de la 11βHSD1 de type triazole a montré qu'ils prolongeaient la rétention de la mémoire chez des souris âgées (Rocha et al., Abstract 231 ACS meeting, Atlanta, 26-30 Mars 2006). Par ailleurs, il a été montré dans des modèles de rongeurs diabétiques que le taux de corticosterone contribuait au développement des pathologies cognitives induites par le diabète (Stranhan et al., Nature neurosc. 11,309 (2008)). Anisi, des inhibiteurs de la 11 bHSD1, permettant une réduction de la régénération du cortisol au niveau de l'hyppocampe, pourraient avoir un rôle bénéfique sur les fonctions cognitives chez les patients diabétiques âgés (Sandeep et al., Proc. Natl. Acad. Sci. 101, 6734 (2004)).

### C. Pression intra-oculaire

Les glucocorticoïdes peuvent être utilisés par voies topique ou systémique pour une grande variété de pathologies de l'ophtalmologie clinique. Une complication particulière de ces traitements est le glaucome induit par l'utilisation de corticostéroïdes. Cette pathologie est caractérisée par l'augmentation de la pression intra-oculaire (PIO). Dans les cas les plus graves et pour les formes non traitées, la PIO peut conduire à une perte de champ de vision partielle et éventuellement à une perte totale de la vision. La PIO est le résultat d'un déséquilibre entre la production d'humeur aqueuse et son drainage. L'humeur aqueuse est produite dans les cellules épithéliales non-pigmentées et le drainage est réalisé au travers des cellules du réseau trabéculaire. La 11βHSD1 est localisée dans les cellules épithéliales non-pigmentées et sa fonction est clairement l'amplification de l'activité des glucocorticoïdes dans ces cellules (Stokes et al., Invest. Ophthalmol. Vis. Sci. 41, 1629 (2000)). Cette notion est confirmée par l'observation que la concentration en cortisol libre est fortement excédentaire par rapport à la cortisone dans l'humeur aqueuse (ratio 14/1). L'activité fonctionnelle de la 11βHSD1 dans les yeux a été évaluée en étudiant l'action de la carbenoxolone chez des volontaires sains. Après sept jours de traitement à la carbenoxolone, la PIO est réduite de 18% (Rauz et al., Invest. Ophtamol. Vis. Sci. 42, 2037 (2001)). L'inhibition de la 11βHSD1 dans les yeux est donc prédite comme réduisant la concentration locale en glucocorticoïdes et la PIO, produisant un effet bénéfique dans le traitement du glaucome et d'autres désordres de la vision.

### D. Hypertension

Les substances hypertensives issues des adipocytes comme la leptine et l'angiotensinogène ont été proposées comme étant des éléments clés dans les pathologies d'hypertension reliées à l'obésité (Wajchenberg et al., Endocr. Rev. 21, 697 (2000)). La leptine qui est secrétée en excès chez les souris aP2-11βHSD1 transgéniques (Masuzaki et al., J. Clinical Invest. 112, 83 (2003)), peut activer différents réseaux de systèmes neuronaux sympathiques, incluant ceux qui régulent la pression artérielle (Matsuzawa et al., Acad. Sci. 892, 146 (1999)). De plus, le système rénine-angiotensine (SRA) a été identifié comme étant une voie déterminante dans la variation de la pression artérielle. L'angiotensinogène, qui est produit dans le foie et le tissu adipeux, est un substrat-clé pour la rénine et est à l'origine de l'activation du SRA. Le niveau plasmatique en angiotensiogène est significativement élevé dans les souris aP2-11βHSD1 transgéniques, comme le sont ceux de l'angiotensine Il et de l'aldostérone (Masuzaki et al., J. Clinical Invest. 112, 83 (2003)) ; ces éléments conduisent à l'élévation de la pression artérielle. Le traitement de ces souris par de faibles doses d'un antagoniste du récepteur de l'angiotensine Il abolit cette hypertension (Masuzaki et al., J. Clinical Invest. 112, 83 (2003)). Ces informations illustrent l'importance de l'activation locale des glucocorticoïdes dans le tissu adipeux et le foie, et suggère que cette hypertension puisse être causée ou exacerbée par l'activité de la 11βHSD1 dans ces tissus. L'inhibition de la 11βHSD1 et la réduction du niveau de glucocorticoïdes dans le tissu adipeux et/ou dans le foie est donc prédit comme ayant un rôle bénéfique pour le traitement de l'hypertension et des pathologies cardiovasculaires associées.

### D2. Hypertension artérielle sensible au sel

On estime qu'environ 30 à 50% de la population générale présente une sensibilité particulière au sel. De nombreuses évidences suggèrent un lien entre la sensibilité au sel et l'hypertension artérielle et les risques cardiovasculaires (Weinberger MH, Curr Opin Cardiol 2004 ; 19 :353-356). Il a été montré que les sujets sensibles au sel présentent une variabilité de la fréquence cardiaque diminuée, ainsi qu'une pression artérielle et une production de cortisol augmentée durant un stress mental, comparativement à des sujets non sensibles (Weber CS et al., Journal of Human Hypertension 2008; 22 :423-431). Par ailleurs, une récente étude de Liu Y et al. (Physiol Genomics 2008 Sept 30) a démontré chez le Dahl salt-sensitive rat, que l'inhibition spécifique de l'expression de la 11β-HSD1 médullaire rénale, par l'utilisation de shRNA, permet de diminuer très nettement chez les animaux l'élévation de la pression artérielle moyenne induite par un régime salé. Ces éléments permettent de penser qu'un inhibiteur de l'enzyme 11β-HSD1 aurait très probablement un effet bénéfique sur cette forme d'hypertension artérielle.

### E. Ostéoporose

Le développement du squelette et les fonctions osseuses sont aussi régulées par l'action des glucocorticoïdes. La 11βHSD1 est présente dans les ostéoclastes et ostéoblastes. Le traitement de volontaires sains par la carbenoxolone a montré une diminution des marqueurs de résorption osseuse sans changement dans les marqueurs de formation des os (Cooper et al., Bone, 27, 375 (2000)). L'inhibition de la 11βHSD1 et la réduction du niveau de glucocorticoïdes dans les os pourraient donc être utilisées comme un mécanisme de protection dans le traitement de l'ostéoporose.

### F. Lipodystrophie associée à une thérapie antirétrovirale hautement active (HAART), ou HAL syndrome.

L'utilisation d'un traitement antiretroviral intensif pour les patients atteints du sida induit souvent un syndrome de lipodystrophie (HAL) ressemblant au syndrome de Cushing, et associant augmentation de la masse grasse abdominale, hypertriglycéridémie et résistance à l'insuline. Il a été montré (Sutinen et al., Diabetologia, 47,1668 (2004)) que cette lipodystrophie (HAL) était associée à une augmentation de l'expression la 11βHSD1 dans le tissu adipeux des patients. Des inhibiteurs de la 11βHSD1, permettant une réduction de la régénération du cortisol au niveau du tissu adipeux, pourraient donc avoir un rôle bénéfique chez les patients atteints de lipodystrophie associée à un traitement intensif du sida par des antitretroviraux (HAL syndrome).

### G. Maladie infectieuses

Certaines intections, comme la tuberculose, sont associées à des dérèglements de la réponse immunitaire (Ellner JJ, J. Lab. Clin. Med, 130, 469, (1997)). Cette particularité, qui s'accompagne le plus souvent de l'augmentation de la sécrétion de certaines cytokines (IL-10, TNFα) et/ou de la réponse à certaines cytokines, semble trouver, au moins en partie, sa cause dans l'exposition tissulaire locale des cellules immunitaires au glucocorticoides. Par ailleurs, l'administration de glucocorticoides de synthèse chez l'homme ou l'animal provoque la reactivation de la tuberculose chez l'homme et chez l'animal (Haanas OC et al. Eur. J. Repir. Dis. 64, 294 (1998), Brown et al. Infect. Immun. 63, 2243, (1995)). De même, les stress divers, activateurs de l'axe HPA, ont pour conséquence une réactivation de cette infection.
En dehors de ces cas particuliers, les taux circulants de glucocorticoides ainsi que l'activation de l'axe HPA, semblent être normaux chez des patients atteints de tuberculose (Baker et al. Am. J Resp.Crit. Care. Med., 162, 1641 (2000)). Par contre, les taux de cortisol versus cortisone dans le liquide brochoalvéolaire, semblent augmentés, traduisant une modulation du métabolisme des glucocorticoides vers la forme active (notamment dépendante de l'activité de la 11βHSD1). L'inhibition de la 11βHSD1 au niveau des tissus périphérique et notamment des poumons, pourrait par conséquent produire un effet bénéfique sur la stabilisation puis sa reversion de l'infection.

### H. Hypertrophie cardiaque et insuffisance cardiaque

Les maladies cardiovasculaires représentent la première cause de morbidité et de mortalité dans les pays industrialisés, et l'hypertrophie ventriculaire gauche (HVG) est un facteur de risque indépendant de mortalité cardiovasculaire (Havranek EP, Am J Med 121 :870-875, 2008). En dehors des causes génétiques, des conditions pathologiques telles que l'hypertension artérielle, l'infarctus du myocarde, ou l'insuffisance rénale, peuvent conduire à une hypertrophie compensatoire, progressant par la suite vers une insuffisance cardiaque chronique. L'activité 11β-HSD1, permettant la conversion de 11-dehydrocorticosterone en corticosterone, est exprimée dans les cardiomyocytes de rats nouveaux-nés, et contribue à l'activité modulatrice des glucocorticoïdes et de l'aldosterone dans le coeur (Sheppard and Autelitano, Endocrinology 143:198-204, 2002). En utilisant ces cellules, Lister et al. (Cardiovascular Research 70: 555-565, 2006) ont montré que l'hypertrophie des cardiomyocytes, induite par des agents pharmacologiques, s'accompagnait d'une augmentation de l'activité de l'enzyme 11β-HSD1. Dans cette même étude, l'utilisation du RU-486, antagoniste spécifique des récepteurs aux glucocorticoïdes, permettait de diminuer l'hypertrophie des cellules.

Des inhibiteurs de l'activité 11β-HSD1 pourraient donc limiter l'hypertrophie cardiaque et ainsi d'éviter la progression vers l'insuffisance cardiaque.

### I. Maladies hépatiques :

### I1. Stéatose hépatique :

Des études chez des obèses sévères (BMI > 35 kg/m2) rapportent une prévalence de 91 % pour la stéatose et de 37 % pour la stéatohépatite *(*Neuschwander-Tetri & Caldwell, Hepatology, 37, 1202 - 1219, 2003*).* Le diabète de type 2 est un autre facteur majeur associé à la stéatose avec une prévalence de 70 % rapportée sur un échantillon de 3000 diabétiques italiens (Targher et al., Diabetes Care, 30, 1212 - 1218, 2007)*.* Par ailleurs, il a été observé une association entre l'insulinorésistance et la stéatose hépatique indépendamment de l'obésité chez les patients atteints de stéatose hépatique non alcoolique (Manchesini et al., Diabetes, 50, 1844 - 1850, 2001*).* Chez les obèses, l'activité 11betaHSD1 paraît modifiée ainsi qu'en témoigne l'activation de la cortisone administrée oralement, l'excrétion urinaire des métabolites du cortisol ou l'expression hépatique tissulaire de la 11 betaHSD1. (Tomlinson et al., Endocrine Rev, 25, 831 - 866, 2004 *;* Rask et al., J. Clin. Endocrin. Metab., 86, 1418 - 1421, 2001*;* Stewart et al., J. Clin. Endocrin. Metabol. 84, 1022-1027, 1999 *;* Valsamakis et al., J. Clin. Endocrinol. Metabol., 89, 4755 - 4761, 2004*)* Les souris transgéniques surexprimant 11 betaHSD1 au niveau du tissu adipeux ou au niveau hépatique développent une stéatose hépatique et une dyslipidémie *(*Masuzaki et al., Sciences 294, 2166 - 2170, 2001 *;* Paterson et al., PNAS, 101, 7088 - 7093, 2004*).* L'inhibition 11 betaHSD1 chez le rat réduit la triglycéridémie à jeûn suite à une diminution de la sécrétion des triglycérides hépatiques et à une augmentation dé la capture et de l'oxydation tissulaire des acides gras, qui se traduit également au niveau hépatique par une réduction significative des triglycérides *(*Berthiaume et al., Am. J. Physiol. Endocrino/. Metab., 293, 1045 - 1052, 2007*).* La réduction locale de glucocorticoïde actif par inhibition de l'activité 11 betaHSD1 est donc envisagée pour diminuer les effets insulinorésistants et lipidiques des glucocorticoïdes et ainsi diminuer la stéatose hépatique.

### 12. Stéatohépatite Métabolique :

La stéatohépatite métabolique représente un stade d'évolution de la stéatose hépatique métabolique chez certaines personnes. Une corrélation est décrite entre le cortisol urinaire, la concentration en cortisol post-dexaméthasone et le grade de nécroinflammation et de fibrose hépatique chez des sujets atteints de stéatohépatite métabolique suggérant l'existence d'un hypercorticolisme subclinique ou locale (Targher et al., Clin. Endocrinol., 64, 337 - 341, 2006).Une correction générale et local (au niveau centrolobulaire) de l'insulinorésistance ainsi qu'une amélioration de l'oxydation des acides gras hépatiques par inhibition de l'activité 11 betaHSD1, et aussi la réduction des effets pro-fibrotiques du cortisol sont donc prédictifs d'une amélioration de l'évolution pathologique.

### 13. Régénération hépatique :

Le foie présente une capacité de régénération importante, tout à fait nécessaire en cas d'agressions d'origines infectieuses ou non, en particulier provenant du tractus digestif. Par exemple une apoptose ou une nécrose hépatique peuvent résulter d'une toxicité médicamenteuse, virale, alcoolique, métabolique, cholestatique ou ischémique vasculaire. Les glucocorticoïdes inhibent la prolifération hépatocytaire et la régénération tissulaire hépatique (Tsukamoto & Kojo, Gut, 30, 387 - 390, 1989 *;* Nagy et al., Hepatology, 28, 423 - 429, 1998 ; Tannuri et al., Pediatr. Transplantation, 12, 73-79, 2008).Une inhibition de l'activité réductase 11betaHSD1 pourrait dans ce contexte diminuer les effets locaux négatifs du cortisol sur la régénération hépatique et sont à mettre en ligne avec les effets pro-angiogéniques de ces inhibiteurs et avec leur action positive sur certains facteurs de croissance.

### J. Cicatrisation des plaies cutanées chroniques :

La cicatrisation des plaies chroniques dépend du contexte pathologique sous-jacent qui modifie et désynchronise les étapes physiologiques de la cicatrisation. Dans l'ulcère chronique du diabétique, l'intérêt potentiel des inhibiteurs de 11 betaHSD1 doit se voir à la fois dans la correction des manifestations du diabète, en tenant compte du rôle pathologique local des corticoides endogènes au niveau de la plaie et de l'état d'avancement pathologique. Il existe un certain nombre d'évidences montrant que les corticoïdes endogènes sont directement impliqués dans l'altération de la cicatrisation des plaies chez l'homme et dans des modèles animaux rongeurs (Goforth et al., J. Foot Surgery, 19, 199 - 2002, 1980 *;* Dostal et al, Arch. Surg, 125, 636 -640, 1990 *;* Bitard, Am. J. Pathology, 152, 547 - 554, 1998). Une production locale de cortisol est prédite par la présence d'une activité réductase 11 betaHSD1 au niveau endothélial, fibroblastique, cutané chez l'homme et chez les rongeurs (Gong et al., Steroids 73, 1187 -1196, 2008 *; ;* Hammami et al., J. Clin. Endocrinol. Metabol., 73, 326 - 334, 1991 *;* Cooper et al., ENDO 2003 *;* Teelucksingh et al., Lancet, 335, 1060 - 1063, 1990). Le cortisol et autres glucocorticoïdes inhibent la cicatrisation de l'ulcère cutané par de nombreux mécanismes et à différents étapes : altération de la vasomotricité microcirculatoire, inhibition de la phase inflammatoire en particulier sur la synthèse de prostaglandines, de leukotriènes, de cytokines, comme TNFalpha et les productions de IL-1beta, IL-4.... et la signalisation de IFNgamma, augmentation de l'infection, réduction de la motilité cellulaire et de la prolifération des kératinocytes, réduction de l'expression de facteurs pro-angiogéniques comme VEGF, suppression de l'expression de TGFbeta 1 et 2 essentiels dans la production de collagène par les fibroblastes et leur transformation en myofibroblastes, suppression de l'expression de MMP1, 2 , 9 et 10 et induction de TIMP bloquant ainsi le remodelage, promotion de la différentiation terminale épidermique mais inhibition des premiers stades de différentiation, avec pour conséquence une fragilisation de l'épiderme (Bitard, Am. J. Pathology, 152, 547 - 554, 1998, Beer et al., Vitam. Horm., 59, 217 - 239, 2000 *;* Rosen & Miner, Endocrine Review, 26, 452 - 464, 2005*,* Stojadinovic et al., J. Biol. Chem , 282, 4021 - 4034, 2007).A l'inverse et comme attendu, une inhibition de l'activité réductase 11 betaHSD1 est décrite pour induire une vasodilatation, un effet pro-angiogénique et anti-infectieux (voir les chapitres correspondants) et dans certaines situations inflammatoires pour produire une exacerbation et une surexpression de facteur de croissance tel que TGFbeta (Zhang et al., J. Immunology, 179, 6325 - 6335, 2007). Des inhibiteurs de 11 HSD1 devraient donc permettre, en agisant ainsi, d'améliorer la cicatrisation des plaies cutanées chroniques.

On a maintenant trouvé des dérivés d'urée de tétrahydroquinoxaline, portant un noyau adamantane, qui modulent l'activité de la 11βHSD1.

La présente invention a pour objet des composés répondant à la formule (I) : 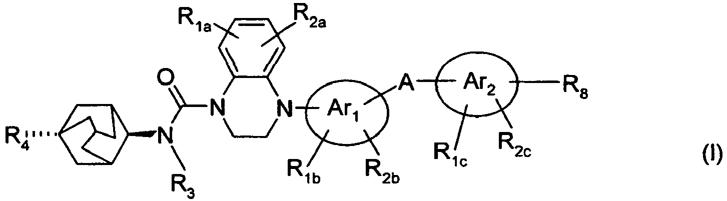 dans laquelle :
- A représente une liaison, un atome d'oxygène ou un groupe -O-CH₂-,
- Ar₁ représente un groupe phényle ou hétéroaryle,
- Ar₂ représente un groupe phényle, un groupe hétéroaryle ou un groupe hétérocycloalkyle,
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, alcoxy-alcoxy, halogénoalkyle, -0-halogénoalkyle, oxo, -CO-alkyle, -CO-alkyle-NR₆R₇, -CO-halogénoalkyle, -COOR₅, alkyl-COOR₅, -O-alkyl-COOR₅, -SO₂-alkyle, -SO₂-cycloalkyle, -SO₂-alkyl-cycloalkyle, -SO₂-alkyle-OR₅, -SO₂-alkyl-COOR₅, -SO₂-alkyl-NR₆R₇, -SO₂-halogénoalkyle, alkyl-SO₂-alkyle, -SO₂-NR₆R₇, -SO₂-alkyl-alcoxy-alcoxy, -CONR₆R₇, -alkyl-CONR₆R₇ ou -O-alkyl-NR₆R₇, ou encore R₁ₐ, R_{1b}, R _{1c} sont liés respectivement à R₂ₐ, R_{2b}, R_{2c} et à l'atome de carbone qui les porte et représentent -O-alkyl-O- ;
- R₃ représente un atome d'hydrogène ou un groupe alkyle,
- R₄ représente un atome d'hydrogène ou d'halogène ou un groupe cyano,-OR₅, hydroxy-alkyle, -COOR₅, -NR₆R₇, -CONR₆R₇, -SO₂-alkyle ou -SO₂-NR₆R₇, -NR₆-COOR₅, -NR₆-COR₅, -CO-NR₆-alkyl-OR₅ ;
- R₅, R₆ et R₇, identiques ou différents, représentent chacun un atome d'hydrogène, un groupe alkyle ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un hétéroaryle ou un hétérocycloalkyle éventuellement substitué par 1 à 3 groupes choisis parmi les groupes alkyles, -SO₂-alkyles et -COOR₅.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou d'acides ou être salifiés par des acides ou des bases, notamment des acides ou des bases pharmaceutiquement acceptables. De tels sels d'addition font partie de l'invention. Ces sels sont avantageusement préparés avec des acides ou des bases pharmaceutiquement acceptables, mais les sels d'autres acides ou bases utiles, par exemple, pour la purification ou l'isolement des composés de formule (I), font également partie de l'invention.

Les composés de formule (I) peuvent également exister sous forme d'hydrates ou de solvats, à savoir sous forme d'associations ou de combinaisons avec une ou plusieurs molécules de solvant. De tels solvats font également partie de l'invention.

Dans le cadre de la présente invention, et sauf mention différente dans le texte, on entend par :
- un atome d'halogène : un fluor, un chlore, un brome ou un iode ;
- un groupe alkyle : un groupe aliphatique saturé linéaire ou ramifié comportant de 1 à 5 atomes de carbone. A titre d'exemples, on peut citer les groupes méthyle, éthyle, propyle, méthylpropyle, isopropyle, butyle, isobutyle, tertbutyle ou pentyle ;
- un groupe cycloalkyle : un groupe alkyle cyclique comportant de 3 à 6 atomes de carbone. A titre d'exemples, on peut citer les groupes cyclopropyle, cyclobutyle, cyclopentyle,
- un groupe alcoxy : un radical de formule -O-alkyle, où le groupe alkyle est tel que défini précédemment ;
- un groupe hydroxy-alkyle : un radical de formule alkyle-OH, où le groupe alkyle est tel que défini précédemment ;
- un groupe alcoxy-alkyle : un radical de formule alkyle-O-alkyle, où les groupes alkyles, identiques ou différents, sont tels que définis précédemment. A titre d'exemples, on peut citer -(CH₂)₂-O-CH₃, -(CH₂)₃-O-CH₃, -CH-(CH₂-O-CH₃)₂ ;
- un groupe alcoxy-alcoxy : un radical de formule -O-alkyl-O-alkyle, où les groupes alkyles, identiques ou différents, sont tels que définis précédemment ;
- un groupe halogénoalkyle : un groupe alkyle tel que défini ci-dessus substitué par 1 à 5 atomes d'halogène, tels que définis précédemment. On citera par exemple le groupe trifluorométhyle ;
- un groupe hétéroaryle : un groupe aromatique comprenant de 5 à 9 atomes, dont 1 à 3 hétéroatomes, tels que l'azote, l'oxygène ou le soufre. On peut notamment citer les groupes pyridinyle, pyrimidinyle, pyridazinyle ou thiazolyle ; et
- un hétérocycloalkyle : un groupe alkyle mono-, bi-cyclique éventuellement ponté comportant de 4 à 9 atomes ou éventuellement partiellement insaturé et dont 1 ou 2 sont des hétéroatomes, tels que l'oxygène, l'azote ou le soufre. On peut notamment citer les groupes pyrrolidinyle, pipéridinyle, pipérazinyle, tétrahydropyranyle, morpholinyle, homopipérazinyle, 3,8-diazabicyclo[3.2.1]octane, thiomorpholinyle et thiomorpholinyl-1,1-dioxide, octahydro-pyrrolo[3,4-c]pyrrole, 1,2,3,6-tetrahydro-pyridine, 2,5-diaza-bicyclo[2.2.1]heptane,
- une fonction carbonyle peut être représentée par CO.

Dans le cadre de la présente invention, R_{1a,b,c} désigne les groupes R₁ₐ, R_{1b} et R_{1c} et R_{2a,b,c} désigne les groupes R₂ₐ, R_{2b} et R_{2c}. Lorsque Ar₂ représente un groupe hétérocycloalkyle, les groupes R_{1c}, R_{2c} et R₈ peuvent être portés par n'importe quel atome dudit hétérocycle, qu'il s'agisse d'un atome de carbone ou d'un hétéroatome (par exemple un atome d'azote), y compris par le même atome dudit hétérocycloalkyle (par exemple lorsqu'il s'agit d'un atome de soufre).

Parmi les composés de formule (I) selon l'invention, on peut citer un sous-groupe de composés dans lesquels :
- A représente une liaison, un atome d'oxygène ou un groupe -O-CH₂-,
- Ar₁ représente un groupe phényle ou hétéroaryle,
- Ar₂ représente un groupe phényle, un groupe hétéroaryle ou un groupe hétérocycloalkyle,
- R_{1a,b,c} et R_{2a,b,c}, identiques ou différents, représentent chacun un atome d'hydrogène ou d'halogène ou un groupe alkyle, -OR₅, hydroxy-alkyle, alcoxy-alkyle, alcoxy-alcoxy, halogénoalkyle, -O-halogénoalkyle, oxo, -CO-alkyle, -CO-halogénoalkyle, -COOR₅, alkyl-COOR₅, -O-alkyl-COOR₅, -SO₂-alkyle, -SO₂-halogénoalkyle, alkyl-SO₂-alkyle, -SO₂-NR₆R₇, -CONR₆R₇, -alkyl-CONR₆R₇ ou -O-alkyl-NR₆R₇,
- R₃ représente un atome d'hydrogène ou un alkyle,
- R₄ représente un atome d'hydrogène ou d'halogène ou un groupe cyano, -OR₅, hydroxy-alkyle, -COOR₅, -NR₆R₇, -CONR₆R₇, -SO₂-alkyle ou -SO₂-NR₆R₇,
- R₅, R₆ et R₇, identiques ou différents, représentent chacun un atome d'hydrogène ou un groupe alkyle, et
- R₈ représente un atome d'hydrogène ou un groupe de formule -B-Het, où B peut être absent ou représenter une liaison, un atome d'oxygène ou un groupe -CO- ou -SO₂- et où Het représente un hétérocycloalkyle éventuellement substitué par 1 à 3 groupes choisis parmi les groupes alkyles, -SO₂-alkyles et -COOR₅.

Parmi les composés de formule (I) selon l'invention, on peut citer un sous-groupe de composés dans lesquels :
- A représente une liaison, un atome d'oxygène ou un groupe -O-CH₂-,
- Ar₁ représente un groupe phényle ou hétéroaryle,
- Ar₂ représente un groupe phényle, un groupe hétéroaryle ou un groupe hétérocycloalkyle,
- R_{1a,b,c} et R_{2a,b,c}, identiques ou différents, représentent chacun un atome d'hydrogène ou d'halogène ou un groupe alkyle, cycloalkyle, -OR₅ (hydroxy ou alcoxy), hydroxy-alkyle, alcoxy-alkyle, alcoxy-alcoxy, halogénoalkyle, -O-halogénoalkyle, oxo, - CO-alkyle, -CO-alkyle-NR₆R₇, -CO-halogénoalkyle, -COOR₅, alkyl-COOR₅, -O-alkyl-COOR₅, -SO₂-alkyle, -SO₂-cycloalkyle, -SO₂-alkyle-cycloalkyle, -SO₂-alkyle-OR₅ -SO₂-alkyle-COOR₅, -SO₂-alkyle-NR₆R₇ -SO₂-halogénoalkyle, alkyl-SO₂-alkyle, -SO₂-NR₆R₇, -SO₂-alkyl-O-alkyl-OR₅, -CONR₆R₇, -alkyl-CONR₆R₇ ou -O-alkyl-NR₆R₇, ou encore R₁ₐ, R_{1b}, R_{1c} sont liés respectivement à R₂ₐ, R _{2b}, R _{2c} et à l'atome de carbone qui les porte et représentent -O-alkyl-O- ;
- R₃ représente un atome d'hydrogène ou un groupe alkyle,
- R₄ représente un atome d'hydrogène ou d'halogène ou un groupe cyano,-OR₅, hydroxy-alkyle, -COOR₅, -NR₆R₇, -CONR₆R₇, -SO₂-alkyle, -SO₂-NR₆R₇, -NR₆-COOR₅ ou -CO-NR₆-alkyl-OR₅ ;
- R₅, R₆ et R₇, identiques ou différents, représentent chacun un atome d'hydrogène, un groupe alkyle ou un groupe -alkyl-phényle, et
- R₈ représente un atome d'hydrogène, un groupe -SO₂-alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un hétéroaryle ou un hétérocycloalkyle éventuellement substitué par 1 à 3 groupes choisis parmi les groupes alkyles, -SO₂-alkyles et -COOR₅.

Parmi les composés de formule (I) selon l'invention, on peut citer un sous-groupe de composés dans lesquels :
- A représente une liaison, un atome d'oxygène ou un groupe -O-CH₂-,
- Ar₁ représente un groupe phényle ou hétéroaryle,
- Ar₂ représente un groupe phényle, un groupe hétéroaryle ou un groupe hétérocycloalkyle,
- R₁ₐ,_{b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyle-NR₆R₇, -COOR₅, -alkyl-COOR₅, -SO₂-alkyle, -SO₂-cycloalkyle, -SO₂-alkyl-cycloalkyle, -SO₂-alkyle-OR₅, -SO₂-alkyl-COOR₅, -SO₂-alkyl-NR₆R₇, -SO₂-halogénoalkyle, alkyl-SO₂-alkyle, -SO₂-alkyl-alcoxy-alcoxy, -CONR₆R₇ ou -O-alkyl-NR₆R₇, ou encore R₁ₐ, R _{1b}, R _{1c} sont liés respectivement à R₂ₐ, R_{2b}, R_{2c} et à l'atome de carbone qui les porte et représentent -O-alkyl-O- ;
- R₃ représente un atome d'hydrogène ou un groupe alkyle,
- R₄ représente un atome d'hydrogène ou d'halogène ou un groupe cyano, - OR₅, hydroxy-alkyle, -COOR₅, -CONR₆R₇, -NR₆-COOR₅, -NR₆-COR₅, -CO-NR₆-alkyl-OR₅ ;
- R₅, R₆ et R₇, identiques ou différents, représentent chacun un atome d'hydrogène, un groupe alkyle ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un hétéroaryle ou un hétérocycloalkyle éventuellement substitué par 1 à 3 groupes choisis parmi les groupes alkyles, -SO₂-alkyles et -COOR₅.

Parmi les composés de formule (I) selon l'invention, on peut citer un sous-groupe de composés dans lesquels
A représente une liaison, un atome d'oxygène ou un groupe -O-CH₂-,
et/ou
Ar₁ représente un groupe phényle ou hétéroaryle,
et/ou
Ar₂ représente un groupe phényle, un groupe hétéroaryle ou un groupe hétérocycloalkyle,
et/ou
R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyle-NR₆R₇, -COOR₅, -alkyl-COOR₅, -SO₂-alkyle, -SO₂-cycloalkyle, -SO₂-alkyl-cycloalkyle, -SO₂-alkyle-OR₅, -SO₂-alkyl-COOR₅, -SO₂-alkyl-NR₆R₇, -SO₂-halogénoalkyle, alkyl-SO₂-alkyle, -SO₂-alkyl-alcoxy-alcoxy, -CONR₆R₇ ou -O-alkyl-NR₆R₇, ou encore R₁ₐ, R _{1b}, R _{1c} sont liés respectivement à R₂ₐ, R _{2b}, R _{2c} et à l'atome de carbone qui les porte et représentent -O-alkyl-O- ;
et/ou
R₃ représente un atome d'hydrogène ou un groupe alkyle,
et/ou
R₄ représente un atome d'hydrogène ou d'halogène ou un groupe cyano, -OR₅, hydroxy-alkyle, -COOR₅, -CONR₆R₇, -NR₆-COOR₅, -NR₆-COR₅, -CO-NR₆-alkyl-OR₅ ; et/ou
R₅, R₆ et R₇, identiques ou différents, représentent chacun un atome d'hydrogène, un groupe alkyle ou un groupe -alkyl-phényle,
et/ou
R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un hétéroaryle ou un hétérocycloalkyle éventuellement substitué par 1 à 3 groupes choisis parmi les groupes alkyles, -SO₂-alkyles et -COOR₅.

Parmi les composés de formule (I) selon l'invention, on peut citer un sous-groupe de composés dans lesquels A représente une liaison.

Un autre sous-groupe de composés de formule (I) selon l'invention est tel que Ar₁ représente un groupe phényle.

Un autre sous-groupe de composés de formule (I) selon l'invention est tel que Ar₁ représente un groupe hétéroaryle. Avantageusement, Ar₁ représente un groupe pyridinyle, pyrimidinyle ou thiazolyle.

Un autre sous-groupe de composés de formule (I) selon l'invention est tel que Ar₁ représente un groupe phényle, pyridinyle, pyrimidinyle, pyridazinyle ou thiazolyle. Avantageusement, Ar₁ représente un groupe phényle, pyridinyle, pyrimidinyle ou thiazolyle.

Un autre sous-groupe de composés de formule (I) selon l'invention est tel que Ar₂ représente un groupe phényle.

Un autre sous-groupe de composés de formule (I) selon l'invention est tel que Ar₂ représente un groupe hétéroaryle.

Un autre sous-groupe de composés de formule (I) selon l'invention est tel que Ar₂ représente un groupe hétérocycloalkyle.

Un autre sous-groupe de composés de formule (I) selon l'invention est tel que Ar₂ représente un groupe phényle, pyridinyle, pyrimidinyle, pipéridinyle, pipérazinyle, homopipérazinyle, 3,8-diazabicyclo[3.2.1]octane, morpholinyle, thiomorpholinyle, octahydro-pyrrolo[3,4-c]pyrrole, 1,2,3,6-tetrahydro-pyridine ou 2,5-diaza-bicyclo[2.2.1]heptane. Avantageusement, Ar₂ représente un groupe phényle, pyridinyle, pyrimidinyle, pipéridinyle ou pipérazinyle.

Parmi les composés de formule (I) selon l'invention dans lesquels Ar₁ représente un groupe phényle ou un hétéroaryle à 6 chaînons, on peut citer ceux dans lesquels la liaison entre les noyaux A-Ar₂ et Ar₁ est en position para par rapport à la liaison entre Ar₁ et l'atome d'azote du noyau tétrahydroquinoxaline auquel il est lié.

Parmi les composés de formule (I) selon l'invention dans lesquels Ar₂ représente un groupe hétéroaryle ou hétérocycloalkyle, on peut citer ceux qui sont liés au groupe A par un hétéroatome.

Un autre sous-groupe de composés de formule (I) selon l'invention est tel que R₁ₐ, R₂ₐ, R_{1b} et R_{2b}, identiques ou différents, représentent chacun un atome d'hydrogène ou d'halogène ou un groupe alkyle ou alcoxy. Avantageusement, R₁ₐ et R₂ₐ, identiques ou différents, représentent chacun un atome d'hydrogène ou d'halogène et R_{1b} et R_{2b} représentent chacun un atome d'hydrogène.

Un autre sous-groupe de composés de formule (I) selon l'invention est tel que R_{1c} et R_{2c}, identiques ou différents, représentent chacun un atome d'hydrogène ou d'halogène ou un groupe alkyle, alcoxy, -SO₂-alkyle, -SO₂-cycloalkyle.

Un autre sous-groupe de composés de formule (I) selon l'invention est tel que R₃ représente un atome d'hydrogène.

Un autre sous-groupe de composés de formule (I) selon l'invention est tel que R₄ représente un atome d'hydrogène ou d'halogène ou un groupe cyano, hydroxy, hydroxy-alkyle, -COOR₅ ou -CONH₂.

Un autre sous-groupe de composés de formule (I) selon l'invention est tel que R₈ représente un atome d'hydrogène, un groupe alkyle ou un groupe de formule -B-Het, où B peut être absent ou représenter une liaison, un atome d'oxygène ou un groupe - CO- ou -SO₂-(CH₂)ₙ avec n égal à 0, 1 ou 2 et où Het représente un groupe pyrrolidinyle, pipéridinyle, pipérazinyle, tétrahydropyranyle, morpholinyle ou pyridinyle, ledit groupe Het étant éventuellement substitué par 1 à 3 groupes choisis parmi les groupes alkyles, -SO₂-alkyles et -COOR₅.

Par ailleurs, B peut être absent et le groupe Het peut se trouver en position spiro sur le noyau Ar₂, lorsque ce dernier représente un groupe hétérocycloalkyle.

Les sous-groupes définis ci-dessus pris séparément ou en combinaison font également partie de l'invention.

On peut notamment citer le sous-groupe de composés de formule (I) selon l'invention dans lequel :
- A représente une liaison, un atome d'oxygène ou un groupe -O-CH₂-,
- Ar₁ représente un groupe phényle, pyridinyle, pyrimidinyle, pyridazinyle ou thiazolyle,
- Ar₂ représente un groupe phényle, pyridinyle, pyrimidinyle, pipéridinyle, pipérazinyle, homopipérazinyle, 3,8-diazabicyclo[3.2.1]octane, morpholinyle, thiomorpholinyle, octahydro-pyrrolo[3,4-c]pyrrole, 1,2,3,6-tetrahydro-pyridine ou 2,5-diaza-bicyclo[2.2.1]heptane,
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyle-NR₆R₇, -COOR₅, alkyl-COOR₅, -SO₂-alkyle, -SO₂-cycloalkyle, -SO₂-alkyl-cycloalkyle, -SO₂-alkyle-OR₅, -SO₂-alkyl-COOR₅, -SO₂-alkyl-NR₆R₇, -SO₂-halogénoalkyle, -alkyl-SO₂-alkyle, -SO₂-alkyl-alcoxy-alcoxy, -CONR₆R₇ ou -O-alkyl-NR₆R₇, ou encore R₁ₐ, R _{1b}, R _{1c} sont liés respectivement à R₂ₐ, R _{2b}, R _{2c} et à l'atome de carbone qui les porte et représentent -O-alkyl-O-,
- R₃ représente un atome d'hydrogène ou un alkyle,
- R₄ représente un atome d'hydrogène ou d'halogène ou un groupe cyano,-OR₅, hydroxy-alkyle, -COOR₅, -CONR₆R₇, -NR₆-COOR₅, -NR₆-COR₅ ou -CO-NR₆-alkyl-OR₅,
- R₅, R₆ et R₇, identiques ou différents, représentent chacun un atome d'hydrogène, un groupe alkyle ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un groupe pyrrolidinyle, pipéridinyle, pipérazinyle, tétrahydropyranyle, morpholinyle ou pyridinyle, ledit groupe Het étant éventuellement substitué par 1 à 3 groupes choisis parmi les groupes alkyles, -SO₂-alkyles et -COOR₅.

On peut également citer le sous-groupe de composés de formule (I) selon l'invention dans lequel :
- A représente une liaison, un atome d'oxygène ou un groupe -O-CH₂-,
- Ar₁ représente un groupe phényle ou hétéroaryle,
- Ar₂ représente un groupe phényle,
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyle-NR₆R₇, -COOR₅, alkyl-COOR₅, -SO₂-alkyle, -SO₂-cycloalkyle, -SO₂-alkyle-cycloalkyle, -SO₂-alkyle-OR₅, -SO₂-alkyle-COOR₅, -SO₂-alkyle-NR₆R₇, -SO₂-halogénoalkyle, alkyl-SO₂-alkyle, -SO₂-alkyl-alcoxy-alcoxy, -CONR₆R₇ ou -O-alkyl-NR₆R₇, ou encore R₁ₐ, R _{1b}, R _{1c} sont liés respectivement à R₂ₐ, R _{2b}, R _{2c} et à l'atome de carbone qui les porte et représentent -O-alkyl-O-,
- R₃ représente un atome d'hydrogène ou un alkyle,
- R₄ représente un atome d'hydrogène ou d'halogène ou un groupe cyano,-OR₅, hydroxy-alkyle, -COOR₅, -CONR₆R₇, -NR₆-COOR₅, -NR₆-COR₅, ou -CO-NR₆-alkyl-OR₅,
- R₅, R₆ et R₇, identiques ou différents, représentent chacun un atome d'hydrogène, un groupe alkyle ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un groupe pyrrolidinyle, pipéridinyle, pipérazinyle, tétrahydropyranyle, morpholinyle ou pyridinyle, ledit groupe Het étant éventuellement substitué par 1 à 3 groupes choisis parmi les groupes alkyles, -SO₂-alkyles et -COOR₅.

On peut encore citer le sous-groupe de composés de formule (I) selon l'invention dans lequel :
- A représente une liaison, un atome d'oxygène ou un groupe -O-CH₂-,
- Ar₁ représente un groupe phényle ou hétéroaryle,
- Ar₂ représente un groupe hétéroaryle (tel qu'un groupe pyridinyle ou pyrimidinyle),
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyle-NR₆R₇, -COOR₅, alkyl-COOR₅, -SO₂-alkyle, -SO₂-cycloalkyle, -SO₂-alkyl-cycloalkyle, -SO₂-alkyl-OR₅, -SO₂-alkyl-COOR₅, -SO₂-alkyl-NR₆R₇, -SO₂-halogénoalkyle, -alkyl-SO₂-alkyle, -SO₂-alkyl-alcoxy-alcoxy, -CONR₆R₇ ou -O-alkyl-NR₆R₇, ou encore R₁ₐ, R _{1b}, R _{1c} sont liés respectivement à R₂ₐ, R _{2b}, R _{2c} et à l'atome de carbone qui les porte et représentent -O-alkyl-O-,
- R₃ représente un atome d'hydrogène ou un alkyle,
- R₄ représente un atome d'hydrogène ou d'halogène ou un groupe cyano,-OR₅, hydroxy-alkyle, -COOR₅, -CONR₆R₇, -NR₆-COOR₅, -NR₆-COR₅ ou -CO-NR₆-alkyl-OR₅,
- R₅, R₆ et R₇, identiques ou différents, représentent chacun un atome d'hydrogène, un groupe alkyle ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un groupe pyrrolidinyle, pipéridinyle, pipérazinyle, tétrahydropyranyle, morpholinyle ou pyridinyle, ledit groupe Het étant éventuellement substitué par 1 à 3 groupes choisis parmi les groupes alkyles, -SO₂-alkyles et -COOR₅.

On peut citer le sous-groupe de composés de formule (I) selon l'invention dans lequel :
- A représente une liaison, un atome d'oxygène ou un groupe -O-CH₂-,
- Ar₁ représente un groupe phényle ou hétéroaryle,
- Ar₂ représente un groupe hétérocycloalkyle (tel qu'un groupe pipéridinyle, pipérazinyle, homopipérazinyle, 3,8-diazabicyclo[3.2.1]octane, morpholinyle, thiomorpholinyle, octahydro-pyrrolo[3,4-c]pyrrole, 1,2,3,6-tetrahydro-pyridine ou 2,5-diaza-bicyclo[2.2.1heptane,),
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyle-NR₆R₇, -COOR₅, alkyl-COOR₅, -SO₂-alkyle, -SO₂-cycloalkyle, -SO₂-alkyl-cycloalkyle, -SO₂-alkyl-OR₅, -SO₂-alkyl-COOR₅, -SO₂-alkyl-NR₆R₇, -SO₂-halogénoalkyle, alkyl-SO₂-alkyle, -SO₂-alkyl-alcoxy-alcoxy, -CONR₆R₇ ou -O-alkyl-NR₆R₇, ou encore R₁ₐ, R _{1b}, R _{1c} sont liés respectivement à R₂ₐ, R _{2b}, R _{2c} et à l'atome de carbone qui les porte et représentent -O-alkyl-O-,
- R₃ représente un atome d'hydrogène ou un alkyle,
- R₄ représente un atome d'hydrogène ou d'halogène ou un groupe cyano,-OR₅, hydroxy-alkyle, -COOR₅, -CONR₆R₇, -NR₆-COOR₅, -NR₆-COR₅, ou -CO-NR6-alkyl-OR5,
- R₅, R₆ et R₇, identiques ou différents, représentent chacun un atome d'hydrogène, un groupe alkyle ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un groupe pyrrolidinyle, pipéridinyle, pipérazinyle, tétrahydropyranyle, morpholinyle ou pyridinyle, ledit groupe Het étant éventuellement substitué par 1 à 3 groupes choisis parmi les groupes alkyles, -SO₂-alkyles et -COOR₅.

On peut enfin citer le sous-groupe de composés de formule (I) selon l'invention dans lequel :
- A représente une liaison,
- Ar₁ représente un groupe phényle ou hétéroaryle,
- Ar₂ représente un groupe hétérocycloalkyle,
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyl-NR₆R₇, -COOR₅, alkyl-COOR₅, -SO₂-alkyle, -SO₂-cycloalkyle, -SO₂-alkyl-cycloalkyle, -SO₂-alkyl-OR₅, -SO₂-alkyl-COOR₅, -SO₂-alkyl-NR₆R₇, -SO₂-halogénoalkyle, -alkyl-SO₂-alkyle, -SO₂-alkyl-alcoxy-alcoxy, -CONR₆R₇ ou -O-alkyl-NR₆R₇, ou encore R₁ₐ, R _{1b}, R _{1c} sont liés respectivement à R₂ₐ, R _{2b}, R _{2c} et à l'atome de carbone qui les porte et représentent -O-alkyl-O-,
- R₃ représente un atome d'hydrogène,
- R₄ représente un groupe OH ou -CONH₂,
- R₅, R₆ et R₇, identiques ou différents, représentent chacun un atome d'hydrogène, un groupe alkyle ou un groupe -alkyl-phényle et
- R₈ représente un atome d'hydrogène.

Parmi les composés de formule (I) objets de l'invention, on peut notamment citer les composés suivants :
. 4-((4-pyridin-2-yl-phenyl)-3,4-dihydro-2H-quinoxaline-1-carboxylic acid adamantan-2-ylamide,
. 4-{4-[1-(tetrahydro-pyran-4-carbonyl)-piperidin-4-yloxy]-phenyl}-3,4-dihydro-2H-quinoxaline-1-carboxylic acid adamantan-2-ylamide,
. 4-[4-(pyrimidin-2-ylmethoxy)-phenyl]-3,4-dihydro-2H-quinoxaline-1-carboxylic acid adamantan-2-ylamide,
. 4-{4-[1-(morpholine-4-carbonyl)-piperidin-4-yl]-phenyl}-3,4-dihydro-2H-quinoxaline-1-carboxylic acid adamantan-2-ylamide,
. 4-[4-(4-methoxy-pyrimidin-2-yloxy)-phenyl]-3,4-dihydro-2H-quinoxaline-1-carboxylic acid adamantan-2-ylamide,
. 4-{4-[4-(morpholine-4-carbonyl)-phenyl]-thiazol-2-yl}-3,4-dihydro-2H-quinoxaline-1-carboxylic acid adamantan-2-ylamide,
. 4-[5-(4-methoxy-phenyl)-pyrimidin-2-yl]-3,4-dihydro-2H-quinoxaline-1-carboxylic acid adamantan-2-ylamide,
. 4-[5-(4-methoxy-phenyl)-pyrimidin-2-yl]-3,4-dihydro-2H-quinoxaline-1-carboxylic acid (5-hydroxy-adamantan-2-yl)-amide,
. trans-4-[4-(4-methanesulfonyl-piperazin-1-yl)-phenyl]-3,4-dihydro-2H-quinoxaline-1-carboxylic acid (5-hydroxy-adamantan-2-yl)-amide,
. ester methylique de l'acide 4-({4-[4-(4-methanesulfonyl-piperazin-1-yl)-phenyl]-3,4-dihydro-2H-quinoxaline-1-carbonyl}-amino)-adamantane-1-carboxylique,
. acide 4-({4-[4-(4-methanesulfonyl-piperazin-1-yl)-phenyl]-3,4-dihydro-2H-quinoxaline-1-carbonyl}-amino)-adamantane-1-carboxylique,
. 4-[4-(4-methanesulfonyl-piperazin-1-yl)-phenyl]-3,4-dihydro-2H-quinoxaline-1-carboxylic acid (5-carbamoyl-adamantan-2-yl)-amide,
. trans-4-[4-(pyridin-2-yloxy)-phenyl]-3,4-dihydro-2H-quinoxaline-1-carboxylic acid (5-hydroxy-adamantan-2-yl)-amide,
. trans-4-[4-(4-trifluoromethyl-pyrimidin-2-yloxy)-phenyl]-3,4-dihydro-2H-quinoxaline-1-carboxylic acid (5-hydroxy-adamantan-2-yl)-amide,
.trans-4-[4-(1-Methanesulfonyl-1,2,3,6-tetrahydro-pyridin-4-yl)-phenyl]-3,4-dihydro-2H-quinoxaline-1-carboxylic acid (5-hydroxy-adamantan-2-yl)-amide,
.trans-4-[4-(4-Hydroxy-1-methanesulfonyl-piperidin-4-yl)-phenyl]-3,4-dihydro-2H-quinoxaline-1-carboxylic acid (5-hydroxy-adamantan-2-yl)-amide,
.trans-4-[2-(4-Methanesulfonyl-piperazin-1-yl)-pyrimidin-5-yl]-3,4-dihydro-2H-quinoxaline-1-carboxylic acid (5-hydroxy-adamantan-2-yl)-amide,
.trans-4-(5-(4-Methanesulfonyl-piperazin-1-yl)-pyrimidin-2-yl]-3,4-dihydro-2H-quinoxaline-1-carboxylic acid (5-hydroxy-adamantan-2-yl)-amide,
.trans-4-{4-[1-(Morpholine-4-carbonyl)-piperidin-4-yl]-phenyl}-3,4-dihydro-2H-quinoxaline-1-carboxylic acid (5-hydroxy-adamantan-2-yl)-amide,
.trans-4-{4-[4-(5-Hydroxy-adamantan-2-ylcarbamoyl)-3,4-dihydro-2H-quinoxalin-1-yl]-phenyl}-piperazine-1-carboxylic acid benzyl ester,
.trans-4-[4-(4-Methanesulfonyl-piperazin-1-yl)-phenyl]-3,4-dihydro-2H-quinoxaline-1-carboxylic acid (5-hydroxymethyl-adamantan-2-yl)-amide,
.trans-4-[4-(5-Fluoro-pyrimidin-2-yloxy)-phenyl]-3,4-dihydro-2H-quinoxaline-1-carboxylic acid (5-hydroxy-adamantan-2-yl)-amide,
.trans-4-[4-(1-Methanesulfonyl-piperidin-4-yloxy)-phenyl]-3,4-dihydro-2H-quinoxaline-1-carboxylic acid (5-hydroxy-adamantan-2-yl)-amide,
.trans-4-(4-Morpholin-4-yl-phenyl)-3,4-dihydro-2H-quinoxaline-1-carboxylic acid (5-hydroxy-adamantan-2-yl)-amide,
.trans-4-[4-(1,1-Dioxo-1lambda6-thiomorpholin-4-yl)-phenyl]-3,4-dihydro-2H-quinoxaline-1-carboxylic acid (5-hydroxy-adamantan-2-yl)-amide,
.trans-4-[4-(5-Isopropoxy-pyridin-2-yl)-phenyl]-3,4-dihydro-2H-quinoxaline-1-carboxylic acid (5-hydroxy-adamantan-2-yl)-amide,
.trans-4-{4-[5-(4-Isopropyl-piperazine-1-carbonyl)-pyridin-2-yl]-phenyl}-3,4-dihydro-2H-quinoxaline-1-carboxylic acid (5-hydroxy-adamantan-2-yl)-amide,
.trans-4-[6-(4-Methanesulfonyl-piperazin-1-yl)-pyridin-3-yl]-3,4-dihydro-2H-quinoxaline-1-carboxylic acid (5-hydroxy-adamantan-2-yl)-amide,
.trans-4-(4-Piperazin-1-yl-phenyl)-3,4-dihydro-2H-quinoxaline-1-carboxylic acid (5-hydroxy-adamantan-2-yl)-amide,
.trans-4-[4-(4,4-Difluoro-piperidin-1-yl)-phenyl]-3,4-dihydro-2H-quinoxaline-1-carboxylic acid (5-hydroxy-adamantan-2-yl)-amide,
.trans-4-[5-(4-Methanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-Methanesulfonyl-piperazin-1-yl)-pyridin-2-yl]-3,4-dihydro-2H-quinoxaline-1-carboxylic acid (5-hydroxy-adamantan-2-yl)-amide,
.trans-4-[2-(1-Methanesulfonyl-piperidin-4-yloxy)-pyrimidin-5-yl]-3,4-dihydro-2H-quinoxaline-1-carboxylic acid (5-hydroxy-adamantan-2-yl)-amide,
.trans-4-[5-(5-Isopropoxy-pyridin-2-yl)-pyrimidin-2-yl]-3,4-dihydro-2H-quinoxaline-1-carboxylic acid (5-hydroxy-adamantan-2-yl)-amide,
.trans-4-[4-(4-Ethanesulfonyl-piperazin-1-yl)-phenyl]-3,4-dihydro-2H-quinoxaline-1-carboxylic acid (5-hydroxy-adamantan-2-yl)-amide,
.trans-4-{4-[4-(2,2,2-Trifluoro-ethanesulfonyl)-piperazin-1-yl]-phenyl}-3,4-dihydro-2H-quinoxaline-1-carboxylic acid (5-hydroxy-adamantan-2-yl)-amide,
.trans-4-{4-[4-(Propane-2-sulfonyl)-piperazin-1-yl]-phenyl}-3,4-dihydro-2H-quinoxaline-1-carboxylic acid (4-hydroxy-adamantan-1-yl)-amide,
.trans-4-(4-{4-[4-(5-Hydroxy-adamantan-2-ylcarbamoyl)-3,4-dihydro-2H-quinoxalin-1-yl]-phenyl}-piperazine-1-sulfonyl)-piperidine-1-carboxylic acid benzyl ester,
.trans-4-{4-[4-(Piperidine-4-sulfonyl)-piperazin-1-yl]-phenyl}-3,4-dihydro-2H-quinoxaline-1-carboxylic acid (5-hydroxy-adamantan-2-yl)-amide,
.trans-4-[4-(4-Cyclopropanesulfonyl-piperazin-1-yl)-phenyl]-3,4-dihydro-2H-quinoxaline-1-carboxylic acid (5-hydroxy-adamantan-2-yl)-amide,
.trans-4-[4-(4-Methanesulfonyl-piperazin-1-yl)-phenyl]-3,4-dihydro-2H-quinoxaline-1-carboxylic acid (5-fluoro-adamantan-2-yl)-amide,
.trans-4-[4-(4-Cyclobutanesulfonyl-piperazin-1-yl)-phenyl]-3,4-dihydro-2H-quinoxaline-1-carboxylic acid (5-hydroxy-adamantan-2-yl)-amide,
.trans-4-{4-[4-(Propane-1-sulfonyl)-piperazin-1-yl]-phenyl}-3,4-dihydro-2H-quinoxaline-1-carboxylic acid (5-hydroxy-adamantan-2-yl)-amide,
.trans-4-{4-[4-(Butane-1-sulfonyl)-piperazin-1-yl]-phenyl}-3,4-dihydro-2H-quinoxaline-1-carboxylic acid (5-hydroxy-adamantan-2-yl)-amide,
.trans-4-{4-[4-(Morpholine-4-sulfonyl)-piperazin-1-yl]-phenyl}-3,4-dihydro-2H-quinoxaline-1-carboxylic acid (5-hydroxy-adamantan-2-yl)-amide,
.trans-4-[4-(4-Trifluoromethanesulfonyl-piperazin-1-yl)-phenyl]-3,4-dihydro-2H-quinoxaline-1-carboxylic acid (5-hydroxy-adamantan-2-yl)-amide,
.trans-4-[4-(1,4-Dioxa-8-aza-spiro[4.5]dec-8-yl)-phenyl]-3,4-dihydro-2H-quinoxaline-1-carboxylic acid (5-hydroxy-adamantan-2-yl)-amide,
.trans-4-[4-(4-Methanesulfonyl-3,3-dimethyl-piperazin-1-yl)-phenyl]-3,4-dihydro-2H-quinoxaline-1-carboxylic acid (5-hydroxy-adamantan-2-yl)-amide,
.trans-4-[4-((3R,5S)-4-Methanesulfonyl-3,5-dimethyl-piperazin-1-yl)-phenyl]-3,4-dihydro-2H-quinoxaline-1-carboxylic acid (5-hydroxy-adamantan-2-yl)-amide,
.trans- (4-{4-[4-(5-Hydroxy-adamantan-2-ylcarbamoyl)-3,4-dihydro-2H-quinoxalin-1-yl]-phenyl}-piperazine-1-sulfonyl)-acetic acid,
.trans- (4-{4-[4-(5-Hydroxy-adamantan-2-ylcarbamoyl)-3,4-dihydro-2H-quinoxalin-1-yl]-phenyl}-piperazine-1-sulfonyl)-acetic acid ethyl ester,
.trans-4-[4-(4-Hydroxy-piperidin-1-yl)-phenyl]-3,4-dihydro-2H-quinoxaline-1-carboxylic acid (5-hydroxy-adamantan-2-yl)-amide,
.trans-4-[4-(4-Methanesulfonyl-[1,4]diazepan-1-yl)-phenyl]-3,4-dihydro-2H-quinoxaline-1-carboxylic acid (5-hydroxy-adamantan-2-yl)-amide,
.trans-2-(4-{4-[4-(5-Hydroxy-adamantan-2-ylcarbamoyl)-3,4-dihydro-2H-quinoxalin-1-yl]-phenyl}-piperazin-1-yl)-2-methyl-propionic acid ethyl ester,
.trans-3-(4-{4-[4-(5-Hydroxy-adamantan-2-ylcarbamoyl)-3,4-dihydro-2H-quinoxalin-1-yl]-phenyl}-piperazine-1-sulfonyl)-propionic acid methyl ester,
.trans-4-[4-(5-Methoxy-pyrimidin-2-yl)-phenyl]-3,4-dihydro-2H-quinoxaline-1-carboxylic acid (5-hyd roxy-adamantan-2-yl)-am ide,
.trans-4-{4-[4-(2-Morpholin-4-yl-ethanesulfonyl)-piperazin-1-yl]-phenyl}-3,4-dihydro-2H-quinoxaline-1-carboxylic acid (5-hydroxy-adamantan-2-yl)-amide,
.trans-4-[6-(4-Methanesulfonyl-piperazin-1-yl)-pyridazin-3-yl]-3,4-dihydro-2H-quinoxaline-1-carboxylic acid (5-hydroxy-adamantan-2-yl)-amide,
.trans-4-[4-(4-Methanesulfonyl-piperazin-1-yl)-phenyl]-3,4-dihydro-2H-quinoxaline-1-carboxylic acid (5-hydroxy-adamantan-2-yl)-methyl-amide,
.trans-4-[4-(4-Methanesulfonyl-3,5-dimethyl-piperazin-1-yl)-phenyl]-3,4-dihydro-2H-quinoxaline-1-carboxylic acid (5-hydroxy-adamantan-2-yl)-amide,
.trans-4-{4-[4-(2-Diethylamino-ethanesulfonyl)-piperazin-1-yl]-phenyl}-3,4-dihydro-2H-quinoxaline-1-carboxylic acid (5-hydroxy-adamantan-2-yl)-amide,
.trans-4-[4-(3-Methanesulfonyl-3,8-diaza-bicyclo[3.2.1]oct-8-yl)-phenyl]-3,4-dihydro-2H-quinoxaline-1-carboxylic acid (5-hydroxy-adamantan-2-yl)-amide,
.trans-4-[5-(4-Methoxy-phenyl)-pyrimidin-2-yl]-3,4-dihydro-2H-quinoxaline-1-carboxylic acid (5-carbamoyl-adamantan-2-yl)-amide,
.trans-4-{4-[4-(2-Methoxy-ethanesulfonyl)-piperazin-1-yl]-phenyl}-3,4-dihydro-2H-quinoxaline-1-carboxylic acid (5-hydroxy-adamantan-2-yl)-amide,
.trans-4-[4-(4-Isopropoxy-piperidin-1-yl)-phenyl]-3,4-dihydro-2H-quinoxaline-1-carboxylic acid (5-hydroxy-adamantan-2-yl)-amide,
.trans-4-[5-(4-Hydroxy-phenyl)-pyrimidin-2-yl]-3,4-dihydro-2H-quinoxaline-1-carboxylic acid (5-hydroxy-adamantan-2-yl)-amide,
.trans-4-(4-Piperazin-1-yl-phenyl)-3,4-dihydro-2H-quinoxaline-1-carboxylic acid (5-carbamoyl-adamantan-2-yl)-amide,
.trans-4-[4-(5-Bromo-pyrimidin-2-yloxy)-phenyl]-3,4-dihydro-2H-quinoxaline-1-carboxylic acid (5-hydroxy-adamantan-2-yl)-amide,
.trans-4-[4-(4-Cyclopropanesulfonyl-piperazin-1-yl)-phenyl]-3,4-dihydro-2H-quinoxaline-1-carboxylic acid (5-carbamoyl-adamantan-2-yl)-amide, trans-4-[4-(4-Cyclopropanesulfonyl-piperazin-1-yl)-phenyl]-3,4-dihydro-2H-quinoxaline-1-carboxylic acid (5-carbamoyl-adamantan-2-yl)-amide,
.trans-6-Chloro-4-[4-(4-methanesulfonyl-piperazin-1-yl)-phenyl]-3,4-dihydro-2H-quinoxaline-1-carboxylic acid (5-hydroxy-adamantan-2-yl)-amide,
.trans-4-[4-(5-Methanesulfonyl-hexahydro-pyrrolo[3,4-c]pyrrol-2-yl)-phenyl]-3,4-dihydro-2H-quinoxaline-1-carboxylic acid (5-hydroxy-adamantan-2-yl)-amide,
.trans-4-{4-[4-(2-Amino-ethanesulfonyl)-piperazin-1-yl]-phenyl}-3,4-dihydro-2H-quinoxaline-1-carboxylic acid (5-hydroxy-adamantan-2-yl)-amide,
.trans-4-(4-Morpholin-4-yl-phenyl)-3,4-dihydro-2H-quinoxaline-1-carboxylic acid (5-carbamoyl-adamantan-2-yl)-amide,
.trans-4-{4-[5-(Piperidin-4-yloxy)-pyridin-2-yl]-phenyl}-3,4-dihydro-2H-quinoxaline-1-carboxylic acid (5-hydroxy-adamantan-2-yl)-amide,
.trans-4-{4-[5-(2-Amino-ethoxy)-pyridin-2-yl]-phenyl}-3,4-dihydro-2H-quinoxaline-1-carboxylic acid (5-hydroxy-adamantan-2-yl)-amide,
.trans-4-{4-[4-(2-Hydroxy-ethanesulfonyl)-piperazin-1-yl]-phenyl}-3,4-dihydro-2H-quinoxaline-1-carboxylic acid (5-hydroxy-adamantan-2-yl)-amide,
.trans-4-[4-(8-Methanesulfonyl-3,8-diaza-bicyclo[3.2.1]oct-3-yl)-phenyl]-3,4-dihydro-2H-quinoxaline-1-carboxylic acid (5-carbamoyl-adamantan-2-yl)-amide,
.trans-4-[4-(8-Methanesulfonyl-3,8-diaza-bicyclo[3.2.1]oct-3-yl)-phenyl]-3,4-dihydro-2H-quinoxaline-1-carboxylic acid (5-hydroxy-adamantan-2-yl)-amide,
.trans-4-[5-(4-Isopropoxy-phenyl)-pyrimidin-2-yl]-3,4-dihydro-2H-quinoxaline-1-carboxylic acid (5-hydroxy-adamantan-2-yl)-amide,
.trans-4-{5-[4-(2,2,2-Trifluoro-ethanesulfonyl)-piperazin-1-yl]-pyridin-2-yl}-3,4-dihydro-2H-quinoxaline-1-carboxylic acid (5-hydroxy-adamantan-2-yl)-amide,
.trans-4-{5-[4-(2,2,2-Trifluoro-ethanesulfonyl)-piperazin-1-yl]-pyridin-2-yl}-3,4-dihydro-2H-quinoxaline-1-carboxylic acid (5-carbamoyl-adamantan-2-yl)-amide,
.trans-3-(4-{4-[4-(5-Hydroxy-adamantan-2-ylcarbamoyl)-3,4-dihydro-2H-quinoxalin-1-yl]-phenyl}-piperazine-1-sulfonyl)-propionic acid,
.trans-4-[4-(4-Methanesulfonyl-piperazin-1-yl)-phenyl]-3,4-dihydro-2H-quinoxaline-1-carboxylic acid [5-(2-hydroxy-ethylcarbamoyl)-adamantan-2-yl]-amide,
.trans-4-[4-(5-isobutoxy-pyridin-2-yl)-phenyl]-3,4-dihydro-2H-quinoxaline-1-carboxylic acid (5-hydroxy-adamantan-2-yl)-amide,
.trans-4-[4-(5-sec-Butoxy-pyridin-2-yl)-phenyl]-3,4-dihydro-2H-quinoxaline-1-carboxylic acid (5-hydroxy-adamantan-2-yl)-amide,
.trans-4-[4-(5-Isopropoxy-pyridin-2-yl)-phenyl]-3,4-dihydro-2H-quinoxaline-1-carboxylic acid (5-carbamoyl-adamantan-2-yl)-amide,
.trans- [4-({4-[4-(4-Methanesulfonyl-piperazin-1-yl)-phenyl]-3,4-dihydro-2H-quinoxaline-1-carbonyl}-amino)-adamantan-1-yl]-carbamic acid methyl ester,
.trans-4-[5-(4-Ethanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-Cyclopropanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-Cyclopropanesulfonyl-piperazin-1-yl)-pyridin-2-yl]-3,4-dihydro-2H-quinoxaline-1-carboxylic acid (5-carbamoyl-adamantan-2-yl)-amide,
.trans-4-{5-[4-(Propane-2-sulfonyl)-piperazin-1-yl]-pyridin-2-yl}-3,4-dihydro-2H-quinoxaline-1-carboxylic acid (5-hydroxy-adamantan-2-yl)-amide,
.trans-4-{4-[4-(Pyridin-4-yloxy)-piperidin-1-yl]-phenyl}-3,4-dihydro-2H-quinoxaline-1-carboxylic acid (5-hydroxy-adamantan-2-yl)-amide,
.trans-4-{4-[4-(2-Methoxy-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(2-Amino-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(2-Diethylamino-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(Piperidine-4-sulfonyl)-piperazin-1-yl]-phenyl}-3,4-dihydro-2H-quinoxaline-1-carboxylic acid (5-carbamoyl-adamantan-2-yl)-amide,
.trans-4-[4-(4-tert-Butyl-piperazin-1-yl)-phenyl]-3,4-dihydro-2H-quinoxaline-1-carboxylic acid (5-carbamoyl-adamantan-2-yl)-amide,
.trans-4-[5-(4-Methanesulfonyl-piperazin-1-yl)-pyridin-2-yl]-3,4-dihydro-2H-quinoxaline-1-carboxylic acid (5-carbamoyl-adamantan-2-yl)-amide,
.trans-4-{5-[4-(Propane-1-sulfonyl)-piperazin-1-yl]-pyridin-2-yl}-3,4-dihydro-2H-quinoxaline-1-carboxylic acid (5-hydroxy-adamantan-2-yl)-amide,
.trans-4-{5-[4-(2-Methyl-propane-1-sulfonyl)-piperazin-1-yl]-pyridin-2-yl}-3,4-dihydro-2H-quinoxaline-1-carboxylic acid (5-hydroxy-adamantan-2-yl)-amide,
.trans-4-(5-Morpholin-4-yl-pyridin-2-yl)-3,4-dihydro-2H-quinoxaline-1-carboxylic acid (5-hydroxy-adamantan-2-yl)-amide,
.trans-4-{4-[4-(2-Hydroxy-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(Piperidin-4-yloxy)-piperidin-1-yl]-phenyl}-3,4-dihydro-2H-quinoxaline-1-carboxylic acid (5-hydroxy-adamantan-2-yl)-amide,
.trans-4-[4-(4-Dimethylcarbamoyl-piperazin-1-yl)-phenyl]-3,4-dihydro-2H-quinoxaline-1-carboxylic acid (5-hydroxy-adamantan-2-yl)-amide,
.trans-4-[6-(4-Cyclopropanesulfonyl-piperazin-1-yl)-pyridin-3-yl]-3,4-dihydro-2H-quinoxaline-1-carboxylic acid (5-carbamoyl-adamantan-2-yl)-amide,
.trans-4-[4-(4-Ethanesulfonyl-piperazin-1-yl)-phenyl]-3,4-dihydro-2H-quinoxaline-1-carboxylic acid (5-carbamoyl-adamantan-2-yl)-amide,
.trans-4-{4-[4-(2,2,2-Trifluoro-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(2-Amino-acetyl)-piperazin-1-yl]-phenyl}-3,4-dihydro-2H-quinoxaline-1-carboxylic acid (5-carbamoyl-adamantan-2-yl)-amide,
.trans-4-[4-(5-Hydroxymethyl-pyridin-2-yl)-phenyl]-3,4-dihydro-2H-quinoxaline-1-carboxylic acid (5-carbamoyl-adamantan-2-yl)-amide,
.trans-4-[5-(4-Cyclopentanesulfonyl-piperazin-1-yl)-pyridin-2-yl]-3,4-dihydro-2H-quinoxaline-1-carboxylic acid (5-hydroxy-adamantan-2-yl)-amide,
.trans-4-[4-(4-Isopropoxy-piperidin-1-yl)-phenyl]-3,4-dihydro-2H-quinoxaline-1-carboxylic acid (5-carbamoyl-adamantan-2-yl)-amide,
.trans-4-{4-[5-(2,2,2-Trifluoro-ethoxy)-pyridin-2-yl]-phenyt}-3,4-dihydro-2H-quinoxaline-1-carboxylic acid (5-hydroxy-adamantan-2-yl)-amide,
.trans-4-{4-[5-(2,2,2-Trifluoro-ethoxy)-pyridin-2-yl]-phenyl}-3,4-dihydro-2H-quinoxaline-1-carboxylic acid (5-carbamoyl-adamantan-2-yl)-amide,
.trans-4-[5-(4-Isopropoxy-phenyl)-pyrimidin-2-yl]-3,4-dihydro-2H-quinoxaline-1-carboxylic acid (5-carbamoyl-adamantan-2-yl)-amide,
.trans-4-{5-[4-(Piperidin-4-yloxy)-phenyl]-pyrimidin-2-yl}-3,4-dihydro-2H-quinoxaline-1-carboxylic acid (5-carbamoyl-adamantan-2-yl)-amide,
.trans-4-[5-(4-Trifluoromethanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-tert-Butyl-piperazin-1-yl)-pyridin-2-yl]-3,4-dihydro-2H-quinoxaline-1-carboxylic acid (5-hydroxy-adamantan-2-yl)-amide,
.trans-4-[6-(4-Ethanesulfonyl-piperazin-1-yl)-pyridin-3-yl]-3,4-dihydro-2H-quinoxaline-1-carboxylic acid (5-carbamoyl-adamantan-2-yl)-amide,
.trans-4-[5-(4-Cyclopropyl-piperazin-1-yl)-pyridin-2-yl]-3,4-dihydro-2H-quinoxaline-1-carboxylic acid (5-hydroxy-adamantan-2-yl)-amide,
.trans-4-[5-(4-Cyclobutanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-Acetyl-piperazin-1-yl)-pyridin-2-yl]-3,4-dihydro-2H-quinoxaline-1-carboxylic acid (5-hydroxy-adamantan-2-yl)-amide,
.trans-4-{4-[4-(1-Ethyl-propyl)-piperazin-1-yl]-phenyl}-3,4-dihydro-2H-quinoxaline-1-carboxylic acid (5-carbamoyl-adamantan-2-yl)-amide,
.trans-4-{5-[4-(2-Methyl-propane-1-sulfonyl)-piperazin-1-yl]-pyridin-2-yl}-3,4-dihydro-2H-quinoxaline-1-carboxylic acid (5-carbamoyl-adamantan-2-yl)-amide,
.trans-4-{5-[4-(3,3,3-Trifluoro-propane-1-sulfonyl)-piperazin-1-yl]-pyridin-2-yl}-3,4-dihydro-2H-quinoxaline-1-carboxylic acid (5-hydroxy-adamantan-2-yl)-amide,
.trans-4-{4-[4-(2,2,2-Trifluoro-ethyl)-piperazin-1-yl]-phenyl}-3,4-dihydro-2H-quinoxaline-1-carboxylic acid (5-carbamoyl-adamantan-2-yl)-amide,
.trans-4-(4-{4-[2-(2-Methoxy-ethoxy)-ethanesulfonyl]-piperazin-1-yl}-phenyl)-3,4-dihydro-2H-quinoxaline-1-carboxylic acid (5-carbamoyl-adamantan-2-yl)-amide,
.trans-4-{5-[4-(2-Isopropoxy-ethanesulfonyl)-piperazin-1-yl]-pyridin-2-yl}-3,4-dihydro-2H-quinoxaline-1-carboxylic acid (5-carbamoyl-adamantan-2-yl)-amide,
.4-[5-(4-Cyclopropylmethanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-tert-Butyl-piperazin-1-yl)-pyridin-2-yl]-3,4-dihydro-2H-quinoxaline-1-carboxylic acid (5-carbamoyl-adamantan-2-yl)-amide,
.trans-4-[5-(4-Cyclopropyl-piperazin-1-yl)-pyridin-2-yl]-3,4-dihydro-2H-quinoxaline-1-carboxylic acid (5-carbamoyl-adamantan-2-yl)-amide,
.trans-4-{4-[4-(Tetrahydro-pyran-4-yl)-piperazin-1-yl]-phenyl}-3,4-dihydro-2H-quinoxaline-1-carboxylic acid (5-carbamoyl-adamantan-2-yl)-amide,
.trans-4-{5-[4-(2-Methoxy-ethanesulfonyl)-piperazin-1-yl]-pyridin-2-yl}-3,4-dihydro-2H-quinoxaline-1-carboxylic acid (5-carbamoyl-adamantan-2-yl)-amide,
trans-4-[4-(7-Methanesulfonyl-2,7-diaza-spiro[4.4]non-2-yl)-phenyl]-3,4-dihydro-2H-quinoxaline-1-carboxylic acid (5-hydroxy-adamantan-2-yl)-amide,
trans-4-{5-[4-(2,2-Dimethyl-propyl)-piperazin-1-yl]-pyridin-2-yl}-3,4-dihydro-2H-quinoxaline-1-carboxylic acid (5-hydroxy-adamantan-2-yl)-amide,
trans-4-{5-[4-(2-Methoxy-1-methoxymethyl-ethyl)-piperazin-1-yl]-pyridin-2-yl}-3,4-dihydro-2H-quinoxaline-1-carboxylic acid (5-hydroxy-adamantan-2-yl)-amide,
trans-4-{5-[4-(3-Methoxy-propyl)-piperazin-1-yl]-pyridin-2-yl}-3,4-dihydro-2H-quinoxaline-1-carboxylic acid (5-hydroxy-adamantan-2-yl)-amide,
trans-4-{5-[4-(3-Methyl-butyryl)-piperazin-1-yl]-pyridin-2-yl}-3,4-dihydro-2H-quinoxaline-1-carboxylic acid (5-hydroxy-adamantan-2-yl)-amide,
trans-4-[4-(4-Methanesulfonyl-piperazin-1-yl)-phenyl]-3,4-dihydro-2H-quinoxaline-1-carboxylic acid (5-acetylamino-adamantan-2-yl)-amide,
trans-4-{5-[4-(2-Methoxy-ethanesulfonyl)-piperazin-1-yl]-pyridin-2-yl}-3,4-dihydro-2H-quinoxaline-1-carboxylic acid (5-hydroxy-adamantan-2-yl)-amide,
trans-4-{5-[4-(2,2-Dimethyl-propionyl)-piperazin-1-yl]-pyridin-2-yl}-3,4-dihydro-2H-quinoxaline-1-carboxylic acid (5-hydroxy-adamantan-2-yl)-amide,
trans-4-(4,4-Difluoro-3,4,5,6-tetrahydro-2H-[1,3']bipyridinyl-6'-yl)-3,4-dihydro-2H-quinoxaline-1-carboxylic acid (5-hydroxy-adamantan-2-yl)-amide,
trans-4-(4-Pyrrolidin-1-yl-3,4,5,6-tetrahydro-2H-[1,3']bipyridinyl-6'-yl)-3,4-dihydro-2H-quinoxaline-1-carboxylic acid (5-hydroxy-adamantan-2-yl)-amide,
trans-4-[5-(5-Cyclopropanesulfonyl-2,5-diaza-bicyclo[2.2.1]hept-2-yl)-pyridin-2-yl]-3,4-dihydro-2H-quinoxaline-1-carboxylic acid (5-hydroxy-adamantan-2-yl)-amide,
trans-4-{6-[4-(5-Hydroxy-adamantan-2-ylcarbamoyl)-3,4-dihydro-2H-quinoxalin-1-yl]-pyridin-3-yl}-piperazine-1-carboxylic acid tert-butyl ester,
trans-4-{4-[4-(2-Methoxy-ethyl)-piperazin-1-yl]-phenyl}-3,4-dihydro-2H-quinoxaline-1-carboxylic acid (5-carbamoyl-adamantan-2-yl)-amide,
trans-4-[5-(4-Methanesulfonyl-piperazin-1-yl)-3-methyl-pyridin-2-yl]-3,4-dihydro-2H-quinoxaline-1-carboxylic acid (5-carbamoyl-adamantan-2-yl)-amide,
trans-2-(4-{4-[4-(5-Carbamoyl-adamantan-2-ylcarbamoyl)-3,4-dihydro-2H-quinoxalin-1-yl]-phenyl}-piperazin-1-yl)-2-methyl-propionic acid,
trans-4-(4-tert-Butyl-3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-(4-Pyrrolidin-1-yl-3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-[5-(4-Ethanesulfonyl-piperazin-1-yl)-pyridin-2-yl]-3,4-dihydro-2H-quinoxaline-1-carboxylic acid (5-carbamoyl-adamantan-2-yl)-amide,
trans-4-[5-(4-Methanesulfonyl-piperazin-1-yl)-6-methyl-pyridin-2-yl]-3,4-dihydro-2H-quinoxaline-1-carboxylic acid (5-carbamoyl-adamantan-2-yl)-amide,
trans-4-(4,4-Difluoro-3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-{4-[4-(2-Hydroxy-1,1-dimethyl-ethyl)-piperazin-1-yl]-phenyl}-3,4-dihydro-2H-quinoxaline-1-carboxylic acid (5-carbamoyl-adamantan-2-yl)-amide,
trans-4-{5-[4-(Propane-2-sulfonyl)-piperazin-1-yl]-pyridin-2-yl}-3,4-dihydro-2H-quinoxaline-1-carboxylic acid (5-carbamoyl-adamantan-2-yl)-amide,
trans-4-(5-Morpholin-4-yl-pyridin-2-yl)-3,4-dihydro-2H-quinoxaline-1-carboxylic acid (5-carbamoyl-adamantan-2-yl)-amide,
trans-4-[5-(4-Methanesulfonyl-piperazin-1-yl)-4-methyl-pyridin-2-yl]-3,4-di hydro-2H-quinoxaline-1-carboxylic acid (5-carbamoyl-adamantan-2-yl)-amide,
trans-4-(4-Trifluoromethyl-3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-(4-tert-Butyl-3,4,5,6-tetrahydro-2H-[1,3']bipyridinyl-6'-yl)-3,4-dihydro-2H-quinoxaline-1-carboxylic acid (5-hydroxy-adamantan-2-yl)-amide,
trans-4-[5-(4-Isobutyl-piperazin-1-yl)-pyridin-2-yl]-3,4-dihydro-2H-quinoxaline-1-carboxylic acid (5-carbamoyl-adamantan-2-yl)-amide,
trans-4-[5-(4-Cyclopropylmethyl-piperazin-1-yl)-pyridin-2-yl]-3,4-dihydro-2H-quinoxaline-1-carboxylic acid (5-carbamoyl-adamantan-2-yl)-amide,
trans-4-{5-[4-(2,2,2-Trifluoro-ethyl)-piperazin-1-yl]-pyridin-2-yl}-3,4-dihydro-2H-quinoxaline-1-carboxylic acid (5-carbamoyl-adamantan-2-yl)-amide,
trans-4-[5-(4-tert-Butyl-piperazin-1-yl)-pyridin-2-yl]-3,4-dihydro-2H-quinoxaline-1-carboxylic acid (5-hydroxymethyl-adamantan-2-yl)-amide,
trans-4-[5-(4-tert-Butyl-piperazin-1-yl)-pyridin-2-yl]-6,7-difluoro-3,4-dihydro-2H-quinoxaline-1-carboxylic acid (5-carbamoyl-adamantan-2-yl)-amide,
trans-4-{5-[4-(2,2-Dimethyl-propionyl)-piperazin-1-yl]-pyridin-2-yl}-3,4-dihydro-2H-quinoxaline-1-carboxylic acid (5-carbamoyl-adamantan-2-yl)-amide,
trans-4-[5-(4-tert-Butyl-piperazin-1-yl)-pyrimidin-2-yl]-3,4-dihydro-2H-quinoxaline-1-carboxylic acid (5-carbamoyl-adamantan-2-yl)-amide,
trans-4-[5-(4-Cyclopropanesulfonyl-piperazin-1-yl)-pyridin-2-yl]-3,4-dihydro-2H-quinoxaline-1-carboxylic acid (5-cyano-adamantan-2-yl)-amide,
trans-4-[5-(1-tert-Butyl-piperidin-4-yloxy)-pyridin-2-yl]-3,4-dihydro-2H-quinoxaline-1-carboxylic acid (5-carbamoyl-adamantan-2-yl)-amide,
Trans-{6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-yl}-acetic acid methyl ester,
trans-6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-carboxylic acid ethyl ester,
trans-4-[5-(4-Isopropyl-piperazin-1-yl)-pyridin-2-yl]-3,4-dihydro-2H-quinoxaline-1-carboxylic acid (5-carbamoyl-adamantan-2-yl)-amide,
trans-4-(1'-tert-Butyl-1',2',3',4',5',6'-hexahydro-[3,4']bipyridinyl-6-yl)-3,4-dihydro-2H-quinoxaline-1-carboxylic acid (5-carbamoyl-adamantan-2-yl)-amide,
trans-{6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-yl}-acetic acid,
trans-6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-carboxylic acid,
trans-2-(4-{6-[4-(5-Carbamoyl-adamantan-2-ylcarbamoyl)-3,4-dihydro-2H-quinoxalin-1-yl]-pyridin-3-yl}-piperazin-1-yl)-2-methyl-propionic acid ethyl ester,
trans-4-(4,4-Dimethyl₋3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-{5-[4-(2,2-Difluoro-cyclopropylmethyl)-piperazin-1-yl]-pyridin-2-yl}-3,4-dihydro-2H-quinoxaline-1-carboxylic acid (5-carbamoyl-adamantan-2-yl)-amide,
trans-4-{5-[4-(Difluoro-methanesulfonyl)-piperazin-1-yl]-pyridin-2-yl}-3,4-dihydro-2H-quinoxaline-1-carboxylic acid (5-carbamoyl-adamantan-2-yl)-amide,
trans-4-{5-[4-(3,3,3-Trifluoro-propyl)-piperazin-1-yl]-pyridin-2-yl}-3,4-dihydro-2H-quinoxaline-1-carboxylic acid (5-carbamoyl-adamantan-2-yl)-amide, *trans* 4-{5-[4-(2-Methanesulfonyl-ethyl)-piperazin-1-yl]-pyridin-2-yl}-3,4-dihydro-2H-quinoxaline-1-carboxylic acid (5-carbamoyl-adamantan-2-yl)-amide,
trans-4-{5-[4-(1-Ethyl-propyl)-piperazin-1-yl]-pyridin-2-yl}-3,4-dihydro-2H-quinoxaline-1-carboxylic acid (5-carbamoyl-adamantan-2-yl)-amide,
trans-4-[5-(1,1-Dioxo-1lambda6-thiomorpholin-4-yl)-pyridin-2-yl]-3,4-dihydro-2H-quinoxaline-1-carboxylic acid (5-carbamoyl-adamantan-2-yl)-amide,
trans-4-{5-[4-(Tetrahydro-pyran-4-yl)-piperazin-1-yl]-pyridin-2-yl}-3,4-dihydro-2H-quinoxaline-1-carboxylic acid (5-carbamoyl-adamantan-2-yl)-amide,
trans-4-[5-(4-Cyclopropanesulfonyl-piperazin-1-yl)-3-methyl-pyridin-2-yl]-3,4-dihydro-2H-quinoxaline-1-carboxylic acid (5-carbamoyl-adamantan-2-yl)-amide,
trans-4-{5-(4-(2-Hydroxy-1,1-dimethyl-ethyl)-piperazin-1-yl]-pyridin-2-yl}-3,4-dihydro-2H-quinoxaline-1-carboxylic acid (5-carbamoyl-adamantan-2-yl)-amide,
trans-4-{5-[4-(2-Fluoro-1,1-dimethyl-ethyl)-piperazin-1-yl]-pyridin-2-yl}-3,4-dihydro-2H-quinoxaline-1-carboxylic acid (5-carbamoyl-adamantan-2-yl)-amide,
trans-4-[5-(4-Trifluoromethanesulfonyl-piperazin-1-yl)-pyridin-2-yl]-3,4-dihydro-2H-quinoxaline-1-carboxylic acid (5-carbamoyl-adamantan-2-yl)-amide.

Il est à noter que les composés ci-dessus ont été nommés en nomenclature IUPAC par l'intermédiaire du logiciel AutoNom (Beilstein Informations system).

Dans ce qui suit, on entend par groupe protecteur (GP) un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 3rd Edition (John Wiley & Sons, Inc., New York).

On entend par groupe partant (Lg, E, J, V, X, Z), dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, paranitrophényle, etc. Des exemples de groupes partants ainsi que des méthodes pour leur préparation sont donnés dans « Advances in Organic Chemistry », J. March, 3rd Edition, Wiley Interscience, p. 310-316.

Conformément à l'invention, on peut préparer les composés de formule générale (I) selon les procédés ci-après.

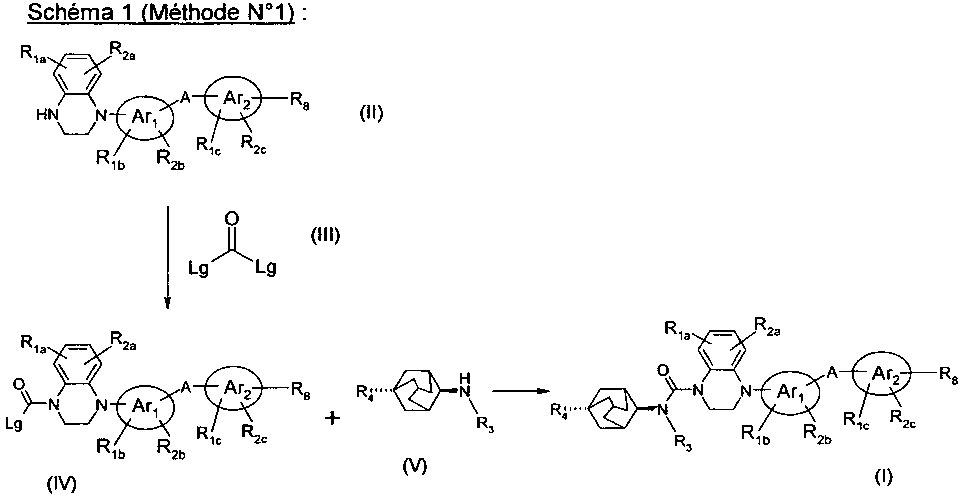

Dans le schéma 1, les composés de formule (IV) peuvent être préparés par réaction entre les intermédiaires de formule (II) et un carbonyle de formule (III) présentant deux groupes partant Lg (par exemple un atome de chlore, un groupe trichlorométhoxy, un groupe para-nitrophényle, un groupe imidazole ou méthyl-imidazolium) en présence d'une base comme la triéthylamine ou la diisopropylamine, dans un solvant tel que le dichlométhane ou le tétrahydrofurane et à une température variant de la température ambiante à 80°C. Les composés de formule (I) sont ensuite obtenus par couplage entre les dérivés activés (IV) et les amines (V) en présence ou non d'une base comme la triéthylamine ou le carbonate de potassium, dans un solvant tel que le tétrahydrofurane, le dichlorométhane, l'acétonitrile, le diméthylformamide ou l'eau, à une température variant de la température ambiante à 100°C.
Dans certains cas, quand R₁ ou R₂ est un alcool ou une amine primaire ou secondaire ou si Ar1 ou Ar2 présente dans le composé (I) une fonction amine secondaire, il est alors nécessaire de réaliser la Méthode N°1 avec un dérivé (II) où les fonctions précédemment citées sont rendues non réactives par la présence d'un groupe protecteur (par exemple pour une amine un groupe Boc, Bn ou CBz, pour un alcool un groupe Bn, pour un acide un groupe ester). Finalement, pour obtenir la fonctionnalité désirée, il faut ensuite réaliser une réaction de déprotection dans des conditions connues par l'homme de l'art.

Les hétérocycles de formule générale (V) sont disponibles commercialement ou peuvent être préparés par des méthodes décrites dans la littérature (par exemple WO 2007/077949, US 2005/0215784 A1, US 2005/0245745 A1, Journal of Organic Chemistry (2005), 70(20), 7919-7924).

Le schéma 2 détaille une synthèse des composés de formule (II).

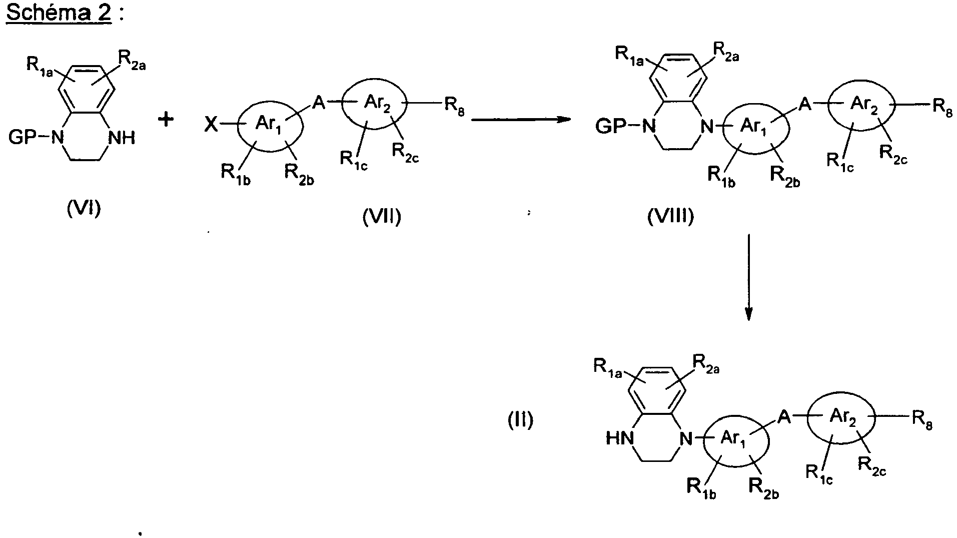

Dans le schéma 2, les composés de formule (VIII) peuvent être préparés par couplage entre une tétrahydro-quinoxaline monoprotégée de formule (VI) et un dérivé (VII) présentant un groupe partant X (par exemple un halogène, un groupe tosylate, triflate ou nanoflate) en présence d'un catalyseur organométallique tel qu'un dérivé du palladium, en présence ou non d'une phosphine telle que la triterbutylphosphine ou la triphénylphosphine, en présence d'une base comme le carbonate de potassium, le fluorure de potassium, le tertbutylate de potassium ou le phosphate de potassium dans un solvant ou mélange de solvants tel que le dioxane, l'éthylène glycol diméthyléther, le toluène, le tétrahydrofurane ou l'eau, à une température variant de la température ambiante à 100°C. Les amines (II) sont obtenues par déprotection de la fonction amine des composés de formule (VIII), par des méthodes choisies parmi celles connues de l'Homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Les hétérocycles de formule générale (VI) sont disponibles commercialement ou peuvent être préparés par des méthodes décrites dans la littérature (par exemple «Comprehensive hétérocyclic chemistry », Katritzky et al., 2rd Edition (Pergamon press) ; Krchnak, V. et coll., Tet. Lett (2001), 42, 2443-2446 ; Eary, C. T. et coll., Tet. Lett. (2006), 47, 6899-6902 ; Savrides, E.-M. et coll., J. Het. Chem. (2005), 42, 1031-1034. De Selms, R.C. et coll J. Het. Chem. (1974), 11 (4), 595-7.

Les composés de formule générale (VII) sont disponibles commercialement ou peuvent être préparés par des méthodes décrites dans la littérature (par exemple Z. Sui et coll., Bioorg. Med. Chem. Lett. (2003), 13, 761-765 ; Chopa, A. B. et coll., J. Organomet. Chem. (2005), 690(17), 3865-3877 ; Düggeli, M. et coll., Org. Biomol. Chem. (2003), 1(11), 1894-1899 ; Gros, P. et coll., J. Org. Chem (2003), 68(5), 2028-2029 ; Bouillon, A. et coll., Tet. (2002), 58(14), 2885-2890 ; Balle, T. et coll., J. Med. Chem. (2006), 49(11), 3159-3171 ; M. A. Ismail et coll., J. Med. Chem. (2006), 49(17), 5324-5332, Gu, Y. G. et coll., J. Med. Chem. (2006), 49(13), 3770-3773 ; Serafin, B. et coll., Eur. J. Med. Chem. (1977), 12(4), 325-31 ; Schmidt, H.-W. et coll., J. Het. Chem. (1987), 24(5), 1305-7 ; Walsh, D. A. et coll., J. Med. Chem. (1990), 33(7), 2028-32 ; WO 2005/042521 ; EP 0 277 725).

Le schéma 3 détaille une synthèse des composés de formule (VIII) dans lesquels Ar₁ représente un noyau pyridine (Y=C) ou pyrimidine (Y=N) ; ces composés seront appelés ci-après composés de formule (IX).

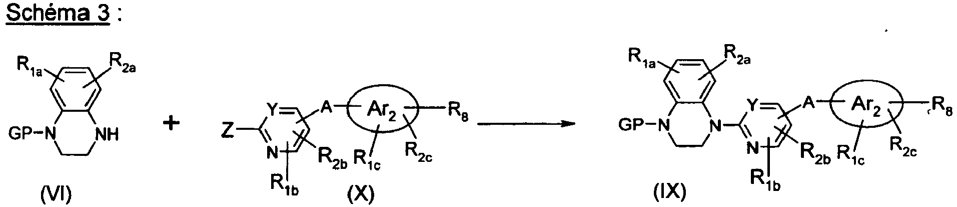

Dans le schéma 3, les composés de formule (IX) peuvent être préparés par une réaction de substitution nucléophile aromatique entre une tétrahydro-quinoxaline monoprotégée de formule (VI) et un dérivé (X) présentant un groupe partant Z (par exemple un halogène ou un groupe alkylsulfonyle) en présence d'une base comme le sel de lithium de l'hexaméthyldisilazane ou l'hydrure de sodium dans un solvant tel que le tétrahydrofurane, la N-méthyle pyrrolidinone, le diméthylsulfoxyde ou le diméthylformamide, à une température variant de la température ambiante à 100°C.

Le schéma 4 détaille une synthèse des composés de formule (VIII) dans lesquels Ar₁ représente un noyau phényle et A représente une liaison ; ces composés seront appelés ci-après composés de formule (XI).

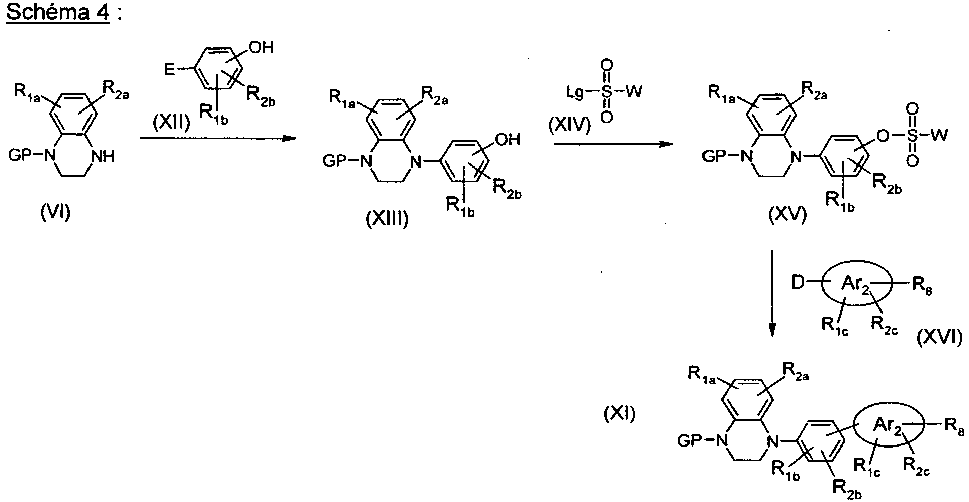

Dans le schéma 4, les composés de formule (XIII) peuvent être préparés par une réaction de couplage entre une tétrahydro-quinoxaline monoprotégée de formule (VI) et un dérivé (XII) présentant un groupe partant E (par exemple un halogène, triflate ou nanoflate) en présence d'un catalyseur organométallique tel qu'un dérivé du palladium, en présence ou non d'une phosphine tel que la triterbutylphosphine ou la triphénylphosphine, en présence d'une base comme le carbonate de potassium ou cesium, le fluorure de potassium, le tertbutylate de potassium ou le phosphate de potassium dans un solvant ou mélange de solvants tel que le dioxane, l'éthylène glycol diméthyléther, le toluène, le tétrahydrofurane ou l'eau, à une température variant de la température ambiante à 100°C. La fonction phénol est ensuite transformée en ester sulfonique (XV) par action d'un dérivé sulfonique (XIV), où Lg représente par exemple un groupe tosylate, triflate ou nanoflate, tel qu'un anhydride sulfonique (Lg=OSO₂W), un fluorure d'acide sulfonique (Lg=F) ou un chlorure d'acide sulfonique (Lg=CI), en présence d'une base ou d'un mélange de bases comme la triéthylamine, la pyridine, la diméthylaminopyridine, la diisopropyléthylamine ou le carbonate de potassium dans un solvant ou mélange de solvants tel que le dichlorométhane, le chloroforme, le toluène, le tétrahydrofurane, le diméthylformamide ou l'acétonitrile, à une température variant de -78°C à 100°C. Finalement, les dérivés (XI) peuvent être obtenus par une réaction de couplage entre un dérivé (XV) et un composé (XVI) présentant un groupe organométallique D (par exemple un dérivé du bore, un dérivé de l'étain ou un organozincique) en présence d'une espèce organométallique telle qu'un dérivé du palladium, en présence ou non d'une phosphine telle que la tricyclohexylphosphine ou la triphénylphosphine, en présence d'une base comme le carbonate de potassium ou le fluorure de potassium dans un solvant ou mélange de solvants tel que le dioxane, le diméthylformamide, l'éthylène glycol diméthyléther, le tétrahydrofurane ou l'eau, à une température variant de la température ambiante à 100°C.

Le schéma 5 présente une synthèse alternative des composés de formule (XI).

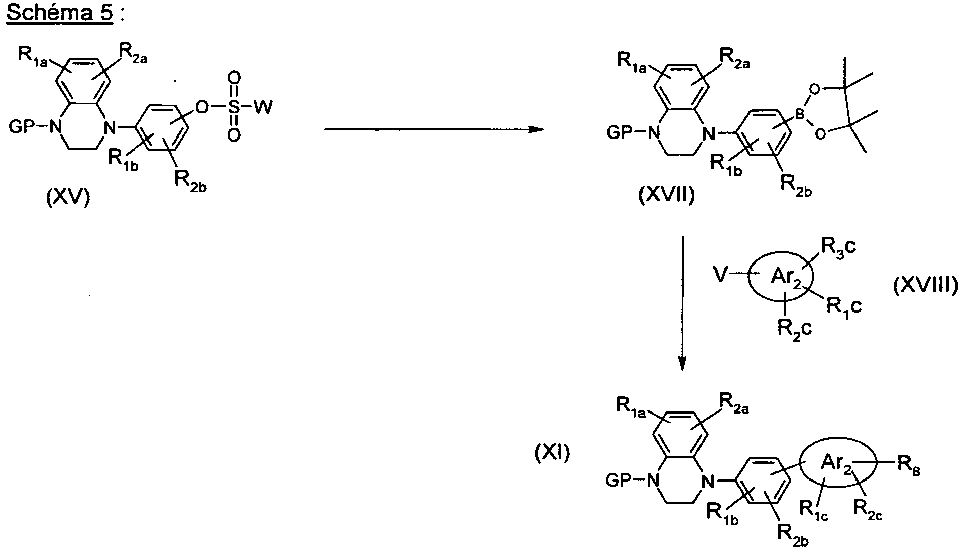

Dans le schéma 5, les composés de formule (XVII) peuvent être préparés par transformation de la fonction ester sulfonique des composé (XV) en un dérivé d'ester boronique (XVII) par une réaction avec le bispinacolatodiborane en présence d'un complexe du palladium tel que le 1,1'-bis(diphénylphosphino)ferrocedichloropalladium (II) en présence d'une base comme l'acétate de potassium et de chlorure de lithium dans un solvant ou mélange de solvants tel que le dichlorométhane, le dioxane ou le diméthylsulfoxide, à une température variant de la température ambiante à 100°C. Dans une deuxieme étape, les dérivés (XI) peuvent être obtenus par une réaction de couplage entre le dérivé (XVII) et un composé (XVIII) présentant un groupe partant V (par exemple un halogène, un triflate, un nonaflate) en présence d'un catalyseur organométallique tel qu'un dérivé du palladium, en présence ou non d'une phosphine telle que la tricyclohexylphosphine ou la triphénylphosphine, en présence d'une base comme le carbonate de sodium ou potassium ou le fluorure de potassium, dans un solvant ou mélange de solvants tel que le dioxane, le diméthylformamide, l'éthylène glycol diméthyléther, le tétrahydrofurane ou l'eau, à une température variant de la température ambiante à 100°C.

Le schéma 6 détaille une synthèse des composés de formule (VIII) dans lesquels Ar₁ représente un noyau pyridine (un seul des deux atomes Y est un azote, l'autre est un carbone) et A représente une liaison ; ces composés seront appelés ci-après composés de formule (XIX).

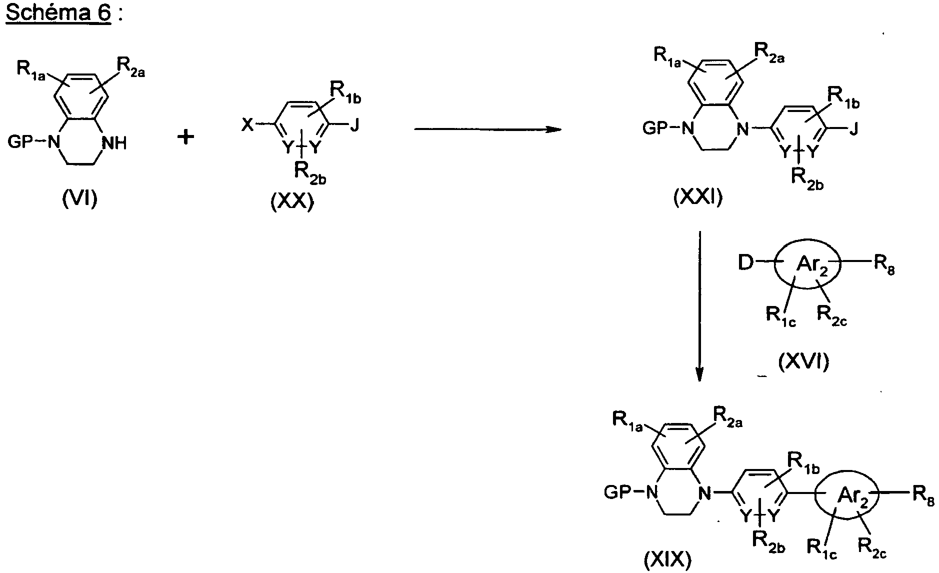

Dans le schéma 6, les composés de formule (XXI) peuvent être préparés par une réaction de substitution nucléophile aliphatique ou aromatique entre une tétrahydro-quinoxaline monoprotégée de formule (VI) et un dérivé (XX) présentant un groupe partant X (par exemple un atome de fluor) et un groupe partant J (par exemple un atome de brome) en présence d'une base comme le tertbutylate de potassium ou l'hydrure de sodium dans un solvant tel que la *N*-méthyle pyrrolidinone ou le diméthylformamide, à une température variant de la température ambiante à 100°C. Finalement, les dérivés (XIX) peuvent être obtenus par une réaction de couplage entre un dérivé (XXI) et un composé (XVI) présentant un groupe organométallique D (par exemple un dérivé du bore, un dérivé de l'étain ou un organozincique) en présence d'un catalyseur organométallique tel qu'un dérivé du palladium, en présence ou non d'une phosphine telle que la tricyclohexylphosphine ou la triphénylphosphine, en présence d'une base comme le carbonate de potasium ou cesium, le triphosphate de potassium ou le fluorure de potassium, dans un solvant ou mélange de solvants tel que le toluène, le dioxane, le diméthylformamide, l'éthylène glycol diméthyléther, le tétrahydrofurane ou l'eau, à une température variant de la température ambiante à 100°C.

Le schéma 7 détaille une synthèse des composés de formule (VIII) dans lesquels A est un atome d'oxygène ou un groupe -O-CH₂-; ces composés seront appelés ci-après composés de formule (XXII).

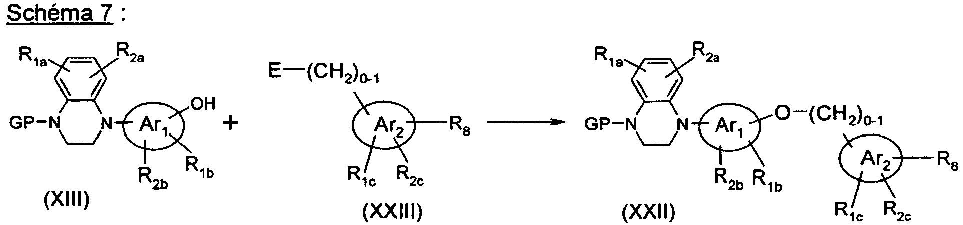

Dans le schéma 7, les composés de formule (XXII) peuvent être préparés par une réaction de substitution nucléophile aromatique ou aliphatique entre une quinoxaline de formule (XIII) et un dérivé (XXIII) présentant un groupe partant E (par exemple un halogène) en présence d'une base comme le tertbutylate de potassium, l'hydrure de sodium ou le carbonate de potassium dans un solvant tel que le diméthylformamide, à une température variant de la température ambiante à 150°C. Dans certains cas, au cours de la réaction, le groupe protecteur GP peut se retirer simultanément à la substitution nucléophile (en particulier si GP est un groupe Boc).

Le schéma 8 détaille une synthèse des composés de formule (VIII) dans lesquels A est une liaison, Ar₁ est un groupe phényle et Ar₂ est un groupe pipéridinyle ; ces composés seront appelés ci-après composés de formule (XXVIII).

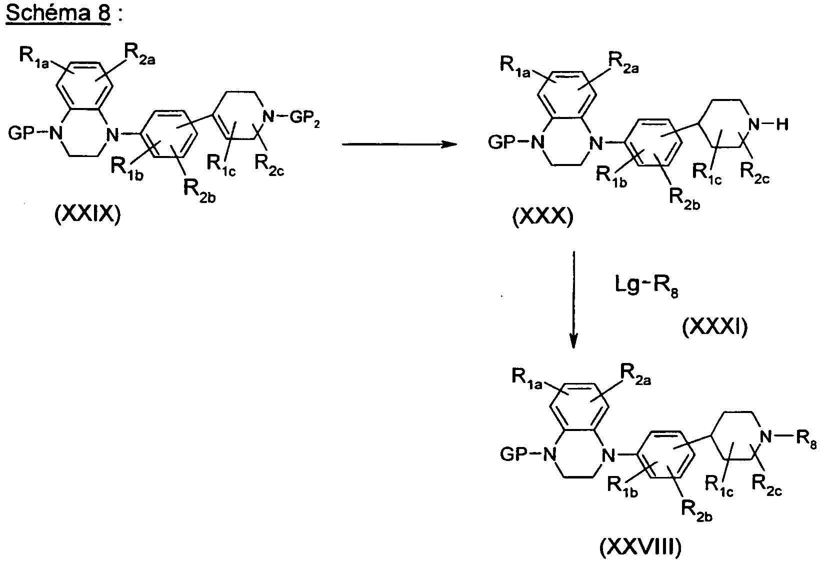

Dans le schéma 8, pour conduire aux dérivés (XXX) le composé de formule (XXIX), où la fonction amine de la 1,2,3,6-tétrahydropipéridine est protégée par un groupe protecteur GP₂ de type benzyle ou carboxybenzyle (le groupe GP étant soit le groupe Fmoc, soit le groupe Boc), peut subir une réaction d'hydrogénation de la double liaison ainsi qu'hydrogénolyse du groupe protecteur GP₂ en présence d'un catalyseur organométallique tel qu'un dérivé du palladium (par exemple du Pd 10% déposé sur charbon, ou du Pd(OAc)₂) dans un solvant ou mélange de solvants tel que le méthanol, l'éthanol ou de l'acétate d'éthyle, à une température variant de la température ambiante à 80°C. Dans la dernière étape, le composé (XXVIII) peut être préparé suivant différentes méthodes. Le composé (XXXI) peut être un dérivé d'acide carboxylique et le dérivé (XXVIII) peut alors être obtenu par une réaction entre (XXX) et (XXXI) dans des conditions de couplage peptidique classiques, en utilisant par exemple comme agent couplant le dicyclocarbodümide, le chlorhydrate du 1-(3-diméthylaminopropyl)-3-éthylcarbodiimide ou le bromo-tris-pyrrolidino-phosphonium hexafluorophosphate, en présence ou non d'hydroxybenzo-triazole, et en utilisant comme base organique la triéthylamine ou la diisopropyl-éthylamine dans un solvant ou mélange de solvants tel que le dioxane, le dichlorométhane ou l'acétonitrile. Le composé (XXXI) peut être un dérivé d'acide tel qu'un chlorure, bromure ou fluorure d'acide, un isocyanate, un chlorure de carbamoyle ou encore un chlorure d'acide sulfonique et le dérivé (XXVIII) peut alors être obtenu par une réaction entre (XXX) et (XXXI) en présence d'une base comme la triéthylamine, la diisopropylamine ou la pyridine, sans solvant ou dans un solvant ou mélange de solvants tel que le dichlométhane, le tétrahydrofurane, l'acétonitrile ou le diméthylformamide à une température variant de 0°C à 160°C. Le composé (XXXI) peut encore être une cétone et le dérivé (XXVIII) peut alors être obtenu par une réaction d'amination réductrice entre (XXX) et (XXXI) en utilisant un réducteur tel que le borohydrure de sodium, le triacétoxyborohydrure de sodium ou le cyanoborohydrure de sodium, en présence ou non d'un acide de Brönsted (tel que l'acide chlorhydrique) ou de Lewis (tel que le tétraisopropoxyde de titane) dans un solvant tel que le dichloroéthane, le dichlorométhane, l'acide acétique ou le méthanol, à des températures comprises entre -10°C et 30°C.

Le schéma 9 détaille une synthèse des composés de formule (VIII) dans lesquels A est une liaison, Ar₁ est un groupe thiazolyle et Ar₂ est un groupe phényle substitué par un groupe -CONR₆R₇; ces composés seront appelés ci-après composés de formule (XXXII).

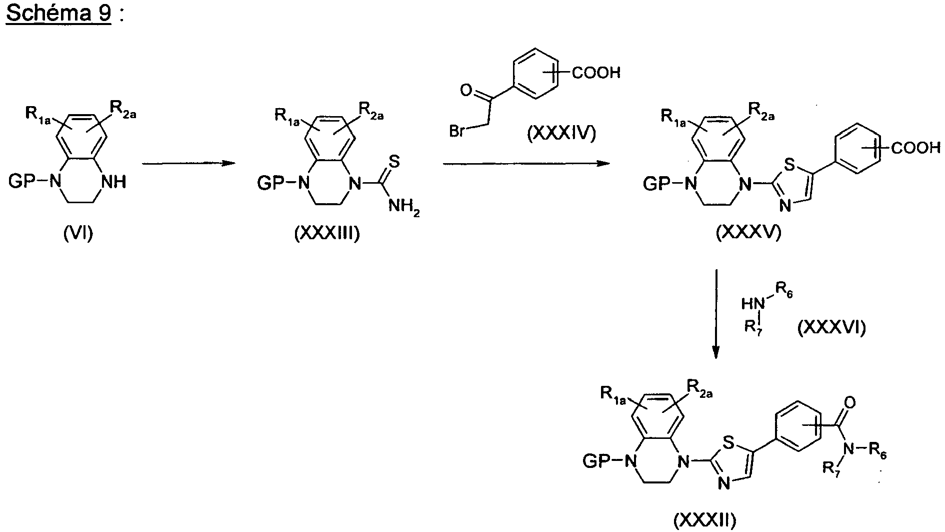

Dans le schéma 9, les composés de formule (XXXIII) peuvent être préparés par une méthodologie telle que décrite dans la demande de brevet WO 2005/007601. Le dérivé de thiazole (XXXV) peut être obtenu de façon classique (cf. Koti, R. S. et coll., Synthetic Communications (2007), 37(1), 99-105; Duggineni, S. et coll., Tetrahedron (2006), 62(14), 3228-3241 ; Balavoine, F. et coll., Bioorganic & Medicinal Chemistry Letters (2007), 17(13), 3754-3759) par condensation du dérivé thiourée (XXXIII) avec un dérivé d'alpha bromo cétone (XXXIV) dans un solvant tel que l'éthanol, l'acétone ou le tétrahydrofurane, à des températures comprises entre la température ambiante et 80°C. Finalement le dérivé (XXXII) peut être obtenu par une réaction entre (XXXV) et (XXXVI) dans des conditions de couplage peptidique classiques, en utilisant par exemple comme agent couplant le dicyclocarbodiimide, le chlorhydrate du 1-(3-diméthylaminopropyl)-3-éthylcarbodiimide ou le bromo-tris-pyrrolidino-phosphonium hexafluorophosphate, en présence ou non d'hydroxybenzo-triazole, et en utilisant comme base organique la triéthylamine ou la diisopropyl-éthylamine dans un solvant ou mélange de solvants tel que le dioxane, le dichlorométhane ou l'acétonitrile.

Le schéma 10 détaille une synthèse alternative des composés de formule (I) dans lesquels A représente une liaison.

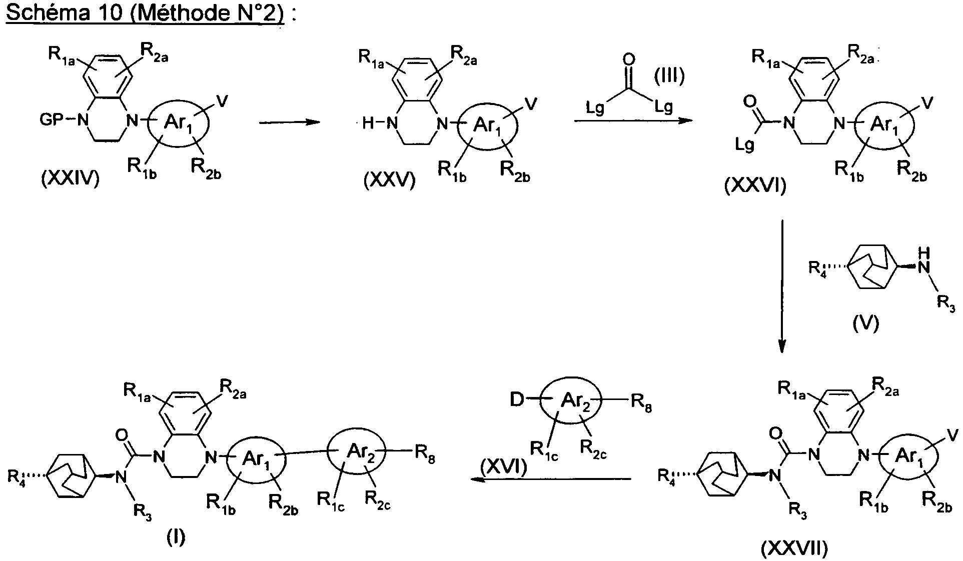

Dans le schéma 10, les amines (XXV) présentant un groupe partant V (par exemple un halogène, un triflate ou un nonaflate) sont obtenues par déprotection de la fonction amine des composés de formule (XXIV) par des méthodes choisies parmi celles connues de l'Homme du métier. Elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C. Dans une étape suivante, les composés de formule (XXVI) peuvent être préparés par réaction entre les intermédiaires de formule (XXV) et un carbonyle de formule (III) présentant deux groupes partant Lg (choisis par exemple parmi des atomes de chlore ou des groupes trichlorométhoxy, para-nitrophényle, imidazole ou méthyl-imidazolium) en présence d'une base comme la triéthylamine ou la diisopropylamine, dans un solvant tel que le dichlométhane ou le tétrahydrofurane, à une température variant de la température ambiante à 80°C. Les composés de formule (XXVII) sont ensuite obtenus par couplage entre les dérivés activés (XXVI) et les amines (V) en présence ou non d'une base comme la triéthylamine ou le carbonate de potassium dans un solvant tel que le tétrahydrofurane, le dichlorométhane, l'acétonitrile, le diméthylformamide ou l'eau, à une température variant de la température ambiante à 100°C. Finalement les composés (I) peuvent être obtenus par une réaction de couplage entre un dérivé (XXVII) et un composé (XVI) présentant un groupe organométallique D (par exemple un dérivé du bore, un dérivé de l'étain ou un organozincique) en présence d'un catalyseur organométallique tel qu'un dérivé du palladium (par exemple le 2'-(diméthylamino)-2-biphénylyl-palladium(II) chloride dinorbornylphosphine), en présence ou non d'une phosphine telle que la tricyclohexylphosphine ou la triphénylphosphine, en présence d'une base comme le carbonate de potasium ou cesium, le triphosphate de potassium ou le fluorure de potassium dans un solvant ou mélange de solvants tel que le toluène, le dioxane, le diméthylformamide, l'éthylène glycol diméthyléther, le tétrahydrofurane ou l'eau, à une température variant de la température ambiante à 100°C, sous chauffage thermique ou micro-onde.

Le schéma 11 détaille une synthèse des composés de formule (I) dans lesquels R₄ représente une fonction amide ; ces composés seront appelés ci-après composés de formule (XXXVII).

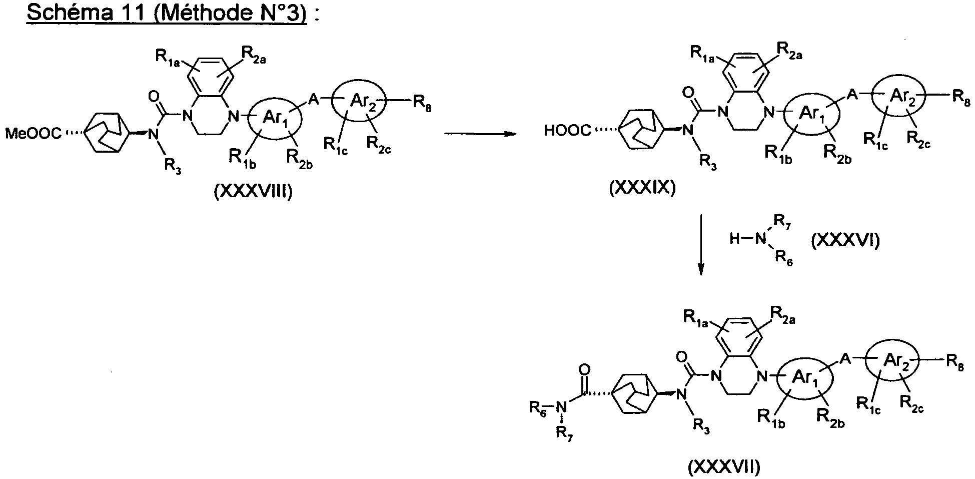

Dans le schéma 11, la fonction ester du composé (XXXVIII) est saponifiée en fonction acide au moyen de soude, de potasse ou de lithine dans un solvant ou mélange de solvants tel qu'un alcool, de l'eau ou du tétrahydrofurane, à une température variant de la température ambiante à 100°C, pour conduire à l'acide (XXXIX). Dans la dernière étape, le composé (XXXVII) peut être préparé par condensation entre l'intermédiaire acide de formule (XXXIX) et une amine (XXXVI) dans des conditions de couplage peptidique classiques, en utilisant par exemple comme agent couplant le dicyclocarbodiimide, le chlorhydrate du 1-(3-diméthylaminopropyl)-3-éthylcarbodiimide ou le bromo-tris-pyrrolidino-phosphonium hexafluorophosphate, en présence ou non d'hydroxybenzo-triazole, et en utilisant comme base organique la triéthylamine ou la diisopropyl-éthylamine dans un solvant ou mélange de solvants tel que le dioxane, le dichlorométhane ou l'acétonitrile.

Le schéma 12 détaille une synthèse alternative des composés de formule (I).

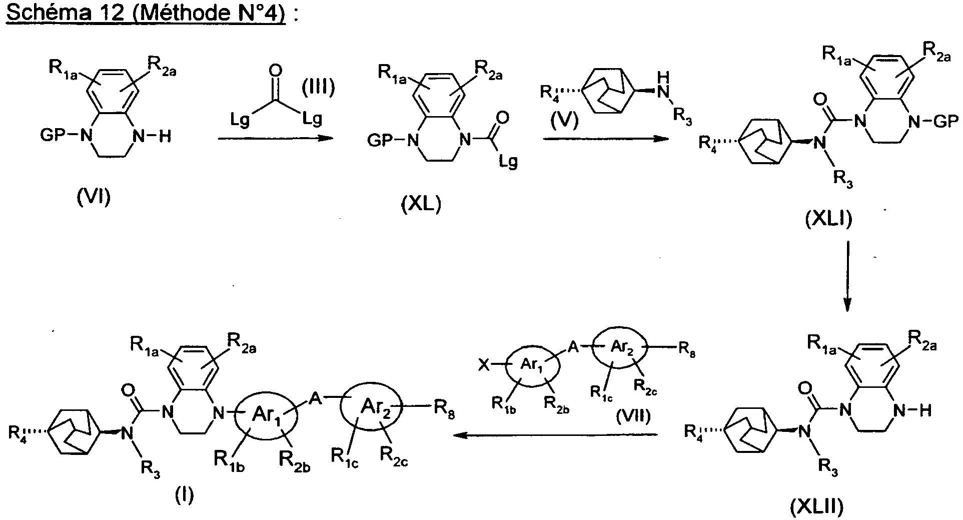

Dans le schéma 12, les composés de formule (XL) peuvent être préparés par réaction entre les intermédiaires de formule (VI) et un carbonyle de formule (III) présentant deux groupes partant Lg (choisis par exemple parmi des atomes de chlore ou des groupes trichlorométhoxy, para-nitrophényle, imidazole ou méthyl-imidazolium) en présence d'une base comme la triéthylamine ou la diisopropylamine, dans un solvant tel que le dichlométhane ou le tétrahydrofurane, à une température variant de la température ambiante à 80°C. Les composés de formule (XLI) sont ensuite obtenus par couplage entre les dérivés activés (XL) et les amines (V) en présence ou non d'une base comme la triéthylamine ou le carbonate de potassium dans un solvant tel que le tétrahydrofurane, le dichlorométhane, l'acétonitrile, le diméthylformamide ou l'eau, à une température variant de la température ambiante à 100°C. Les amines (XLII) sont ensuites obtenues par déprotection de la fonction amine des composés de formule (XLI), par des méthodes choisies parmi celles connues de l'Homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C. Finalement les composés (I) peuvent être obtenus par couplage entre le dérivé (XLII) et un dérivé (VII) présentant un groupe partant X (par exemple un halogène, un groupe tosylate, triflate ou nanoflate) en présence d'un catalyseur organométallique tel qu'un dérivé du palladium, en présence ou non d'une phosphine telle que la triterbutylphosphine ou la triphénylphosphine, en présence d'une base comme le carbonate de potassium, le fluorure de potassium, le tertbutylate de potassium ou le phosphate de potassium dans un solvant ou mélange de solvants tel que le dioxane, l'éthylène glycol diméthyléther, le toluène, le tétrahydrofurane ou l'eau, à une température variant de la température ambiante à 100°C

Le schéma 13 détaille une synthèse des composés de formule (VIII) dans lesquels Ar₁ représente un noyau pyridine (un des deux Y est un azote, l'autre est un carbone) ou pyrimidine (les deux Y sont des azotes) et A représente une liaison ; ces composés seront appelés ci-après composés de formule (XLIII).

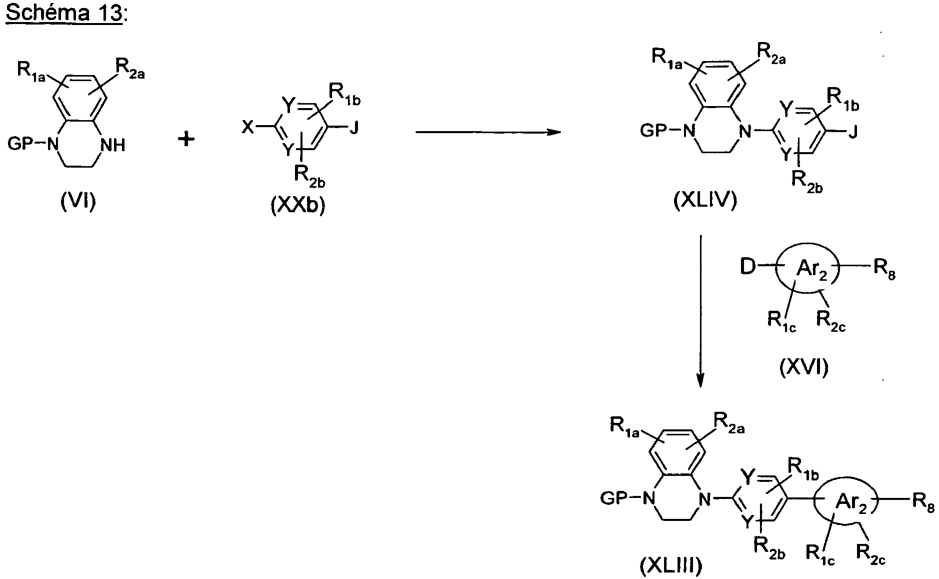

Dans le schéma 13, les composés de formule (XLIV) peuvent être préparés par une réaction de substitution nucléophile aromatique entre une tétrahydro-quinoxaline monoprotégée de formule (VI) et un dérivé (XXb) présentant un groupe partant X (par exemple un atome de fluor, de clhlore ou un groupe SO₂Me) et un autre groupe partant J (par exemple un atome de brome ou d'iode) en présence d'une base comme le tertbutylate de potassium, le n-butyle lithium ou l'hydrure de sodium dans un solvant tel que la N-méthyle pyrrolidinone, le tétrahydrofurane ou le diméthylformamide, à une température variant de -70°C à 100°C. Finalement, les dérivés (XLIII) peuvent être obtenus par une réaction de couplage entre un dérivé (XLIV) et un composé (XVI) dans lequel D est soit un groupe organométallique (par exemple un dérivé du bore, un dérivé de l'étain, un organozincique) soit un atome d'hydrogène d'une amine secondaire cyclique (par exemple Ar₂ est un dérivé de piperazine ou de piperidine) en présence d'un catalyseur organométallique tel qu'un dérivé du palladium, en présence ou non d'une phosphine telle que la tricyclohexylphosphine, la tritertbutylphoshine, la triphénylphosphine ou la 2-dicyclohexylphosphino-2',6'-dimethoxybiphenyl, en présence d'une base comme le carbonate de potasium ou cesium, le triphosphate de potassium ou le fluorure de potassium, dans un solvant ou mélange de solvants tel que le toluène, le dioxane, le diméthylformamide, l'éthylène glycol diméthyléther, le tétrahydrofurane ou l'eau, à une température variant de la température ambiante à 100°C.

Le schéma 14 détaille une synthèse des composés de formule (I) dans lesquels R₄ représente un groupe CH₂OH ; ces composés seront appelés ci-après composés de formule (XLV).

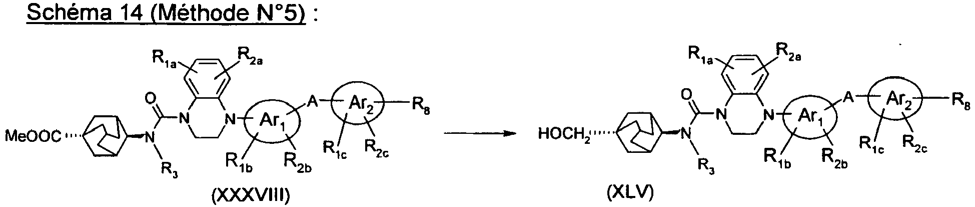

Dans le schéma 14, les dérivés (XLV) sont obtenus par réduction de la fonction ester du composé (XXXVIII) en fonction alcool au moyen d'un hydrure tel que LiAlH₄, LiBH₄, DiBal dans un solvant ou mélange de solvants tel que le tetrahydrofurane, l'éther ou le toluène, à une température variant de la -78°C à 40°C.

Le schéma 15 détaille une synthèse des composés de formule (I) dans lesquels Ar₂ est un groupe pipérazine, A est une liaison simple connectée directement sur un des deux azotes de la pipérazine, R1c est connecté sur l'autre atome d'azote de la piperazine; ces composés seront appelés ci-après composés de formule (XLVI). Le même schéma réactionnel peut aussi s'appliquer à la synthèse de composés où la position entre R_{1c} et R₈ est interchangée. Les groupes Lg-R_{1c} et O=R_{1c} sont alors remplacés respectivement par les groupes Lg-R₈ et O=R₈.

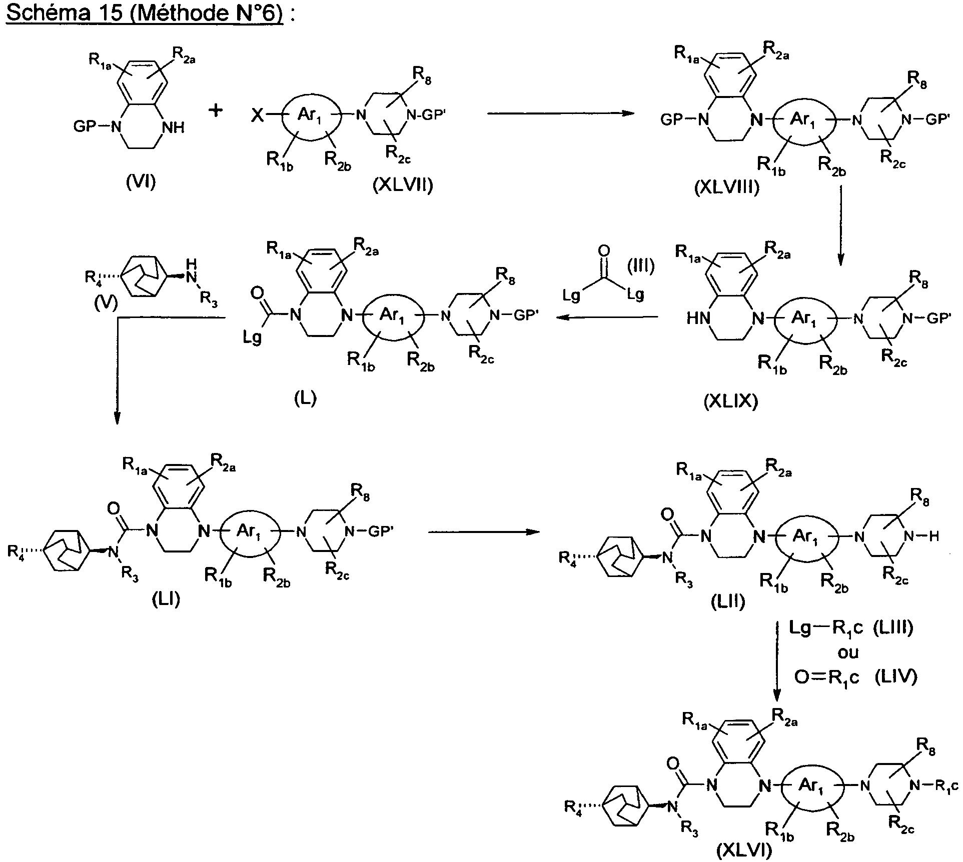

Dans le schéma 12, les composés de formule (XLVIII) peuvent être préparés par couplage entre une tétrahydro-quinoxaline monoprotégée de formule (VI) et un dérivé (XLVII) présentant un groupe partant X (par exemple un halogène, un groupe tosylate, triflate ou nanoflate) en présence d'un catalyseur organométallique tel qu'un dérivé du palladium, en présence ou non d'une phosphine telle que la triterbutylphosphine ou la triphénylphosphine, en présence d'une base comme le carbonate de potassium, le fluorure de potassium, le tertbutylate de potassium ou le phosphate de potassium dans un solvant ou mélange de solvants tel que le dioxane, l'éthylène glycol diméthyléther, le toluène, le tétrahydrofurane ou l'eau, à une température variant de la température ambiante à 100°C. Les deux composés (VI) et (XLVII) présentent deux fonctions amines protégées. Pour obtenir une déprotection sélective du composé (XLVIII) il faut que les deux groupes protecteurs portés par les composés (VI) et (XLVII) soient différents. De plus, chaque groupe protecteur doit pouvoir être résistant aux conditions de déprotection de son voisin. Par exemple, GP peux être un groupe Boc ou fmoc et GP' peut être un groupe benzyl ou carboxybenzyl. Les amines (XLIX) sont obtenues par déprotection sélective des groupes GP des composés de formule (XLVIII), par des méthodes choisies parmi celles connues de l'Homme du métier; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C. Dans l'étape suivante, les composés de formule (L) peuvent être préparés par réaction entre les intermédiaires de formule (XLIX) et un carbonyle de formule (III) présentant deux groupes partant Lg (choisis par exemple parmi des atomes de chlore ou des groupes trichlorométhoxy, para-nitrophényle, imidazole ou méthyl-imidazolium) en présence d'une base comme la triéthylamine ou la diisopropylamine, dans un solvant tel que le dichlométhane ou le tétrahydrofurane, à une température variant de la température ambiante à 80°C. Les composés de formule (LI) sont ensuite obtenus par couplage entre les dérivés activés (L) et les amines (V) en présence ou non d'une base comme la triéthylamine ou le carbonate de potassium dans un solvant tel que le tétrahydrofurane, le dichlorométhane, l'acétonitrile, le diméthylformamide ou l'eau, à une température variant de la température ambiante à 100°C. Les amines (LII) sont ensuites obtenues par déprotection de la fonction amine des composés de formule (LI), par exemple si PG' est un groupe benzyle ou carboxybenzyle, les méthodes de déprotection possibles comprennent entre autres l'utilisation d'hydrogène en présence d'un catalyseur dérivé du palladium pour réaliser une réaction d'hydrogénolyse, dans un solvant ou mélange de solvants tel que le méthanol, l'ethanol, l'acétate d'éthyle, le tétrahydrofurane, sous une pression d'hydrogène comprise entre 1 et 10 bar à une température variant de la température ambiante à 80°C. Une méthode alternative pour réaliser une hydrogénolyse de groupement Bn ou Cbz est d'utiliser une catalyse au palladium (par exemple avec du palladium adsorbé sur du charbon) en présence de formiate d'ammonium au reflux d'un solvant tel que le méthanol. Dans la dernière étape, les composés (XLVI) peuvent être obtenus suivant différentes réactions :
- On peut faire réagir sur le composé (LII), un dérivé (LIII) de type chlorure de sulfonyle, chlorure d'acide ou chlorure de carbamoyle (Lg est alors un atome de chlore) en présence d'une base telle que la triéthylamine, la diisopropyl éthylamine ou la pyridine, avec ou sans solvant tel que le dichlorométhane, le chloroforme, le tétrahydrofurane ou le dioxane à une température variant de 0 à 40°C.
- Une réaction d'alkylation est aussi possible entre le composé (LII) et un groupe alkyle (LIII) substitué ou non dans lequels Lg est par exemple un atome de chlore, de brome ou d'iode, un groupe tosylate ou triflate, en présence d'une base telle que la triéthylamine, la diisopropyl éthylamine, dans un solvant tel que le tétrahydrofurane ou le dioxane à une température variant de 0 à 80°C.
- Une réaction d'amination réductrice peut aussi être réalisé entre le composé (LII) et un dérivé (LIV) de type aldéhyde ou cétone, en utilisant un réducteur tel que le borohydrure de sodium, le triacétoxyborohydrure de sodium ou le cyanoborohydrure de sodium, en présence ou non d'un acide de Bronsted (tel que l'acide chlorhydrique) ou de Lewis (tel que le tétraisopropoxyde de titane) dans un solvant tel que le dichloroéthane, le dichlorométhane, l'acide acétique ou le méthanol, à des températures comprises entre -10°C et 30°C.
Dans certain cas, une étape supplémentaire de déprotection est nécéssaire afin d'obtenir la fonctionalité désirée sur R_{1c} ou R₈ (par exemple une fonction alcool ou amine primaire ou secondaire).

Le schéma 16 détaille une synthèse des composés de formule (I) dans lesquels R₄ représente un atome d'halogène tel qu'un atome de fluor ; ces composés seront appelés ci-après composés de formule (LV).

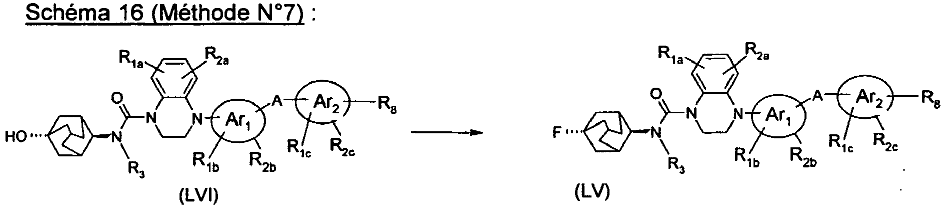

Dans le schéma 16, les dérivés (LV) sont obtenus par transformation de la fonction alcool du composé (LVI) en atome d'halogène tel qu'un atome de fluor au moyen d'un réactif d'halogénation tel que le (diethylamino)sulfur triofluoride (DAST) ou le bis(2-methoxyethyl)aminosulfur trifluoride (Deoxo-Fluor^{®}) dans un solvant tel que dichlorométhane ou le toluène à une température variantde -78°C à la température ambiante.

Le schéma 17 détaille une synthèse des composés de formule (I) dans lesquels R_{1c} représente un groupe -SO₂-(CH₂)₁₋₃-CO₂H ; ces composés seront appelés ci-après composés de formule (LVI').

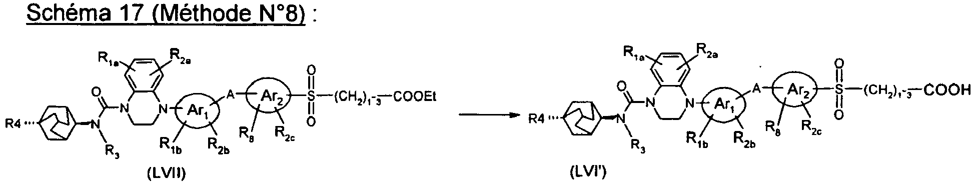

Dans le schéma 17, les dérivés (LVI) sont obtenus par hydrolyse de la fonction ester (par exemple un ester ethylique) du composé (LVII) au moyen d'une base minérale telle que la soude, la potasse ou la lithine dans un solvant tel qu'un alcool ou de l'eau à une température variant de la température ambiante à 100°C pour conduire aux acides (LVI).

Le schéma 18 détaille une synthèse des composés de formule (I) dans lesquels Ar₂ est un groupe pipéridine, A est une liaison simple connectée directement sur l'azote de la pipéridine, R1c est une fonction alcool et est connectée en position 4 de l'atome d'azote de la pipéridine ; ces composés seront appelés ci-après composés de formule (LVIII).

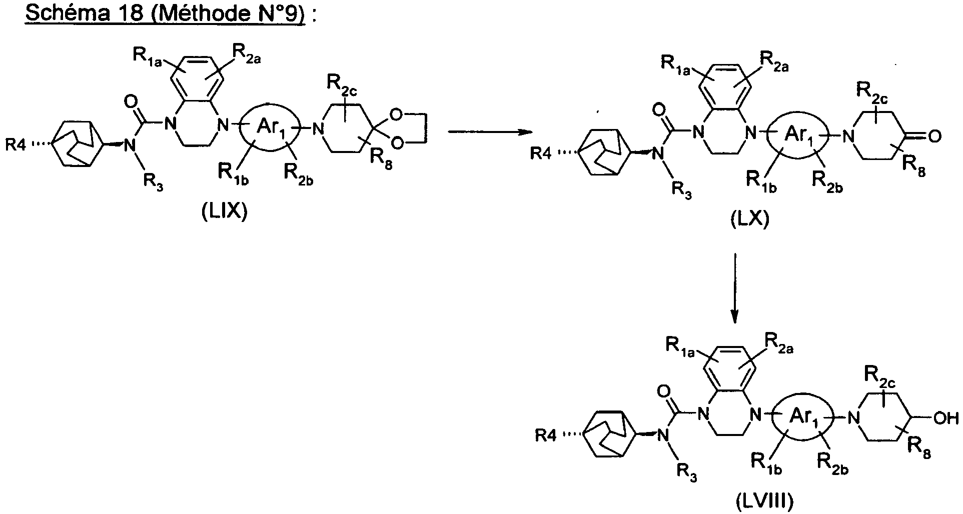

Dans le schéma 18, les dérivés (LX) sont obtenus par hydrolyse de la fonction acétal cyclique du composé (LIX) au moyen d'un acide tel que l'acide chlorhydrique dans un solvant ou mélange de solvants tel que de l'eau, un alcool, du dioxane à une température variant de la température ambiante à 100°C pour conduire aux cétones (LX). La dernière étape consiste à réduire le groupe carbonyle en fonction alcool au moyen d'un agent de réduction tel qu'un hydrure, par exemple le borohydrure de sodium ou l'hydrure double de lithium et d'aluminium dans un solvant tel que le méthanol ou l'éthanol, à une température variant de 0°C à la température ambiante, pour conduire aux composés (LVIII).

Le schéma 19 détaille une synthèse des composés de formule (VIII) dans lesquels Ar₂ est un groupe piperazine, A est une liaison simple connectée directement sur un des deux azotes de la pipérazine, R_{1c} est un groupe -SO₂(CH₂)₂-Nu (Nu est un groupe OR₅ ou NR₆R₇) et est connecté sur l'autre atome d'azote de la pipérazine; ces composés seront appelés ci-après composés de formule (LXI).

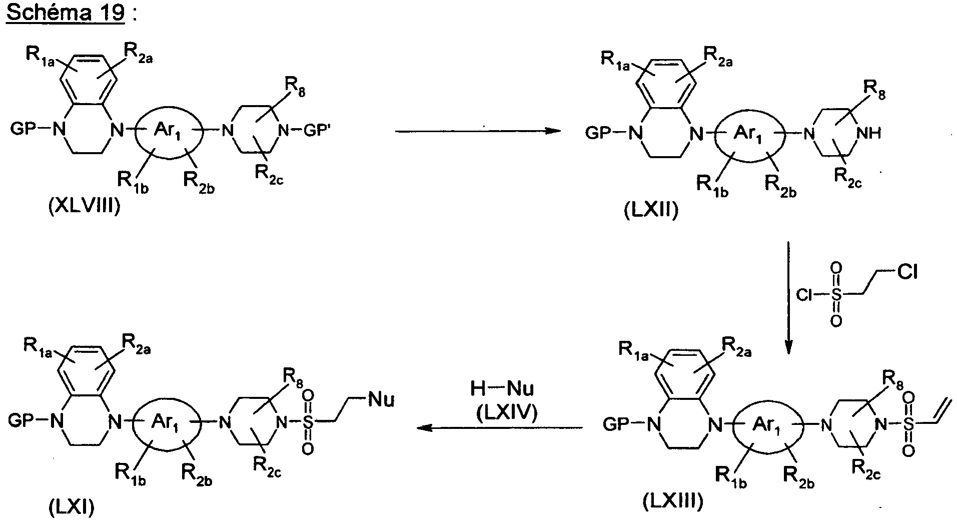

Dans le schéma 19, les composés de formule (LXII) peuvent être obtenus par une déprotection sélective du groupe GP' par rapport au groupe GP. Par exemple si GP est un groupe Boc et GP' est un groupe CBz, une réaction d'hydrogénolyse du dérivé (XLVIII) réalisé par exemple soit sous pression d'hydrogène soit avec du formiate d'amonium avec une catalyse au palladium conduit au dérivé monoprotégé (LXII). Dans l'étape suivante le composé (LXIII) est obtenu par mise en réaction du dérivé (LXII) avec le chlorure de l'acide 2-chloroéthanesulfonique selon les conditions décritent par Golding et coll.dans l'article Org. Biomol. Chem., 2007, 5, (132-138). Les intermédiaires (LXI) sont finalement obtenus après une réaction de type Michael entre le dérivé (LXIII) et les nucléophiles H-Nu (LXIV). Les nucléophiles (LXIV) peuvent être de nature variés comme des amines secondaires, des alcools ou des phtalimides et la réaction peut être conduite dans des conditions décritent par B.T. Golding et coll.dans l'article Org. Biomol. Chem.,2007, 5, (132-138) ou par J. Marchand-Brynaert dans l'article Tet. 1996, 52, (5591-5606).

Le schéma 20 détaille une synthèse des composés de formule (I) dans lesquels R_{1c} représente un groupe -SO₂(CH₂)₂₋₃CH₂OH ; ces composés seront appelés ci-après composés de formule (LXV).

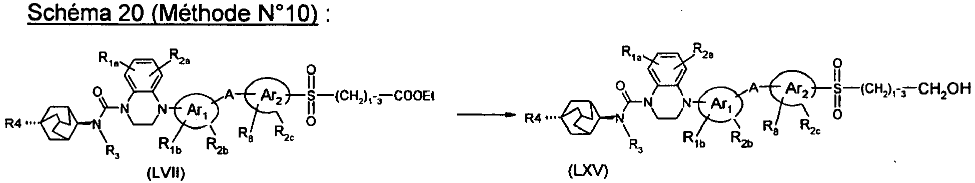

Dans le schéma 20, les dérivés (LXV) sont obtenus par réduction de la fonction ester du composé (LVII) en fonction alcool au moyen d'un agent de réduction tel qu'un hydrure par exemple LiAlH₄, LiBH₄, DIBAL dans un solvant ou mélange de solvants tel que le tetrahydrofurane, l'éther ou le toluène, à une température variant de -78°C à 40°C.

Le schéma 21 détaille une synthèse des composés de formule (I) dans lesquels Rₐ représente un groupe 4-hydroxypiperidine et R₈ est connecté à Ar₂ par l'atome d'oxygène ; ces composés seront appelés ci-après composés de formule (LXVI).

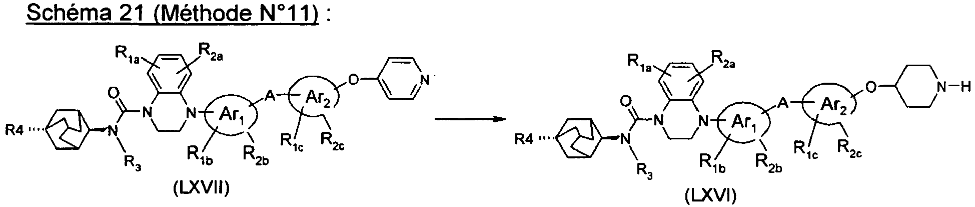

Dans le schéma 21, les dérivés (LXVI) sont obtenus par réduction du noyau pyridine en pipéridine du composé (LXVIII) au moyen d'un catalyseur tel que des dérivé du pallladium ou du platine en présence ou non d'un acide tel que l'acide para toluene sulfonique, HCl ou H₂SO₄, sous une pression d'hydrogène comprise entre 1 et 50 bar dans un solvant ou mélange de solvants comme un alcool ou de l'eau.

Le schéma 22 détaille une synthèse des composés de formule (VIII) dans lesquels Ar₂ est un groupe piperazine, A est une liaison simple connectée directement sur un des deux azotes de la pipérazine, R_{1c} est connecté sur l'autre atome d'azote de la pipérazine; ces composés seront appelés ci-après composés de formule (LXVIII).

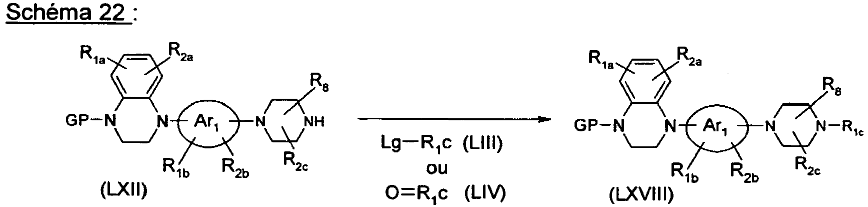

Les composés (XLVIII) peuvent être obtenus suivant différentes réactions :
- On peut faire réagir sur le composé (LXII), un dérivé (LIII) de type chlorure de sulfonyle, chlorure d'acide ou chlorure de carbamoyle (Lg est alors un atome de chlore) en présence d'une base telle que la triéthylamine, la diisopropyl éthylamine ou la pyridine, avec ou sans solvant tel que le dichlorométhane, le chloroforme, le tétrahydrofurane ou le dioxane à une température variant de 0 à 40°C.
- Une réaction d'alkylation est aussi possible entre le composé (LXII) et un groupe alkyle (LIII) substitué ou non dans lequels Lg est par exemple un atome de chlore, de brome ou d'iode, un groupe tosylate ou triflate, en présence d'une base telle que la triéthylamine, la diisopropyl éthylamine, dans un solvant tel que le tétrahydrofurane ou le dioxane à une température variant de 0 à 80°C.
- Une réaction d'amination réductrice peut aussi être réalisé entre le composé (LXII) et un dérivé (LIV) de type aldéhyde ou cétone, en utilisant un réducteur tel que le borohydrure de sodium, le triacétoxyborohydrure de sodium ou le cyanoborohydrure de sodium, en présence ou non d'un acide de Bronsted (tel que l'acide chlorhydrique) ou de Lewis (tel que le tétraisopropoxyde de titane) dans un solvant tel que le dichloroéthane, le dichlorométhane, l'acide acétique ou le méthanol, à des températures comprises entre -10°C et 30°C.

Le schéma 23 détaille une synthèse des composés de formule (V) dans lesquels R₃ est un hydrogène et R₄ un groupe benzyl carbamate ; ces composés seront appelés ci-après composés de formule (LXIX).

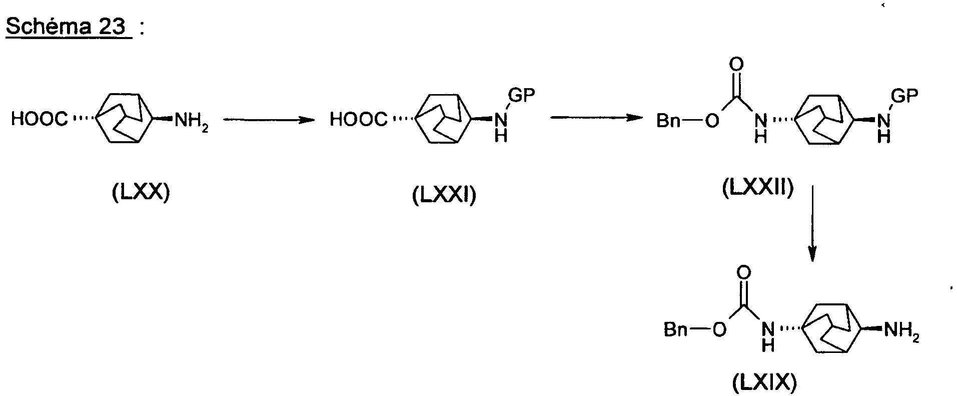

Dans le schéma 23, les dérivés (LXXI) sont obtenus par protection de la fonction amine du dérivé (LXX), par exemple par un groupe Boc au moyen des méthodes connus par l'Homme de l'art. Dans une deuxième étape, la fonction acide des dérivés (LXXI) subit une réaction de Curtius tel que par exemple décrit avec des dérivés d'adamantane dans Maison et coll. J.Org.Chem. 2008 pp1056 ou dans Broadhurst et coll. J.Med.Chem. 2004 pp 4975. Les amines (LXIX) sont obtenues par déprotection de la fonction amine des composés de formule (LXXII), par des méthodes choisies parmi celles connues de l'Homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Le schéma 24 détaille une synthèse des composés de formule (I) dans lesquels R₃ est un hydrogène et R₄ un groupe -NHCOR₆, ces composés sont appelés ci-après composés de formule (LXXIII).

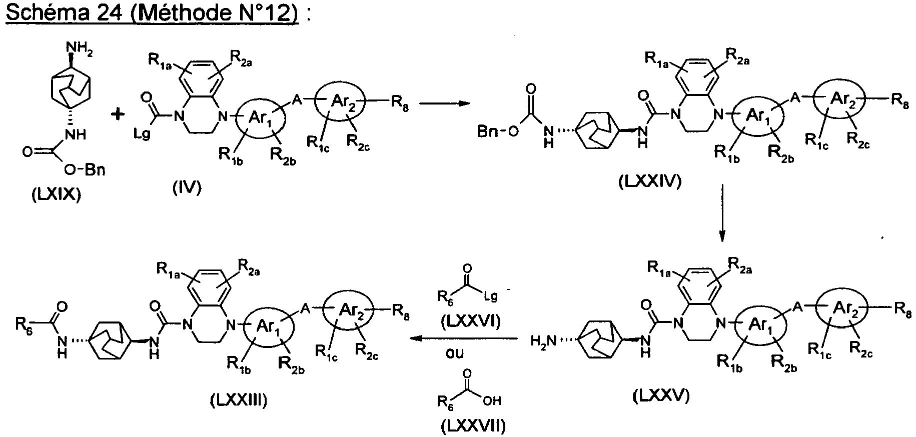

Dans le schéma 24, les dérivés (LXXIV) sont obtenus par couplage entre les dérivés activés (IV) et l'amine (LXIX) en présence ou non d'une base comme la triéthylamine ou le carbonate de potassium, dans un solvant tel que le tétrahydrofurane, le dichlorométhane, l'acétonitrile, le diméthylformamide ou l'eau, à une température variant de la température ambiante à 100°C. Les amines (LXXV) sont ensuites obtenues par déprotection de la fonction carboxybenzyle. Les méthodes de déprotection possibles comprennent entre autres l'utilisation d'hydrogène en présence d'un catalyseur dérivé du palladium pour réaliser une réaction d'hydrogénolyse, dans un solvant ou mélange de solvants tel que le méthanol, l'ethanol, l'acétate d'éthyle, le tétrahydrofurane, sous une pression d'hydrogène comprise entre 1 et 10 bar à une température variant de la température ambiante à 80°C. Une méthode alternative pour réaliser une hydrogénolyse de groupement Cbz est d'utiliser une catalyse au palladium (par exemple avec du palladium adsorbé sur du charbon) en présence de formiate d'ammonium au reflux d'un solvant tel que le méthanol. Dans la dernière étape, la formation de la liaison amide des composés (LXXIII) peut être obtenue soit par la réaction des amines (LXXV) avec un dérivé d'acide de formule (LXXVI) présentant un groupe partant Lg (par exemple un atome de chlore, un groupe trichlorométhoxy, un groupe para-nitrophényle, un groupe imidazole, ou méthyl-imidazolium) en présence d'une base comme la triéthylamine ou la diisopropylamine dans un solvant tel que le dichlométhane, le tétrahydrofurane à une température variant de la température ambiante à 80°C ; soit par condensation entre les acides de formule (LXXVII) et les amines (LXXV) dans des conditions de couplage peptidique classiques, en utilisant par exemple comme agent couplant le dicyclocarbodiimide, le chlorhydrate du 1-(3-diméthylaminopropyl)-3-éthylcarbodiimide ou le bromo-tris-pyrrolidino-phosphonium hexafluorophosphate, en présence ou non d'hydroxybenzo-triazole, et en utilisant comme base organique la triéthylamine ou la diisopropyl-éthylamine dans un solvant ou mélange de solvant tel que le dioxane, le dichlorométhane ou l'acétonitrile.

Le schéma 25 détaille une synthèse des composés de formule (I) dans lesquels R_{1c} représente un groupe Alkyl-CO₂H ; ces composés seront appelés ci-après composés de formule (LXXVIII).

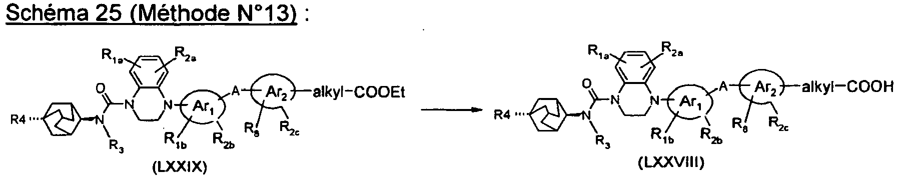

Dans le schéma 25, les dérivés (LXXVIII) sont obtenus par hydrolyse de la fonction ester (par exemple un ester ethylique) du composé (LXXIX) au moyen d'une base minérale telle que la soude, la potasse ou la lithine dans un solvant tel qu'un alcool ou de l'eau à une température variant de la température ambiante à 100°C.

Le schéma 26 détaille une synthèse des composés de formule (I) dans lesquels R_{1c} représente un groupe -Alkyl-CH₂OH ; ces composés seront appelés ci-après composés de formule (LXXX).

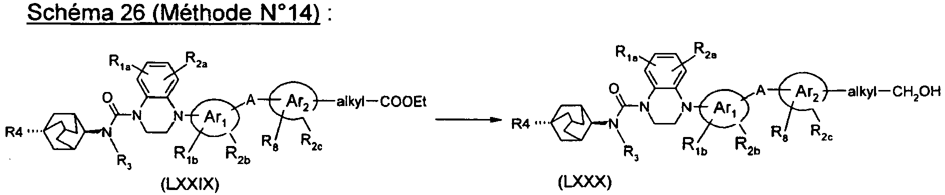

Dans le schéma 26, les dérivés (LXXX) sont obtenus par réduction de la fonction ester du composé (LXXIX) en fonction alcool au moyen d'un agent de réduction tel qu'un hydrure par exemple LiAlH₄, LiBH₄, DIBAL dans un solvant ou mélange de solvants tel que le tetrahydrofurane, l'éther ou le toluène, à une température variant de -78°C à 40°C.

Le schéma 27 détaille une synthèse des composés de formule (I) dans lesquels R₃ est un hydrogène et R₄ un groupe -CN, ces composés sont appelés ci-après composés de formule (LXXXI).

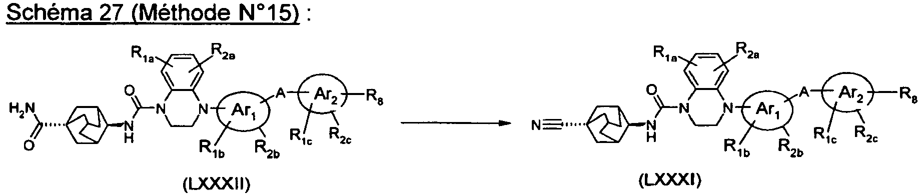

Dans le schéma 27, les dérivés (LXXXI) sont obtenus par déshydratation de la fonction amide primaire du composé (LXXXII) en fonction nitrile au moyen d'un agent de dehydratation tel que l'anhydride trifluoracétique, l'anhydride trifluorométhanesulfonique, SOCl₂, POCl₃, P₂O₅ dans un solvant ou mélange de solvants tel que le dichlorométhane, le dioxanne, le dichloroéthane, le diméthylformamide, en présence ou non d'une base tel que la pyridine ou la triéthylamine à une température variant de -0°C à 70°C.

Le schéma 28 détaille une synthèse des composés de formule (VIII) dans lesquels A est une liaison, Ar₁ est un groupe pyridine et Ar₂ est un groupe pipéridinyle, ; ces composés seront appelés ci-après composés de formule (LXXXIII).

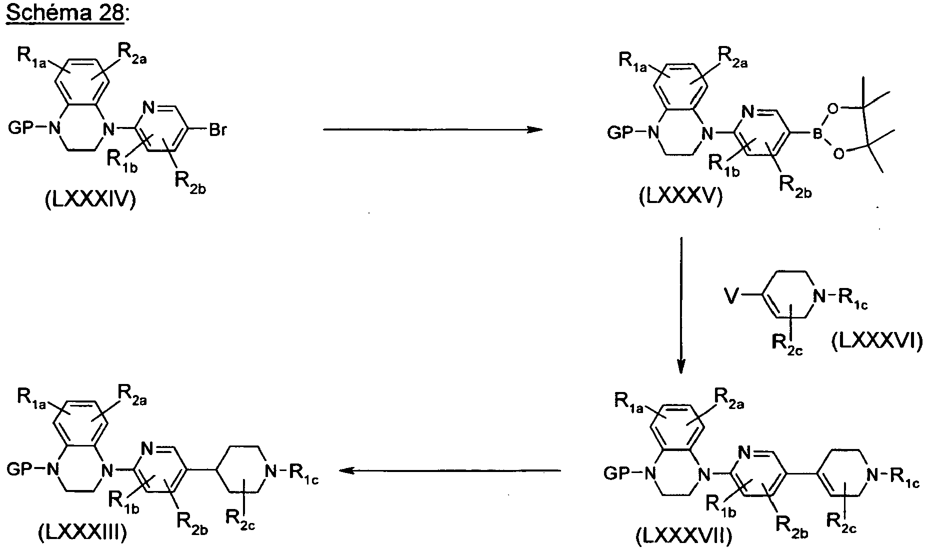

Dans le schéma 27, les composés de formule (LXXXV) peuvent être préparés par transformation de la fonction bromo des composé (LXXXIV) en un dérivé d'ester boronique (LXXXV) par une réaction avec le bispinacolatodiborane en présence d'un complexe du palladium tel que le 1,1'-bis(diphénylphosphino)ferrocedichloropalladium (II) en présence d'une base comme l'acétate de potassium et de chlorure de lithium dans un solvant ou mélange de solvants tel que le dichlorométhane, le dioxane ou le diméthylsulfoxide, à une température variant de la température ambiante à 100°C. Dans une deuxieme étape, les dérivés (LXXXVII) peuvent être obtenus par une réaction de couplage entre le dérivé (LXXXV) et un composé (LXXXVI) présentant un groupe partant V (par exemple un halogène, un triflate, un nonaflate) en présence d'un catalyseur organométallique tel qu'un dérivé du palladium, en présence ou non d'une phosphine telle que la tricyclohexylphosphine ou la triphénylphosphine, en présence d'une base comme le carbonate de sodium ou potassium ou le fluorure de potassium, dans un solvant ou mélange de solvants tel que le dioxane, le diméthylformamide, l'éthylène glycol diméthyléther, le tétrahydrofurane ou l'eau, à une température variant de la température ambiante à 100°C. Dans la dernière étape, la double liaison du composé (LXXXVII) subir une réaction d'hydrogénation en présence d'un catalyseur organométallique tel qu'un dérivé du palladium (par exemple du Pd 10% déposé sur charbon, ou du Pd(OAc)2) dans un solvant ou mélange de solvants tel que le méthanol, l'éthanol ou de l'acétate d'éthyle, à une température variant de la température ambiante à 80°C.

Le schéma 29 détaille une synthèse des composés de formule (VIII) dans lesquels Ar₂ est un groupe piperazine, A est une liaison simple connectée directement sur un des deux azotes de la pipérazine, R1c est un groupe alkyl sulfone connecté sur l'autre atome d'azote de la pipérazine; ces composés seront appelés ci-après composés de formule (LXXXVIII).

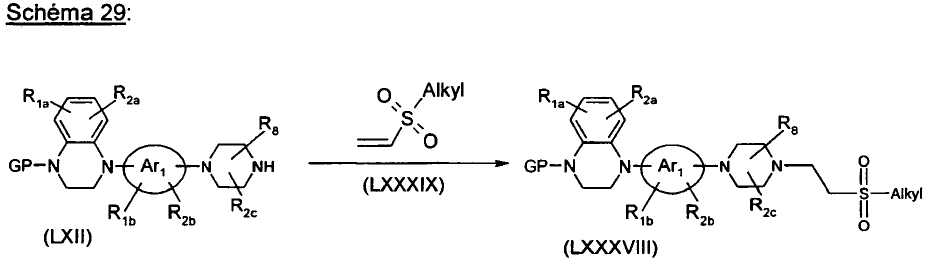

Dans le schéma 29, les dérivés (LXXXVIII) sont obtenus par une réaction de type Michaël entre le composé (LXII) et l'accepteur de Michaël (LXXXIX) en présence ou absence d'une base tel que la triéthylamine dans un solvant tel qu'un alcool à une température variant de 0°C à la température ambiante pour conduire aux dérivés (LXXXVIII).

Dans les schémas qui précèdent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

L'invention, selon un autre de ses aspects, a également pour objet les composés de formules (II), (IV), (XXVII), (XXXIX), (XXXVIII), (XLI), (XLII), (LI), (LII), (LVI), (LVII), (LX), (LXIII), (LXVII), (LXXIV), (LXXV) définies ci-avant. Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les abréviations et formules brutes suivantes sont utilisées :
- °C: Degré Celsius
- DME: diméthoxyéthane
- DMF: diméthylformamide
- DMSO: diméthyl sulfoxide
- h: heure(s)
- H₂: dihydrogène
- H₂O: eau
- HCl: acide chlorhydrique
- K₂CO₃: Carbonate de potassium
- LC/MS: chromatographie liquide/ spectrométrie de masse
- ml ou mL: millilitre(s)
- mmol: millimole(s)
- MHz: MegaHertz
- MgSO₄: Sulfate de magnésium
- N: normal
- NMP: N-méthylmorpholine
- NaHCO₃: Hydrogénocarbonate de sodium
- Pd/C: Palladium sur charbon
- P₂O₅: phosphorus pentoxide
- ppm: parties par millions
- psi: pound per square inch
- SO₂: dioxyde de soufre

### Exemple 1 : 4-[4-(pyrimidin-2-ylmethoxy)-phenyl]-3,4-dihydro-2H-quinoxaline-1-carboxylic acid adamantan-2-ylamide (composé n° 3)

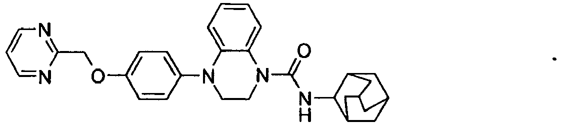

### 1.1: Ester tert-butylique de l'acide 4-(4-hydroxy-phenyl)-3,4-dihydro-2H-quinoxaline-1-carboxylique

On place 0,3 g de 3,4-dihydro-2H-quinoxaline-1-carboxylic acid tert-butyl ester, 0,183 g de 4-bromophénol, 0,0079 g de complexe de 2'-(dimethylamino)-2-biphenylyl-palladium(II) chloride dinorbornylphosphine, 0,363 g de triphosphate de potassium dans 3,4 ml d'éthylèneglycol diméthyl éther sous atmosphère inerte. On ajoute 4,16 ml de lithium bis(trimethylsilyl)amide et 2,17 ml d'éthylène glycol diméthyléther. Le mélange réactionnel est agité à 80°C pendant 4h. Après refroidissement, le milieu réactionnel est repris au dichlorométhane. Une solution aqueuse 1 N d'acide chlorhydrique est ajoutée jusqu'à pH 1 puis le pH est ramené à 8 avec une solution aqueuse d'hydrogénocarbonate de sodium saturée et le mélange est extrait au dichlorométhane. Les phases organiques sont rassemblées, lavées à l'eau et avec une solution aqueuse saturée en chlorure de sodium, séchées sur sulfate de magnésium. Après concentration à sec, le brut obtenu est chromatographié sur gel de silice en éluant avec un gradient de méthanol dans le dichlorométhane variant de 0% à 2,5%. On obtient 0,34 g de l'ester *tert*-butylique de l'acide 4-(4-hydroxy-phenyl)-3,4-dihydro-2H-quinoxaline-1-carboxylique.
M+H⁺ = 327

### 1.2: 1-[4-(pyrimidin-2-ylmethoxy)-phenyl]-1,2,3,4-tetrahydro-quinoxaline

On place 0,3 g de l'ester *tert*-butylique de l'acide 4-(4-hydroxy-phenyl)-3,4-dihydro-2H-quinoxaline-1-carboxylique dans 4,6 ml de diméthylformamide sous azote. On ajoute 0,167 g de 2(chloromethyl)-pyrimidine et 0,508 g de carbonate de potassium. Le milieu réactionnel est chauffé à 100°C pendant 4 h. On ajoute 0,254 g de carbonate de potassium et le chauffage est maintenu 1h30. Après hydrolyse, le milieu réactionnel est extrait à l'acétate d'éthyle jusqu'à épuisement de la phase aqueuse. Les phases organiques sont rassemblées, lavées avec une solution aqueuse saturée de chlorure de sodium, séchées sur sulfate de magnésium et concentrées à sec. Le brut obtenu est chromatographié sur gel de silice en éluant avec un gradient de méthanol dans le dichlorométhane variant de 0% à 9/1. On obtient 0,095 g de 1-[4-(pyrimidin-2-ylmethoxy)-phenyl]-1,2,3,4-tetrahydro-quinoxaline.
M+H⁺ = 319

### 1.3: 4-[4-(pyrimidin-2-ylmethoxy)-phenyl]-3,4-dihydro-2H-quinoxaline-1-carboxylic acid adamantan-2-ylamide

On place 0,095 g de 1-[4-(pyrimidin-2-ylmethoxy)-phenyl]-1,2,3,4-tetrahydro-quinoxaline dans 3 ml de dichlorométhane à 0°C sous azote. On ajoute 0,09 ml de triéthylamine et 0,031 g de triphosgène. On agite 2h30 à température ambiante, puis on ajoute 0,01 g de triphosgène et l'agitation est maintenue 2h. On ajoute alors 0,12 ml de triéthylamine et 0,062 g de chlorhydrate d'adamantan-2-ylamine. L'agitation est maintenue 1 h à température ambiante. Après hydrolyse, le milieu réactionnel est extrait à l'acétate d'éthyle jusqu'à épuisement de la phase aqueuse. Les phases organiques sont rassemblées, lavées avec une solution aqueuse saturée de chlorure de sodium, séchées sur sulfate de magnésium et concentrées à sec. Le brut obtenu est chromatographié sur gel de silice en éluant avec un gradient de méthanol dans le dichlorométhane variant de 0% à 9/1. On obtient 0,03 g de 4-[4-(pyrimidin-2-ylmethoxy)-phenyl]-3,4-dihydro-2H-quinoxaline-1-carboxylic acid adamantan-2-ylamide, qui est trituré dans un mélange d'acétate d'éthyle et de diisopropyléther.
Point de fusion = 123-125°C ; M+H⁺ = 496 ; RMN ¹H (DMSO, 400 MHz), δ(ppm) : 1,91 (dl, J=12Hz, 2H) ; 1,66-1,97 (m, 11H) ; 2,09 (dl, J=12Hz, 2H) ; 3,69 (m, 4H) ; 4,72 (s, 2H) ; 6,4 (m, 1H) ; 6,64-6,53 (m, 3H) ; 7,07 (m, J=9Hz, 2H) ; 7,15 (m, J=9Hz, 2H) ; 7,39 (t, J=4.9Hz, 1H) ; 7,76 (d, J=7Hz,1H); 8,79 (d, J=4.8Hz, 2H).

### Exemple 2 : Trans-4-[4-(4-methanesulfonyl-piperazin-1-yl)-phenyl]-3,4-dihydro-2H-quinoxaline-1-carboxylic acid (5-hydroxy-adamantan-2-yl)-amide (composé n°9)

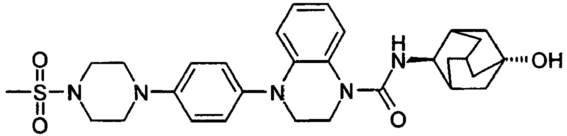

### 2.1: 1-(4-bromo-phenyl)-4-methanesulfonyl-piperazine

A une solution 20 g de 1-(4-bromo-phenyl)-piperazine et de 17,34 ml de triéthylamine dans 394 ml de dichlorométhane sont ajoutés 7,7 ml de chlorure de méthane sulfonyle. Le mileu réactionnel est mis sous agitation pendant 4h à température ambiante. De l'eau est ensuite ajoutée et la phase organique est lavée deux fois à l'eau, séchée sur sulfate de magnésium, puis le solvant est évaporé sous pression réduite pour conduire à 26,2 g de 1-(4-bromo-phenyl)-4-methanesulfonyl-piperazine. Le produit est utilisé ensuite tel quel sans autre purification.
M+H⁺ = 321, 4

### 2.2: Ester tert-butylique de l'acide 4-[4-(4-methanesulfonyl-piperazin-1-yl)-phenyl]-3,4-dihydro-2H-quinoxaline-1-carboxylique

A une solution 19,23 g 3,4-dihydro-2*H*-quinoxaline-1-tert-butyl carbamate et de 26,2 g de 1-(4-bromo-phenyl)-4-methanesulfonyl-piperazine dans 410 ml de xylène sont ajoutés 11,8 g de *tert*-butylate de sodium, 0,737 g de diacétate de palladium et 0,664 g de tri-tert-butylphosphine. Le milieu réactionnel est chauffé à 150°C pendant 4h, puis de l'eau et de l'acétate d'éthyle sont ensuite ajoutés. La phase organique est lavée deux fois à l'eau, séchée sur sulfate de magnésium, puis le solvant est évaporé sous pression réduite. Le résidu est chromatographié sur gel de silice par un gradient de mélange d'heptane/acétate d'éthyle 8/2 à 0/1. On obtient 31,5 g d'ester *tert*-butylique de l'acide 4-[4-(4-methanesulfonyl-piperazin-1-yl)-phenyl]-3,4-dihydro-2H-quinoxaline-1-carboxylique.
M+H⁺ = 473,0

### 2.3: 1-[4-(4-methanesulfonyl-piperazin-1-yl)-phenyl]-1,2,3,4-tetrahydro-quinoxaline

On place 31,5 g de l'ester tert-butylique de l'acide 4-[4-(4-methanesulfonyl-piperazin-1-yt)-phenyl]-3,4-dihydro-2H-quinoxaline-1-carboxylique dans 400 ml de dichlorométhane, puis 333 ml d'acide chlorhydrique 4N dans le dioxane sont ajoutés. Après 16h d'agitation sous atmosphère inerte, les solvants sont évaporés sous pression réduite. De l'eau, une solution aqueuse saturée en hydrogénocarbonate de sodium et du dichlorométhane sont ajoutés. La phase aqueuse est extraite deux fois au dichlorométhane. Les phases organiques sont rassemblées, lavées à l'eau, séchées sur sulfate de magnésium, puis le solvant est évaporé sous pression réduite. On obtient 23,2 g de 1-[4-(4-methanesulfonyl-piperazin-1-yl)-phenyl]-1,2,3,4-tetrahydro-quinoxaline. Le produit est utilisé ensuite tel quel sans autre purification.
M+H⁺ = 373,5

### 2.4 : Trans-4-[4-(4-methanesulfonyl-piperazin-1-yl)-phenyl]-3,4-dihydro-2H-quinoxaline-1-carboxylic acid (5-hydroxy-adamantan-2-yl)-amide

On place 0,6 g de 1-[4-(4-methanesulfonyl-piperazin-1-yl)-phenyl]-1,2,3,4-tetrahydro-quinoxaline dans 5 ml de dichlorométhane à 0°C sous azote. On ajoute 0,45 ml de triéthylamine et 0,192 g de triphosgène. On agite 3 h à température ambiante, puis on ajoute 15 ml de diméthylformamide, 0,45 ml de triéthylamine et 0,427 g de chlorhydrate de 4-amino-adamantan-1-ol et l'agitation est maintenue 18h. Après hydrolyse, le milieu réactionnel est extrait à l'acétate d'éthyle jusqu'à épuisement de la phase aqueuse. Les phases organiques sont rassemblées, lavées avec une solution aqueuse saturée de chlorure de sodium, séchées sur sulfate de magnésium et concentrées à sec. Le brut obtenu est chromatographié sur gel de silice en éluant avec un gradient de méthanol et d'acétate d'éthyle dans le dichlorométhane variant de 0/0/1 à 2,5/0,5/7. On obtient 0,24 g de trans-4-[4-(4-methanesulfonyl-piperazin-1-yl)-phenyl]-3,4-dihydro-2H-quinoxaline-1-carboxylic acid (5-hydroxy-adamantan-2-yl)-amide, qui est trituré dans un mélange d'acétate d'éthyle et de diisopropyléther.
Point de fusion = 160°C ; M+H⁺ = 566 ; RMN ¹H (DMSO, 400MHz), δ(ppm) : 1,38 (dl, J=12Hz, 2H) ; 1,58-1,737 (m, 9H) ; 2,02 (m, 2H) ; 2,94 (s, 3H) ; 3,27 (m, 8H) ; 3,53 (m, 2H), 3,71 (m, 1 H), 3,78 (m, 1H); 4,4 (s, 1H); 5,95 (d, J=6,1 Hz); 6,52 (dd, J=8,2 et 1,3Hz, 1H) ; 6,67 (m, J=7,5 et 1,4Hz, 1H) ; 6,84 (m, J=7,8 et 1,5 Hz, 1H) ; 7,05 (m, J=9 Hz, 2H) ; 7,16 (m, J=9Hz, 1 H) ; 7,28 (d, J=7,9 et 1,5 Hz,1H).

### Exemple 3 : Trans-4-[4-(4-Ethanesulfonyl-piperazin-1-yl)-phenyl]-3,4-dihydro-2H-quinoxaline-1-carboxylic acid (5-hydroxy-adamantan-2-yl)-amide (composé n°34)

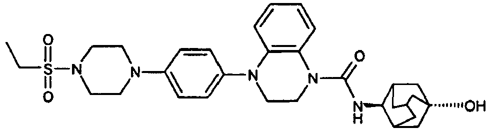

### 3.1 : 4-(4-Bromo-phenyl)-piperazine-1-carboxylic acid benzyl ester

On place 5 g de 4-bromophénylpipérazine dans un tricol de 500 ml, sous atmosphère azotée. On ajoute 4,34 ml de triéthylamine. On refroidit au bain de glace et on ajoute 3,24 ml de chloroformiate de benzyle. On laisse le mélange revenir à température ambiante. On dilue au dichlorométhane puis on hydrolyse doucement. On extrait la phase aqueuse au dichlorométhane. Les phases organiques sont réunies, lavées avec une solution de NaCl saturée, séchées sur sulfate de sodium, puis filtrées sur fritté et concentrées sous vide. On obtient 8,43 g de 4-(4-Bromo-phenyl)-piperazine-1-carboxylic acid benzyl ester.
M+H⁺ =376

### 3.2 : 4-[4-(4-Benzyloxycarbonyl-piperazin-1-yl)-phenyl]-3,4-dihydro-2H-quinoxaline-1-carboxylic acid tert-butyl ester

On place 8,43 g de 4-(4-bromo-phenyl)-piperazine-1-carboxylic acid benzyl ester dans un tricol de 500 ml, sous atmosphère azotée. On ajoute 170 ml d'o-xylène anhydre, 5,01 g de 3,4-dihydro-2H-quinoxaline-1-carboxylic acid tert-butyl ester, puis 3,08 g de tert-butylate de sodium, suivis de 0,48 g d'acétate de palladium puis on termine par 0,53 ml de tri-tert-butylphosphine. On chauffe le mélange réactionnel pendant 18 h à 150°C. Le chauffage est arrêté et on revient à température ambiante. On évapore l'oxylène et on reprend le mélange à l'acétate d'éthyle avant de le filtrer sur célite. On lave la phase organique à l'eau puis avec une aqueuse saturée en chlorure de sodium. On extrait la phase aqueuse à l'acétate d'éthyle. Les phases organiques sont réunies puis séchées sur sulfate de sodium, filtrées sur fritté et concentrées sous vide. On obtient 8,9 g de 4-[4-(4-Benzyloxycarbonyl-piperazin-1-yl)-phenyl]-3,4-dihydro-2H-quinoxaline-1-carboxylic acid tert-butyl ester après purification sur colonne de silice en éluant avec un gradient de solvant heptane/acétate d'éthyle variant de 95/5 à 60/40).
M+H⁺=529

### 3.3: 4-[4-(3,4-Dihydro-2H-quinoxalin-1-yl)-phenyl]-piperazine-1-carboxylic acid benzyl ester

On place 8,9 g de 4-[4-(4-Benzyloxycarbonyl-piperazin-1-yl)-phenyl]-3,4-dihydro-2H-quinoxaline-1-carboxylic acid tert-butyl ester dans 220 ml de dichlorométhane. On ajoute lentement à 0°C, 55 ml d'une solution d'acide chlorhydrique 4N dans le dioxane. On agite pendant 5 minutes à froid puis on laisse le mélange remonter à température ambiante. On agite 18 h à température ambiante puis on dilue le mélange réactionnel par du dichlorométhane. On ajoute une solution aqueuse saturée en carbonate de sodium puis on poursuit avec du carbonate de sodium solide jusqu'à pH=7-8. On extrait la phase aqueuse au dichlorométhane. Les phases organiques sont réunies, lavées à l'eau, séchées sur sulfate de sodium, filtrées sur fritté et concentrées sous vide. On obtient 7,2 g de 4-[4-(3,4-Dihydro-2H-quinoxalin-1-yl)-phenyl]-piperazine-1-carboxylic acid benzyl ester sous forme d'une huile.
M+H⁺=429

### 3.4 : Trans-4-{4-[4-(5-Hydroxy-adamantan-2-ylcarbamoyl)-3,4-dihydro-2H-quinoxalin-1-yl]-phenyl}-piperazine-1-carboxylic acid benzyl ester

Dans un tricol de 250 ml sous atmosphère inerte d'azote, on introduit 4 g de 4-[4-(3,4-Dihydro-2H-quinoxalin-1-yl)-phenyl]-piperazine-1-carboxylic acid benzyl ester dans 93 ml de dichlorométhane anhydre. On ajoute à 0°C, 5,2 ml de triéthylamine. On additionne ensuite 1,11 g de triphosgène. On agite 1 h à température ambiante, et on ajoute ensuite 1,56 g de trans-4-Amino-adamantan-1-ol et 15 ml de diméthylformamide anhydre. On agite 18 h à température ambiante. On évapore les solvants et on reprend à l'eau. On dilue au dichlorométhane, on lave à l'eau puis avec une solution saturée d'hydrogenocarbonate de sodium. La phase aqueuse est extraite au dichlorométhane. Les phases organiques sont réunies, lavées à l'eau, séchées sur sulfate de sodium, filtrées sur fritté et évaporées à sec. On obtient 2,34 g de trans-4-{4-[4-(5-Hydroxy-adamantan-2-ylcarbamoyl)-3,4-dihydro-2H-quinoxalin-1-yl]-phenyl}-piperazine-1-carboxylic acid benzyl ester.
M+H⁺=622

### 3.5 : Trans-4-(4-piperazin-1-yl-phenyl)-3,4-dihydro-2H-quinoxaline-1-carboxylic acid (5-hydroxy-adamantan-2-yl)-amide

On place 4,1 g de de trans-4-{4-[4-(5-hydroxy-adamantan-2-ylcarbamoyl)-3,4-dihydro-2H-quinoxalin-1-yl]-phenyl}-piperazine-1-carboxylic acid benzyl ester dans un tricol de 250 ml sous atmosphère inerte. On ajoute 66 ml de méthanol, puis 1,66 g de formiate d'ammonium suivis de 1,40 g de Pd/C 10% (50% dans l'eau). Le mélange est porté au reflux du méthanol pendant une heure puis ramené à température ambiante. On filtre sous atmosphère inerte sur filtre Whateman puis on rince de nombreuses fois au méthanol. Le méthanol est évaporé sous pression réduite. On reprend au dichlorométhane et on lave la phase organique avec un minimum d'eau. La phase organiques est séchée sur sulfate de sodium, filtrée sur fritté et évaporée sous pression réduite. On obtient 3,15 g de trans-4-(4-Piperazin-1-yl-phenyl)-3,4-dihydro-2H-quinoxaline-1-carboxylic acid (5-hydroxy-adamantan-2-yl)-amide sous forme d'un solide blanc.
M+H⁺ =488

### 3.6: Trans-4-[4-(4-Ethanesulfonyl-piperazin-1-yl)-phenyl]-3,4-dihydro-2H-quinoxaline-1-carboxylic acid (5-hydroxy-adamantan-2-yl)-amide

On place 0,2 g de trans-4-(4-Piperazin-1-yl-phenyl)-3,4-dihydro-2H-quinoxaline-1-carboxylic acid (5-hydroxy-adamantan-2-yl)-amide dans un ballon de 25 ml. On ajoute 4 ml de dichlorométhane puis 0,09 ml de triéthylamine et enfin 0,04 ml de chloroéthylsulfonyle. Le mélange réactionnel est agité 18 h à température ambiante puis dilué au dichlorométhane, lavé avec une solution aqueuse d'HCl 1 N. On extrait la phase aqueuse au dichlorométhane puis on regroupe les phases organiques. Les phases organiques sont lavées à l'eau, séchées sur sulfate de sodium, filtrées sur fritté, évaporées sous pression réduite. On obtient 0,21 g de trans-4-[4-(4-Ethanesulfonyl-piperazin-1-yl)-phenyl]-3,4-dihydro-2H-quinoxaline-1-carboxylic acid (5-hydroxy-adamantan-2-yl)-amide sous forme d'un solide blanc, après purification sur colonne de silice en éluant avec un gradient heptane/acétate d'éthyle variant de 95/5 à 40/60 puis cristallisation dans l'éther éthylique.
Point de fusion = 138-160°C ; M+H⁺ = 579 ; RMN ¹H (400 MHz, DMSO-d6) δ ppm 7,28 (dd, J = 7,8 and 1,5 Hz, 1H) ; 7,16 (m, J = 9 Hz, 2H) ; 7,04 (m, J = 9 Hz, 2H) ; 6,84 (m, J = 7,8 and 1,5 Hz, 1H) ; 6,67 (m, J = 7,8 and 1,4 Hz, 1H ) ; 6,52 (dd, J = 8,2 and 1,3 Hz, 1H) ; 5,94 (d, J = 6,1 Hz, 1 H) ; 4,4 (s, 1 H) ; 3,79 (m, 2H) ; 3,71 (m, 1H) ; 3,53 (m, 2H) ; 3,36-3,27(m, 4H) ; 3,24 (m, 4H) ; 3,13 (q, J = 7,9 Hz, 2H) ; 2,08-1,96 (m, 3H); 1,74-1,57 (m, 8H); 1,38 (m, 2H); 1,26 (t, J = 7,9 Hz, 3H).

### Exemple 4 : Trans- 4-{4-[4-(2-Methoxy-ethanesulfonyl)-piperazin-1-yl]-phenyl}-3,4-dihydro-2H-quinoxaline-1-carboxylic acid (5-hydroxy-adamantan-2-yl)-amide (composé n°63)

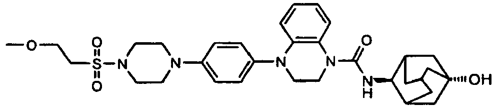

### 4.1 : 4-(4-Piperazin-1-yl-phenyl)-3,4-dihydro-2H-quinoxaline-1-carboxylic acid tert-butyl ester

On place 3,5 g de 4-[4-(4-Benzyloxycarbonyl-piperazin-1-yl)-phenyl]-3,4-dihydro-2H-quinoxaline-1-carboxylic acid tert-butyl ester dans un tricol de 250 ml sous atmosphère inerte. On ajoute 66 ml de MeOH puis 1,67 g de formiate d'ammonium suivis de 1,41 g de Pd/C 10% (50% dans l'eau). Le mélange est porté au reflux du méthanol pendant une heure trente puis ramené à température ambiante. On le filtre sous atmosphère inerte sur filtre Whateman puis on le rince de nombreuses fois au méthanol. On évapore le méthanol sous pression réduite. Le mélange est repris au dichlorométhane et la phase organique est lavée avec un minimum d'eau. La phase organique est séchée sur sulfate de sodium, filtrée sur fritté et évaporée sous pression réduite. On obtient 2,5 g de 4-(4-Piperazin-1-yl-phenyl)-3,4-dihydro-2H-quinoxaline-1-carboxylic acid tert-butyl ester sous forme d'un solide blanc.
M+H⁺=395

### 4.2 : 4-[4-(4-Ethenesulfonyi-piperazin-1-yl)-phenyl]-3,4-dihydro-2H-quinoxaline-1-carboxylic acid tert-butyl ester

On place 2 g 4-(4-Piperazin-1-yl-phenyl)-3,4-dihydro-2H-quinoxaline-1-carboxylic acid tert-butyl ester dans un ballon de 250 ml, sous atmosphère inerte. On ajoute 72 ml de dichlorométhane anhydre puis on additionne à 0°C, 1,22 ml de chloroéthylchlorosulfonyle, puis 3,67 ml de triéthylamine. On laisse le mélange réactionnel remonter doucement à température ambiante. Le mélange réactionnel est agité 3 h à température ambiante avant d'être dilué au dichlorométhane. La phase organique est lavée avec une solution aqueuse saturée d'hydrogénocarbonate de sodium et la phase aqueuse est extraite au dichlorométhane. Les phases organiques sont réunies et lavées à l'eau puis séchées sur sulfate de sodium, filtrées sur fritté et concentrées sous pression réduite. On obtient 1,01 g de 4-[4-(4-ethenesulfonyl-piperazin-1-yl)-phenyl]-3,4-dihydro-2H-quinoxaline-1-carboxylic acid tert-butyl ester sous forme d'un solide blanc, après purification sur colonne de silice en éluant avec un gradient heptane/acétate d'éthyle variant de 95/5 à 40/60.
M+H⁺=485

### 4.3: 4-{4-[4-(2-Methoxy-ethanesulfonyl)-piperazin-1-yl]-phenyl}-3,4-dihydro-2H-quinoxaline-1-carboxylic acid tert-butyl ester

On place 0,3 g de 4-[4-(4-ethenesulfonyl-piperazin-1-yl)-phenyl]-3,4-dihydro-2H-quinoxaline-1-carboxylic acid tert-butyl ester dans un tricol de 100 ml, sous atmosphère inerte. On ajoute lentement 23,5 ml de méthanolate de sodium 0,5N dans le méthanol. On agite 1 h au reflux (65°C) puis on stoppe le chauffage. Le méthanol est évaporé et le mélange est repris au dichlorométhane. La phase organique est lavée avec une solution aqueuse saturée de chlorure d'amonium. La phase aqueuse est extraite au dichlorométhane. Les phases organiques réunies sont lavées à l'eau, séchées sur sulfate de sodium, filtrées sur fritté et concentrées sous vide. On obtient 0,3 g de 4-{4-[4-(2-Methoxy-ethanesulfonyl)-piperazin-1-yl]-phenyl}-3,4-dihydro-2H-quinoxaline-1-carboxylic acid tert-butyl ester sous forme d'un solide blanc.
M+H⁺=517

### 4.4 : 1-{4-[4-(2-Methoxy-ethanesulfonyl)-piperazin-1-yl]-phenyl}-1,2,3,4-tetrahydro-quinoxaline

On place 0,35 g 4-{4-[4-(2-methoxy-ethanesulfonyl)-piperazin-1-yl]-phenyl}-3,4-dihydro-2H-quinoxaline-1-carboxylic acid tert-butyl ester dans 7 ml de dichlorométhane. On ajoute à 0°C, lentement 2,5 ml d'une solution d'HCl 4N dans le dioxane. On agite pendant 5 minutes à froid puis on laisse le mélange remonter à température ambiante. Le mélange réactionnel est agité 18 h à température ambiante puis est dilué par du dichlorométhane. On ajoute une solution aqueuse saturée d'hydrogénocarbonate de sodium jusqu'à pH=7-8. On extrait la phase aqueuse au dichlorométhane. Les phases organiques sont réunies, lavées à l'eau, séchées sur sulfate de sodium, filtrées sur fritté, et concentrées sous pression réduite. On obtient 0,246 g de 1-{4-[4-(2-Methoxy-ethanesulfonyl)-piperazin-1-yl]-phenyl}-1,2,3,4-tetrahydro-quinoxaline sous forme d'une huile.
M+H⁺=417

### 4.5 : Trans- 4-{4-[4-(2-Methoxy-ethanesulfonyl)-piperazin-1-yl]-phenyl}-3,4-dihydro-2H-quinoxaline-1-carboxylic acid (5-hydroxy-adamantan-2-yl)-amide

On place 0,246 g de 1-{4-[4-(2-Methoxy-ethanesulfonyl)-piperazin-1-yl]-phenyl}-1,2,3,4-tetrahydro-quinoxaline dans 6 ml de dichlorométhane anhydre, dans un tricol de 50 ml sous atmosphère inerte. On ajoute à 0°C, 0,33 ml (2,36 mmol, 4 éq) de triéthylamine puis on additionne 0,07 g (0,24 mmol, 0,4 éq) de triphosgène. On agite 1 h à température ambiante puis on ajoute 0,4 g de trans-4-amino-adamantan-1-ol et 15 ml de diméthylformamide anhydre. On agite une nuit à température ambiante puis on évapore le mélange de solvants sous pression réduite et on reprend à l'eau. On dilue au dichlorométhane. La phase organique est lavé à l'eau puis avec une solution aqueuse saturée d'hydrogénocarbonate de sodium. On extrait la phase aqueuse au dichlorométhane. Les phases organiques réunies sont lavées à l'eau, séchées sur sulfate de sodium, filtrées sur fritté, évaporées sous pression réduite. On obtient 0,04 g de trans- 4-{4-[4-(2-methoxy-ethanesulfonyl)-piperazin-1-yl]-phenyl}-3,4-dihydro-2H-quinoxaline-1-carboxylic acid (5-hydroxy-adamantan-2-yl)-amide sous forme d'un solide blanc, après purification sur colonne de silice en éluant avec un gradient dichlorométhanelméthanol/ammoniaque variant de 1/0/0 à 99/1/0,1.
M+H⁺= 610 ; RMN ¹H (400 MHz, DMSO-d6) δ ppm : 7,28 (dd, J = 7,9 et 1,5 Hz, 1H); 7,16 (m, J = 9 Hz, 2H); 7,04 (m, J = 9 Hz, 2H); 6,84 (m, J = 7,8 et 1,6 Hz, 1H); 6,67 (m, J = 7,6 et 1,6 Hz, 1H); 6,51 (dd, J = 8,3 and 1,4 Hz, 1H); 5,94 (d, J = 6,3 Hz, 1H); 4,40 (s, 1 H); 3,79 (m, 2H); 3,70 (m, 3H); 3,53 (m, 2H); 3,39 (t, J = 6 Hz, 2H); 3,34-3,27 (m, 7H); 3,24 (m, 4H); 2,02 (m, 3H); 1,74-1,58 (m, 8H); 1,38 (m, 2H).

### Exemple 5 : 4-[4-(4-Methanesulfonyl-piperazin-1-yl)-phenyl]-3,4-dihydro-2H-quinoxaline-1-carboxylic acid (5-carbamoyl-adamantan-2-yl)-amide (composé n°12)

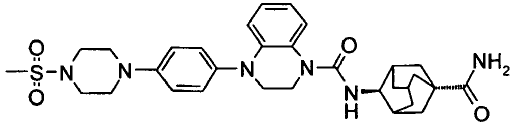

### 5,1 : 4-Amino-adamantane-1-carboxylic acid methyl ester

On place 5 g de 4-oxo-adamantane-1-carboxylic acid methyl ester dans 26 ml de méthanol sous azote. On ajoute 86 ml d'une solution 7N d'ammoniaque dans le méthanol. Le milieu réactionnel est placé sous agitation pendant 18 h, puis on ajoute à 0°C, 4 g de borhohydrure de sodium et le milieu réactionnel est remis sous agitation pendant 3 h. Après concentration à sec sous pression réduite et addition d'eau et d'acétate d'éthyle, la phase aqueuse est extraite trois fois à l'acétate d'éthyle. Les phases organiques sont rassemblées, séchées sur sulfate de magnésium et concentrées à sec. Le brut obtenu est chromatographié sur gel de silice en éluant avec un gradient de solvant d'heptane/acétone/méthanol/NH₄OH de 1/0/0 à 4/5/1/0,1. On obtient 1,5 g de 4-amino-adamantane-1-carboxylic acid methyl ester sous forme d'un mélange d'isomère cis/trans (1/3).
M+H⁺=210

### 5.2 : 4-({4-[4-(4-Methanesulfonyl-piperazin-1-yl)-phenyl]-3,4-dihydro-2H-quinoxaline-1-carbonyl}-amino)-adamantane-1-carboxylic acid methyl ester

On place 1,2 g de 1-[4-(4-methanesulfonyl-piperazin-1-yl)-phenyl]-1,2,3,4-tetrahydro-quinoxaline dans 32 ml de dichlorométhane à 0°C sous azote. On ajoute 0,99 ml de triéthylamine et 0,38 g de triphosgène. On agite 4 h à température ambiante, puis on ajoute 24 ml de diméthylformamide, 1,35 ml de triéthylamine et 0,742 g de mélange cis/trans 4-amino-adamantane-1-carboxylic acid methyl ester (1/3) et l'agitation est maintenue 18 h. Après hydrolyse, le milieu réactionnel est extrait trois fois à l'acétate d'éthyle. Les phases organiques sont rassemblées, lavées avec une solution aqueuse saturée de chlorure de sodium, séchées sur sulfate de magnésium et concentrées à sec. Le brut obtenu est chromatographié sur gel de silice en éluant avec un gradient de solvant heptane/acétate d'éthyle/méthanol de 1/0/0 à 4/5/1. On obtient 1,91 g d'un mélange cis/trans (1/3) de 4-({4-[4-(4-Methanesulfonyl-piperazin-1-yl)-phenyl]-3,4-dihydro-2H-quinoxaline-1-carbonyl}-amino)-adamantane-1-carboxylic acid methyl ester.
M+H⁺=608

### 5.3 : Trans 4-({4-[4-(4-methanesulfonyl-piperazin-1-yl)-phenyl]-3,4-dihydro-2H-quinoxaline-1-carbonyl}-amino)-adamantane-1-carboxylic acid

On place 1,65 g de 4-({4-[4-(4-methanesulfonyl-piperazin-1-yl)-phenyl]-3,4-dihydro-2H-quinoxaline-1-carbonyl}-amino)-adamantane-1-carboxylic acid methyl ester dans 27 ml d'un mélange de tétrahydrofurane/méthanol/eau (1/1/1) et on ajoute 0,193 g de monohydrate de lithine. Le milieu réactionnel est mis sous agitation pendant 18 h, puis on rajoute 0,04 g de monohydrate de lithine et l'agitation est reprise pendant 20 minutes. Les solvants organiques sont évaporés sous pression réduite et on ajoute de l'eau et du dichlorométhane. La phase aqueuse est acidifiée jusqu'à pH=1 avec une solution aqueuse concentrée d'acide chlorhydrique et ensuite extraite au dichlorométhane puis deux fois à l'acétate d'éthyle. Les phases organiques sont rassemblées, séchées sur sulfate de magnésium et concentrées à sec. Le brut obtenu est chromatographié sur gel de silice en éluant avec un mélange heptane/acétate d'éthyle/méthanol (4/5/1) puis est rechromatographié sur gel de silice en éluant avec un gradient de solvant dichlorométhane/acétone/méthanol de 1/0/0 à 4/5/1. Après cristallisation dans l'acétate d'éthyle, on obtient 0,5 g de trans 4-({4-[4-(4-methanesulfonyl-piperazin-1-yl)-phenyl]-3,4-dihydro-2H-quinoxaline-1-carbonyl}-amino)-adamantane-1-carboxylic acid.
M+H⁺=594

### 5.4 : 4-[4-(4-Methanesulfonyl-piperazin-1-yl)-phenyl]-3,4-dihydro-2H-quinoxaline-1-carboxylic acid (5-carbamoyl-adamantan-2-yl)-amide

On place 10,3 g de trans 4-({4-[4-(4-Methanesulfonyl-piperazin-1-yl)-phenyl]-3,4-dihydro-2H-quinoxaline-1-carbonyl}-amino)-adamantane-1-carboxylic acid dans 8,5 ml de diméthylformamide et on ajoute 0,3 ml d'une solution 7,5N d'ammoniaque dans l'eau, 0,136 g de 1-hydroxybenzotriazole, 0,29 g de chlorhydrate de 1-ethyl-3-(3-dimethylaminopropyl)carbodiimide et 0,17 ml de düsopropyléthylamine. Le milieu réactionnel est mis sous agitation pendant 3 h, puis on ajoute de l'eau. La phase aqueuse est extraite deux fois à l'acétate d'éthyle. Les phases organiques sont rassemblées, séchées sur sulfate de magnésium et concentrées à sec. Le brut obtenu est chromatographié sur gel de silice en éluant avec un gradient de solvant dichlorométhane/acétone/méthanol de 1/0/0 à 70/25/5. On obtient 0,171 g de trans 4-[4-(4-methanesulfonyl-piperazin-1-yl)-phenyl]-3,4-dihydro-2H-quinoxaline-1-carboxylic acid (5-carbamoyl-adamantan-2-yl)-amide.
Point de fusion = 247-250°C ; M+H⁺= 593 ; RMN ¹H (300 MHz, DMSO-d6) δ ppm 7,29 (d, J = 7,9 Hz, 1 H) ; 7,16 (m, 2H); 7,04 (m, 2H); 6,97 (m, 1 H); 6,83 (m, 1 H); 6,68 (m, 2H); 6,5 (m, J = 8,5 Hz, 1H); 6,0 (d, J = 6,6 Hz, 1H); 3,77 (m, 3H); 3,53 (m, 2H); 3,25 (m, 8H); 2,94 (s, 3H); 2,02-1,63 (m, 11 H); 1,46 (m, 2H).

### Exemple 6 : Trans 4-[4-(4-Methanesulfonyl-piperazin-1-yl)-phenyl]-3,4-dihydro-2H-quinoxaline-1-carboxylic acid (5-fluoro-adamantan-2-yl)-amide (composé n°40)

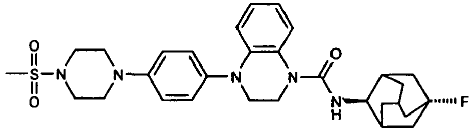

On place 0,2 g de trans-4-[4-(4-methanesulfonyl-piperazin-1-yl)-phenyl]-3,4-dihydro-2H-quinoxaline-1-carboxylic acid (5-hydroxy-adamantan-2-yl)-amide (exemple 2) dans 7 ml de dichlorométhane à 0°C sous azote. On ajoute 0,09 ml de (diethylamino)sulfur trifluoride. On agite 2 h à température ambiante, puis on ajoute de la glace et une solution saturée d'hydrogénocarbonate de sodium. Le mélange est extrait trois fois au dichlorométhane. Les phases organiques sont rassemblées, lavées à l'eau, puis avec une solution aqueuse saturée de chlorure de sodium, séchées sur sulfate de magnésium et concentrées à sec. Le brut obtenu est chromatographié sur gel de silice en éluant avec un gradient de solvant dichlorométhane/acétate d'éthyle/méthanol variant de 1/0/0 à 7/2,5/05. Après cristallisation dans l'acétate d'éthyle, on obtient 0,07 g de trans de 4-[4-(4-methanesulfonyl-piperazin-1-yl)-phenyl]-3,4-dihydro-2H-quinoxaline-1-carboxylic acid (5-fluoro-adamantan-2-yl)-amide.
Point de fusion = 165-200°C ; M+H⁺ = 568 ; RMN ¹H (400 MHz, DMSO-d6) δ ppm 7,30 (dd, J = 7,8 et 1,5 Hz, 1H); 7,17 (m, J = 9 Hz, 2H); 7,05 (m, J = 9, 2H); 6,83 (m, J = 7,7 et 1,5 Hz, 1H); 6,67 (m, J = 7,7 et 1,5 Hz, 1H); 6,51 (dd, J = 8,1 et 1,3 Hz, 1H); 6,03 (d, J = 5,8 Hz, 1H); 3,79 (m, 3H); 3,54 (m, 2H); 3,27 (m, 8H); 2,94 (s, 3H); 2,24-2,12 (m, 3H); 1,99-1,82 (m, 6H); 1,70 (m, 2H); 1,43 (m, 2H).

### Exemple 7: trans (4-{4-[4-(5-Hydroxy-adamantan-2-ylcarbamoyl)-3,4-dihydro-2H-quinoxalin-1-yl]-phenyl}-piperazine-1-sulfonyl)-acetic acid (composé n°49)

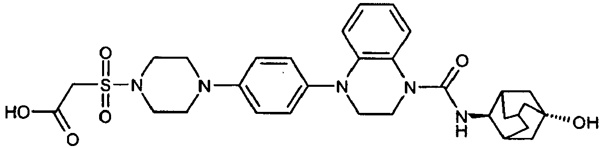

### 7.1 : Trans (4-{4-[4-(5-hydroxy-adamantan-2-ylcarbamoyl)-3,4-dihydro-2H-quinoxalin-1-yl]-phenyl}-piperazine-1-sulfonyl)-acetic acid ethyl ester

On place 0,35 g de trans-4-(4-piperazin-1-yl-phenyl)-3,4-dihydro-2H-quinoxaline-1-carboxylic acid (5-hydroxy-adamantan-2-yl)-amide (intermédiaire 3.5) dans 5,5 ml de dichlorométhane. On additionne 0,15 ml de triéthylamine puis 0,154 g de chlorosulfonyl-acetic acid ethyl ester. On agite 3 h à température ambiante puis on dilue au dichlorométhane et on lave à l'eau. La phase organique est séchée sur sulfate de sodium puis évaporée sous pression réduite. On obtient 0,055 g de trans (4-{4-[4-(5-hydroxy-adamantan-2-ylcarbamoyl)-3,4-dihydro-2H-quinoxalin-1-yl]-phenyl}-piperazine-1-sulfonyl)-acetic acid ethyl ester sous forme d'un solide blanc, après purification sur colonne de silice en éluant avec un gradient dichlorométhane/méthanol/ammoniaque variant de 99/1/0,1 à 94/6/0,6 et une purification sur phase inverse C18 en éluant avec un gradient eau/acétonitrile variant de 90/10 à 5/95.
M+H⁺=638

### 7.2 : Trans (4-{4-[4-(5-Hydroxy-adamantan-2-ylcarbamoyl)-3,4-dihydro-2H-quinoxalin-1-yl]-phenyl}-piperazine-1-sulfonyl)-acetic acid

On place 0,041 g de trans (4-{4-[4-(5-hydroxy-adamantan-2-ylcarbamoyl)-3,4-dihydro-2H-quinoxalin-1-yl]-phenyl}-piperazine-1-sulfonyl}-acetic acid ethyl ester dans 1 ml d'un mélange de tetrahydrofurane/méthanol/eau (2/1/1) et on ajoute 0,008 g de monohydrate de lithine. Le milieu réactionnel est mis sous agitation pendant 2 h. Les solvants organiques sont évaporés sous pression réduite et on ajoute de l'eau. Le mélange est acidifié jusqu'à pH=6 avec une solution aqueuse à 10% d'acide citrique, puis est extrait au dichlorométhane. La phase organique est séchée sur sulfate de sodium et concentrée à sec sous pression réduite. On obtient 0,015 g de trans (4-{4-[4-(5-hydroxy-adamantan-2-ylcarbamoyl)-3,4-dihydro-2H-quinoxalin-1-yl]-phenyl}-piperazine-1-sulfonyl)-acetic acid.
M+H⁺ = 610 ; RMN ¹H (400 MHz, DMSO-d6) δ ppm 13,28 (s, 1 H), 7,29 (dd, J = 7,8 et 1,5 Hz, 1H); 7,17 (m, J = 9 Hz, 2H); 7,05 (m, J = 9 Hz, 2H); 6,84 (m, J = 7,8 et 1,5 Hz, 1 H); 6,67 (m, J = 7,5 et 1,3 Hz, 1H ); 6,52 (dd, J = 8,3 et 1,4 Hz, 1 H); 5,95 (d, J = 6,1 Hz, 1H); 4,40 (s, 1H); 4,21 (s, 2H); 3,79 (m, 2H); 3,71 (m, 1H); 3,53 (m, 2H); 3,39 (m, 4H); 3,25 (m, 4H); 2,02 (m, 3H); 1,75-1,56 (m, 8H); 1,37 (m, 2H).

### Exemple 8 : Trans 4-[4-(4-methanesulfonyl-piperazin-1-yl)-phenyl]-3,4-dihydro-2H-quinoxaline-1-carboxylic acid (5-hydroxymethyl-adamantan-2-yl)-amide (composé n°21)

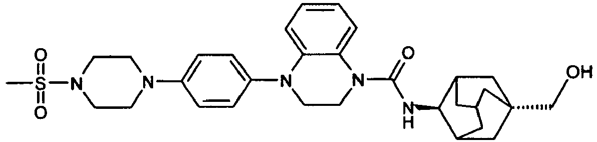

On place 0,7 g de l'intermédiaire 5.2 dans 11 ml de tetrahydrofurane, puis on ajoute à 0°C, 2,3 ml d'une solution 1N d'hydrure double de lithium et d'aluminium dans l'éther. Le milieu réactionnel est mis sous agitation pendant 2 h à température ambiante. De l'eau et de l'acétate d'éthyle sont ajoutés. La phase aqueuse est extraite trois fois à l'acétate d'éthyle. Les phases organiques sont rassemblées, lavées à l'eau, séchées sur sulfate de magnésium et concentrées à sec. Le brut obtenu est chromatographié deux fois sur gel de silice en éluant pour la première purification avec un gradient de solvant dichlorométhane/acetone/méthanol variant de 1/0/0 à 85/15/3, puis pour la seconde purification avec un gradient de solvant dichlorométhane/acetone/méthanol variant de 1/0/0 à 90/10/2. Après cristallisation dans l'éthanol, on obtient 0,415 g de trans 4-[4-(4-methanesulfonyl-piperazin-1-yl)-phenyl]-3,4-dihydro-2H-quinoxaline-1-carboxylic acid (5-hydroxymethyl-adamantan-2-yl)-amide.
Point de fusion = 160°C ; M+H⁺= 580 ; RMN ¹H (400 MHz, DMSO-d6) δ ppm 7,28 (dd, J = 7,9 et 1,5 Hz, 1H); 7,15 (m, J = 9 Hz, 2H); 7,03 (m, J = 9 Hz, 2H); 6,83 (m, J = 7,7 et 1,5 Hz, 1H); 6,66 (m, J = 7,6 et 1,4 Hz, 1H); 6,51 (dd, J = 8,3 et 1,3 Hz, 1H); 5,96 (d, J = 6,7 Hz, 1H); 4,31 (m, 1H); 3,77 (m, 2H); 3,69 (m, 1H); 3,52 (m, 2H); 3,25 (m, 8H); 2,99 (d, J = 5,8 Hz, 2H); 2,93 (s, 3H); 1,94 (m, 2H); 1,82 (m, 1H); 1,69 (m, 2H); 1,54-1,37 (m, 8H).

### Exemple 9 : trans 4-{4-[4-(2-Hydroxy-ethanesulfonyl)-piperazin-1-yl]-phenyl}-3,4-dihydro-2H-quinoxaline-1-carboxylic acid (5-hydroxy-adamantan-2-yl)-amide (composé n°75)

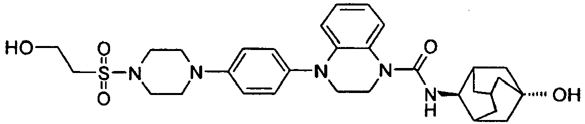

On place 0,145 g de l'intermédiaire 7.1 dans 2,3 ml de tetrahydrofurane, puis on ajoute à 0°C, 0,14 ml d'une solution 2N d'hydrure double de lithium et d'aluminium dans le tetrahydrofurane. Le milieu réactionnel est mis sous agitation pendant 18 h à température ambiante. On ajoute ensuite à 0°C, 5 ml d'une solution à 10% d'hydrogénosulfate de potassium dans l'eau et on laisse le milieu sous agitation à la même température pendant 20 minutes. On filtre le mélange hétérogène, puis la phase aqueuse est extraite à l'acétate d'éthyle. La phase organique est lavée à l'eau, séchée sur sulfate de magnésium et concentrée sous pression réduite. Le brut obtenu est chromatographié sur gel de silice en éluant avec un gradient de solvant dichlorométhane/méthanol/ammoniaque de 1/0/0 à 90/10/1. On obtient 0,003 g de trans 4-{4-[4-(2-Hydroxy-ethanesulfonyl)-piperazin-1-yl]-phenyl}-3,4-dihydro-2H-quinoxaline-1-carboxylic acid (5-hydroxy-adamantan-2-yl)-amide
M+H⁺ = 596 ; RMN ¹H (400 MHz, DMSO-d6) δ ppm 7,28 (dd, J = 7,8 et 1,5 Hz, 1H); 7,16 (m, J = 9 Hz, 2H); 7,04 (m, J = 9 Hz, 2H); 6,84 (m, J = 7,8 and 1,5 Hz, 1 H); 6,67 (m, J = 1,3 Hz, 1H ); 6,51 (dd, J = 8,3 and 1,4 Hz, 1H); 5,94 (d, J = 6,2 Hz, 1H); 5,04 (t, J = 5,4 Hz, 1H); 4,40 (s, 1H); 3,79 (m, 3H); 3,71 (m, 1H); 3,53 (m, 2H); 3,46-3,17 (m, 10H); 2,02 (m, 3H); 1,74-1,57 (m, 8H); 1,38 (m, 2H).

### Exemple 10 : trans 4-[4-(4-hydroxy-piperidin-1-yl)-phenyl]-3,4-dihydro-2H-quinoxaline-1-carboxylic acid (5-hydroxy-adamantan-2-yl)-amide (composé n°51)

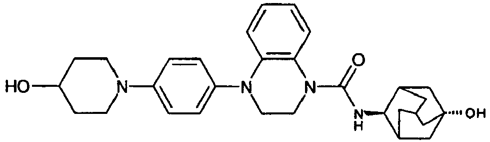

### 10.1 : 8-(4-Bromo-phenyl)-1,4-dioxa-8-aza-spiro[4.5]decane

On place 0,5 g de 1-bromo-4-iodo-benzene et 0,3 g de 1,4-dioxa-8-aza-spiro[4.5]decane dans 10 ml de toluène, puis on ajoute 0,08 g de tris(dibenzylideneacetone)dipalladium (0), 0,061 g de 4,5-bis(diphenylphosphino)-9,9-dimethylxanthene et 0,255 g de terbutylate de sodium. Le milieu réactionnel est mis au reflux du solvant pendant 4 h. On ajoute ensuite de l'acétate d'éthyle et le mélange est lavée deux fois à l'eau et une fois avec une solution aqueuse saturée de chlorure de sodium. La phase organique est séchée sur sulfate de magnésium et concentrée sous pression réduite. Le brut obtenu est chromatographié sur gel de silice en éluant avec un mélange de solvant heptane / acétate d'éthyle (4/1). On obtient 0,31 g de 8-(4-Bromo-phenyl)-1,4-dioxa-8-aza-spiro[4.5]decane.
M+H⁺ = 300

### 10.2 : 4-[4-(1,4-Dioxa-8-aza-spiro[4.5]dec-8-yl)-phenyl]-3,4-dihydro-2H-quinoxaline-1-carboxylic acid tert-butyl ester

On place 0,31 g de 8-(4-bromo-phenyl)-1,4-dioxa-8-aza-spiro[4.5]decane dans 5 ml d'o-xylène anhydre. On additionne 0,24 g de 3,4-dihydro-2H-quinoxaline-1-carboxylic acid tert-butyl ester, puis on ajoute 0,15 g de tert-butylate de sodium, suivis de 0,009 g d'acétate de palladium puis on termine par 0,0084 g de tri-tert-butylphosphine. Le mélange réactionnel est chauffé pendant 4h à 150°C puis on arrête le chauffage et on revient à température ambiante. On ajoute de l'acétate d'éthyle et le mélange est lavé deux fois à l'eau puis deux fois avec une solution aqueuse saturée en chlorure de sodium. La phase organique est séchée sur sulfate de sodium et concentrée sous pression réduite. Le brut obtenu est chromatographié sur gel de silice en éluant avec un mélange de solvant heptane / acétate d'éthyle (4/1). On obtient 0,27 g de 4-[4-(1,4-dioxa-8-aza-spiro[4.5]dec-8-yl)-phenyl]-3,4-dihydro-2H-quinoxaline-1-carboxylic acid tert-butyl ester.
M+H⁺=452

### 10.3 : 1-[4-(1,4-Dioxa-8-aza-spiro[4.5]dec-8-yl)-phenyl]-1,2,3,4-tetrahydro-quinoxaline

On place 0,27 g de 4-[4-(1,4-dioxa-8-aza-spiro[4.5]dec-8-yl)-phenyl]-3,4-dihydro-2H-quinoxaline-1-carboxylic acid tert-butyl ester dans 3 ml d'une solution d'acide chlorhydrique 4N dans le dioxane. On agite 28 h à température ambiante. Le mélange réactionnel est dilué par du dichlorométhane puis on ajoute une solution aqueuse saturée en hydrogenocarbonate de sodium. La phase aqueuse est extraite trois fois au dichlorométhane. Les phases organiques sont réunies, séchées sur sulfate de sodium et concentrées sous vide. On obtient 0,21 g de 1-[4-(1,4-Dioxa-8-aza-spiro[4.5]dec-8-yl)-phenyl]-1,2,3,4-tetrahydro-quinoxaline.
M+H⁺=352

### 10.4 : trans 4-[4-(1,4-Dioxa-8-aza-spiro[4.5]dec-8-yl)-phenyl]-3,4-dihydro-2H-quinoxaline-1-carboxylic acid (5-hydroxy-adamantan-2-yl)-amide

On place 0,21 g de 1-[4-(1,4-Dioxa-8-aza-spiro[4.5]dec-8-yl)-phenyl]-1,2,3,4-tetrahydro-quinoxaline dans 5 ml de dichlorométhane à 0°C sous azote. On ajoute 0,33 ml de triéthylamine et 0,071 g de triphosgène. On agite 3 h à température ambiante, puis on ajoute 1 ml de diméthylformamide et 0,1 g de trans 4-amino-adamantan-1-ol et l'agitation est maintenue pendant 3 jours. Le milieu réactionnel est lavé avec une solution aqueuse saturée d'hydrogénocarbonate de sodium. La phase organique est séchée sur sulfate de magnésium et concentrée à sec. Le brut obtenu est chromatographié sur gel de silice en éluant avec un gradient d'un mélange dichlorométhane/methanol (99/1 à 96/4). On obtient 0,21 g de trans 4-[4-(1,4-dioxa-8-aza-spiro[4.5]dec-8-yl)-phenyl]-3,4-dihydro-2H-quinoxaline-1-carboxylic acid (5-hydroxy-adamantan-2-yl)-amide.
M+H⁺=545,7

### 10.5 : trans 4-[4-(4-Oxo-piperidin-1-yl)-phenyl]-3,4-dihydro-2H-quinoxaline-1-carboxylic acid (5-hydroxy-adamantan-2-yl)-amide

On place 0,20 g de 4-[4-(1,4-Dioxa-8-aza-spiro[4.5]dec-8-yl)-phenyl]-3,4-dihydro-2H-quinoxaline-1-carboxylic acid (5-hydroxy-adamantan-2-yl)-amide dans 4 ml d'une solution d'acide chlorhydrique 5N dans l'eau. Le milieu réactionnel est agité pendant 36 h, puis neutralisé à 0°C avec une solution aqueuse saturée en hydrogenocarbonate de sodium. On extrait la phase aqueuse trois fois au dichlorométhane. Les phases organiques sont réunies, séchées sur sulfate de sodium et concentrées sous vide. Le brut obtenu est chromatographié sur gel de silice en éluant avec un gradient d'un mélange dichlorométhane/méthanol (98/2 à 97/3). On obtient 0,145 g de trans 4-[4-(4-oxo-piperidin-1-yl)-phenyl]-3,4-dihydro-2H-quinoxaline-1-carboxylic acid (5-hydroxy-adamantan-2-yl)-amide.
M+H⁺=519,7 (hydrate)

### 10.6 : trans 4-[4-(4-Hydroxy-piperidin-1-yl)-phenyl]-3,4-dihydro-2H-quinoxaline-1-carboxylic acid (5-hydroxy-adamantan-2-yl)-amide

On place 0,145 g de trans 4-[4-(4-oxo-piperidin-1-yl)-phenyl]-3,4-dihydro-2H-quinoxaline-1-carboxylic acid (5-hydroxy-adamantan-2-yl)-amide dans 2,9 ml de méthanol, puis on ajoute à 0°C, 0,015 g borohydrure de sodium. Le milieu réactionnel est mis sous agitation pendant 4 h à température ambiante. On ajoute ensuite à 0°C, de l'eau et on concentre sous pression réduite. Le brut obtenu est chromatographié sur gel de silice avec un gradient de solvant dichlorométhane/méthanol (98/2 à 96/4). On obtient 0,058 g de trans 4-[4-(4-Hydroxy-piperidin-1-yl)-phenyl]-3,4-dihydro-2H-quinoxaline-1-carboxylic acid (5-hydroxy-adamantan-2-yl)-amide
Point de fusion = 131°C ; M+H⁺ = 503 ; RMN ¹H (400 MHz, DMSO-d6) δ ppm 7,27 (dd, J = 7,7 et 1,5 Hz, 1H); 7,10 (m, J = 9 Hz, 2H); 6,99 (m, J = 9 Hz, 2H); 6,83 (m, J = 7,8 et 1,5 Hz, 1H); 6,66 (m, J = 7,6 et 1,3 Hz, 1H ); 6,49 (dd, J = 8,3 et 1,3 Hz, 1H); 5,93 (d, J = 6,2 Hz, 1H); 4,66 (d, J = 4,5 Hz, 1H); 4,40 (s, 1 H), 3,78 (m, 2H); 3,71 (m, 1H); 3,64 (m, 1 H); 3,53 (m, 4H); 3,28 (m, 4H); 2,86 (m, 2H); 2,02 (m, 3H); 1,84 (m, 2H); 1,74-1,57 (m, 8H); 1,49 (m, 2H); 1,38 (m, 2H).

### Exemple 11 : trans 4-[4-(4-Hydroxy-1-methanesulfonyl-piperidin-4-yl)-phenyl]-3,4-dihydro-2H-quinoxaline-1-carboxylic acid (5-hydroxy-adamantan-2-yl)-amide (composé n°16)

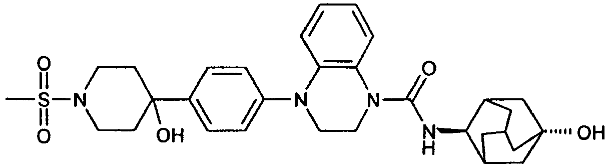

### 11.1 : 4-(4-Bromo-phenyl)-1-methanesulfonyl-piperidin-4-ol

On place 1 g de 4-(4-bromo-phenyl)-piperidin-4-ol dans 20 ml de dichlorométhane. On additionne 0,65 ml de triéthylamine puis 0,33 ml methanesulfonyl chloride. On agite 2 h à température ambiante. Le mélange réactionnel est lavé à l'eau, puis avec une solution aqueuse saturére en chlorure de sodium. La phase organique est séchée sur sulfate de sodium, puis évaporée sous pression réduite. Le brut obtenu est chromatographié sur gel de silice en éluant avec un mélange de solvant dichlorométhane/méthanol (99/1). On obtient 1,1 g de 4-(4-bromo-phenyl)-1-methanesulfonyl-piperidin-4-ol.
M+H⁺=335

### 11.2: Trans 4-(5-Hydroxy-adamantan-2-ylcarbamoyl)-3,4-dihydro-2H-quinoxaline-1-carboxylic acid tert-butyl ester

On place 0,4 g de 3,4-dihydro-2H-quinoxaline-1-carboxylic acid tert-butyl ester dans 8 ml de dichlorométhane à 0°C sous azote. On ajoute 0,69 ml de triéthylamine et 0,202 g de triphosgène. On agite 3 h à température ambiante, puis on ajoute 1 ml de diméthylformamide et 0,285 g de trans 4-amino-adamantan-1-ol et l'agitation est maintenue pendant 18 h. Le milieu réactionnel est lavé avec une solution aqueuse saturée d'hydrogénocarbonate de sodium. La phase organique est séchée sur sulfate de magnésium et concentrée à sec. Le brut obtenu est chromatographié sur gel de silice en éluant avec un gradient d'un mélange dichlorométhane/methanol (99/1 à 95/5). On obtient 0,54 g de Trans 4-(5-Hydroxy-adamantan-2-ylcarbamoyl)-3,4-dihydro-2H-quinoxaline-1-carboxylic acid tert-butyl ester.
M+H⁺=428

### 11.3 : Trans 3,4-Dihydro-2H-quinoxaline-1-carboxylic acid (5-hydroxy-adamantan-2-yl)-amide

On place 0,54 g de trans 4-(5-hydroxy-adamantan-2-ylcarbamoyl)-3,4-dihydro-2H-quinoxaline-1-carboxylic acid tert-butyl ester dans 5 ml d'une solution d'acide chlorhydrique 4N dans le dioxane. On agite 3,5 h à température ambiante. Le mélange réactionnel est dilué par du dichlorométhane. On ajoute une solution aqueuse saturée en hydrogenocarbonate de sodium. La phase aqueuse est extraite trois fois au dichlorométhane. Les phases organiques sont réunies, séchées sur sulfate de sodium, et concentrées sous vide. On obtient 0,41 g trans 3,4-dihydro-2H-quinoxaline-1-carboxylic acid (5-hydroxy-adamantan-2-yl)-amide
M+H⁺=328

### 11.4: trans 4-[4-(4-Hydroxy-1-methanesulfonyl-piperidin-4-yl)-phenyl]-3,4-dihydro-2H-quinoxaline-1-carboxylic acid (5-hydroxy-adamantan-2-yl)-amide

On place 0,210 g de 4-(4-Bromo-phenyl)-1-methanesulfonyl-piperidin-4-ol dans 4 ml de dioxane. On additionne 0,206 g de Trans 3,4-dihydro-2H-quinoxaline-1-carboxylic acid (5-hydroxy-adamantan-2-yl)-amide, puis on ajoute 0,4 g de K₃PO₄, suivis de 0,0353 g de Chloro(Di-2-Norbornylphosphino)(2'-Dimethylamino-1,1'-Biphenyl-2-YI)Palladium (II). Le mélange réactionnel est chauffé au reflux du solvant pendant 20h. Ensuite, on arrête le chauffage et on revient à température ambiante. On ajoute de l'eau et de l'acétate d'éthyle puis on extrait deux fois la phase aqueuse à l'acétate d'éthyle. Les phases organiques sont réunies, lavées avec une solution aqueuse saturée en chlorure de sodium, séchées sur sulfate de sodium et concentrées sous pression réduite. Le brut obtenu est chromatographié sur gel de silice en éluant avec un gradient d'un mélange dichlorométhane/methanol (99/1 à 95/5). On obtient 0,114 g de trans 4-[4-(4-Hydroxy-1-methanesulfonyl-piperidin-4-yl)-phenyl]-3,4-dihydro-2H-quinoxaline-1-carboxylic acid (5-hydroxy-adamantan-2-yl)-amide.
Point de fusion = 160°C ; M+H⁺ = 581 ; RMN ¹H (400 MHz, DMSO-d6) δ ppm 7,54 (m, J = 8,8 Hz, 2H) ; 7,35 (dd, J = 7,9 et 1,5 Hz, 1H); 7,24 (m, J = 8,8 Hz, 2H); 6,88 (m, J = 7,7 et 1,5 Hz, 1H); 6,75 (m, J = 7,5 et 1,4 Hz, 1H); 6,70 (dd, J = 8,2 et 1,4 Hz, 1H); 5,99 (dd, J = 6 Hz, 1 H); 5,12 (s, 1H); 4,40 (s, 1H); 3,80 (m, 2H); 3,71 (m, 1H); 3,61 (m, 2H); 3,49 (m, 2H); 3,28 (m, 8H); 3,13 (m, 2H); 2,92 (s, 3H); 2,08-1,94 (m, 6H); 1,79-1,59 (m, 9H); 1,37 (m, 2H).

### Exemple 12 : Trans 4-{4-[4-(Piperidin-4-yloxy)-piperidin-1-yl]-phenyl}-3,4-dihydro-2H-quinoxaline-1-carboxylic acid (5-hydroxy-adamantan-2-yl)-amide (composé n°102)

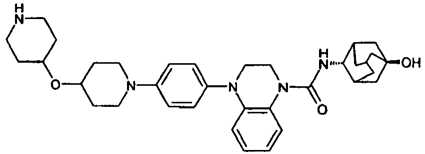

### 12.1 : 4-[1-(4-Bromo-phenyl)-piperidin-4-yloxy]-pyridine

On place 2 g de 1-bromo-4-iodo-benzène et 1,386 g de 4-(piperidin-4-yloxy)-pyridine dans 35 ml de toluène, puis on ajoute 0,324 g de tris(dibenzylideneacetone)dipalladium (0), 0,245 g de 4,5-bis(diphenylphosphino)-9,9-dimethylxanthene et 1,019 g de terbutylate de sodium. Le milieu réactionnel est chauffé à 110°C pendant 18 h. On ajoute ensuite de l'acétate d'éthyle et le mélange est lavé deux fois à l'eau et une fois avec une solution aqueuse saturée de chlorure de sodium. La phase organique est séchée sur sulfate de magnésium et concentrée sous pression réduite. Le brut obtenu est chromatographié sur gel de silice en éluant avec un gradient d'un mélange dichlorométhane/methanol (99/1 à 98/2). On obtient 1,2 g de 4-[1-(4-bromo-phenyl)-piperidin-4-yloxy]-pyridine.
M+H⁺ = 335

### 12.2 : 4-{4-[4-(Pyridin-4-yloxy)-piperidin-1-yl]-phenyl}-3,4-dihydro-2H-quinoxaline-1-carboxylic acid tert-butyl ester

On place 1,2 g de 4-[1-(4-bromo-phenyl)-piperidin-4-yloxy]-pyridine dans 20 ml d'o-xylène anhydre. On additionne 0,844 g de 3,4-dihydro-2H-quinoxaline-1-carboxylic acid tert-butyl ester, puis on ajoute 0,519 g de tert-butylate de sodium, suivis de 0,032 g d'acétate de palladium puis on termine par 0,029 g de tri-tert-butylphosphine. Le mélange réactionnel est chauffé pendant 6 h à 150°C. Ensuite, on arrête le chauffage, on revient à température ambiante et on ajoute de l'acétate d'éthyle. Le mélange est lavér deux fois à l'eau puis deux fois avec une solution aqueuse saturée en chlorure de sodium. La phase organique est séchée sur sulfate de sodium et concentrée sous pression réduite. Le brut obtenu est chromatographié sur gel de silice en éluant avec un gradient d'un mélange dichlorométhane/methanol (99/1 à 97/3). On obtient 1,1 g de 4-{4-[4-(Pyridin-4-yloxy)-piperidin-1-yl]-phenyl}-3,4-dihydro-2H-quinoxaline-1-carboxylic acid tert-butyl ester.
M+H⁺=487

### 12.3: 1-{4-[4-(Pyridin-4-yloxy)-piperidin-1-yl]-phenyl)-1,2,3,4-tetrahydro-quinoxaline

On place 1,1 g de trans 4-{4-[4-(pyridin-4-yloxy)-piperidin-1-yl]-phenyl}-3,4-dihydro-2H-quinoxaline-1-carboxylic acid tert-butyl ester dans 15 ml d'une solution d'acide chlorhydrique 4N dans le dioxane. On agite 3 h à température ambiante. Le mélange réactionnel est dilué par du dichlorométhane puis on ajoute une solution aqueuse saturée en hydrogénocarbonate de sodium. La phase aqueuse est extraite trois fois au dichlorométhane. Les phases organiques sont réunies, séchées sur sulfate de sodium, et concentrées sous vide. On obtient 0,87 g 1-{4-[4-(pyridin-4-yloxy)-piperidin-1-yl]-phenyl}-1,2,3,4-tetrahydro-quinoxaline.
M+H⁺=387

### 12.4 : trans 4-{4-[4-(Pyridin-4-yloxy)-piperidin-1-yl]-phenyl}-3,4-dihydro-2H-quinoxaline-1-carboxylic acid (5-hydroxy-adamantan-2-yl)-amide

On place 0,4 g de 1-{4-[4-(pyridin-4-yloxy)-piperidin-1-yl]-phenyl}-1,2,3,4-tetrahydro-quinoxaline dans 6 ml de dichlorométhane à 0°C. On ajoute 0,58 ml de triéthylamine et 0,123 g de triphosgène. On agite 3 h à température ambiante, puis on ajoute 1,5 ml de diméthylformamide et 0,173 g de trans 4-amino-adamantan-1-ol et l'agitation est maintenue pendant 22 h. Le milieu réactionnel est lavé avec une solution aqueuse saturée d'hydrogénocarbonate de sodium. La phase organique est séchée sur sulfate de magnésium et concentrée à sec. Le brut obtenu est chromatographié sur gel de silice en éluant avec un gradient d'un mélange dichlorométhane/méthanol (98/2 à 90/10). On obtient 0,045 g de trans 4-{4-[4-(pyridin-4-yloxy)-piperidin-1-yl]-phenyl}-3,4-dihydro-2H-quinoxaline-1-carboxylic acid (5-hydroxy-adamantan-2-yl)-amide.
M+H⁺=580

### 12.5 : trans 4-{4-[4-(Piperidin-4-yloxy)-piperidin-1-yl]-phenyl}-3,4-dihydro-2H-quinoxaline-1-carboxylic acid (5-hydroxy-adamantan-2-yl)-amide

Dans un flacon de parr, on place 0,045 g de trans 4-{4-[4-(pyridin-4-yloxy)-piperidin-1-yl]-phenyl}-3,4-dihydro-2H-quinoxaline-1-carboxylic acid (5-hydroxy-adamantan-2-yl)-amide dans 7 ml d'éthanol. On ajoute 0,005 g d'oxyde de platine et 0,015 g d'acide paratoluene soulfonique monohydrate. On agite le milieu réactionnel sous 50 psi d'hydrogène pendant 12 h à température ambiante, puis 22 h à 40°C. Le milieu réactionnel est versé à 0°C sur 2 ml d'une solution aqueuse 2N de soude, filtré puis concentré sous pression réduite. De l'eau et du dichlorométhane sont ajoutés. La phase aqueuse est extraite deux fois au dichlorométhane. Les phases organiques sont rassemblées, séchées sur sulfate de magnésium et concentrées à sec. Le brut obtenu est chromatographié sur gel de silice en éluant avec un gradient d'un mélange dichlorométhane/méthanol/amoniaque (98/2/0,2 à 90/10/1). On obtient 0,003 g de trans 4-{4-[4-(piperidin-4-yloxy)-piperidin-1-yl]-phenyl}-3,4-dihydro-2H-quinoxaline-1-carboxylic acid (5-hydroxy-adamantan-2-yl)-amide.
M+H⁺ = 586 ; RMN ¹H (400 MHz, DMSO-d6) δ ppm 7,27 (d, J = 7,9 Hz, 1 H) ; 7,11 (m, 2H); 7 (m, 2H), 6,83 (m, J = 7,8 et 1,5 Hz, 1H); 6,66 (m, 1H); 6,49 (m, J = 8,2 Hz, 1H); 5,93 (d, J = 6,2 Hz, 1H); 4,40 (m, 1H); 3,84-3,42 (m, 9H); 3,21-2,73 (m, 6H); 2,12-1,11 (m, 21H).

### Exemple 13 : Trans 4-[4-(4-Methanesulfonyl-piperazin-1-yl)-phenyl]-3,4-dihydro-2H-quinoxaline-1-carboxylic acid (5-acetylamino-adamantan-2-yl)-amide (composé n°137)

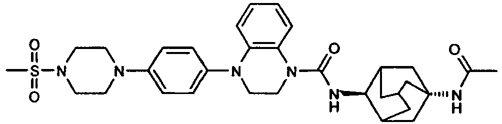

### 13.1 : trans-4-Amino-adamantane-1-carboxylic acide

Le 4-oxo-adamantane-1-carboxylic acid (8 g, 41,2 mmol) est agité sous atmosphère d'H₂ en présence de palladium sur charbon 10% (0,5g) dans 160 mL d'une solution méthanolique d'ammoniac 7N pendant 19h. Le solide en suspension est tout d'abord essoré et lavé avec du méthanol, puis il est repris avec 200 mL d'eau et le palladium sur charbon est filtré. On concentre à sec, on reprend dans un peu de méthanol puis on essore le solide blanc qui est ensuite séché sur P₂O₅. On obtient 5,4 g de trans-4-amino-adamantane-1-carboxylic acid attendu.
[M+H⁺] = 196

### 13.2 : trans 4-tert-Butoxycarbonylamino-adamantane-1-carboxylic acide

On dissout le trans-4-amino-adamantane-1-carboxylic acid (7,74 g, 39,6 mmol) dans 70 mL d'une solution de soude aqueuse 1 N puis on ajoute 70 mL de dioxane. On additionne le di-t-butoxycarbonate (25,9 g, 118,9 mmol). Le milieu réactionnel est agité 16h à température ambiante. Après évaporation du dioxane, la phase aqueuse est extraite au dichlorométhane. Puis on ajoute une solution aqueuse d'HCl 1 N jusqu'à pH 4. On extrait au dichlorométhane. La phase organique est séchée sur MgSO₄ et concentrée à sec. On obtient 10,5 g de trans 4-t-butoxycarbonylamino-adamantane-1-carboxylic acid attendu.
[M+H⁺] = 296

### 13.3 : trans (4-tert-Butoxycarbonylamino-adamantan-1-yl)-carbamic acid benzyl ester

On place le *trans*-4-t-butoxycarbonylamino-adamantane-1-carboxylic acid (2,0 g, 6,77 mmol) dans 13 mL de toluène anhydre sous azote. On ajoute la triéthylamine (1,04 mL, 7,45 mmol) puis le diphénylphosphorylazide (1,61 mL, 7,45 mmol). Le milieu réactionnel est chauffé au reflux pendant 2h. On ajoute alors le sodium benzyloxide 1 N dans l'alcool benzylique (3,4 mL, 3,39 mmol). Le chauffage au reflux est poursuivi pendant 4h. Après hydrolyse, et extraction à l'acétate d'éthyle, la phase organique est lavée avec une solution aqueuse saturée en chlorure de sodium, séchée sur MgSO₄ et concentrée à sec. Le brut obtenu est chromatographié sur gel de silice en éluant avec un gradient de dichlorométhane/acétate d'éthyle/méthanol dans l'heptane variant de 100/0/0/0 à 0/7/2,5/0,5. On obtient 1,85 g de trans (4-tert-Butoxycarbonylamino-adamantan-1-yl)-carbamic acid benzyl ester en mélange avec 0,5 mole d'alcool benzylique.
[M+H⁺] = 456

### 13.4 : Chlorydrate du trans (4-Amino-adamantan-1-yl)-carbamic acid benzyl ester

On place le trans (4-tert-Butoxycarbonylamino-adamantan-1-yl)-carbamic acid benzyl (1,85 g, 4,07 mmol) dans du HCl 4N dans le dioxane (17 mL, 69 mmol). Le milieu réactionnel est agité 4h à température ambiante. Le précipité obtenu est essoré et rincé au dioxane. On obtient 1,85 g de chlorhydrate du trans (4-Amino-adamantan-1-yl)-carbamic acid benzyl ester.
[M+H⁺] = 301

### 13.5 : trans [4-({4-[4-(4-Methanesulfonyl-piperazin-1-yl)-phenyl]-3,4-dihydro-2H-quinoxaline-1-carbonyl}-amino)-adamantan-1-yl]-carbamic acid benzyl ester

On place le 1-[4-(4-Methanesulfonyl-piperazin-1-yl)-phenyl]-1,2,3,4-tetrahydro-quinoxaline (1,1 g, 2,95 mmol) dans 90 mL d'un mélange 50/50 de dichlorométhane/solution aqueuse saturée en NaHCO₃ à 0°C. On ajoute le phosgène 20% dans le toluène (2,33 mL, 2,95 mmol). Après 1h30 d'agitation, on extrait la phase aqueuse au dichlorométhane. La phase organique est séchée sur MgSO₄. Le brut obtenu est placé dans 45 mL de NMP anhydre sous azote. On ajoute la triéthylamine (0,82 mL, 5,91 mmol) suivie du chlorhydrate du trans (4-amino-adamantan-1-yl)-carbamic acid benzyl ester (1,15 g, 2,95 mmol). Le milieu réactionnel est agité 48h puis chauffé à 50°C pendant 2h. Après hydrolyse et extraction à l'acétate d'éthyle, la phase organique est lavée avec une solution aqueuse saturée en chlorure de sodium et séchée sur MgSO₄. Après concentration à sec, le brut obtenu est chromatographié sur gel de silice en éluant avec un gradient de méthanol dans du dichlorométhane variant de 0% à 10%. On obtient 0,37g [4-({4-[4-(4-Methanesulfonyl-piperazin-1-yl)-phenyl]-3,4-dihydro-2H-quinoxaline-1-carbonyl}-amino)-adamantan-1-yl]-carbamic acid benzyl ester.
[M+H⁺] = 699

### 13.6: trans 4-[4-(4-methanesulfonyl-piperazin-1-yl)-phenyl]-3,4-dihydro-2H-quinoxaline-1-carboxylic acid (trans-5-amino-adamantan-2-yl)-amide

On place le trans [4-({4-[4-(4-methanesulfonyl-piperazin-1-yl)-phenyl]-3,4-dihydro-2H-quinoxaline-1-carbonyl}-amino)-adamantan-1-yl]-carbamic acid benzyl ester (1,34 g, 1,92 mmol) dans 19 mL de méthanol. On ajoute Pd/C 10% à 50% humide (0,27g) et le formiate d'ammonium (1,21 g, 19,2 mmol). Le milieu réactionnel est chauffé au reflux pendant 2h. Le Pd/ C est filtré puis rincé au méthanol. Par ailleurs, le Pd/C en mélange avec le produit attendu est repris par un mélange d'eau et de dichlorométhane. Le mélange hétérogène est filtré puis rincé au dichlorométhane. La phase aqueuse du filtrat est basifié avec une solution aqueuse saturée en hydrogénocarbonate de sodium, puis est extraite au dichlorométhane. L'ensemble des phases organiques sont rassemenblés, séchés sur sulfate de magnésium puis concentrés sous pression réduite.. Le brut obtenu est chromatographié sur gel de silice en éluant avec un gradient de méthanol/ammoniaque dans du dichlorométhane variant de 100/0/0 à 90/10/1 (dichlorométhane/méthanol/ammoniaque). On obtient 0,77 g de 4-[4-(4-methanesulfonyl-piperazin-1-yl)-phenyl]-3,4-dihydro-2H-quinoxaline-1-carboxylic acid (trans-5-amino-adamantan-2-yl)-amide.
[M+H⁺ ] = 565

### 13.7 : trans 4-[4-(4-Methanesulfonyl-piperazin-1-yl)-phenyl]-3,4-dihydro-2H-quinoxaline-1-carboxylic acid (trans-5-acetylamino-adamantan-2-yl)-amide

On place le 4-[4-(4-methanesulfonyl-piperazin-1-yl)-phenyl]-3,4-dihydro-2H-quinoxaline-1-carboxylic acid (trans-5-amino-adamantan-2-yl)-amide (0,26 g, 0,46 mmol) dans 4 mL de dichlorométhane anhydre sous azote. Puis on ajoute la diisopropylethylamine (0,18 mL, 1,01 mmol) et le chlorure d'acétyle (0,03 mL, 0,48 mmol). Le milieu réactionnel est agité 18h à température ambiante. Après hydrolyse avec une solution aqueuse saturée en hydrogénocarbonate de sodium, la phase aqueuse est extraite à l'acétate d'éthyle. Les phases organiques sont rassemblées, puis lavées avec une solution aqueuse saturée en chlorure de sodium, séchées sur MgSO₄ et concentrées à sec. Le brut obtenu est chromatographié sur gel de silice en éluant avec un gradient de méthanol dans le dichlorométhane variant de 0% à 10%. Après trituration dans l'acétonitrile, on obtient 0,184 g 4-[4-(4-methanesulfonyl-piperazin-1-yl)-phenyl]-3,4-dihydro-2H-quinoxaline-1-carboxylic acid (trans-5-acetylamino-adamantan-2-yl)-amide.
PF : 250°C ; [M+H⁺] = 607 ; RMN ¹H (400 MHz, DMSO-d6) δ ppm 7,34 (m, 1H); 7,29 (dd, J = 8 Hz et 1,4 Hz, 1 H); 7,16 (m, 2H); 7,05 (m, 2H); 6,83 (m, J = 7,8 Hz et 1,5 Hz, 1H); 6,77 (m, J = 7,7 Hz et 1,4 Hz, 1H); 6,52 (dd, J = 8,2 Hz et 1, Hz, 1H); 5,8 (d, J = 6, Hz, 1 H); 3,9 (m, 2H); 3,5 (m, 1 H); 3,4 (m, 2H); 3,7 (m, 8H); 2,4 (s, 3H); 2,6-1,0 (m, 9H); 1,6 (s, 3H); 1,67 (m, 2H); 1,5 (m, 2H).

### Exemple 14: trans 4-[5-(4-Ethanesulfonyl-piperazin-1-yl)-pyridin-2-yl]-3,4-dihydro-2H-quinoxaline-1-carboxylic acid (5-carbamoyl-adamantan-2-yl)-amide (composé n°149)

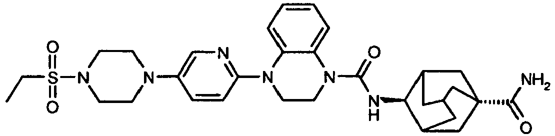

### 14.1 : 4-(5-Bromo-pyridin-2-yl)-3,4-dihydro-2H-quinoxaline-1-carboxylic acid tert-butyl ester

On place 30 g de 3,4-dihydro-2H-quinoxaline-1-carboxylic acid tert-butyl ester dans 430 ml de N-Méthyl pyrrolidinone à 0°C sous azote. On ajoute par portion 30 g de tert-butylate de potassium en maintenant la température en dessous de 10°C. On agite 1h30 à température ambiante, puis à 0°C on ajoute 850 ml d'eau et 800 ml d'éther éthylique. La phase aqueuse est extraite par 800 ml d'ether éthylique, puis par 400 ml d'ether éthylique. Les phases organiques sont rassemblées, puis séchées sur sulfate de magnésium et concentrée à sec. On ajoute 300 mL de pentane au brut réactionnel et on sonique sous ultra-sons pendant 5 min le mélange hétérogène obtenu. Le mélange est placé pendant 48h à 5°C, puis le solide est filtré, lavé trois fois au pentane puis séché à 40°C pendant 5h. On obtient 35 g de 4-(5-Bromo-pyridin-2-yl)-3,4-dihydro-2H-quinoxaline-1-carboxylic acid tert-butyl ester
M+H⁺=392.0

### 14.2 : 4-[5-(4-Benzyloxycarbonyl-piperazin-1-yl)-pyridin-2-yl]-3,4-dihydro-2H-quinoxaline-1-carboxylic acid tert-butyl ester

On place 10,12 g de 4-(5-Bromo-pyridin-2-yl)-3,4-dihydro-2H-quinoxaline-1-carboxylic acid tert-butyl ester et 5,7 g de 4-carboxybenzyle piperazine dans 118 ml de toluène, puis on ajoute 0,95 g de tris(dibenzylideneacetone)dipalladium (0), 1,7 g de 2-dicyclohexylphosphino-2',6'-dimethoxybiphenyl et 3,5 g de terbutylate de sodium. Le milieu réactionnel est chauffé à 110°C pendant 3 h. On ajoute ensuite de l'acétate d'éthyle et le mélange est lavé une fois à l'eau et une fois avec une solution aqueuse saturée de chlorure de sodium. La phase organique est séchée sur sulfate de magnésium et concentrée sous pression réduite. Le brut obtenu est chromatographié sur gel de silice en éluant avec un gradient d'un mélange d'heptane/acétate d'éthyle (90/10 à 0/100). On obtient 10,16 g de 4-[5-(4-Benzyloxycarbonyl-piperazin-1-yl)-pyridin-2-yl]-3,4-dihydro-2H-quinoxaline-1-carboxylic acid tert-butyl ester.
M+H⁺= 530,5

### 14.3 : 4-(5-Piperazin-1-yl-pyridin-2-yl)-3,4-dihydro-2H-quinoxaline-1-carboxylic acid tert-butyl ester

Dans un flacon de parr, on place 5,08 g de 4-[5-(4-Benzyloxycarbonyl-piperazin-1-yl)-pyridin-2-yl]-3,4-dihydro-2H-quinoxaline-1-carboxylic acid tert-butyl ester dans 240 ml d'éthanol. On ajoute 2 g de Pd/C 10% (50% dans l'eau). On agite le milieu réactionnel à 35°C, sous 45 psi d'hydrogène pendant 3,5h. On filtre sous atmosphère inerte sur filtre Whatman puis on rince de nombreuses fois au méthanol. Le méthanol est évaporé sous pression réduite. On obtient 3,57 g de 4-(5-Piperazin-1-yl-pyridin-2-yl)-3,4-dihydro-2H-quinoxaline-1-carboxylic acid tert-butyl ester.
M+H⁺= 396,6

### 14.4 : 4-[5-(4-Ethanesulfonyl-piperazin-1-yl)-pyridin-2-yl]-3,4-dihydro-2H-quinoxaline-1-carboxylic acid tert-butyl ester

On place 0,8 g de 4-(5-Piperazin-1-yl-pyridin-2-yl)-3,4-dihydro-2H-quinoxaline-1-carboxylic acid tert-butyl ester dans un ballon de 25 ml. On ajoute 10 ml de dichlorométhane puis 0,37 ml de triéthylamine et enfin 0,23 ml de chloroéthylsulfonyle. Le mélange réactionnel est agité 3 h à température ambiante puis, lavé deux fois à l'eau et une fois par une solution saturé en chlorure de sodium, puis évaporé sous pression réduite. On obtient 0,98 g 4-[5-(4-Ethanesulfonyl-piperazin-1-yl)-pyridin-2-yl]-3,4-dihydro-2H-quinoxaline-1-carboxylic acid tert-butyl ester.
M+H⁺ = 488.0

### 14.4: 1-[5-(4-Ethanesulfonyl-piperazin-1-yl)-pyridin-2-yl]-1,2,3,4-tetrahydro-quinoxaline

On place 0,98 g de 4-[5-(4-Ethanesulfonyl-piperazin-1-yl)-pyridin-2-yl]-3,4-dihydro-2H-quinoxaline-1-carboxylic acid tert-butyl ester dans 3 ml d'une solution d'acide chlorhydrique 4N dans le dioxane. On agite 3 h à température ambiante. Le mélange réactionnel est ensuite évaporé sous pression réduite, puis on ajoute une solution aqueuse saturée en hydrogenocarbonate de sodium. La phase aqueuse est extraite trois fois au dichlorométhane. Les phases organiques sont réunies, séchées sur sulfate de sodium et concentrées sous vide. On obtient 0,7 g de 1-[5-(4-Ethanesulfonyl-piperazin-1-yl)-pyridin-2-yl]-1,2,3,4-tetrahydro-quinoxaline.
M+H⁺=388,6

### 14.5: trans 4-[5-(4-Ethanesulfonyl-piperazin-1-yl)-pyridin-2-yl]-3,4-dihydro-2H-quinoxaline-1-carboxylic acid (5-carbamoyl-adamantan-2-yl)-amide

On place 0,33 g de 1-[5-(4-Ethanesulfonyl-piperazin-1-yl)-pyridin-2-yl]-1,2,3,4-tetrahydro-quinoxaline dans un mélange de 14 ml d'une solution aqueuse saturée d'hydrogénocarbonate de sodium et 14 ml de dichlorométhane à 0°C. On ajoute 0,67 ml d'une solution à 20% de phosgène dans le toluène. On agite 15 min à température ambiante, puis on décante les deux phases. La phase aqueuse est extraite au dichlorométhane. Les phases organiques sont réunies, séchées sur sulfate de magnésium et concentrées sous vide. Le brut réactionnel est repris par 18 ml de diméthylformamide, puis on ajoute 0,74 mL de diisopropylethylamine ainsi que 0,22 g du chlorhydrate de *trans* 4-Amino-adamantane-1-carboxylic acide amide. On agite 48 h à température ambiante, puis on ajoute de l'eau. La phase aqueuse est extraite deux fois à l'acétate d'éthyle. Les phases organiques sont réunies, lavées trois fois à l'eau, séchées sur sulfate de sodium et concentrées sous vide. Le brut obtenu est chromatographié sur gel de silice en éluant avec un gradient d'un mélange dichlorométhane/méthanol (99/1 à 90/10). On obtient 0,185 g de trans 4-[5-(4-Ethanesulfonyl-piperazin-1-yl)-pyridin-2-yl]-3,4-dihydro-2H-quinoxaline-1-carboxylic acid (5-carbamoyl-adamantan-2-yl)-amide après trituration dans un mélange d'acétate d'éthyle et d'éther éthylique.
Point de fusion = 175°C ; M+H⁺= 608 ; RMN ¹H (400 MHz, DMSO-d6) δ ppm 8,07 (d, J = 3 Hz, 1 H) ; 7,47 (dd, J = 8 Hz and 1,6 Hz, 1 H) ; 7,42 (dd, J = 9 Hz and 3 Hz, 1H), 7,17-7,11 (m, 2H) ; 6,99-6,85 (m, 3H); 6,69 (sl, 1H); 6,07 (d, J = 6 Hz, 1H); 3,81 (s, 4H); 3,75 (m, 1H); 3,34 (m, 4H); 3,20 (m, 4H); 3,13 (q, J = 7,4 Hz, 2H); 1,99 (m, 2H); 1,91-1,72 (m, 9H); 1,45 (m, 2H); 1,26 (t, J = 7,4 Hz, 3H).

### Exemple 15: trans-2-(4-{4-[4-(5-Carbamoyl-adamantan-2-ylcarbamoyl)-3,4-dihydro-2H-quinoxalin-1-yl]-phenyl}-piperazin-1-yl)-2-methyl-propionic acid (composé n°146)

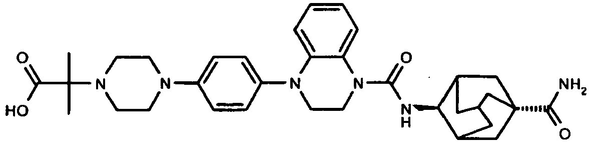

### 15.1 : trans 4-{4-[4-(5-Carbamoyl-adamantan-2-ylcarbamoyl)-3,4-dihydro-2H-quinoxalin-1-yl]-phenyl}-piperazine-1-carboxylic acid benzyl ester

Dans un tricol de 100 ml sous atmosphère inerte d'azote, on introduit 1,17 g de l'intermédiaire 3.3 dans 28 ml de dichlorométhane anhydre. On ajoute à 0°C, 0,56 ml de triéthylamine. On additionne ensuite 0,32 g de triphosgène. On agite 2 h à température ambiante, et on dilue au dichlorométhane, on lave avec une solution saturée d'hydrogenocarbonate de sodium puis à l'eau. La phase organique est séchée sur sulfate de sodium, filtrées sur fritté et évaporées à sec. Le brut est ensuite dissous dans 25 ml de diméthylformamide et on ajoute ensuite 1,56 g du chlorhydrate de *trans* 4-amino-adamantane-1-carboxylic acid amide. On agite 18 h à température ambiante. On évapore les solvants et on reprend à l'eau. La phase aqueuse est extraite au dichlorométhane. La phase organique est séchée sur sulfate de sodium, filtrées sur fritté et évaporées à sec. Le brut obtenu est chromatographié sur gel de silice en éluant avec un gradient d'un mélange dichlorométhane/méthanol/ammoniaque (99/1/0,1 à 92/8/0,8). On obtient 1,09 g de *trans* 4-{4-[4-(5-Carbamoyl-adamantan-2-ylcarbamoyl)-3,4-dihydro-2H-quinoxalin-1-yl]-phenyl}-piperazine-1-carboxylic acid benzyl ester.
M+H⁺=649

### 15.2 : trans 4-(4-Piperazin-1-yl-phenyl)-3,4-dihydro-2H-quinoxaline-1-carboxylic acid (5-carbamoyl-adamantan-2-yl)-amide

On place 1,08 g de *trans* 4-{4-[4-(5-Carbamoyl-adamantan-2-ylcarbamoyl)-3,4-dihydro-2H-quinoxalin-1-yl]-phenyl}-piperazine-1-carboxylic acid benzyl ester dans un tricol de 100 ml sous atmosphère inerte. On ajoute 15 ml de méthanol, puis 0,42 g de formiate d'ammonium suivis de 0,35 g de Pd/C 10% (50% dans l'eau). Le mélange est porté au reflux du méthanol pendant une heure puis ramené à température ambiante. On filtre sous atmosphère inerte sur filtre Whateman puis on rince de nombreuses fois au méthanol. Le méthanol est évaporé sous pression réduite. On reprend au dichlorométhane et on lave la phase organique avec un minimum d'eau. La phase organiques est séchée sur sulfate de sodium, filtrée sur fritté et évaporée sous pression réduite. On obtient 0,7 g de trans 4-(4-Piperazin-1-yl-phenyl)-3,4-dihydro-2H-quinoxaline-1-carboxylic acid (5-carbamoyl-adamantan-2-yl)-amide.
M+H⁺=515

### 15.3: trans 2-(4-{4-[4-(5-Carbamoyl-adamantan-2-ylcarbamoyl)-3,4-dihydro-2H-quinoxalin-1-yl]-phenyl}-piperazin-1-yl)-2-methyl-propionic acid ethyl ester

On place 0,7 g de *trans* 4-(4-Piperazin-1-yl-phenyl)-3,4-dihydro-2H-quinoxaline-1-carboxylic acid (5-carbamoyl-adamantan-2-yl)-amide dans un ballon de 100 ml sous atmosphère inerte. On ajoute 14 ml de diméthylformamide, 0,36 g de carbonate de potassium, puis 0,21 ml de 2-Bromo-2-methyl-propionic acide ethyl ester. Le mélange est agité pendant 4 h à température ambiante puis est chauffé pendant 18 heures à 50°C. On ajoute 0,36 g de carbonate de potassium, puis 0,2 ml de 2-Bromo-2-methyl-propionic acide ethyl ester, puis le mélange est chauffé pendant 32 heures à 50°C. On évapore les solvants et on reprend à l'eau. La phase aqueuse est extraite au dichlorométhane. La phase organique est lavée à l'eau, séchée sur sulfate de sodium, filtrées sur fritté et évaporées à sec. Le brut obtenu est chromatographié sur gel de silice en éluant avec un gradient d'un mélange dichlorométhane/méthanol/ammoniaque (99/1/0,1 à 95/5/0,5). On obtient 0,37 g de *trans* 2-(4-{4-[4-(5-Carbamoyl-adamantan-2-ylcarbamoyl)-3,4-dihydro-2H-quinoxalin-1-yl]-phenyl}-piperazin-1-yl)-2-methyl-propionic acid ethyl ester.
M+H⁺=629

### 15.4: trans-2-(4-{4-[4-(5-Carbamoyl-adamantan-2-ylcarbamoyl)-3,4-dihydro-2H-quinoxalin-1-yl]-phenyl}-piperazin-1-yl)-2-methyl-propionic acid

On place 0,15 g de *trans* 2-(4-{4-[4-(5-Carbamoyl-adamantan-2-ylcarbamoyl)-3,4-dihydro-2H-quinoxalin-1-yl]-phenyl}-piperazin-1-yl)-2-methyl-propionic acid ethyl ester. dans un ballon de 25 ml. On ajoute 2 ml de tetrahydrofurane et 1 ml d'éthanol. A 0°C, on additionne une solution de 0,031 g d'hydroxide de lithium dans 1 ml d'eau. Le mélange est agité pendant 24 heures à température ambiante puis est chauffé pendant 8 heures à 50°C. On évapore les solvants et on reprend à l'eau, puis on basifie au moyen d'une solution aqueuse 1 N de soude. On acidie ensuite jusqu'à pH=1 au moyen d'une solution à 6% de SO₂ dans l'eau. La phase aqueuse est extraite au dichlorométhane. La phase organique est séchée sur sulfate de sodium, filtrées sur fritté et évaporées à sec. Le brut obtenu est chromatographié sur gel de silice en éluant avec un gradient d'un mélange dichlorométhane/méthanol/ammoniaque (99/1/0,1 à 80/20). On obtient 0,01 g de *trans*-2-(4-{4-[4-(5-Carbamoyl-adamantan-2-ylcarbamoyl)-3,4-dihydro-2H-quinoxalin-1-yl]-phenyl}-piperazin-1-yl)-2-methyl-propionic acide après trituration dans l'éther.
M+H⁺ = 601 ; RMN ¹H (400 MHz, DMSO-d6) δ ppm: 7,29 (m, 1 H); 7,22-6,94 (m, 5H); 6,84 (m, 1H); 6,74-6,22 (m, 2H); 6,51 (m, 1H); 6 (m, 1H); 3,77 (m, 3H); 3,56-3,02 (m, 8H); 2,77 (m, 2H); 1,99 (m, 2H); 1,94-1,64 (m, 11 H); 1,47 (m, 2H); 1,27 (m, 6H).

### Exemple 16: trans-4-{4-[4-(2-Hydroxy-1,1-dimethyl-ethyl)-piperazin-1-yl]-phenyl}-3,4-dihydro-2H-quinoxaline-1-carboxylic acid (5-carbamoyl-adamantan-2-yl)-amide (composé n°152)

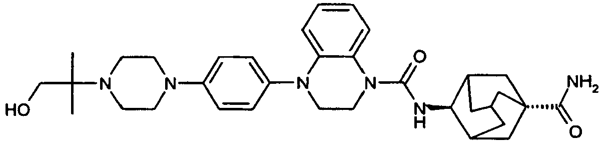

On place 0,19 g de l'intermédiaire 15.3 dans 3 ml de tetrahydrofurane, puis on ajoute à 0°C, 0,36 ml d'une solution 2N d'hydrure double de lithium et d'aluminium dans le tetrahydrofurane. Le milieu réactionnel est mis sous agitation pendant 2 h à une température de 0-5°C. On ajoute ensuite à 0°C, 1,5 ml d'une solution à 10% d'hydrogénosulfate de potassium dans l'eau et on laisse le milieu sous agitation à la même température pendant 10 minutes. La phase aqueuse est extraite à l'acétate d'éthyle. Les phases organiques sont régroupées, lavées à l'eau, séchées sur sulfate de sodium et concentrée sous pression réduite. Le brut obtenu est chromatographié sur gel de silice en éluant avec un gradient de solvant dichlorométhane/méthanol/ammoniaque de 99/1/0,1 à 95/5/0,5. On obtient 0,071 g de *trans*-4-{4-[4-(2-Hydroxy-1,1-dimethyl-ethyl)-piperazin-1-yl]-phenyl}-3,4-dihydro-2H-quinoxaline-1-carboxylic acid (5-carbamoyl-adamantan-2-yl)-amide après trituration dans l'éther.
Point de fusion = 194-200°C ; M+H⁺= 587 ; RMN ¹H (400 MHz, DMSO-d6) δ ppm 8,02 (s, 1 H); 7,45 (d, J = 8 Hz, 1 H); 7,36 (d, J = 8,4 Hz, 1 H); 7,10 (m, 2H); 7-6,82 (m, 3H); 6,68 (s, 1 H); 6,05 (d, J = 5,9 Hz, 1 H); 4,26 (m, 1 H); 3,79 (s, 4H); 3,75 (m, 1 H); 3,32 (m, 2H); 3,10 (m, 4H); 2,71 (m, 4H); 2,02-1,70 (m, 11H); 1,45 (m, 2H); 0,99 (s, 6H).

### Exemple 17: trans 4-[5-(4-Isobutyl-piperazin-1-yl)-pyridin-2-yl]-3,4-dihydro-2H-quinoxaline-1-carboxylic acid (5-carbamoyl-adamantan-2-yl)-amide (composé n°158)

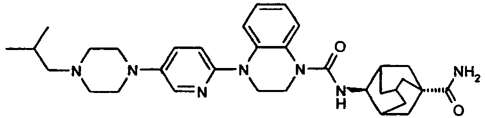

### 17.1 : 4-[5-(4-Isobutyl-piperazin-1-yl)-pyridin-2-yl]-3,4-dihydro-2H-quinoxaline-1-carboxylic acid tert-butyl ester

On place l'intermédiaire 14.3, (0,4 g, 1,01 mmol) dans 5 mL de dichlorométhane. On ajoute l'isobutyraldehyde (0,090 mL, 1,01 mmol) et le triacétoxyborohydrure de sodium (0,279 g, 1,31 mmol). Le milieu réactionnel est agité 18h à température ambiante sous azote. Après hydrolyse et extraction au dichlorométhane, la phase organique est séchée sur MgSO₄ et concentrée à sec. Le brut obtenu est chromatographié sur gel de silice en éluant avec un gradient de méthanol dans du dichlorométhane variant de 0% à 5%. On obtient 0,39 g de 4-[5-(4-Isobutyl-piperazin-1-yl)-pyridin-2-yl]-3,4-dihydro-2H-quinoxaline-1-carboxylic acid tert-butyl ester.
[M+H⁺ ] = 452

### 17.2 : 1-[5-(4-Isobutyl-piperazin-1-yl)-pyridin-2-yl]-1,2,3,4-tetrahydro-quinoxaline

On place le 4-[5-(4-Isobutyl-piperazin-1-yl)-pyridin-2-yl]-3,4-dihydro-2H-quinoxaline-1-carboxylic acid tert-butyl ester (0,39 g, 0,86 mmol) dans 1,7 mL de dichlorométhane puis on ajoute HCl 4N dans le dioxane (3,24 mL, 12,95 mmol). Le milieu réactionnel est agité 18h à température ambiante. Après hydrolyse avec une solution de soude aqueuse 1N jusqu'à pH 10, on extrait au dichlorométhane. La phase organique est lavée avec une solution aqueuse saturée en chlorure de sodium puis séchée sur MgSO₄ et concentrée à sec. Le brut obtenu est chromatographié sur gel de silice en éluant avec un gradient de méthanol dans du dichlorométhane variant de 0% à 10%. On obtient 0,27 g de 1-[5-(4-Isobutyl-piperazin-1-yl)-pyridin-2-yl]-1,2,3,4-tetrahydroquinoxaline.
[M+H⁺] = 352

### 17.3 : 4-[5-(4-Isobutyl-piperazin-1-yl)-pyridin-2-yl]-3,4-dihydro-2H-quinoxaline-1-carboxylic acid (5-carbamoyl-adamantan-2-yl)-amide

On place la 1-[5-(4-Isobutyl-piperazin-1-yl)-pyridin-2-yl]-1,2,3,4-tetrahydro-quinoxaline (0,25 g, 0,71 mmol) dans 7 mL de dichlorométhane à 0°C sous azote. On ajoute la triéthylamine (0,2 mL, 1,43 mmol) puis le triphosgène (0,084 g, 0,28 mmol). Le milieu réactionnel est agité 3h à température ambiante. On ajouté alors 7,1 mL de DMF, la diisopropylamine (0,31 mL, 1,78 mmol), puis le chlorhydrate du 4-Amino-adamantane-1-carboxylic acid amide (0,164 g, 0,71 mmol). Le milieu réactionnel est chauffé à 50°C pendant 18h soous azote. Après hydrolyse sur 50 mL de glace, et extraction à l'acétate d'éthyle, la phase organique est lavée à l'eau puis avec une solution aqueuse saturée en chlorure de sodium, séchée sur MgSO₄ et concentrée à sec. Le brut obtenu est chromatographié sur gel de silice en éluant avec un gradient de acétate d'éthyle/méthanol/ammoniaque dans du dichlorométhane variant de 100/0/0/0 à 7/2,5/0,5/0,5 (dichlorométhane/acétate d'éthyle/méthanol/ammoniaque). On obtient 0,236 g de 4-[5-(4-Isobutyl-piperazin-1-yl)-pyridin-2-yl]-3,4-dihydro-2H-quinoxaline-1-carboxylic acid (5-carbamoyl-adamantan-2-yl)-amide trituré ensuite dans le diéthyléther. Les cristaux obtenus sont essorés et séchés.
[M+H⁺ ] = 572 ; PF : 209 °C ; RMN ¹H (400 MHz, DMSO-d6) δ ppm 8,03 (d, J = 3 Hz, 1 H) ; 7,45 (dd, J = 8 Hz and 1,5 Hz, 1H) ; 7,38 (dd, J = 8,9 Hz et 3 Hz, 1 H); 7,10 (m, 2H); 6,97 (m, 1H); 6,94 (m, J = 7,6 Hz et 1,5 Hz, 1H); 6,86 (m, J = 7,6 Hz et 1,5 Hz, 1H); 6,69 (m, 1H); 6,06 (d, J = 6 Hz, 1H); 3,79 (s, 4H); 3,74 (m, 1H); 3,13 (m, 4H); 2,49 (m, 4H); 2,10 (d, J = 7,2 Hz, 2H); 1,99 (m, 2H); 1,91-1,71 (m, 10H); 1,45 (m, 2H); 0,89 (d, J = 6,5 Hz, 6H).

### Exemple 18: trans 4-(1'-tert-Butyl-1',2',3',4',5',6'-hexahydro-[3,4']bipyridinyl-6-yl)-3,4-dihydro-2H-quinoxaline-1-carboxylic acid (5-carbamoyl-adamantan-2-yl)-amide (composé n°170)

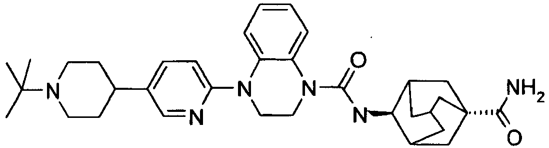

### 18.1 : 4-[5-(4,4,5,5-Tetramethyl-[1,3,2]dioxaborolan-2-yl)-pyridin-2-yl]-3,4-dihydro-2H-quinoxaline-1-carboxylic acid tert-butyl ester

On place le 4-(5-bromo-pyridin-2-yl)-3,4-dihydro-2H-quinoxaline-1-carboxylic acid tert-butyl ester (2 g, 5,12 mmol), 25 mL de DME. On ajoute le bis(pinacolato)diboron (1,56 g, 6,15 mmol), l'acétate de potassium (1,51 g, 15,4 mmol) et le dichlorodiphenylphosphinoferrocene palladium (0,418 g, 0,51 mmol). Le milieu réactionnel est chauffé au reflux pendant 2h. Après hydrolyse et addition d'acétate d'éthyle, le mélange est filtré sur célite®, la phase aqueuse est extraite à l'acétate d'éthyle, les phases organiques sont rassemblées, puis lavées avec une solution aqueuse saturée en chlorure de sodium, séchées sur MgSO₄ et concentrée à sec. Le brut obtenu est chromatographié sur gel de silice en éluant avec un gradient d'acétate d'éthyle dans l'heptane variant de 10% à 30%. On obtient 1,75 g de 4-[5-(4,4,5,5-tetramethyl-[1,3,2]dioxaborolan-2-yl)-pyridin-2-yl]-3,4-dihydro-2H-quinoxaline-1-carboxylic acid tert-butyl ester.
[M+H⁺] = 438

### 18.2 : 4-(1'-tert-Butyl-1',2',3',6'-tetrahydro-[3,4']bipyridinyl-6-yl)-3,4-dihydro-2H-quinoxaline-1-carboxylic acid tert-butyl ester

On place le 4-[5-(4,4,5,5-tetramethyl-[1,3,2]dioxaborolan-2-yl)-pyridin-2-yl]-3,4-dihydro-2H-quinoxaline-1-carboxylic acid tert-butyl ester (0,3 g, 0,69 mmol), le methanesulfonic acid 1-tert-butyl-1,2,3,6-tetrahydro-pyridin-4-yl ester (0,22 g, 0, 75 mmol), le tetrakistriphenylphosphine palladium (0,04 g, 0,03 mmol), et une solution aqueuse d'hydrogénocarbonate de sodium 2N (1,03 mL, 2,06 mmol) dans 2,3 mL de DME. Le milieu réactionnel est chauffé pendant 20 min au micro-onde Biotage® à 100°C. Après addition de 50 mL d'H₂O, extraction à l'acétate d'éthyle, la phase organique est lavée avec une solution aqueuse saturée en chlorure de sodium, séchée sur MgSO₄ et concentrée à sec. Le brut obtenu est chromatographié sur gel de silice en éluant avec un gradient de méthanol dans le dichlorométhane variant de 0% à 10%. On obtient 0,44 g de 4-(1'-tert-butyl-1',2',3',6'-tetrahydro-[3,4']bipyridinyl-6-yl)-3,4-dihydro-2H-quinoxaline-1-carboxylic acid tert-butyl ester.
[M+H⁺ ] = 448

### 18.3 : 4-(1'-tert-Butyl-1',2',3',4',5',6'-hexahydro-[3,4']bipyridinyl-6-yl)-3,4-dihydro-2H-quinoxaline-1-carboxylic acid tert-butyl ester

On place le 4-(1'-tert-butyl-1',2',3',6'-tetrahydro-[3,4']bipyridinyl-6-yl)-3,4-dihydro-2H-quinoxaline-1-carboxylic acid tert-butyl ester (0,44 g, 0,98 mmol) dans 60 mL de acétate d'éthyle dans une fiole pour appareil de Parr. On ajoute l'oxyde de platine (0,089 g, 0,39 mmol). Après agitation sous 32 psi d'H₂ pendant 4h, le catalyseur est filtré, rincé à l'acétate d'éthyle. Le filtrat est concentré à sec, puis agité à nouveau dans 60 mL d'acétate d'éthyle en présence de 90 mg d'oxyde platine sous 35 psi pendant 18h. L'opération est renouvelée pendant 5h puis 7h. Apès filtration du catalyseur et rinçage à l'acétate d'éthylen, le filtrat est concentré puis chromatographié sur gel de silice en éluant avec une gradient de méthanol/ammoniaque dans du dichlorométhane variant de 100/0/0 à 9/1/0,1 (dichlorométhane /méthanol/ammoniaque). On obtient 0,166 g de 4-(1'-tert-Butyl-1',2',3',4',5',6'-hexahydro-[3,4']bipyridinyl-6-yl)-3,4-dihydro-2H-quinoxaline-1-carboxylic acid tert-butyl ester.
[M+H⁺] = 451

### 18.4 : 1-(1'-tert-Butyl-1',2',3',4',5',6'-hexahydro-[3,4']bipyridinyl-6-yl)-1,2,3,4-tetrahydro-quinoxaline

On place le 4-(1'-tert-Butyl-1',2',3',4',5',6'-hexahydro-[3,4']bipyridinyl-6-yl)-3,4-dihydro-2H-quinoxaline-1-carboxylic acid tert-butyl ester (0,166 g, 0,37 mmol) dans 0,65 mL de dichlorométhane à 0°C puis on ajoute HCl 4N dans le dioxane (1,4mL, 5,59 mmol). Le milieu réactionnel est agité 18h à température ambiante. Après concentration à sec, le milieu réactionnel est hydrolysé avec 20 mL d'H₂O puis on ajoute K₂CO₃ jusqu'à pH 10. La phase aqueuse est extraite à l'acétate d'éthyle. Puis la phase organique est lavée avec une solution aqueuse saturée en chlorure de sodium puis séchée sur MgSO₄ et concentrée à sec. On obtient 0,12 g de 1-(1'-tert-Butyl-1',2',3',4',5',6'-hexahydro-[3,4']bipyridinyl-6-yl)-1,2,3,4-tetrahydro-quinoxaline.
[M+H⁺ ] = 351

### 18.5 : 4-(1'-tert-Butyl-1',2',3',4',5',6'-hexahydro-[3,4']bipyridinyl-6-yl)-3,4-dihydro-2H-quinoxaline-1-carboxylic acid (5-carbamoyl-adamantan-2-yl)-amide

On place le 1-(1'-tert-butyl-1',2',3',4',5',6'-hexahydro-[3,4']bipyridinyl-6-yl)-1,2,3,4-tetrahydro-quinoxaline (0,12 g, 0,35 mmol) dans 3,5 mL de dichorométhane à 0°C. On ajoute la triéthylamine (0,10 mL, 0,7 mmol) puis le triphosgène (0,041 g, 0,14 mmol). Le milieu réactionnel est agité 3h à température ambiante. On ajouté alors 3,5 mL de DMF, la diisopropylethylamine (0,15 mL, 0,87 mmol), puis le chlorhydrate du 4-amino-adamantane-1-carboxylic acid amide (0,08 g, 0,35 mmol). Le milieu réactionnel est chauffé à 50°C pendant 18h sous azote. Après hydrolyse sur 40 mL d'H₂O, et extraction à l'acétate d'éthyle, la phase organique est lavée à l'eau puis avec une solution aqueuse saturée en chlorure de sodium, séchée sur MgSO₄ et concentrée à sec. Le brut obtenu est chromatographié sur gel de silice en éluant avec un gradient de acétate d'éthyle/méthanol/ammoniaque dans du dichlorométhane variant de 100/0/0/0 à 7/2,5/0,5/0,5 (dichlorométhane/acétate d'éthyle/méthanol/ammoniaque). On obtient 0,09 g de 4-(1'-tert-butyl-1',2',3',4',5',6'-hexahydro-[3,4']bipyridinyl-6-yl)-3,4-dihydro-2H-quinoxaline-1-carboxylic acid (5-carbamoyl-adamantan-2-yl)-amide, trituré ensuite dans le diéthyléther. Les cristaux obtenus sont essorés et séchés.
[M+H⁺ ] = 571 ; PF : 228°C ; RMN ¹H (400 MHz, DMSO-d6) d ppm 8,16 (d, J = 2,5 Hz, 1H); 7,55 (m, 2H); 7,33 (m, 1H); 7,10 (d, J = 8,6 Hz, 2H); 7,02-6,94 (m, 3H); 6,68 (sl, 1H); 6,10 (d, J = 6 Hz, 1H); 3,91 (m, 2H); 3,81 (m, 2H); 3,74 (m, 1H); 3,11 (m, 1H); 2,44 (m, 1H); 2,13 (m, 2H); 1,98 (m, 2H); 1,93-1,71 (m, 10H); 1,59 (m, 2H); 1,44 (m, 2H); 1,06 (s, 9H).

### Exemple 19: trans 4-[5-(4-Cyclopropanesulfonyl-piperazin-1-yl)-pyridin-2-yl]-3,4-dihydro-2H-quinoxaline-1-carboxylic acid (5-cyano-adamantan-2-yl)-amide (composé n°165)

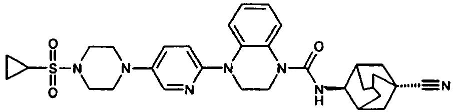

### 19.1 4-[5-(4-Cyclopropylsulfonyl-piperazin-1-yl)-pyridin-2-yl]-3,4-dihydro-2H-quinoxaline-1-carboxylic acid tert-butyl ester

On place 10 g de 4-(5-Piperazin-1-yl-pyridin-2-yl)-3,4-dihydro-2H-quinoxaline-1-carboxylic acid tert-butyl ester dans un ballon de 500 ml. On ajoute 126 ml de dichlorométhane puis 4,23 ml de triéthylamine et enfin 2,7 ml de chlorocyclopropylsulfonyle. Le mélange réactionnel est agité 16 h à température ambiante puis, lavé à l'eau et une fois par une solution saturé en chlorure de sodium, séché sur sulfate de magnésium puis évaporé sous pression réduite. Le brut obtenu est chromatographié sur gel de silice en éluant avec un gradient de méthanol dans le dichlorométhane variant de 0% à 10%. On obtient On obtient 10,9 g de 4-[5-(4-cyclopropylsulfonyl-piperazin-1-yl)-pyridin-2-yl]-3,4-dihydro-2H-quinoxaline-1-carboxylic acid tert-butyl ester
M+H⁺= 500,6

### 19.2 4-[5-(4-Cyclopropylsulfonyl-piperazin-1-yl)-pyridin-2-yl]-3,4-dihydro-2H-quinoxaline

On place 10,3 g de 4-[5-(4-cyclopropylsulfonyi-piperazin-1-yl)-pyridin-2-yl]-3,4-dihydro-2H-quinoxaline-1-carboxylic acid tert-butyl ester dans 204 ml d'une solution d'acide chlorhydrique 4N dans le dioxane. On agite 1,5 h à température ambiante. Le mélange réactionnel est ensuite évaporé sous pression réduite, puis on ajoute une solution aqueuse saturée en hydrogenocarbonate de sodium. La phase aqueuse est extraite trois fois au dichlorométhane. Les phases organiques sont réunies, séchées sur sulfate de sodium et concentrées sous vide. On obtient 7,96 g de 4-[5-(4-Cyclopropylsulfonyl-piperazin-1-yl)-pyridin-2-yl]-3,4-dihydro-2H-quinoxaline.
M+H⁺=400,5

### 19.3 trans 4-[5-(4-Cyclopropanesulfonyl-piperazin-1-yl)-pyridin-2-yl]-3,4-dihydro-2H-quinoxaline-1-carboxylic acid (5-carbamoyl-adamantan-2-yl)-amide

On place 0,39 g de 4-[5-(4-cyclopropylsulfonyl-piperazin-1-yl)-pyridin-2-yl]-3,4-dihydro-2H-quinoxaline dans 6,1 ml de dichlorométhane et 0,54 ml de triéthylamine. A 0°C, on ajoute ensuite 0,12 g de triphosgène. On agite 2h à température ambiante, puis on lave le milieu réactionnel avec une solution aqueuse saturée en hydrogénocarbonate de sodium. On sèche la phase organique sur sulfate de sodium et on concentre sous vide. Le brut réactionnel est dilué par 8 mL de diméthylformamide, puis on ajoute 0,34 ml de triéthylamine ainsi que 0,25 g du chlorhydrate de *trans* 4-Amino-adamantane-1-carboxylic acide amide. On agite 16 h à température ambiante, puis on ajoute de l'eau et de l'acétate d'éthyle. La phase organique est lavée trois fois à l'eau, une fois avec une solution aqueuse saturée en hydrogénocarbonate de sodium, séchées sur sulfate de sodium et concentrées sous vide. Le brut obtenu est chromatographié sur gel de silice en éluant avec un gradient d'un mélange dichlorométhane/acétone/méthanol (88/10/2 à 70/25/5). On obtient 0,34 g de *trans* 4-[5-(4-Cyclopropanesulfonyl-piperazin-1-yl)-pyridin-2-yl]-3,4-dihydro-2H-quinoxaline-1-carboxylic acid (5-carbamoyl-adamantan-2-yl)-amide après trituration dans un mélange d'acétate d'éthyle et d'éther éthylique.
M+H⁺=620,8

### 19.4 trans 4-[5-(4-Cyclopropanesulfonyl-piperazin-1-yl)-pyridin-2-yl]-3,4-dihydro-2H-quinoxaline-1-carboxylic acid (5-cyano-adamantan-2-yl)-amide

On place 0,1g de l'intermédiaire 19.3 dans 0,85 mL de dichlorométhane à 0°C. On ajoute 0,04 ml de triéthylamine puis 0,03 ml d'anhydride trifluoracétique. Le milieu réactionnel est agité 2h20 à 0°C. On hydrolyse le milieu réactionnel avec une solution saturée aqueuse d'hydrogénocarbonnate de sodium, puis on ajoute du dichlorométhane. La phase aqueuse est extraite trois fois au dichlorométhane, puis la phase organique est séchée sur MgSO₄ et concentrée à sec. Le brut obtenu est chromatographié sur gel de silice en éluant avec un gradient de méthanol dans du dichlorométhane variant de 100/0 à 90/10 (dichlorométhane/méthanol). On obtient 0,045 g de *trans* 4-[5-(4-Cyclopropanesulfonyl-piperazin-1-yl)-pyridin-2-yl]-3,4-dihydro-2H-quinoxaline-1-carboxylic acid (5-cyano-adamantan-2-yl)-amide
[M+H⁺ ] = 602 ; PF : 188°C ; RMN ¹H (400 MHz, DMSO-d6) δ d ppm 8,07 (m, 1 H); 7,47 (m, J = 8 Hz, 1 H); 7,42 (m, J = 9,2 Hz, 1 H); 7,14 (m, 2H); 6,94 (m, 1 H); 6,87 (m, 1H); 6,16 (d, J = 5,7 Hz, 1H); 3,87-3,74 (m, 5H); 3,41-3,16 (m, 8H); 2,67 (m, 1H); 2,13-1,94 (m, 8H); 1,90 (sl, 1H); 1,81 (m, 2H); 1,52 (m, 2H); 1,08-0,93 (m, 4H).

### Exemple 20: trans 4-{5-[4-(2-Methanesulfonyl-ethyl)-piperazin-1-yl]-pyridin-2-yl}-3,4-dihydro-2H-quinoxaline-1-carboxylic acid (5-carbamoyl-adamantan-2-yl)-amide (composé n°178)

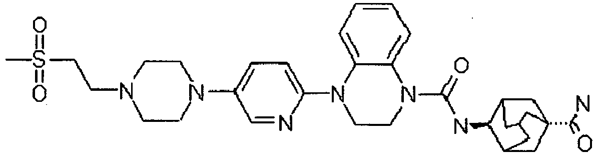

### 20.1 : 4-{5-[4-(2-Methanesulfonyl-ethyl)-piperazin-1-yl]-pyridin-2-yl}-3,4-dihydro-2H-quinoxaline-1-carboxylic acid tert-butyl ester

On place l'intermédiaire 14.3 (0,3 g, 0,76mmol) dans 7,5 mL de méthanol. On ajoute la méthylvinylsulfone (0,17 mL, 1,94 mmol). Le milieu réactionnel est agité 18h à température ambiante sous azote. Le milieu réactionnel est concentré à sec, puis chromatographié sur gel de silice en éluant avec un gradient de méthanol dans du dichlorométhane, variant de 1% à 10%. On obtient 0,38 g de 4-{5-[4-(2-Methanesulfonyl-ethyl)-piperazin-1-yl]-pyridin-2-yl}-3,4-dihydro-2H-quinoxaline-1-carboxylic acid tert-butyl ester.
[M+H⁺] = 502

### 20.2 : 1-{5-[4-(2-Methanesulfonyl-ethyl)-piperazin-1-yl]-pyridin-2-yl}-1,2,3,4-tetrahydro-quinoxaline

On place le 4-{5-[4-(2-Methanesulfonyl-ethyl)-piperazin-1-yl]-pyridin-2-yl}-3,4-dihydro-2H-quinoxaline-1-carboxylic acid tert-butyl ester (0,38 g, 0,76 mmol) dans 5 mL de dioxane, puis on ajoute HCl 4N dans le dioxane (2,84 mL, 11,36 mmol). Le milieu réactionnel est agité 18h à température ambiante. On ajoute à nouveau HCl 4N dans le dioxane (2,84 mL, 11,36 mmol) et le milieu réactionnel est agité 18h supplémentaires à température ambiante. Après concentration à sec, le milieu réactionnel est dilué par 50 mL d'HCl 1 N dans l'eau et extrait par 100 mL d'éther diéthylique. La phase organique est relavée par 50 mL d'HCl 1 N dans l'eau. Les phases aqueuses sont combinées, basifiées par K₂CO₃ en poudre jusqu'à pH 10, extraites 3 fois par 50 mL de dichlorométhane. Les phases organiques résultantes sont combinées, lavées par une solution aqueuse saturée en chlorure de sodium, séchées sur MgSO₄ et concentrées à sec. On obtient 0,3 g de 1-{5-[4-(2-Methanesulfonyl-ethyl)-piperazin-1-yl]-pyridin-2-yl}-1,2,3,4-tetrahydro-quinoxaline.
[M+H⁺] = 402

### 20.3 : trans 4-{5-[4-(2-Methanesulfonyl-ethyl)-piperazin-1-yl]-pyridin-2-yl}-3,4-dihydro-2H-quinoxaline-1-carboxylic acid (5-carbamoyl-adamantan-2-yl)-amide

On place le 1-{5-[4-(2-Methanesulfonyl-ethyl)-piperazin-1-yl]-pyridin-2-yl}-1,2,3,4-tetrahydro-quinoxaline (0,3 g, 0,75 mmol) dans 5mL de dichlorométhane à 0°C. On ajoute la triéthylamine (0,21 mL, 1,50 mmol) puis le triphosgène (0,09 g, 0,3 mmol). Le milieu réactionnel est agité 30min sous azote à 0°C, puis 3 heures à température ambiante. On ajoute alors le chlorhydrate du 4-amino-adamantane-1-carboxylic acid amide (0,19 g, 0,82 mmol), la triéthylamine (0,26 mL, 1,89 mmol) et 5 mL de DMF. Le milieu réactionnel est agité 18h à température ambiante sous azote. Après hydrolyse avec 100 ml d'H₂O, le milieu est extrait 2 fois par 50 mL de dichlorométhane. Les phases organiques sont combinées, lavées 2 fois par 100 mL d'H₂O, puis par 100 mL d'une solution aqueuse saturée en chlorure de sodium, séchées sur MgSO₄ et concentrées à sec. Le brut obtenu est chromatographié sur gel de silice en éluant avec un gradient de méthanol dans du dichlorométhane variant de 1% à 10%. Après trituration dans l'éther diéthylique, filtration et séchage, on obtient 0,23 g de *trans* 4-{5-[4-(2-Methanesulfonyl-ethyl)-piperazin-1-yl]-pyridin-2-yl}-3,4-dihydro-2H-quinoxaline-1-carboxylic acid (5-carbamoyl-adamantan-2-yl)-amide.
[M+H⁺] = 622 ; Pf = 204°C ; RMN ¹H (400 MHz, DMSO-d6) δ ppm 8,05 (s, 1 H); 7,45 (d, J = 8,2 Hz, 1H); 7,40 (d, J = 9 Hz, 1 H); 7,11 (d, J = 8,4 Hz, 2H); 7-6,82 (m, 3H); 6,68 (s, 1H); 6,06 (d, J = 5,8 Hz, 1H); 3,79 (s, 4H); 3,75 (sl, 1H); 3,75 (m, 2H); 3,15 (m, 4H); 3,06 (s, 3H); 2.78 (broad triplet, J = 6 Hz, 2H); 2,61 (m, 4H); 2,03-1,70 (m, 11 H); 1,45 (m, 2H).

Le tableau qui suit illustre les structures chimiques et les propriétés physiques de quelques composés selon l'invention, répondant à la formule (I bis), dans laquelle R_{1b} et R_{2b} représentent des atomes d'hydrogène, et se présentant sous forme de bases libres (composés non salifiés), sauf pour le composé N°155 qui se présente sous forme d'un chlorhydrate équimolaire. Dans ce tableau :
- dans la colonne « A », « - » représente une liaison simple ;
- Me représente un groupe méthyle ;
- Et représente un groupe éthyle ;
- iPr représente un groupe iso-propyle ;
- tBu représente un groupe tert-butyle ;
- Ph représente un groupe phényle ;
- PF représente le point de fusion du composé, exprimé en degrés Celsius ;
- M+H⁺ représente la masse du composé, obtenue par LC-MS (Liquid Chromatography
- Mass Spectroscopy).

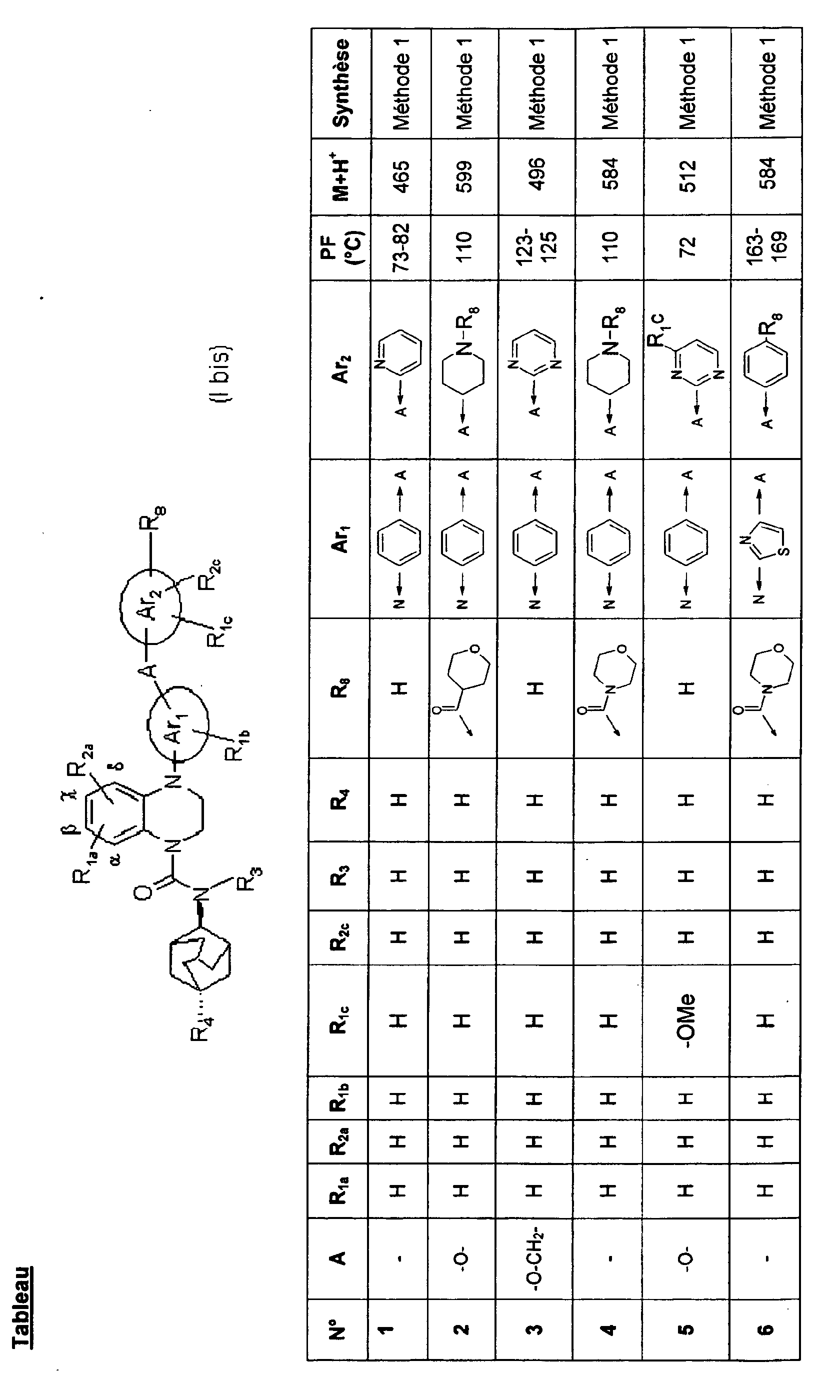

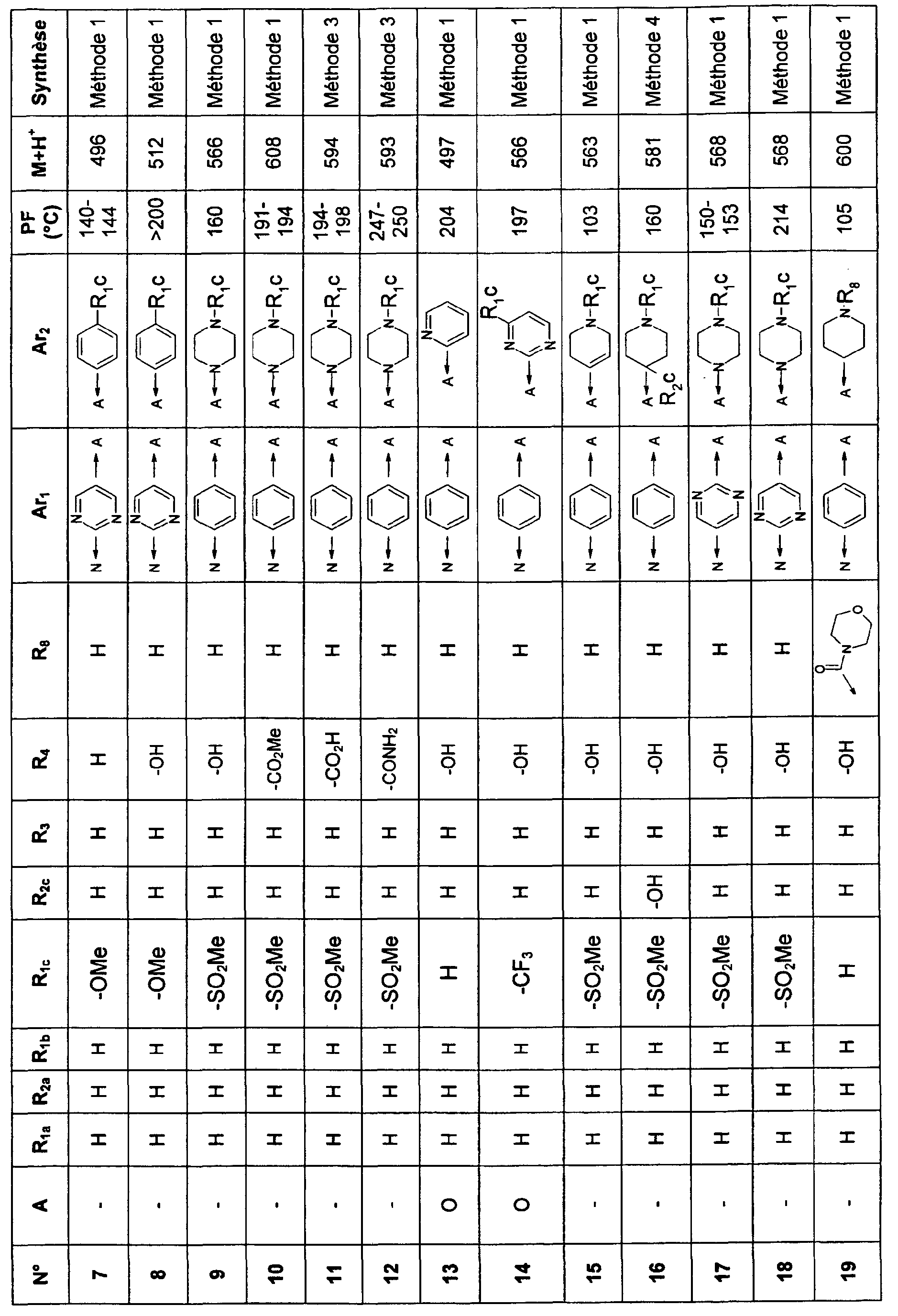

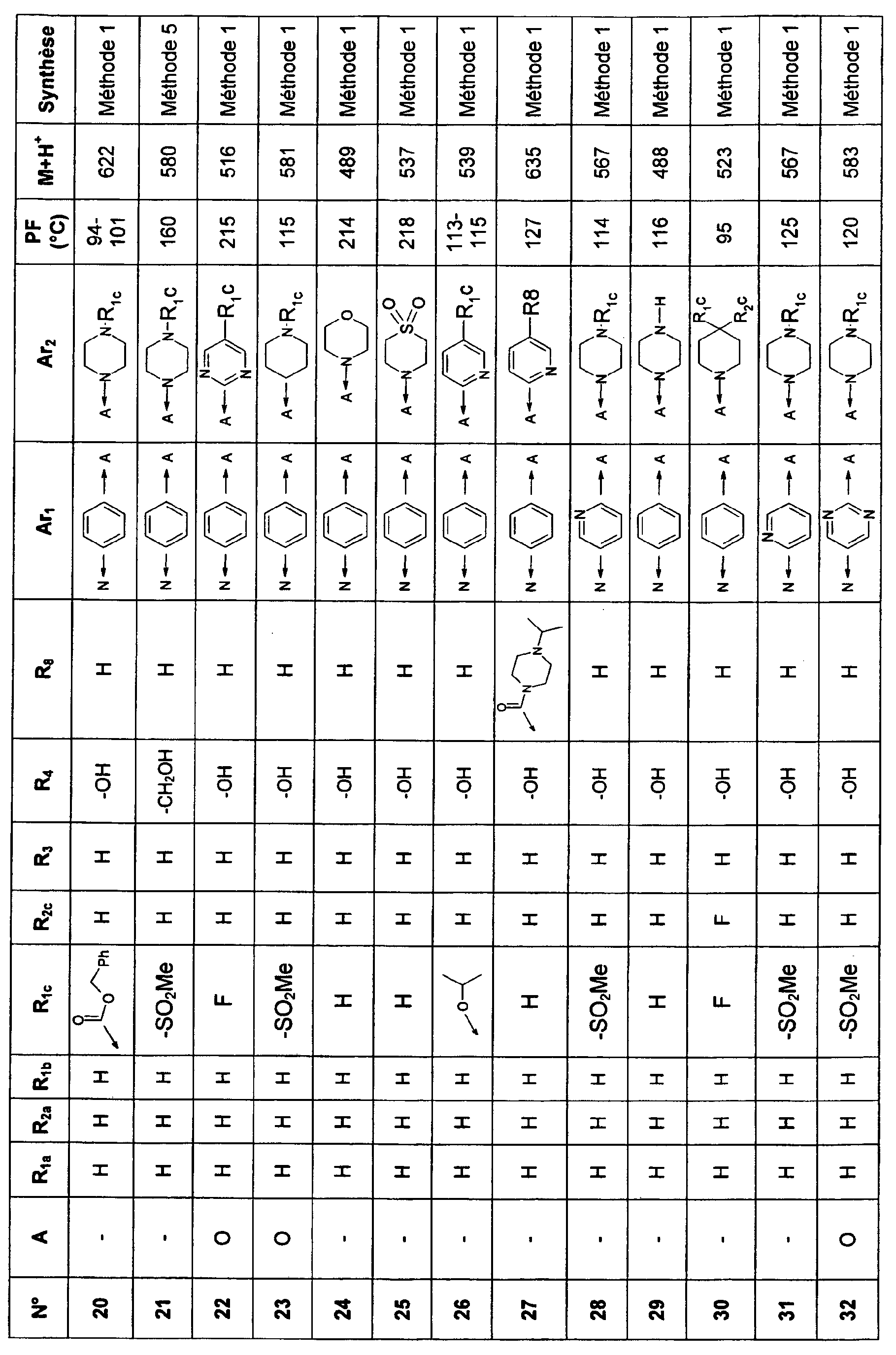

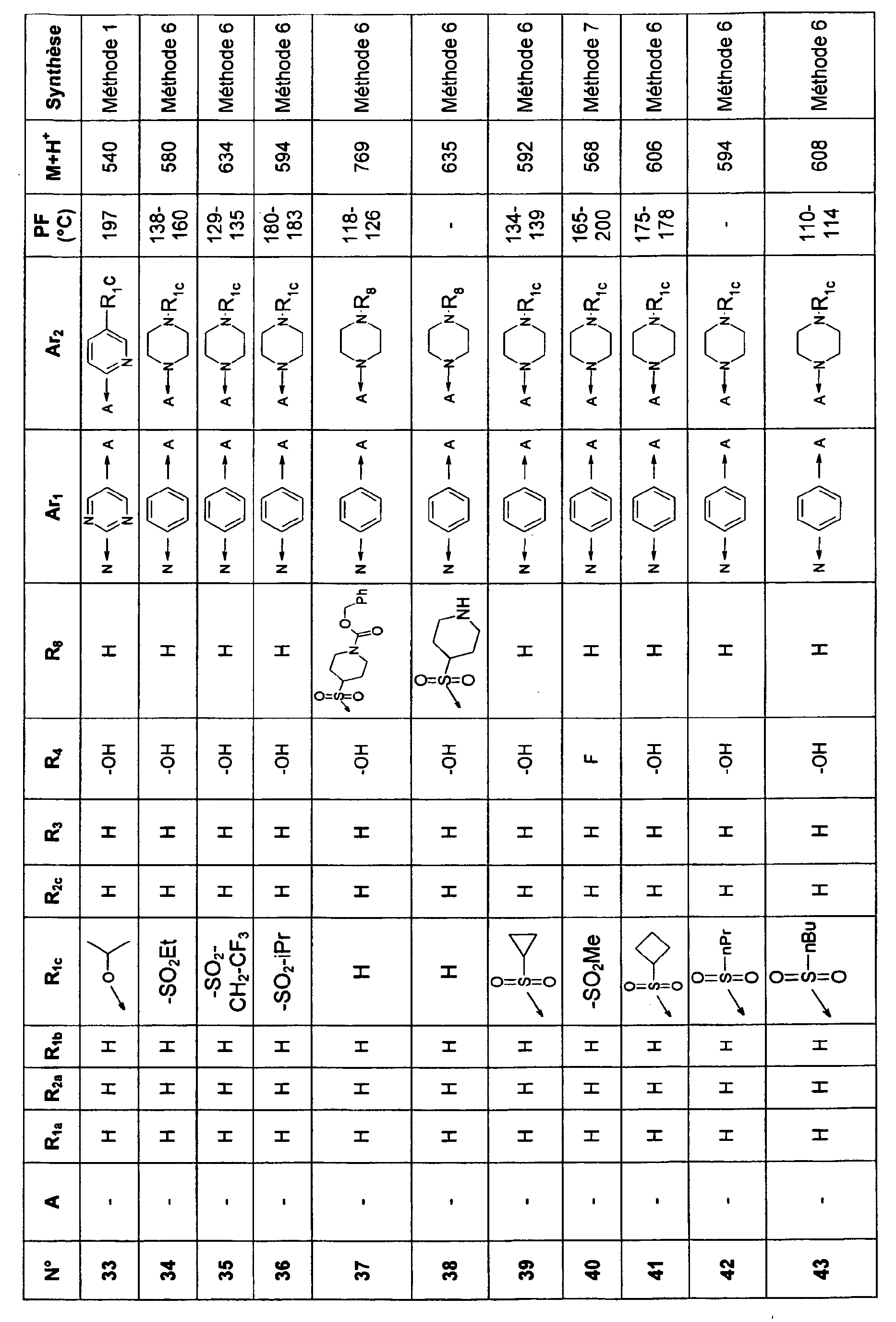

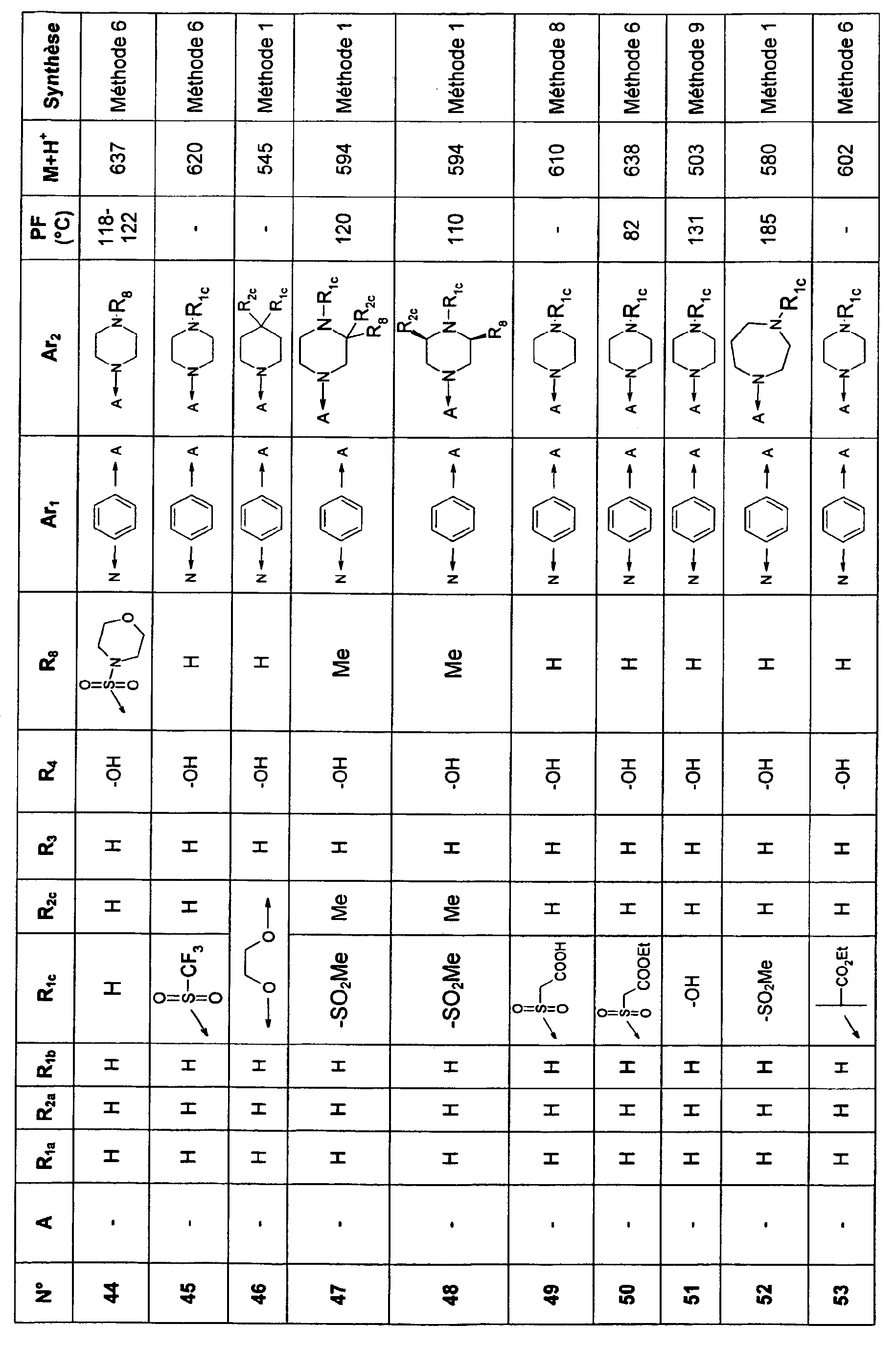

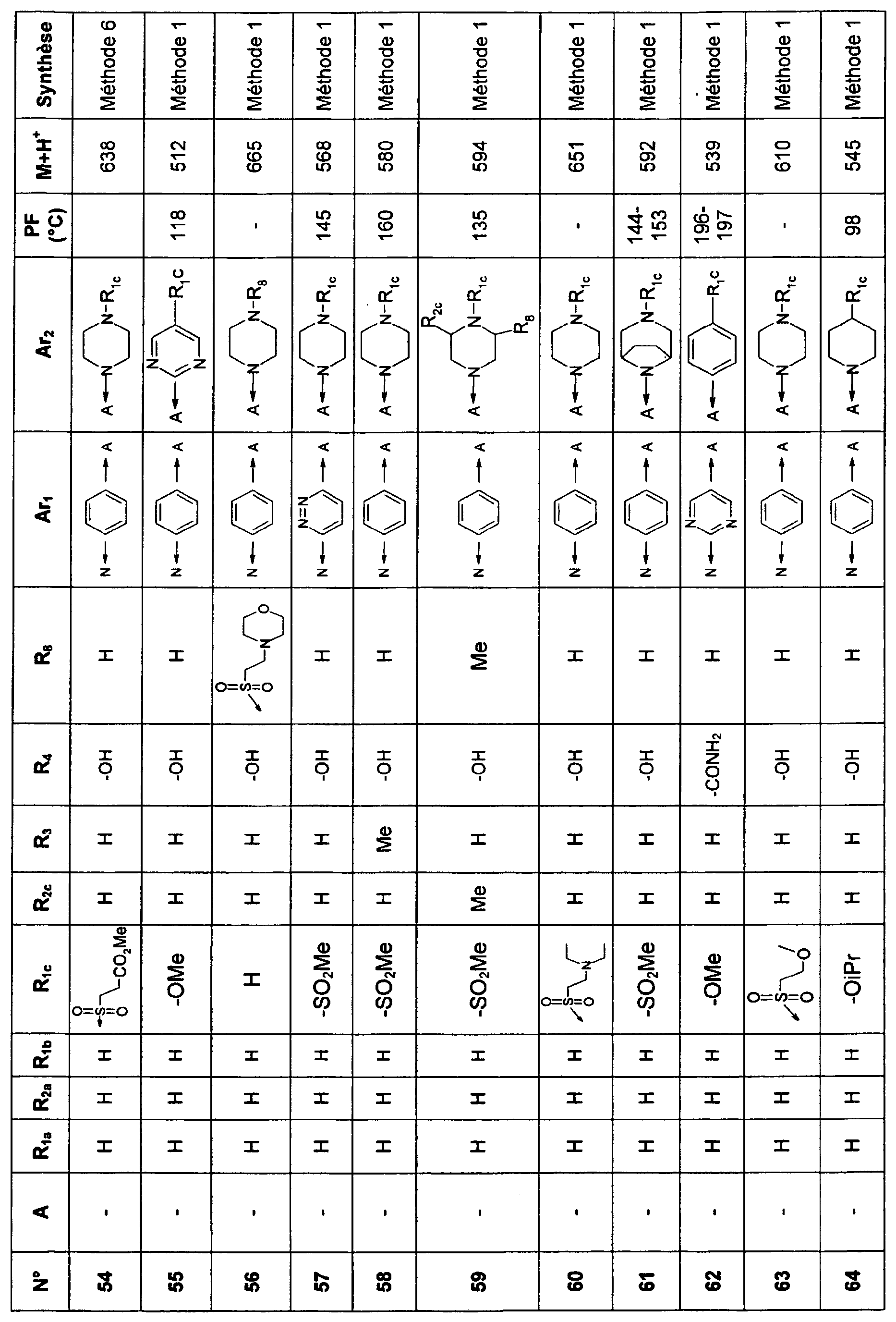

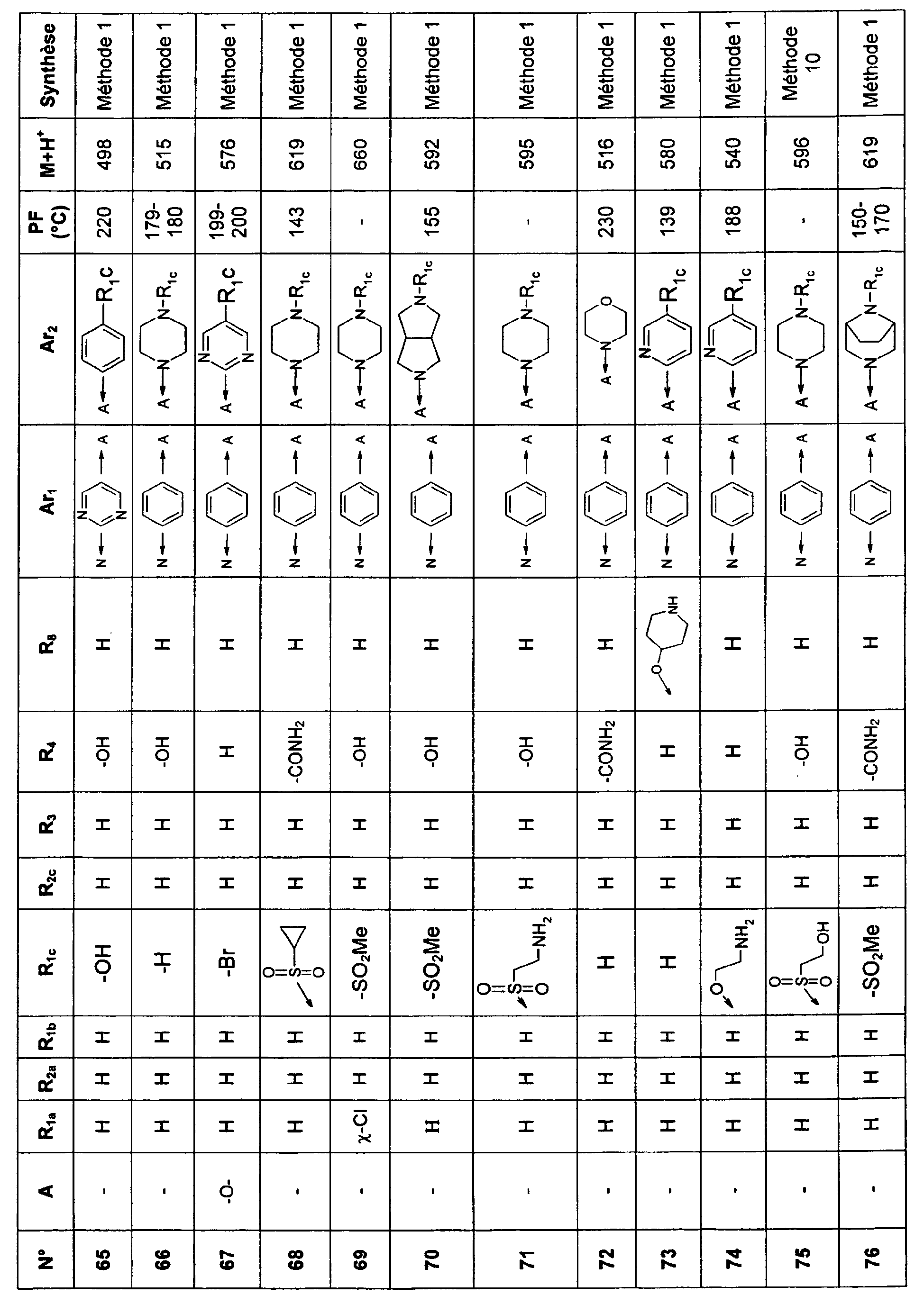

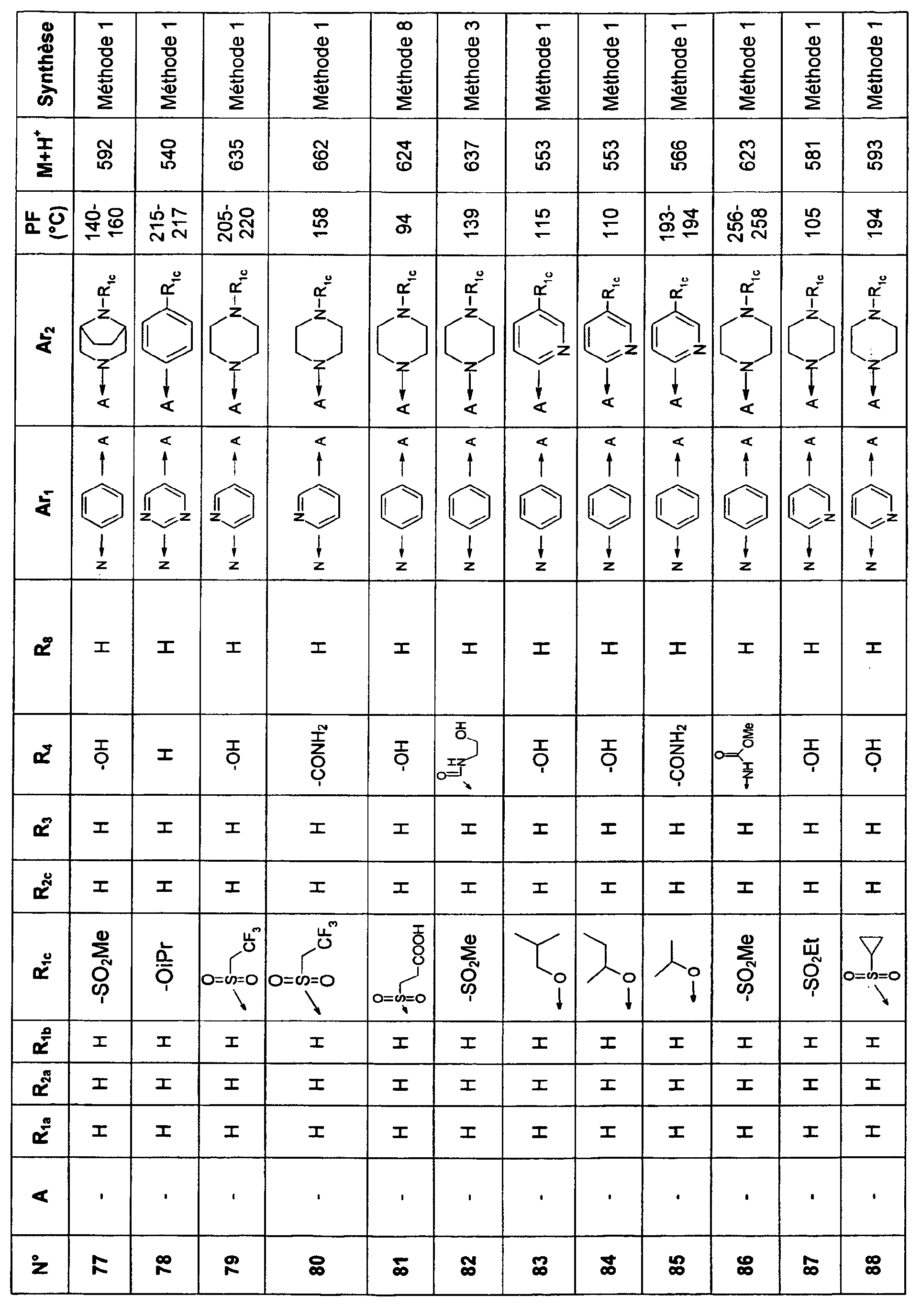

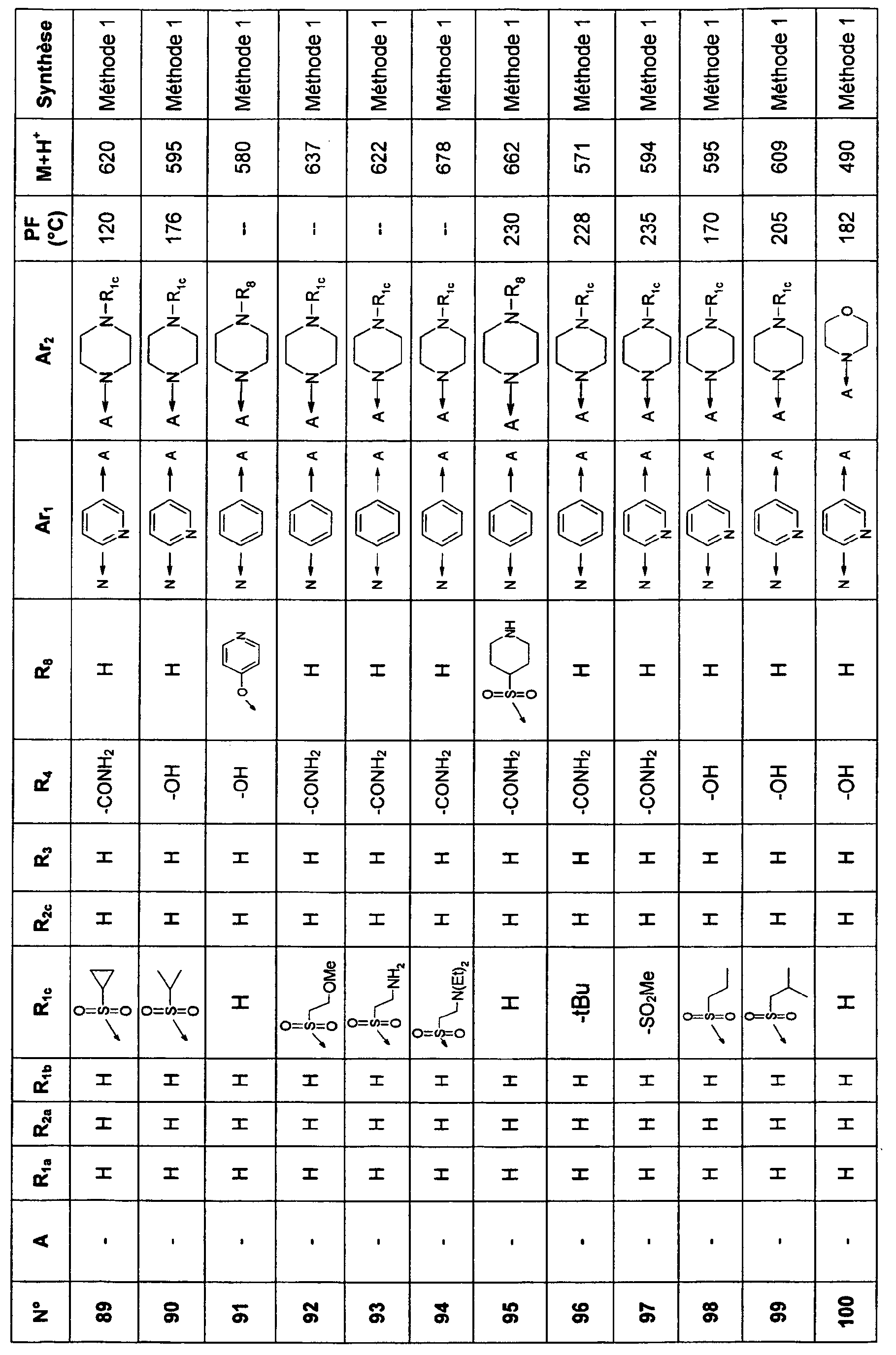

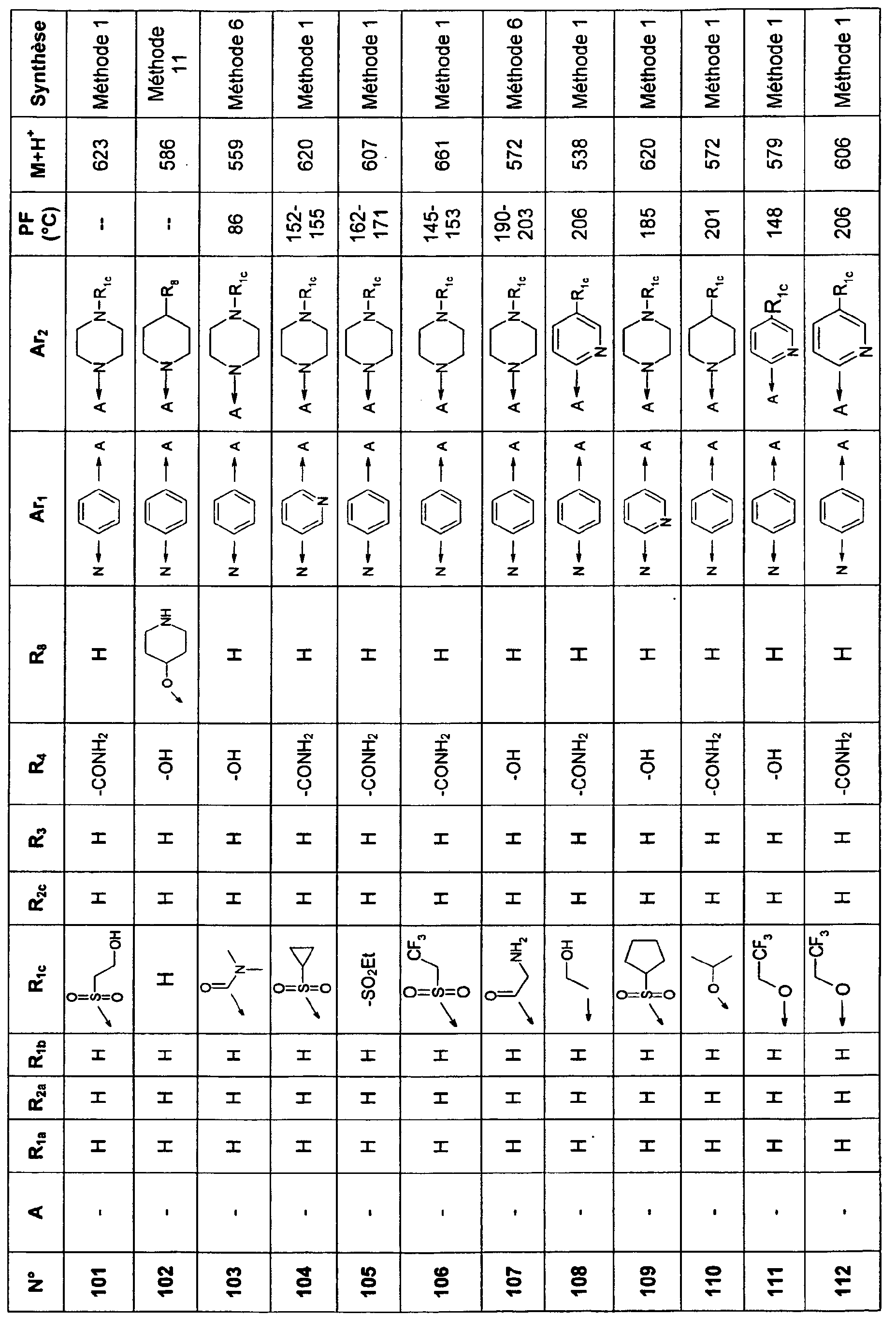

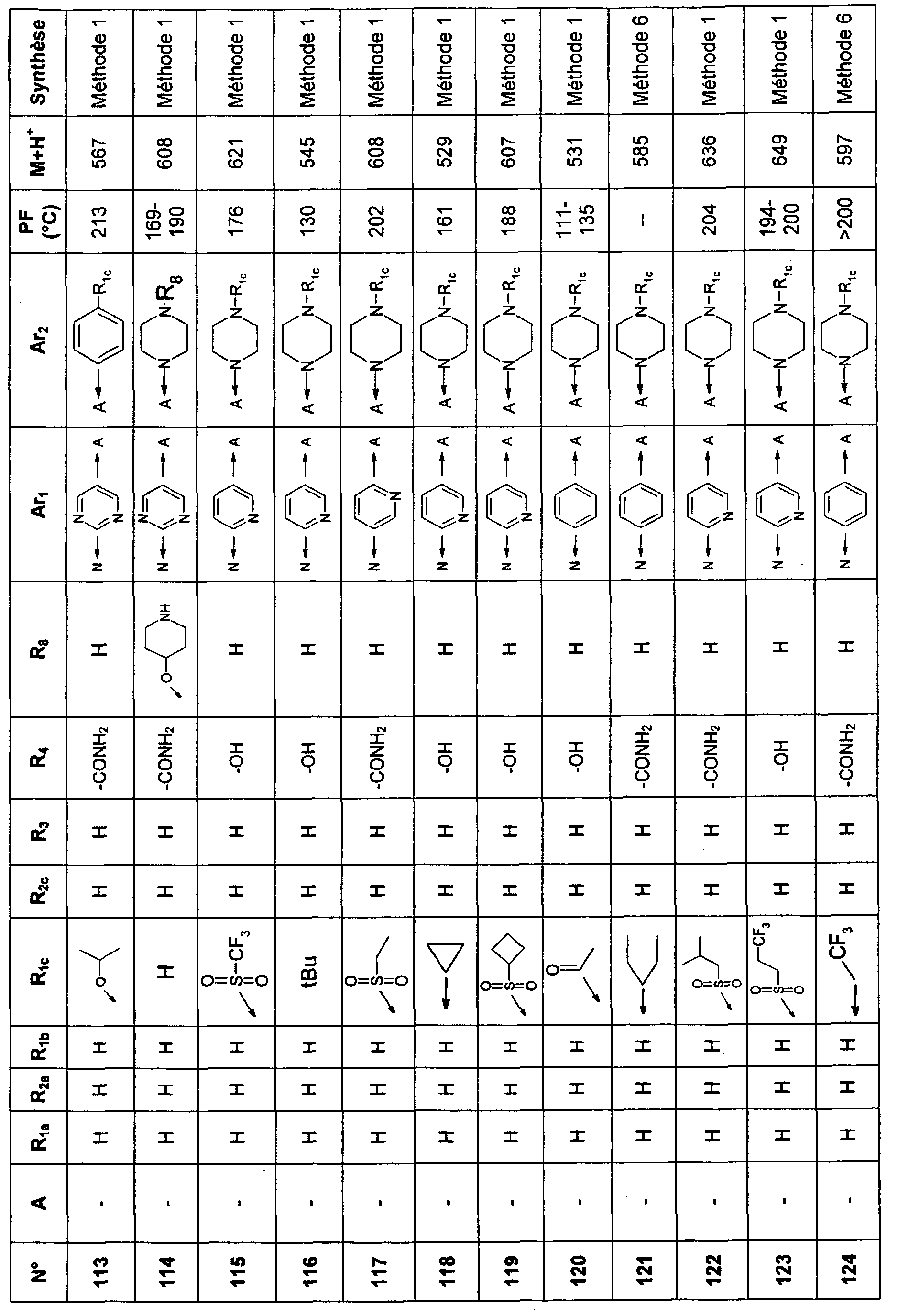

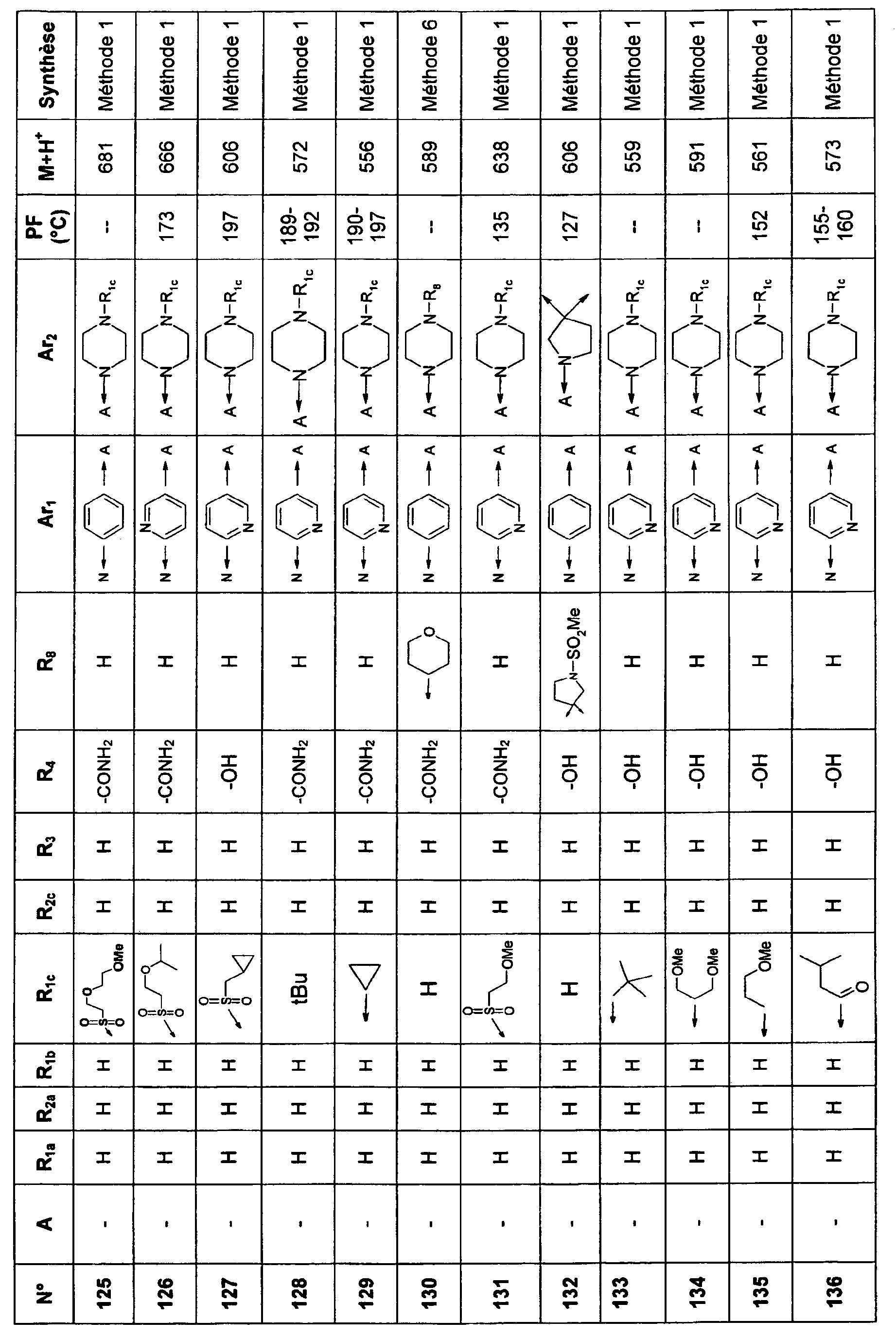

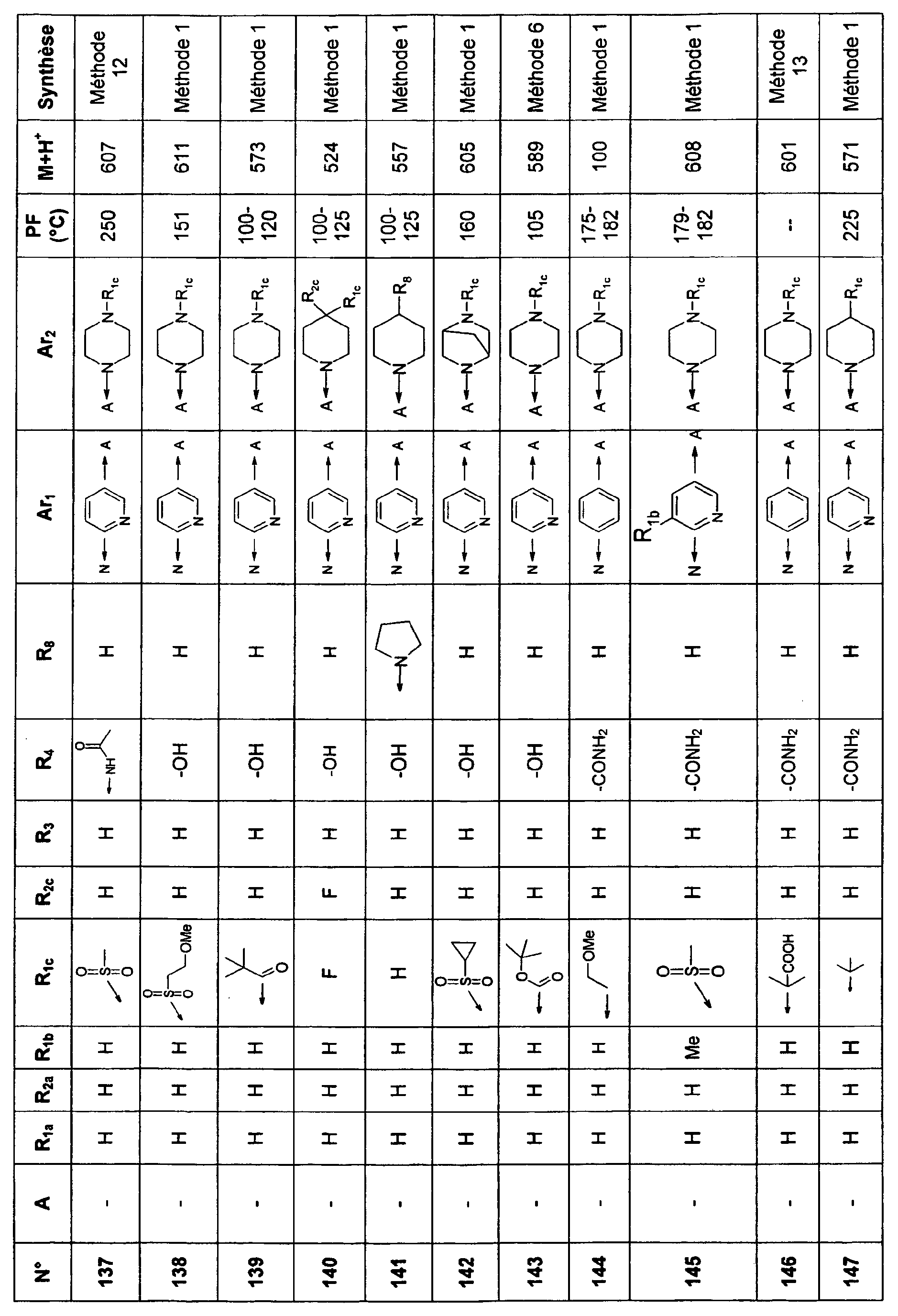

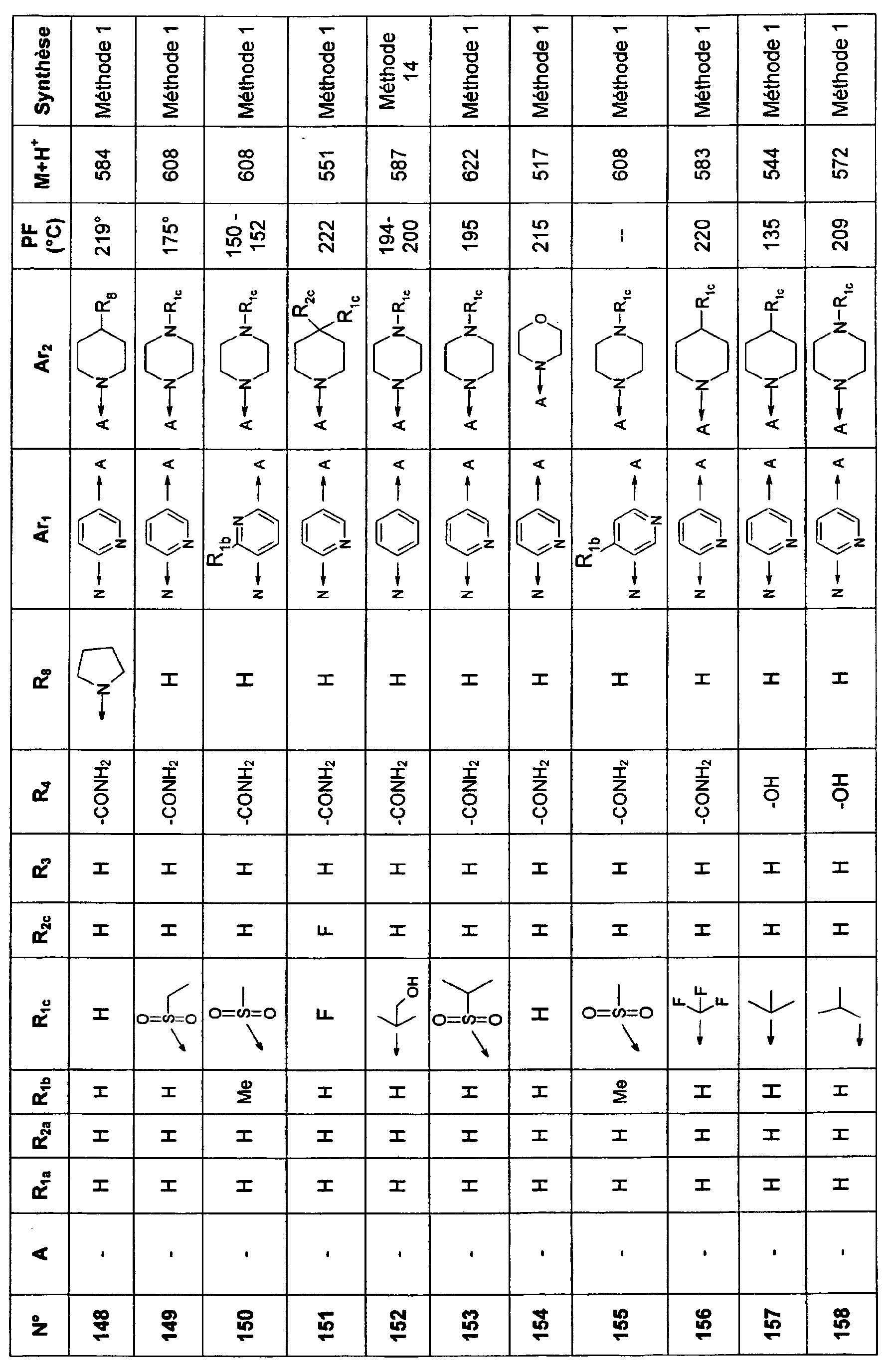

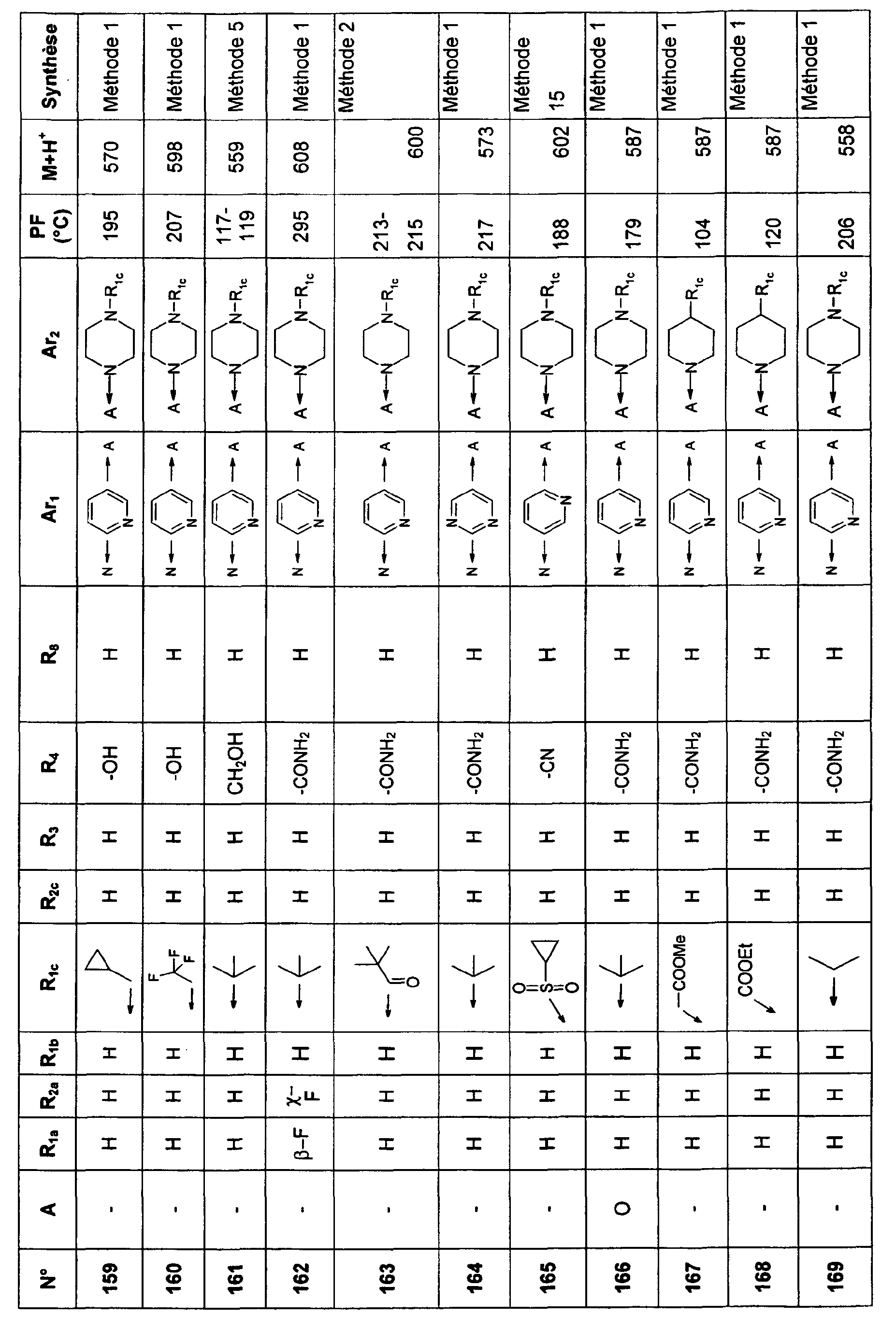

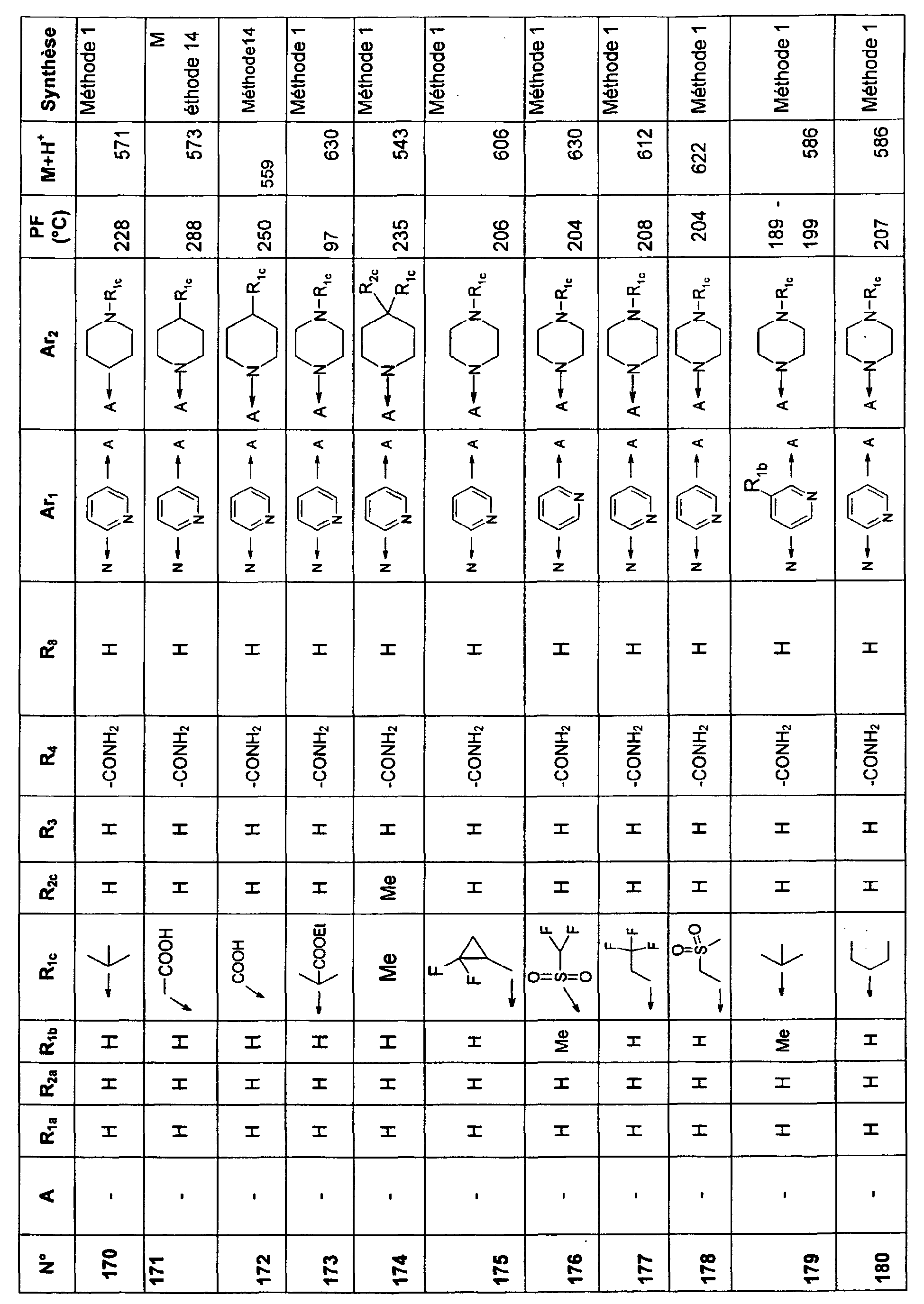

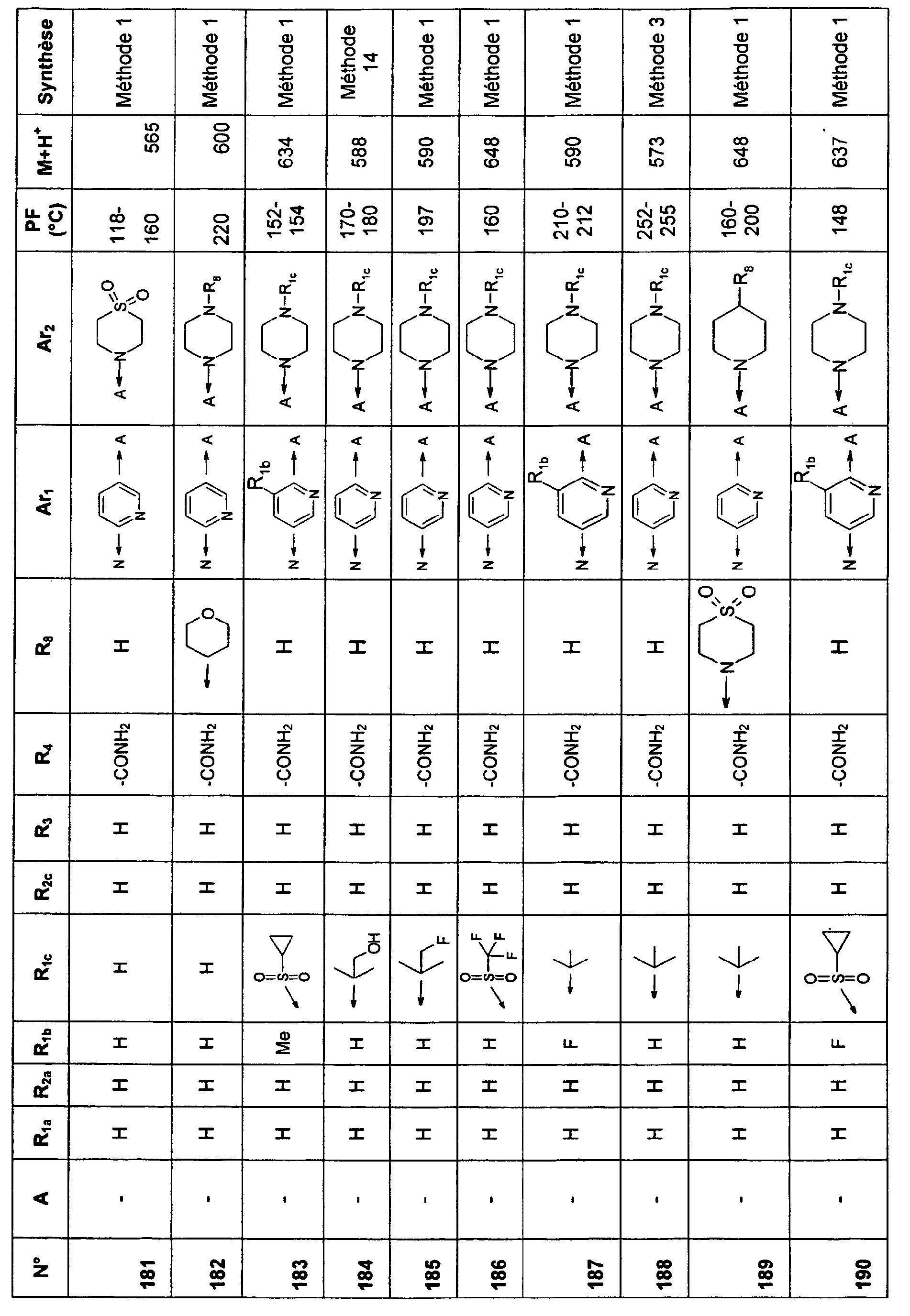

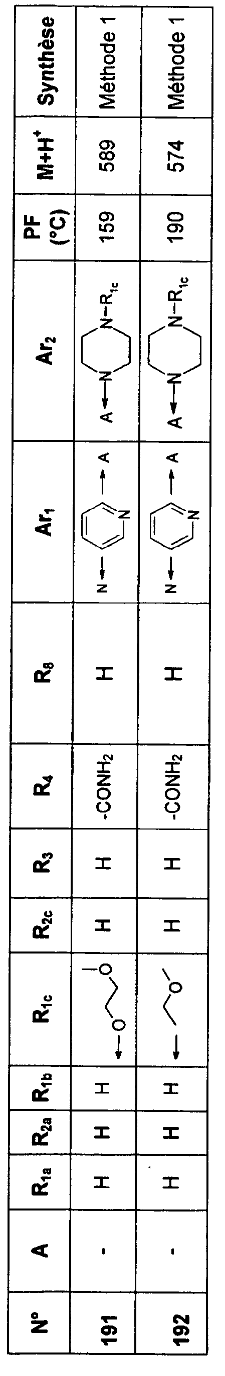

Les composés selon l'invention ont fait l'objet d'essais pharmacologiques permettant de déterminer leur effet inhibiteur de l'enzyme 11 beta-HSD1, qui intervient dans le métabolisme des lipides et le métabolisme du glucose.
Ces essais ont consisté à mesurer l'activité inhibitrice *in vitro* des composés de l'invention sur l'enzyme 11 beta-HSD1 grâce à un test SPA (Scintillation Proximity Assay) en format 384 puits, La protéine 11 beta-HSD1 recombinante a été produite en levure *S,cerevisiae,* La réaction a été réalisée en incubant l'enzyme en présence de ³H-cortisone et de NADPH, en absence ou en présence de concentration croissante d'inhibiteur, Des billes SPA couplées à un anticorps anti-souris, préincubées avec un anticorps anti-cortisol, ont permis de mesurer la quantité de cortisol formé au cours de la réaction.
L'activité inhibitrice vis-à-vis de l'enzyme 11 beta-HSD1 est donnée par la concentration qui inhibe 50% de l'activité de 11 beta-HSD1 (CI₅₀).
Les CI₅₀ des composés selon l'invention sont inférieures à 1 µM, Par exemple, les CI₅₀ des composés n° 3, 6, 9, 14, 15, 16, 27, 30, 31, 85, 89, 95, 96, 118, 119, 125, 128, 132 et 137 sont respectivement de 0,405 ; 0,624 ; 0,052 ; 0,028 ; 0,049 ; 0,028 ; 0,017; 0,094; 0,023; 0,007; 0,004; 0,008; 0,019; 0,008; 0,007, 0,007; 0,004; 0,187 et 0,130 µM.

Afin d'évaluer les propriétés pharmaco-dynamiques d'inhibiteurs de 11-beta HSD1, un test ex vivo permettant de mesurer l'activité de cette enzyme dans des tissus animaux intacts, a été utilisé.Le composé à évaluer est administré oralement à des souris C57bl/6J à la dose de 10 mg/kg. 16h après le traitement, les animaux sont euthanasiés par dislocation cervicale. 50 mg de foie et 200 mg de tissu adipeux sous-cutané sont prélevés sur chaque animal et placés sur de la glace en plaque 24 puits, dans 500 µl de PBS contenant 2 mM d'EDTA et des inhibiteurs de protéases. Les tissus sont ensuite coupés au ciseau en morceaux d'environ 3 mm³, puis pré-incubés à 37 °C pendant 10 minutes. Il est ensuite ajouté par puits, 500 µL de PBS additionné de 2 mM d'EDTA, d'inhibiteurs de protéases, et de 1,5 µCi de H³-Cortisone (à 50Ci/mmol). Les tissus sont ainsi incubés à 37°C sous agitation douce en présence de substrat radiomarqué pendant 15 minutes dans le cas des foies, ou 2h dans le cas des tissus adipeux. La réaction enzymatique est arrêtée par ajout de 500 µL de méthanol/HCl 1N (1 :1). Le surnageant de réaction est collecté et congelé sur carboglace, puis centrifugé (20 minutes à 20000 g) après décongélation afin d'éliminer le maximum de débris cellulaires. Les échantillons sont filtrés (sur filtres Acroprep 24 type GHP0.2µm) avant analyse sur chaine HPLC (Alliance 2695 de Waters), couplée à un détecteur de radioactivité (Radioamatic 625TR de perkin Elmer). L'analyse est réalisée sur 100 µl d'échantillon injecté sur colonne C18 (4,6x75 ; 3,5 µm SUNFIRE de Waters), puis élué par un solvant isocratique composé de H₂O/acide trifluoroacétique/méthanol (44,95/0,05/55), pendant 8 minutes au débit de 1 ml/minute. Les temps de rétention des pics cortisone et cortisol sont respectivement de 3,5 et 4,5 minutes dans ces conditions. Le logiciel Empower Pro2 développé par Waters a été utilisé pour l'analyse des chromatogrammes. Les pics de traceur radiomarqué sont convertis en « pmol de cortisol formé/ µg de tissu » pour calculer l'inhibition de la conversion de la cortisone en cortisol par la 11-beta HSD1.

Par exemple, les composés 92 et 99 présentent dans les tissus cibles les taux d'inhibition suivants :

| *ex-vivo* 16h inhib. | Foie | Gras sous cutané |
|---|---|---|
| Composé n°99 | 85% | 80% |
| Composé n°92 | 85% | 60% |

Il apparaît donc que les composés selon l'invention ont une activité inhibitrice de l'enzyme 11beta-HSD1. Les composés selon l'invention peuvent donc être utilisés pour la préparation de médicaments, en particulier de médicaments inhibiteurs de l'enzyme 11 beta-HSD1.

Ainsi, selon un autre de ses aspects, l'invention a pour objet des médicaments qui comprennent un composé de formule (I), ou un sel d'addition de ce dernier à un acide ou à une base pharmaceutiquement acceptable, ou encore un hydrate ou un solvat du composé de formule (I).

Ces médicaments trouvent leur emploi en thérapeutique, notamment dans le traitement et la prévention de l'obésité, des diabètes, des troubles de la microcirculation, de la résistance à l'insuline, du syndrome métabolique, du syndrome de Cushing, de l'hypertension, de l'athérosclérose, de la cognition et de la démence, des glaucomes, de l'ostéoporose, de la lipodystrophie, de l'hypertrophie cardiaque, de l'insuffisance cardiaque, de maladies hépatiques, de certaines maladies infectieuses en augmentant l'efficacité du système immunitaire ou encore pour favoriser la cicatrisation de plaies.

Selon un autre de ses aspects, la présente invention concerne des compositions pharmaceutiques comprenant, en tant que principe actif, un composé selon l'invention, Ces compositions pharmaceutiques contiennent une dose efficace d'au moins un composé selon l'invention ou un sel pharmaceutiquement acceptable, un hydrate ou solvat dudit composé, ainsi qu'au moins un excipient pharmaceutiquement acceptable, Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale, telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants, Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention a un rapport également avec une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) : 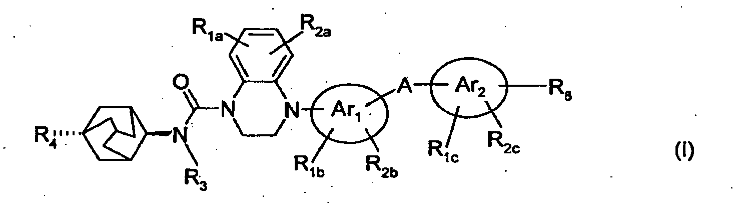 dans laquelle :
- A représente une liaison, un atome d'oxygène ou un groupe -O-CH₂-,
- Ar₁ représente un groupe phényle ou hétéroaryle,
- Ar₂ représente un groupe phényle, un groupe hétéroaryle ou un groupe hétérocycloalkyle,
- R_{1a,b,c} et R_{2a,b,c,}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, alcoxy-alcoxy, halogénoalkyle, -O-halogénoalkyle, oxo, -CO-alkyle, -CO-alkyle-NR₆R₇, -CO-halogénoalkyle, -COOR₅, alkyl-COOR₅, -O-alkyl-COOR₅, -SO₂-alkyle, -SO₂-cycloalkyle, -SO₂-alkyl-cycloalkyle, -SO₂-alkyle-OR₅, -SO₂-alkyl-COOR₅, -SO₂-alkyl-NR₆R₇, -SO₂-halogénoalkyle, alkyl-SO₂-alkyle, -SO₂-NR₆R₇, -SO₂-alkyl-alcoxy-alcoxy, -CONR₆R₇, -alkyl-CONR₆R₇ ou -O-alkyl-NR₆R₇, ou encore R₁ₐ, R _{1b}, R _{1c} sont liés respectivement à R₂ₐ, R_{2b}, R_{2c} et à l'atome de carbone qui les porte et représentent -O-alkyl-O- ;
- R₃ représente un atome d'hydrogène ou un groupe alkyle,
- R₄ représente un atome d'hydrogène ou d'halogène ou un groupe cyano, - OR₅, hydroxy-alkyle, -COOR₅, -NR₆R₇, -CONR₆R₇, -SO₂-alkyle ou -SO₂-NR₆R₇, -NR₆-COOR₅, -NR₆-COR₅, -CO-NR₆-alkyl-OR₅ ;
- R₅, R₆ et R₇, identiques ou différents, représentent chacun un atome d'hydrogène, un groupe alkyle ou un groupe -alkyl-phényle, et
R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un hétéroaryle ou un hétérocycloalkyle éventuellement substitué par 1 à 3 groupes choisis parmi les groupes alkyles, -SO₂-alkyles et -COOR₅,
à l'état de base ou d'acide ou de sel d'addition à un acide ou à une base.

2. Composé de formule (I) selon la revendication 1, **caractérisé en ce que** A représente une liaison.

3. Composé de formule (I) selon la revendication 1 ou la revendication 2, **caractérisé en ce que** Ar₁ représente un groupe phényle, pyridinyle, pyrimidinyle, pyridazinyle ou thiazolyle.

4. Composé de formule (I) selon l'une quelconque des revendications précédentes, **caractérisé en ce que** Ar₂ représente un groupe phényle, pyridinyle, pyrimidinyle, pipéridinyle, pipérazinyle, homopipérazinyle, 3,8-diazabicyclo[3,2,1]octane, morpholinyle, thiomorpholinyle, octahydro-pyrrolo[3,4-c]pyrrole, 1,2,3,6-tetrahydro-pyridine ou 2,5-diaza-bicyclo[2.2.1]heptane.

5. Composé de formule (I) selon l'une quelconque des revendications précédentes, **caractérisé en ce que** R₁ₐ, R₂ₐ, R_{1b} et R_{2b}, identiques ou différents, représentent chacun un atome d'hydrogène ou d'halogène ou un groupe alkyle ou alcoxy.

6. Composé de formule (I) selon l'une quelconque des revendications précédentes, **caractérisé en ce que** R_{1c} et R_{2c}, identiques ou différents, représentent chacun un atome d'hydrogène ou d'halogène ou un groupe alkyle, alcoxy. ou -SO₂-alkyle, -SO₂-cycloalkyle.

7. Composé de formule (I) selon l'une quelconque des revendications précédentes, **caractérisé en ce que** R₃ représente un atome d'hydrogène.

8. Composé de formule (I) selon l'une quelconque des revendications précédentes, **caractérisé en ce que** R₄ représente un atome d'hydrogène ou d'halogène ou un groupe cyano, hydroxy, hydroxy-alkyle; -COOR₅ ou -CONH₂.

9. Composé de formule (I) selon l'une quelconque des revendications précédentes, **caractérisé en ce que** R₈ représente un atome d'hydrogène, un groupe alkyle ou un groupe de formule -B-Het, où B peut être absent ou représenter une liaison, un atome d'oxygène ou un groupe -CO- ou -SO₂-(CH2)ₙ- avec n égal à 0,1 ou 2 et où Het représente un groupe pyrrolidinyle, pipéridinyle, pipérazinyle, tétrahydropyranyle, morpholinyle ou pyridinyle, ledit groupe Het étant éventuellement substitué par un groupe alkyle, -SO₂-alkyle ou -COOR₅.

10. Composé de formule (I) selon la revendication 1, **caractérisé en ce que** :
- A représente une liaison, un atome d'oxygène ou un groupe -O-CH₂-,
- Ar₁ représente un groupe phényle ou hétéroaryle,
- Ar₂ représente un groupe phényle,
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyle-NR₆R₇, -COOR₅, alkyl-COOR₅. -SO₂-alkyle, -SO₂-cycloalkyle, -SO₂-alkyle-cycloalkyle, -SO₂-alkyle-OR₅, -SO₂-alkyle-COOR₅, -SO₂-alkyle-NR₆R₇, -SO₂-halogénoalkyle, alkyl-SO₂-alkyle, -SO₂-alkyl-alcoxy-alcoxy, -CON R₆R₇ ou -O-alkyl-NR₆R₇, ou encore R₁ₐ, R_{1b}, R_{1c} sont liés respectivement à R₂ₐ, R_{2b}, R_{2c} et à l'atome de carbone qui les porte et représentent -O-alkyl-O-,
- R₃ représente un atome d'hydrogène ou un alkyle,
- R₄ représente un atome d'hydrogène ou d'halogène ou un groupe cyano, - OR₅, hydroxy-alkyle, -COOR₅, -CONR₆R₇, -NR_{O}-COOR₅, -NR₆-COR₅, ou -CO-NR₆-alkyl-OR₅,
- R₅, R₆ et R₇, identiques ou différents, représentent chacun un atome d'hydrogène, un groupe alkyle ou un groupe -alkyl-phényle, et
R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un groupe pyrrolidinyle, pipéridinyle, pipérazinyle, tétrahydropyranyle, morpholinyle ou pyridinyle, ledit groupe Het étant éventuellement substitué par 1 à 3 groupes choisis parmi les groupes alkyles, -SO₂-alkyles et -COOR₅,
à l'état de base ou d'acide ou de sel d'addition à un acide ou à une base.

11. Composé de formule (I) selon la revendication 1, **caractérisé en ce que** :
- A représente une liaison, un atome d'oxygène ou un groupe -O-CH₂-,
- Ar₁ représente un groupe phényle ou hétéroaryle,
- Ar₂ représente un groupe hétéroaryle,
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyle-NR₆R₇, -COOR₅, alkyl-COOR₅, -SO₂-alkyle, -SO₂-cycloalkyle, -SO₂-alkyl-cycloalkyle, -SO₂-alkyl-OR₅, -SO₂-alkyl-COOR₅, -SO₂-alkyl-NR₆R₇, -SO₂-halogénoalkyle, -alkyl-SO₂-alkyle, -SO₂-alkyl-alcoxy-alcoxy, -CONR₆R₇ ou -O-alkyl-NR₆R₇, ou encore R₁ₐ, R_{1b,} R_{1c} sont liés respectivement à R₂ₐ, R_{2b}, R_{2c} et à l'atome de carbone qui les porte et représentent -O-alkyl-O-,
- R₃ représente un atome d'hydrogène ou un alkyle,
- R₄ représente un atome d'hydrogène ou d'halogène ou un groupe cyano,-OR₅, hydroxy-alkyle, -COOR₅, -CONR₆R₇, -NR₆-COOR₅, -NR₆-COR₅ ou-CO-NR₆-alkyl-OR₅,
- R₅, R₆ et R₇, identiques ou différents, représentent chacun un atome d'hydrogène, un groupe alkyle ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un groupe pyrrolidinyle, pipéridinyle, pipérazinyle, tétrahydropyranyle, morpholinyle ou pyridinyle, ledit groupe Het étant éventuellement substitué par 1 à 3 groupes choisis parmi les groupes alkyles, -SO₂-alkyles et -COOR₅,
à l'état de base ou d'acide ou de sel d'addition à un acide ou à une base.

12. Composé de formule (I) selon la revendication 1, **caractérisé en ce que** :
- A représente une liaison, un atome d'oxygène ou un groupe -O-CH₂-,
- Ar₁ représente un groupe phényle ou hétéroaryle,
- Ar₂ représente un groupe hétérocycloalkyle (tel qu'un groupe pipéridinyle, pipérazinyle, homopipérazinyle, 3,8-diazabicydo[3.2.1]octane, morpholinyle, thiomorpholinyle, octahydro-pyrrolo[3,4-c]pyrrole, 1,2,3,6-tetrahydro-pyridine ou 2,5-diaza-bicyclo[2.2.1]heptane,),
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, halogénoalkyle, -O-halogénoalkyle, oxo, -CO-alkyle, -CO-alkyle-NR₆R₇, -COOR₅, alkyl-COOR₅, -SO₂-alkyle, -SO₂-cycloalkyle, -SO₂-alkyl-cycloalkyle, -SO₂-alkyl-OR₅, -SO₂-alkyl-COOR₅, -SO₂-alkyl-NR₆R₇, -SO₂-halogénoalkyle, alkyl-SO₂-alkyle, -SO₂-alkyl-alcoxy-alcoxy, -CONR₆R₇ ou -O-alkyl-NR₆R₇, ou encore R_{1a,} R_{1b,} R_{1c} sont liés respectivement à R₂ₐ, R_{2b}, R_{2c} et à l'atome de carbone qui les porte et représentent --O-alkyl-O-,
- R₃ représente un atome d'hydrogène ou un alkyle,
- R₄ représente un atome d'hydrogène ou d'halogène ou un groupe cyano,-OR₅, hydroxy-alkyle, -COOR₅, -CONR₆R₇. -NR₆-COOR₅, -NR₆-COR₅, ou -CO-NR6-alkyl-OR5,
- R₅, R₆ et R₇, identiques ou différents, représentent chacun un atome d'hydrogène, un groupe alkyle ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un groupe pyrrolidinyle, pipéridinyle, pipérazinyle, tétrahydropyranyle, morpholinyle ou pyridinyle, ledit groupe Het étant éventuellement substitué par 1 à 3 groupes choisis parmi les groupes alkyles, -SO₂-alkyle et -COOR₅,
à l'état de base ou d'acide ou de sel d'addition à un acide ou à une base.

13. Composé de formule (I) selon la revendication 1, **caractérisé en ce qu'**il est choisi parmi :
. 4-((4-pyridin-2-yl-phenyl)-3,4-dihydro-2H-quinoxaline-1-carboxylic acid adamantan-2-ylamide,
. 4-{4-[1-(tetrahydro-pyran-4-carbonyl)-piperidin-4-yloxy]-phenyl}-3,4-dihydro-2H-quinoxaline-1-carboxylic acid adamantan-2-ylamide,
. 4-[4-(pyrimidin-2-ylmethoxy)-phenyl]-3,4-dihydro-2H-quinoxaline-1-carboxylic acid adamantan-2-ylamide,
. 4-{4-[1-(morpholine-4-carbonyl)-piperidin-4-yl]-phenyl}-3,4-dihydro-2H-quinoxaline-1-carboxylic acid adamantan-2-ylamide,
. 4-[4-(4-methoxy-pyrimidin-2-yloxy)-phenyl]-3,4-dihydro-2H-quinoxaline-1-carboxylic acid adamantan-2-ylamide,
. 4-{4-[4-(morpholine-4-carbonyl)-phenyl]-thiazol-2-yl}-3,4-dihydro-2H-quinoxaline-1-carboxylic acid adamantan-2-ylamide,
. 4-[5-(4-methoxy-phenyl)-pyrimidin-2-yl]-3,4-dihydro-2H-quinoxaline-1-carboxylic acid adamantan-2-ylamide,
. 4-[5-(4-methoxy-phenyl)-pyrimidin-2-yl]-3,4-dihydro-2H-quinoxaline-1-carboxylic acid (5-hydroxy-adamantan-2-yl)-amide,
. trans-4-[4-(4-methanesulfonyl-piperazin-1-yl)-phenyl]-3,4-dihydro-2H-quinoxaline-1 - carboxylic acid (5-hydroxy-adamantan-2-yl)-amide,
. ester methylique de l'acide 4-({4-[4-(4-methanesulfonyl-piperazin-1-yl)-phenyl]-3,4-dihydro-2H-quinoxaline-1-carbonyl}-amino)-adamantane-1-carboxylique,
. acide 4-({4-[4-(4-methanesulfonyl-piperazin-1-yl)-phenyl]-3,4-dihydro-2H-quinoxaline-1-carbonyl}-amino)-adamantane-1-carboxylique,
. 4-[4-(4-methanesulfonyl-piperazin-1-yl)-phenyl]-3,4-dihydro-2H-quinoxaline-1-carboxylic acid (5-carbamoyl-adamantan-2-yl)-amide,
. trans-4-[4-(pyridin-2-yloxy)-phenyl]-3,4-dihydro-2H-quinoxaline-1-carboxylic acid (5-hydroxy-adamantan-2-yl)-amide,
. trans-4-[4-(4-trifluoromethyl-pyrimidin-2-yloxy)-phenyl]-3,4-dihydro-2H-quinoxaline-1-carboxylic acid (5-hydroxy-adamantan-2-yl)-amide,
. trans-4-[4-(1-Methanesulfonyl-1,2,3,6-tetrahydro-pyridin-4-yl)-phenyl]-3,4-dihydro-2H-quinoxaline-1-carboxylic acid (5-hydroxy-adamantan-2-yl)-amide,
. trans-4-[4-(4-Hydroxy-1-methanesulfonyl-piperidin-4-yl)-phenyl]-3,4-dihydro-2H-quinoxaline-1-carboxylic acid (5-hydroxy-adamantan-2-yl)-amide,
. trans-4-[2-(4-Methanesulfonyl-piperazin-1-yl)-pyrimidin-5-yl]-3,4-dihydro-2H-quinoxaline-1-carboxylic acid (5-hydroxy-adamantan-2-yl)-amide,
. trans-4-[5-(4-Methanesulfonyl-piperazin-1-yl)-pyrimidin-2-yl]-3,4-dihydro-2H-quinoxaline-1-carboxylic acid (5-hydroxy-adamantan-2-yl)-amide,
. trans-4-{4-[1-(Morpholine-4-carbonyl)-piperidin-4-yl]-phenyl}-3,4-dihydro-2H-quinoxaline-1-carboxylic acid (5-hydroxy-adamantan-2-yl)-amide,
. trans-4-{4-[4-(5-Hydroxy-adamantan-2-ylcarbamoyl)-3,4-dihydro-2H-quinoxalin-1-yl]-phenyl}-piperazine-1-carboxylic acid benzyl ester,
. trans-4-[4-(4-Methanesulfonyl-piperazin-1-yl)-phenyl]-3,4-dihydro-2H-quinoxaline-1-carboxylic acid (5-hydroxymethyl-adamantan-2-yl)-amide,
. trans-4-[4-(5-Fluoro-pyrimidin-2-yloxy)-phenyl]-3,4-dihydro-2H-quinoxaline-1-carboxylic acid (5-hydroxy-adamantan-2-yl)-amide,
. trans-4-[4-(1-Methanesulfonyl-piperidin-4-yloxy)-phenyl]-3,4-dihydro-2H-quinoxaline-1-carboxylic acid (5-hydroxy-adamantan-2-yl)-amide,
.trans-4-(4-Morpholin-4-yl-phenyl)-3,4-dihydro-2H-quinoxaline-1-carboxylic acid (5-hydroxy-adamantan-2-yl)-amide,
. trans-4-[4-(1,1-Dioxo-1 lambda6-thiomorpholin4-yl)-phenyl]-3,4-dihydro-2H-quinoxaline-1-carboxylic acid (5-hydroxy-adamantan-2-yl)-amide,
. trans-4-[4-(5-Isopropoxy-pyridin-2-yl)-phenyl]-3,4-dihydro-2H-quinoxaline-1-carboxylic-acid (5-hydroxy-adamantan-2-yl)-amide,
. trans-4-{4-[5-(4-Isopropyl-piperazine-1-carbonyl)-pyridin-2-yl]-phenyl}-3,4-dihydro-2H-quinoxaline-1-carboxylic acid (5-hydroxy-adamantan-2-yl)-amide,
. trans-4-[6-(4-Methanesulfonyl-piperazin-1-yl)-pyridin-3-yl]-3,4-dihydro-2H-quinoxaline-1-carboxylic acid (5-hydroxy-adamantan-2-yl)-amide,
. trans-4-(4-Piperazin-1-yl-phenyl)-3,4-dihydro-2H-quinoxaline-1-carboxylic acid (5-hydroxy-adamantan-2-yl)-amide,
. trans-4-[4-(4,4-Difluoro-piperidin-1-yl)-phenyl]-3,4-dihydro-2H-quinoxaline-1-carboxylic acid (5-hydroxy-adamantan-2-yl)-amide,
. trans-4-[5-(4-Methanesulfonyl-piperazin-1-yl)-pyridin-2-yl]-3,4-dihydro-2H-quinoxaline-1-carboxylic acid (5-hydroxy-adamantan-2-yl)-amide,
. trans-4-[5-(4-Methanesulfonyl-piperazin-1-yl)-pyridin-2-yl]-3,4-dihydro-2H-quinoxaline-1-carboxylic acid (5-hydroxy-adamantan-2-yl)-amide,
.trans-4-[2-(1-Methanesulfonyl-piperidin-4-yloxy)-pyrimidin-5-yl]-3,4-dihydro-2H-quinoxaline-1-carboxylic acid (5-hydroxy-adamantan-2-yl)-amide,
. trans-4-[5-(5-Isopropoxy-pyridin-2-yl)-pyrimidin-2-yl]-3,4-dihydro-2H-quinoxaline-1-carboxylic acid (5-hydroxy-adamantan-2-yl)-amide,
. trans-4-[4-(4-Ethanesulfonyl-piperazin-1-yl)-phenyl]-3,4-dihydro-2H-quinoxaline-1-carboxylic acid (5-hydroxy-adamantan-2-yl)-amide,
. trans-4-{4-[4-(2,2,2-Trifluoro-ethanesulfonyl)-piperazin-1-yl]-phenyl}-3,4-dihydro-2H-quinoxaline-1-carboxylic acid (5-hydroxy-adamantan-2-yl)-amide,
. trans-4-{4-[4-(Propane-2-sulfonyl)-piperazin-1-yl]-phenyl}-3,4-dihydro-2H-quinoxaline-1-carboxylic acid (4-hydroxy-adamantan-1-yl)-amide,
. trans-4-(4-{4-[4-(5-Hydroxy-adamantan-2-ylcarbamoyl)-3,4-dihydro-2H-quinoxalin-1-yl]-phenyl}-piperazine-1-sulfonyl)-piperidine-1-carboxylic acid benzyl ester,
. trans-4-{4-[4-(Piperidine-4-sulfonyl)-piperazin-1-yl]-phenyl}-3,4-dihydro-2H-quinoxaline-1-carboxylic acid (5-hydroxy-adamantan-2-yl)-amide,
. trans-4-[4-(4-Cyclopropanesulfonyl-piperazin-1-yl)-phenyl]-3,4-dihydro-2H-quinoxaline-1-carboxylic acid (5-hydroxy-adamantan-2-yl)-amide,
. trans-4-[4-(4-Methanesulfonyl-piperazin-1-yl)-phenyl]-3,4-dihydro-2H-quinoxaline-1-carboxylic acid (5-fluoro-adamantan-2-yl)-amide,
. trans-4-[4-(4-Cyclobutanesulfonyl-piperazin-1-yl)-phenyl]-3,4-dihydro-2H-quinoxaline-1-carboxylic acid (5-hydroxy-adamantan-2-yl)-amide,
. trans-4-{4-[4-(Propane-1-sulfonyl)-piperazin-1-yl]-phenyl}-3,4-dihydro-2H-quinoxaline-1-carboxylic acid (5-hydroxy-adamantan-2-yl)-amide,
. trans-4-{4-[4-(Butane-1-sulfonyl)-piperazin-1-yl]-phenyl}-3,4-dihydro-2H-quinoxaline-1-carboxylic acid (5-hydroxy-adamantan-2-yl)-amide,
. trans-4-{4-[4-(Morpholine-4-sulfonyl)-piperazin-1-yl]-phenyl}-3,4-dihydro-2H-quinoxaline-1-carboxylic acid (5-hydroxy-adamantan-2-yl)-amide,
. trans-4-[4-(4-Trifluoromethanesulfonyl-piperazin-1-yl)-phenyl]-3,4-dihydro-2H-quihoxaline-1-carboxylic acid (5-hydroxy-adamantan-2-yl)-amide,
. trans-4-[4-(1,4-Dioxa-8-aza-spiro[4.5]dec-8-yl)-phenyl]-3,4-dihydro-2H-quinoxaline-1-carboxylic acid (5-hydroxy-adamantan-2-yl)-amide,
. trans-4-[4-(4-Methanesulfonyl-3,3-dimethyl-piperazin-1-yl)-phenyl]-3,4-dihydro-2H-quinoxaline-1-carboxylic acid (5-hydroxy-adamantan-2-yl)-amide,
.trans-4-[4-((3R,5S)-4-Methanesulfonyl-3,5-dimethyl-piperazin-1-yl)-phenyl]-3,4-dihydro-2H-quinoxaline-1-carboxylic acid (5-hydroxy-adamantan-2-yl)-amide,
.trans- (4-{4-[4-(5-Hydroxy-adamantan-2-ylcarbamoyl)-3,4-dihydro-2H-quinoxalin-1-yl]-phenyl}-piperazine-1-sulfonyl)-acetic acid,
.trans-(4-{4-[4-(5-Hydroxy-adamantan-2-ylcarbamoyl)-3,4-dihydro-2H-quinoxalin-1-yl]-phenyl}-piperazine-1-sulfonyl)-acetic acid ethyl ester,
.trans-4-[4-(4-Hydroxy-piperidin-1-yl)-phenyl]-3,4-dihydro-2H-quinoxaline-1-carboxylic acid (5-hydroxy-adamantan-2-yl)-amide,
.trans-4-[4-(4-Methanesulfonyl-[1,4]diazepan-1-yl)-phenyl]-3,4-dihydro-2H-quinoxaline-1-carboxylic acid (5-hydroxy-adamantan-2-yl)-amide,
.trans-2-(4-{4-[4-(5-Hydroxy-adamantan-2-ylcarbamoyl)-3,4-dihydro-2H-quinoxalin-1-yl]-phenyl}-piperazin-1-yl)-2-methyl-propionic acid ethyl ester,
.trans-3-(4-{4-[4-(5-Hydroxy-adamantan-2-ylcarbamoyl)-3,4-dihydro-2H-quinoxalin-1-yl]-phenyl}-piperazine-1-sulfonyl)-propionic acid methyl ester,
.trans-4-[4-(5-Methoxy-pyrimidin-2-yl)-phenyl]-3,4-dihydro-2H-quinoxaline-1-carboxylic acid (5-hydroxy-adamantan-2-yl)-amide,
.trans-4-{4-[4-(2-Morpholin-4-yl-ethanesulfonyl)-piperazin-1-yl]-phenyl}-3,4-dihydro-2H-quinoxaline-1-carboxylic acid (5-hydroxy-adamantan-2-yl)-amide,
.trans-4-[6-(4-Methanesulfonyl-piperazin-1-yl)-pyridazin-3-yl]-3,4-dihydro-2H-quinoxaline-1-carboxylic acid (5-hydroxy-adamantan-2-yl)-amide,
.trans-4-[4-(4-Methanesulfonyl-piperazin-1-yl)-phenyl]-3,4-dihydro-2H-quinoxaline-1-carboxylic acid (5-hydroxy-adamantan-2-yl)-methyl-amide,
.trans-4-[4-(4-Methanesulfonyl-3,5-dimethyl-piperazin-1-yl)-phenyl]-3,4-dihydro-2H-quinoxaline-1-carboxylic acid (5-hydroxy-adamantan-2-yl)-amide,
.trans-4-{4-[4-(2-Diethylamino-ethanesulfonyl)-piperazin-1-yl]-phenyl}-3,4-dihydro-2H-quinoxaline-1-carboxylic acid (5-hydroxy-adamantan-2-yl)-amide,
.trans-4-[4-(3-Methanesulfonyl-3,8-diaza-bicyclo[3.2.1]oct-8-yl)-phenyl]-3,4-dihydro-2H-quinoxaline-1-carboxylic acid (5-hydroxy-adamantan-2-yl)-amide,
.trans-4-[5-(4-Methoxy-phenyl)-pyrimidin-2-yl]-3,4-dihydro-2H-quinoxaline-1-carboxylic acid (5-carbamoyl-adamantan-2-yl)-amide,
.trans-4-{4-[4-(2-Methoxy-ethanesulfonyl)-piperazin-1-yl]-phenyl}-3,4-dihydro-2H-quinoxaline-1-carboxylic acid (5-hydroxy-adamantan-2-yl)-amide,
.trans-4-[4-(4-Isopropoxy-piperidin-1-yl)-phenyl]-3,4-dihydro-2H-quinoxaline-1-carboxylic acid (5-hydroxy-adamantan-2-yl)-amide,
.trans-4-[5-(4-Hydroxy-phenyl)-pyrimidin-2-yl]-3,4-dihydro-2H-quinoxaline-1-carboxylic acid (5-hydroxy-adamantan-2-yl)-amide,
.trans-4-(4-Piperazin-1-yl-phenyl)-3,4-dihydro-2H-quinoxaline-1-carboxylic acid (5-carbamoyl-adamantan-2-yl)-amide,
.trans-4-[4-(5-Bromo-pyrimidin-2-yloxy)-phenyl]-3,4-dihydro-2H-quinoxaline-1-carboxylic acid (5-hydroxy-adamantan-2-yl)-amide,
.trans-4-[4-(4-Cyclopropanesulfonyl-piperazin-1-yl)-phenyl]-3,4-dihydro-2H-quinoxaline-1-carboxylic acid (5-carbamoyl-adamantan-2-yl)-amide,
.trans-4-[4-(4-Cyclopropanesulfonyl-piperazin-1-yl)-phenyl]-3,4-dihydro-2H-quinoxaline-1-carboxylic acid (5-carbamoyl-adamantan-2-yl)-amide,
.trans-6-Chloro-4-[4-(4-methanesulfonyl-piperazin-1-yl)-phenyl]-3,4-dihydro-2H-quinoxaline-1-carboxylic acid (5-hydroxy-adamantan-2-yl)-amide,
.trans-4-[4-(5-Methanesulfonyl-hexahydro-pyrrolo[3,4-c]pyrrol-2-yl)-phenyl]-3,4-dihydro-2H-quinoxaline-1-carboxylic acid (5-hydroxy-adamantan-2-yl)-amide,
.trans-4-{4-[4-(2-Amino-ethanesulfonyl)-piperazin-1-yl]-phenyl}-3,4-dihydro-2H-quinoxaline-1-carboxylic acid (5-hydroxy-adamantan-2-yl)-amide,
.trans-4-(4-Morpholin-4-yl-phenyl)-3,4-dihydro-2H-quinoxaline-1-carboxylic acid (5-carbamoyl-adamantan-2-yl)-amide,
.trans-4-{4-[5-(Piperidin-4-yloxy)-pyridin-2-yl]-phenyl}-3,4-dihydro-2H-quinoxaline-1-carboxylic acid (5-hydroxy-adamantan-2-yl)-amide,
.trans-4-{4-[5-(2-Amino-ethoxy)-pyridin-2-yl]-phenyl}-3,4-dihydro-2H-quinoxaline-1-carboxylic acid (5-hydroxy-adamantan-2-yl)-amide,
.trans-4-{4-[4-(2-Hydroxy-ethanesulfonyl)-piperazin-1-yl]-phenyl}-3,4-dihydro-2H-quinoxaline-1-carboxylic acid (5-hydroxy-adamantan-2-yl)-amide,
.trans-4-[4-(8-Methanesulfonyl-3,8-diaza-bicyclo[3.2.1]oct-3-yl)-phenyl]-3,4-dihydro-2H-quinoxaline-1-carboxylic acid (5-carbamoyl-adamantan-2-yl)-amide,
.trans-4-[4-(8-Methanesulfonyl-3,8-diaza-bicyclo[3.2.1]oct-3-yl)-phenyl]-3,4-dihydro-2H-quinoxaline-1-carboxylic acid (5-hydroxy-adamantan-2-yl)-amide,
.trans-4-[5-(4-Isopropoxy-phenyl)-pyrimidin-2-yl]-3,4-dihydro-2H-quinoxaline-1-carboxylic acid (5-hydroxy-adamantan-2-yl)-amide,
.trans-4-{5-[4-(2,2,2-Trifluoro-ethanesulfonyl)-piperazin-1-yl]-pyridin-2-yl}-3,4-dihydro-2H-quinoxaline-1-carboxylic acid (5-hydroxy-adamantan-2-yl)-amide,
.trans-4-{5-[4-(2,2,2-Trifluoro-ethanesulfonyl)-piperazin-1-yl]-pyridin-2-yl}-3,4-dihydro-2H-quinoxaline-1-carboxylic acid (5-carbamoyl-adamantan-2-yl)-amide,
.trans-3-(4-{4-[4-(5-Hydroxy-adamantan-2-ylcarbamoyl)-3,4-dihydro-2H-quinoxalin-1-yl]-phenyl}-piperazine-1-sulfonyl)-propionic acid,
.trans-4-[4-(4-Methanesulfonyl-piperazin-1-yl)-phenyl]-3,4-dihydro-2H-quinoxaline-1-carboxylic acid [5-(2-hydroxy-ethylcarbamoyl)-adamantan-2-yl]-amide,
.trans-4-[4-(5-Isobutoxy-pyridin-2-yl)-phenyl]-3,4-dihydro-2H-quinoxaline-1-carboxylic acid (5-hydroxy-adamantan-2-yl)-amide,
.trans-4-[4-(5-sec-Butoxy-pyridin-2-yl)-phenyl]-3,4-dihydro-2H-quinoxaline-1-carboxylic acid (5-hydroxy-adamantan-2-yl)-amide,
.trans-4-[4-(5-Isopropoxy-pyridin-2-yl)-phenyl]-3,4-dihydro-2H-quinoxaline-1-carboxylic acid (5-carbamoyl-adamantan-2-yl)-amide,
.trans-[4-({4-[4-(4-Methanesulfonyl-piperazin-1-yl)-phenyl]-3,4-dihydro-2H-quinoxaline-1-carbonyl}-amino)-adamantan-1-yl]-carbamic acid methyl ester, trans-4-[5-(4-Ethanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-Cyclopropanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-Cyclopropanesulfonyl-piperazin-1-yl)-pyridin-2-yl]-3,4-dihydro-2H-quinoxaline-1-carboxylic acid (5-carbamoyl-adamantan-2-yl)-amide,
.trans-4-{5-[4-(Propane-2-sulfonyl)-piperazin-1-yl]-pyridin-2-yl}-3,4-dihydro-2H-quinoxaline-1-carboxylic acid (5-hydroxy-adamantan-2-yl)-amide,
.trans-4-{4-[4-(Pyridin-4-yloxy)-piperidin-1-yl]-phenyl}-3,4-dihydro-2H-quinoxaline-1-carboxylic acid (5-hydroxy-adamantan-2-yl)-amide,
.trans-4-{4-[4-(2-Methoxy-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(2-Amino-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(2-Diethylamino-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(Piperidine-4-sulfonyl)-piperazin-1-yl]-phenyl}-3,4-dihydro-2H-quinoxaline-1-carboxylic acid (5-carbamoyl-adamantan-2-yl)-amide,
.trans-4-[4-(4-tert-Butyl-piperazin-1-yl)-phenyl]-3,4-dihydro-2H-quinoxaline-1-carboxylic acid (5-carbamoyl-adamantan-2-yl)-amide,
.trans-4-[5-(4-Methanesulfonyl-piperazin-1-yl)-pyridin-2-yl]-3,4-dihydro-2H-quinoxaline-1-carboxylic acid (5-carbamoyl-adamantan-2-yl)-amide,
.trans-4-{5-[4-(Propane-1-sulfonyl)-piperazin-1-yl]-pyridin-2-yl}-3,4-dihydro-2H-quinoxaline-1-carboxylic acid (5-hydroxy-adamantan-2-yl)-amide,
.trans-4-{5-[4-(2-Methyl-propane-1-sulfonyl)-piperazin-1-yl]-pyridin-2-yl}-3,4-dihydro-2H-quinoxaline-1-carboxylic acid (5-hydroxy-adamantan-2-yl)-amide,
.trans-4-(5-Morpholin-4-yl-pyridin-2-yl)-3,4-dihydro-2H-quinoxaline-1-carboxylic acid (5-hydroxy-adamantan-2-yl)-amide,
.trans-4-{4-[4-(2-Hydroxy-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(Piperidin-4-yloxy)-piperidin-1-yl]-phenyl}-3,4-dihydro-2H-quinoxaline-1-carboxylic acid (5-hydroxy-adamantan-2-yl)-amide,
.trans-4-[4-(4-Dimethylcarbamoyl-piperazin-1-yl)-phenyl]-3,4-dihydro-2H-quinoxaline-1-carboxylic acid (5-hydroxy-adamantan-2-yl)-amide,
.trans-4-[6-(4-Cyclopropanesulfonyl-piperazin-1-yl)-pyridin-3-yl]-3,4-dihydro-2H-quinoxaline-1-carboxylic acid (5-carbamoyl-adamantan-2-yl)-amide,
.trans-4-[4-(4-Ethanesulfonyl-piperazin-1-yl)-phenyl]-3,4-dihydro-2H-quinoxaline-1-carboxylic acid (5-carbamoyl-adamantàn-2-yl)-am ide,
.trans-4-{4-[4-(2,2,2- Trifluoro-ethanesulfonyl)-piperazin-1-yl]-phenyl}-3,4-dihydro-2H-quinoxaline-1-carboxylic acid (5-carbamoyl-adamantan-2-yl)-amide,
.trans-4-{4-[4-(2-Amino-acetyl)-piperazin-1-yl]-phenyl}-3,4-dihydro-2H-quinoxaline-1-carboxylic acid (5-carbamoyl-adamantan-2-yl)-amide,
.trans-4-[4-(5-Hydroxymethyl-pyridin-2-yl)-phenyl]-3,4-dihydro-2H-quinoxaline-1-carboxylic acid (5-carbamoyl-adamantan-2-yl)-amide,
.trans-4-[5-(4-Cyclopentanesulfonyl-piperazin-1-yl)-pyridin-2-yl]-3,4-dihydro-2H-quinoxaline-1-carboxylic acid (5-hydroxy-adamantan-2-yl)-amide,
.trans-4-[4-(4-Isopropoxy-piperidin-1-yl)-phenyl]-3,4-dihydro-2H-quinoxaline-1-carboxylic acid (5-carbamoyl-adamantan-2-yl)-amide,
.trans-4-{4-[5-(2,2,2-Trifluoro-ethoxy)-pyridin-2-yl]-phenyl}-3,4-dihydro-2H-quinoxaline-1-carboxylic acid (5-hydroxy-adamantan-2-yl)-amide,
.trans-4-{4-(5-(2,2,2-Trifluoro-ethoxy)-pyridin-2-yl]-phenyl}-3,4-dihydro-2H-quinoxaline-1-carboxylic acid (5-carbamoyl-adamantan-2-yl)-amide,
.trans-4-[5-(4-Isopropoxy-phenyl)-pyrimidin-2-yl]-3,4-dihydro-2H-quinoxaline-1-carboxylic acid (5-carbamoyl-adamantan-2-yl)-amide,
.trans-4-{5-[4-(Piperidin-4-yloxy)-phenyl]-pyrimidin-2-yl}-3,4-dihydro-2H-quinoxaline-1-carboxylic acid (5-carbamoyl-adamantan-2-yl)-amide,
.trans-4-[5-(4-Trifluoromethanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-tert-Butyl-piperazin-1-yl)-pyridin-2-yl]-3,4-dihydro-2H-quinoxaline-1-carboxylic acid (5-hydroxy-adamantan-2-yl)-amide,
.trans-4-[6-(4-Ethanesulfonyl-piperazin-1-yl)-pyridin-3-yl]-3,4-dihydro-2H-quinoxaline-1-carboxylic acid (5-carbamoyl-adamantan-2-yl)-amide,
.trans-4-[5-(4-Cyclopropyl-piperazin-1-yl)-pyridin-2-yl]-3,4-dihydro-2H-quinoxaline-1-carboxylic acid (5-hydroxy-adamantan-2-yl)-amide,
.trans-4-[5-(4-Cyclobutanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-Acetyl-piperazin-1-yl)-pyridin-2-yl]-3,4-dihydro-2H-quinoxaline-1-carboxylic acid (5-hydroxy-adamantan-2-yl)-amide,
.trans-4-{4-[4-(1-Ethyl-propyl)-piperazin-1-yl]-phenyl}-3,4-dihydro-2H-quinoxaline-1-carboxylic acid (5-carbamoyl-adamantan-2-yl)-amide,
.trans-4-{5-[4-(2-Methyl-propane-1-sulfonyl)-piperazin-1-yl]-pyridin-2-yl}-3,4-dihydro-2H-quinoxaline-1-carboxylic acid (5-carbamoyl-adamantan-2-yl)-amide,
.trans-4-{5-[4-(3,3,3-Trifluoro-propane-1-sulfonyl)-piperazin-1-yl]-pyridin-2-yl}-3,4-dihydro-2H-quinoxaline-1-carboxylic acid (5-hydroxy-adamantan-2-yl)-amide,
.trans-4-{4-[4-(2,2,2-Trifluoro-ethyl)-piperazin-1-yl]-phenyl}-3,4-dihydro-2H-quinoxaline-1-carboxylic acid (5-carbamoyl-adamantan-2-yl)-amide,
.trans-4-(4-{4-[2-(2-Methoxy-ethoxy)ethanesulfonyl]-piperazin-1-yl}-phenyl)-3,4-dihydro-2H-quinoxaline-1-carboxylic acid (5-carbamoyl-adamantan-2-yl)-amide,
.trans-4-{5-[4-(2-Isopropoxy-ethanesulfonyl)-piperazin-1-yl]-pyridin-2-yl}-3,4-dihydro-2H-quinoxaline-1-carboxylic acid (5-carbamoyl-adamantan-2-yl)-amide,
.4-[5-(4-Cyclopropylmethanesulfonyl-piperazin-1-yl)-pyridin-2-yl]-3,4-dihydro-2H-quinoxaline-1-carboxylic acid (5-hydroxy-adamantan-2-yl)-amide,
.trans-4-[5-(4-tert-Butyl-piperazin-1-yl)-pyridin-2-yl]-3,4-dihydro-2H-quinoxaline-1-carboxylic acid (5-carbamoyl-adamantan-2-yl)-amide,
.trans-4-[5-(4-Cyclopropyl-piperazin-1-yl)-pyridin-2-yl]-3,4-dihydro-2H-quinoxaline-1-carboxylic acid (5-carbamoyl-adamantan-2-yl)-amide,
.trans-4-{4-[4-(Tetrahydro-pyran-4-yl)-piperazin-1-yl]-phenyl}-3,4-dihydro-2H-quinoxaline-1-carboxylic acid (5-carbamoyl-adamantan-2-yl)-amide,
.trans-4-{5-[4-(2-Methoxy-ethanesulfonyl)-piperazin-1-yl]-pyridin-2-yl}-3,4-dihydro-2H-quinoxaline-1-carboxylic acid (5-carbamoyl-adamantan-2-yl)-amide,
.trans-4-[4-(7-Methanesulfonyl-2,7-diaza-spiro[4.4]non-2-yl)-phenyl]-3,4-dihydro-2H-quinoxaline-1-carboxylic acid (5-hydroxy-adamantan-2-yl)-amide,
trans-4-{5-[4-(2,2-Dimethyl-propyl)-piperazin-1-yl]-pyridin-2-yl}-3,4-dihydro-2H-quinoxaline-1-carboxylic acid (5-hydroxy-adamantan-2-yl)-amide,
trans-4-{5-[4-(2-Methoxy-1-methoxymethyl-ethyl)-piperazin-1-yl]-pyridin-2-yl}-3,4-dihydro-2H-quinoxaline-1-carboxylic acid (5-hydroxy-adamantan-2-yl)-amide,
trans-4-{5-[4-(3-Methoxy-propyl)-piperazin-1-yl]-pyridin-2-yl}-3,4-dihydro-2H-quinoxaline-1-carboxylic acid (5-hydroxy-adamantan-2-yl)-amide,
trans-4-{5-[4-(3-Methyl-butyryl)-piperazin-1-yl]-pyridin-2-yl}-3,4-dihydro-2H-quinoxaline-1-carboxylic acid (5-hydroxy-adamantan-2-yl)-amide,
trans-4-[4-(4-Methanesulfonyl-piperazin-1-yl)-phenyl]-3,4-dihydro-2H-quinoxaline-1-carboxylic acid (5-acetylamino-adamantan-2-yl)-amide,
trans-4-{5-[4-(2-Methoxy-ethanesulfonyl)-piperazin-1-yl]-pyridin-2-yl}-3,4-dihydro-2H-quinoxaline-1-carboxylic acid (5-hydroxy-adamantan-2-yl)-amide,
trans-4-{5-[4-(2,2-Dimethyl-propionyl)-piperazin-1-yl]-pyridin-2-yl}-3,4-dihydro-2H-quinoxaline-1-carboxylic acid (5-hydroxy-adamantan-2-yl)-amide,
trans-4-(4,4-Difluoro-3,4,5,6-tetrahydro-2H-[1,3']bipyridinyl-6'-yl)-3.4-dihydro-2H-quinoxaline-1-carboxylic acid (5-hydroxy-adamantan-2-yl)-amide,
trans-4-(4-Pyrrolidin-1-yl-3,4,5,6-tetrahydro-2H-[1,3']bipyridinyt-6'-yl)-3,4-dihydro-2H-quinoxaline-1-carboxylic acid (5-hydroxy-adamantan-2-yl)-amide,
trans-4-[5-(5-Cyclopropanesulfonyl-2,5-diaza-bicyclo[2.2.1]hept-2-yl)-pyridin-2-yl]-3,4-dihydro-2H-quinoxaline-1-carboxylic acid (5-hydroxy-adamantan-2-yl)-amide,
trans-4-{6-[4-(5-Hydroxy-adamantan-2-ylcarbamoyl}-3,4-dihydro-2H-quinoxalin-1-yl]-pyridin-3-yl}-piperazine-1-carboxyfic acid tert-butyl ester,
trans-4-{4-[4-(2-Methoxy-ethyl)-piperazin-1-yl]-phenyl}-3,4-dihydro-2H-quinoxaline-1-carboxylic acid (5-carbamoyl-adamantan-2-yl)-amide,
trans-4-[5-(4-Methanesulfonyl-piperazin-1-yl)-3-methyl-pyridin-2-yl]-3,4-dihydro-2H-quinoxaline-1-carboxylic acid (5-carbamoyl-adamantan-2-yl)-amide,
trans-2-(4-{4-[4-(5-Carbamoyl-adamantan-2-ylcarbamoyl)-3,4-dihydro-2H-quinoxalin-1-yl]-phenyl}-piperazin-1-yl)-2-methyl-propionic acid,
trans-4-(4-tert-Butyl-3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-(4-Pyrrolidin-1-yl-3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-[5-(4-Ethanesulfonyl-piperazin-1-yl)-pyridin-2-yl]-3,4-dihydro-2H-quinoxaline-1-carboxylic acid (5-carbamoyl-adamantan-2-yl)-amide,
trans-4-[5-(4-Methanesulfonyl-piperazin-1-yl)-6-methyl-pyridin-2-yl]-3,4-dihydro-2H-quinoxaline-1-carboxylic acid (5-carbamoyl-adamantan-2-yl)-amide,
trans-4-(4,4-Difluoro-3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-{4-[4-(2-Hydroxy-1,1-dimethyl-ethyl)-piperazin-1-yl]-phenyl}-3,4-dihydro-2H-quinoxaline-1-carboxylic acid (5-carbamoyl-adamantan-2-yl)-amide,
trans-4-{5-[4-(Propane-2-sulfonyl)-piperazin-1-yl]-pyridin-2-yl}-3,4-dihydro-2H-quinoxaline-1-carboxylic acid (5-carbamoyl-adamantan-2-yl)-amide,
trans-4-(5-Morpholin-4-yl-pyridin-2-yl)-3,4-dihydro-2H-quinoxaline-1-carboxylic acid (5-carbamoyl-adamantan-2-yl)-amide,
trans-4-[5-(4-Methanesulfonyl-piperazin-1-yl)-4-methyl-pyridin-2-yl]-3,4-dihydro-2H-quinoxaline-1-carboxylic acid (5-carbamoyl-adamantan-2-yl)-amide,
trans-4-(4-Trrfluoromethyl-3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-(4-tert-Butyl-3,4,5,6-tetrahydro-2H-[1,3']bipyridinyl-6'-yl)-3,4-dihydro-2H-quinoxaline-1-carboxylic acid (5-hydroxy-adamantan-2-yl)-amide,
trans-4-[5-(4-Isobutyl-piperazin-1-yl)-pyridin-2-yl]-3,4-dihydro-2H-quinoxaline-1-carboxylic acid (5-carbamoyl-adamantan-2-yl)-amide,
trans-4-[5-(4-Cyclopropylmethyl-piperazin-1-yl)-pyridin-2-yl]-3,4-dihydro-2H-quinoxaline-1-carboxylic acid (5-carbamoyl-adamantan-2-yl)-amide,
trans-4-{5-[4-(2,2,2-Trifluoro-ethyl)-piperazin-1-yl]-pyridin-2-yl}-3,4-dihydro-2H-quinoxaline-1-carboxylic acid (5-carbamoyl-adamantan-2-yl)-amide,
trans-4-[5-(4-tert-Butyl-piperazin-1-yl)-pyridin-2-yl]-3,4-dihydro-2H-quinoxaline-1-carboxylic acid (5-hydroxymethyl-adamantan-2-yl)-amide,
trans-4-[5-(4-tert-Butyl-piperazin-1-yl)-pyridin-2-yl]-6,7-difluoro-3,4-dihydro-2H-quinoxaline-1-carboxylic acid (5-carbamoyl-adamantan-2-yl)-amide,
trans-4-{5-[4-(2,2-Dimethyl-propionyl)-piperazin-1-yl]-pyridin-2-yl}-3,4-dihydro-2H-quinoxaline-1-carboxylic acid (5-carbamoyl-adamantan-2-yl)-amide,
trans-4-[5-(4-tert-Butyl-piperazin-1-yl)-pyrimidin-2-yl]-3,4-dihydro-2H-quinoxaline-1-carboxylic acid (5-carbamoyl-adamantan-2-yl)-amide,
trans-4-[5-(4-Cyclopropanesulfonyl-piperazin-1-yl)-pyridin-2-yl]-3,4-dihydro-2H-quinoxaline-1-carboxylic acid (5-cyano-adamantan-2-yl)-amide,
trans-4-[5-(1-tert-Butyl-piperidin-4-yloxy)-pyridin-2-yl]-3,4-dihydro-2H-quinoxaline-1-carboxylic acid (5-carbamoyl-adamantan-2-yl)-amide,
Trans-{6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-yl}-acetic acid methyl ester,
trans-6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-carboxylic acid ethyl ester,
trans-4-[5-(4-Isopropyl-piperazin-1-yl)-pyridin-2-yl]-3,4-dihydro-2H-quinoxaline-1-carboxylic acid (5-carbamoyl-adamantan-2-yl)-amide,
trans-4-(1'-tert-Butyl-1',2',3',4',5',6'-hexahydro-[3,4']bipyridinyl-6-yl)-3,4-dihydro-2H-quinoxaline-1-carboxylic acid (5-carbamoyl-adamantan-2-yl)-amide,
trans-{6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-yl}-acetic acid,
trans-6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-carboxylic acid,
trans-2-(4-{6-[4-(5-Carbamoyl-adamantan-2-ylcarbamoyl)-3,4-dihydro-2H-quinoxalin-1-yl]-pyridin-3-yl}-piperazin-1-yl)-2-methyl-propionic acid ethyl ester,
trans-4-(4,4-Dimethyl-3,4,5,6-tetrahydro-2H-[1,3']bipyridinyl-6'-yl)-3,4-dihydro-2H-quinoxaline-1-carboxylic acid (5-carbamoyl-adamantan-2-yl)-amide,
trans-4-{5-[4-(2,2-Difluoro-cyclopropylmethyl)-piperazin-1-yl]-pyridin-2-yl}-3,4-dihydro-2H-quinoxaline-1-carboxylic acid (5-carbamoyhadamantan-2-yl)-amide,
trans-4-{5-[4-(Difluoro-methanesûlfonyl)-piperazin-1-yl]-pyridin-2-yl}-3,4-dihydro-2H-quinoxaline-1-carboxylic acid (5-carbamoyl-adamantan-2-yl)-amide,
trans-4-{5-[4-(3,3,3-Trifluoro-propyl)-piperazin-1-yl]-pyridin-2-yl}-3,4-dihydro-2H-quinoxaline-1-carboxylic acid (5-carbamoyl-adamantan-2-yl)-amide, *trans* 4-{5-[4-(2-Methanesulfonyl-ethyl)-piperazin-1-yl]-pyridin-2-yl}-3,4-dihydro-2H-quinoxaline-1-carboxylic acid (5-carbamoyl-adamantan-2-yl)-amide,
trans-4-{5-[4-(1-Ethyl-propyl)-piperazin-1-yl]-pyridin-2-yl}-3,4-dihydro-2H-quinoxaline-1-carboxylic acid (5-carbamoyl-adamantan-2-yl)-amide,
trans-4-[5-(1,1-Dioxo-1lambda6-thiomorpholin-4-yl)-pyridin-2-yl]-3,4-dihydro-2H-quinoxaline-1-carboxylic acid (5-carbamoyl-adamantan-2-yl)-amide,
trans-4-{5-[4-(Tetrahydro-pyran-4-yl)-piperazin-1-yl]-pyridin-2-yl}-3,4-dihydro-2H-quinoxaline-1-carboxylic acid (5-carbamoyl-adamantan-2-yl)-amide,
trans-4-[5-(4-Cyclopropanesulfonyl-piperazin-1-yl)-3-methyf-pyridin-2-yl]-3,4-dihydro-2H-quinoxaline-1-carboxylic acid (5-carbamoyl-adamantan-2-yl)-amide,
trans-4-{5-[4-(2-Hydroxy-1,1-dimethyl-ethyl)-piperazin-1-yl]-pyridin-2-yl}-3,4-dihydro-2H-quinoxaline-1-carboxylic acid (5-carbamoyl-adamantan-2-yl)-amide,
trans-4-{5-[4-(2-Fluoro-1,1-dimethyl-ethyl)-piperazin-1-yl]-pyridin-2-yl}-3,4-dihydro-2H-quinoxaline-1-carboxylic acid (5-carbamoyl-adamantan-2-yl)-amide,
trans-4-[5-(4-Trifluoromethanesulfonyl-piperazin-1-yl)-pyridin-2-yl]-3,4-dihydro-2H-quinoxaline-1-carboxylic acid (5-carbamoyl-adamantan-2-yl)-amide.

14. Procédé de préparation d'un composé de formule (I) selon l'une quelconque des revendications 1 à 13, **caractérisé en ce que** l'on fait réagir un composé de formule (IV): 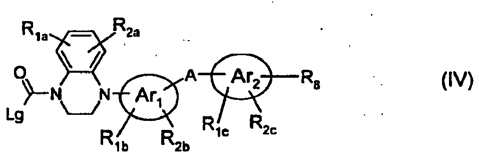 dans laquelle A, Ar₁, Ar₂, R_{1a,b,c}, R_{2a,b,c} et R₈ sont tels que définis dans l'une quelconque des revendications 1 à 6 et 9 à 12 et Lg représente un groupe partant, avec un composé de formule (V) : 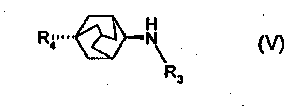 dans laquelle R₃ et R₄ sont tels que définis dans l'une quelconque des revendications 1, 7, 8 et 10 à 12.

15. Procédé de préparation selon la revendication 14, **caractérisé en ce que** le composé de formule (IV) est obtenu en faisant réagir un composé de formule (II) : 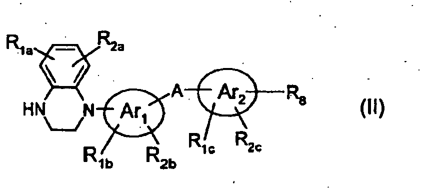 dans laquelle A, Ar₁, Ar₂, R_{1a,b,c}, R_{2a,b,c} et R₈ sont tels que définis dans l'une quelconque des revendications 1 à 6 et 9 à 12, avec un composé de formule Lg-CO-Lg, où Lg représente des groupes partants, en présence d'une base.

16. Procédé de préparation d'un composé de formule (I) selon l'une quelconque des revendications 1 à 13, dans lequel A représente une liaison, **caractérisé en ce que** l'on fait réagir un composé de formule (XXVII) : 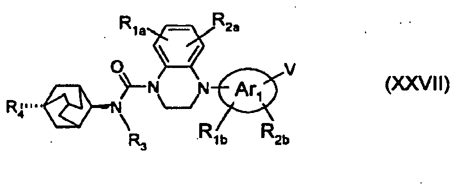 dans laquelle Ar₁, R_{1a,b,} R_{2a,b}, R₃ et R₄ sont tels que définis dans l'une quelconque des revendications 1, 3, 5, 7, 8 et 10 à 13 et V représente un groupe partant, avec un composé de formule (XVI) : 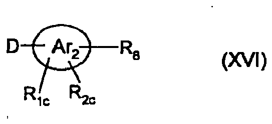 dans laquelle Ar₂, R_{1c}, R_{2c} et R₈ sont tels que définis dans l'une quelconque des revendications 1, 4, 6, 9 et 10 à 13 et D représente un groupe organométallique, en présence d'un catalyseur organométallique et d'une base.

17. Procédé de préparation d'un composé de formule (I) selon l'une quelconque des revendications 1 à 13, dans lequel R₄ représente une fonction amide, **caractérisé en ce que** l'on fait réagir un composé de formule (XXXIX) : 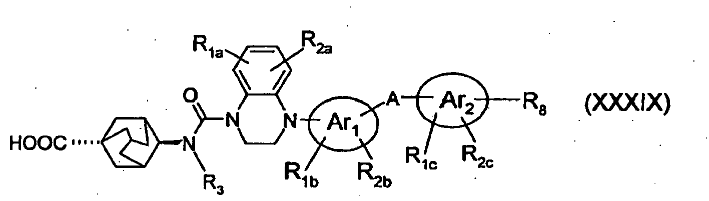 dans laquelle A, Ar1, Ar₂, R_{1a,b,c}, R_{2a,b,c}, R₃ et R₈ sont tels que définis dans l'une quelconque des revendications 1 à 7 et 9 à 13,
avec un composé de formule (XXXVI), H-NP₆R₇, où R₆ et R₇ sont tels que définis dans la revendication 1, en présence d'un agent de couplage peptidique et d'une base.

18. Procédé de préparation d'un composé de formule (I) selon l'une quelconque des revendications 1 à 13, **caractérisé en ce que** l'on fait réagir un composé de formule (XLII) : 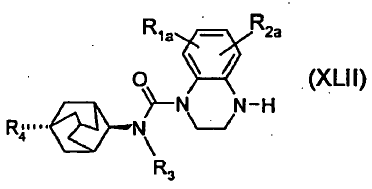 dans laquelle R_{1a,} R_{2a,} R₃ et R₄ sont tels que définis dans l'une quelconque des revendications 1, 5, 7, 8 et 10 à 12,
avec un composé de formule (VII) : 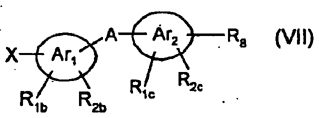 dans laquelle dans laquelle A, Ar1, Ar₂, R_{1b,c}, R_{2b,c} et R₈ sont tels que définis dans l'une quelconque des revendications 1, 2, 3, 4, 5, 6, 9 et 10 à 12 et X est un groupe partant, en présence d'un catalyseur organométallique et d'une base.

19. Procédé de préparation d'un composé de formule (I) selon l'une quelconque des revendications 1 à 13, dans lequel Ar₂ est un groupe pipérazine, A est une liaison simple connectée directement sur un des deux azotes de la pipérazine, R_{1c} est connecté sur l'autre atome d'azote de la pipérazine; **caractérisé en ce que** l'on fait réagir un composé de formule (LII) : 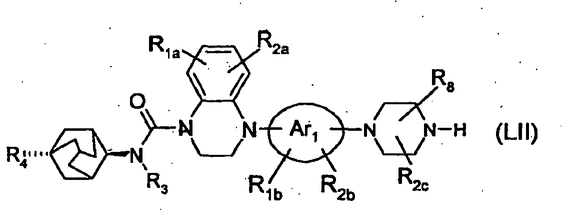 dans laquelle Ar1, R_{1a,b}, R_{2a,b,c}, R₃, R₄ et R₈ sont tels que définis dans l'une quelconque des revendications 1, 4, 6, 9 et 10 à 12 avec un composé de formule (LIII) Lg-R_{1C} en présence d'une base ou avec un composé (LIV) O=R_{1C} en présence d'un réducteur.

20. Procédé de préparation d'un composé de formule (I) selon l'une quelconque des revendications 1 à 13, dans lequel R₄ est un atome d'halogène, **caractérisé en ce que** l'on fait réagir un composé de formule (LVI) : 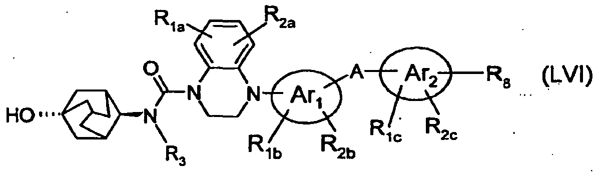 dans laquelle A, Ar1, Ar₂, R_{1a,b,c}, R_{2a,b,c}, R₃ et R₈ sont tels que définis dans l'une quelconque des revendications 1 à 7 et 9 à 12 avec un réactif d'halogénation.

21. Composés de formules (II) et (IV) : 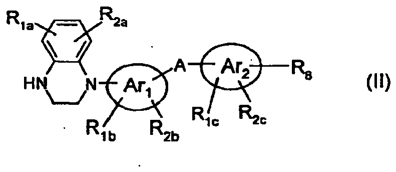 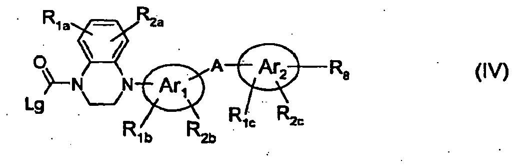 dans lesquelles A, Ar₁, Ar₂, R_{1a,b,c}, R_{2a,b,c} et R₈ sont tels que définis dans l'une quelconque des revendications 1 à 6 et 9 à 12 et Lg représente un groupe partant.

22. Composé de formule (XXVII) : 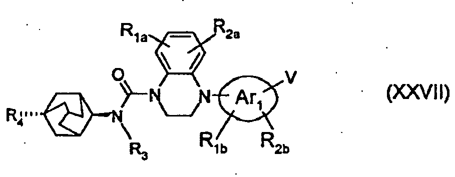 dans laquelle Ar₁, R_{1a,b}, R_{2a,b}, R₃ et R₄ sont tels que définis dans l'une quelconque des revendications 1, 3, 5, 7, 8 et 10 à 12 et V représente un groupe partant,

23. Médicament, **caractérisé en ce qu'**il comprend un composé de formule (I) selon l'une quelconque des revendications 1 à 13, ou un sel d'addition de ce composé à un acide ou à une base pharmaceutiquement acceptable, ou encore un solvat du composé de formule (I).

24. Composition pharmaceutique, **caractérisée en ce qu'**elle comprend un composé de formule (I) selon l'une quelconque des revendications 1 à 13, ou un sel pharmaceutiquement acceptable ou un solvat de ce composé, ainsi qu'au moins un excipient pharmaceutiquement acceptable.

25. Composé de formule (I) selon l'une quelconque des revendications 1 à 13 pour son utilisation dans le traitement et la prévention de l'obésité, des diabètes, des troubles de la microcirculation, de la résistance à l'insuline, du syndrome métabolique, du syndrome de Cushing, de l'hypertension, de l'athérosclérose, de la cognition et de la démence, des glaucomes, de l'ostéoporose, de la lipodystrophie, de l'hypertrophie cardiaque, de l'insuffisance cardiaque, de maladies hépatiques, de certaines maladies infectieuses en augmentant l'efficacité du système immunitaire ou des plaies en favorisant la cicatrisation.

## Patentansprüche

1. Verbindung entsprechend der Formel (I): 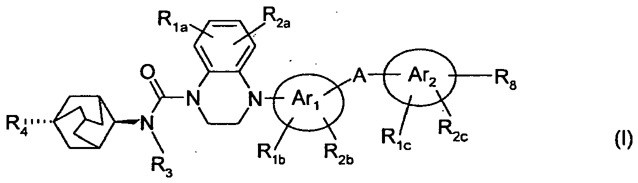 worin:
- A eine Bindung, ein Sauerstoffatom oder eine -O-CH₂-Gruppe darstellt,
- Ar₁ eine Phenyl- oder Heteroarylgruppe darstellt,
- Ar₂ eine Phenylgruppe, eine Heteroarylgruppe oder eine Heterocycloalkylgruppe darstellt,
- R_{1a,b,c} und R_{2a,b,c}, die gleich oder verschieden sind, jeweils ein Wasserstoff- oder Halogenatom oder eine Alkyl-, Cycloalkyl-, -Alkyl-cycloalkylgruppe, gegebenenfalls mit einem oder mehreren Halogenatom(en) substituiert, -OR₅ (Hydroxy oder Alkoxy), Hydroxy-alkyl, Alkoxy-alkyl, Alkoxy-alkoxy, Halogenalkyl, -O-halogenalkyl, Oxo, -CO-alkyl, -CO-alkyl-NR₆R₇, -CO-halogenalkyl, -COOR₅, Alkyl-COOR₅, -O-alkyl-COOR₅, -SO₂-alkyl, -SO₂-cycloalkyl, -SO₂-alkyl-cycloalkyl, -SO₂-alkyl-OR₅, -SO₂-alkyl-COOR₅, -SO₂-alkyl-NR₆R₇, -SO₂-halogenalkyl, Alkyl-SO₂-alkyl, -SO₂-NR₆R₇, -SO₂-alkyl-alkoxy-alkoxy, -CONR₆R₇, -Alkyl-CONR₆R₇ oder -O-alkyl-NR₆R₇ darstellen oder R₁ₐ, R_{1b}, R_{1c} jeweils an R₂ₐ, R_{2b}, R_{2c} und an das Kohlenstoffatom, das sie trägt, gebunden sind und -O-alkyl-O-darstellen;
- R₃ ein Wasserstoffatom oder eine Alkylgruppe darstellt,
- R₄ ein Wasserstoff- oder Halogenatom oder eine Cyanogruppe, -OR₅, Hydroxy-alkyl, -COOR₅, -NR₆R₇, -CONR₆R₇, -SO₂-alkyl oder -SO₂-NR₆R₇, -NR₆-COOR₅, -NR₆-COR₅, -CO-NR₆-alkyl-OR₅ darstellt;
- R₅, R₆ und R₇, die gleich oder verschieden sind, jeweils ein Wasserstoffatom, eine Alkylgruppe oder eine -Alkyl-phenylgruppe darstellen, und
- R₈ ein Wasserstoffatom, eine Alkylgruppe oder eine Gruppe der Formel -B-Het darstellt, worin B abwesend sein oder eine Bindung, ein Sauerstoffatom oder eine Gruppe -CO- oder -SO₂-(CH₂)ₙ mit n gleich 0, 1 oder 2 darstellen kann und worin Het ein Heteroaryl oder ein Heterocycloalkyl, gegebenenfalls substituiert mit 1 bis 3 Gruppe(n), ausgewählt aus den Gruppen Alkyl, -SO₂-alkyl und -COOR₅, darstellt,
in Form einer Base oder einer Säure oder in Form eines Säure- oder Basenadditionssalzes.

2. Verbindung der Formel (I) gemäß Anspruch 1, **dadurch gekennzeichnet, dass** A eine Bindung darstellt.

3. Verbindung der Formel (I) gemäß Anspruch 1 oder Anspruch 2, **dadurch gekennzeichnet, dass** Ar₁ eine Phenyl-, Pridinyl-, Pyrimidinyl-, Pyridazinyl- oder Thiazolylgruppe darstellt.

4. Verbindung der Formel (I) gemäß einem der vorhergehenden Ansprüche, **dadurch gekennzeichnet, dass** Ar₂ eine Phenyl-, Pyridinyl-, Pyrimidinyl-, Piperidinyl-, Piperazinyl-, Homopiperazinyl-, 3,8-Diazabicyclo[3.2.1]octan-, Morpholinyl, Thiomorpholinyl-, Octahydro-pyrrolo[3,4-c]pyrrol-, 1,2,3,6-Tetrahydro-pyridin- oder 2,5-Diaza-bicyclo[2.2.1]heptangruppe darstellt.

5. Verbindung der Formel (I) gemäß einem der vorhergehenden Ansprüche, **dadurch gekennzeichnet, dass** R₁ₐ, R₂ₐ, R_{1b} und R_{2b}, die gleich oder verschieden sind, jeweils ein Wasserstoff- oder Halogenatom oder eine Alkyl- oder Alkoxygruppe darstellen.

6. Verbindung der Formel (I) gemäß einem der vorhergehenden Ansprüche, **dadurch gekennzeichnet, dass** R_{1c} und R_{2c}, die gleich oder verschieden sind, jeweils ein Wasserstoff- oder Halogenatom oder eine Alkyl-, Alkoxy- oder -SO₂-alkyl, -SO₂-cycloalkylgruppe darstellen.

7. Verbindung der Formel (I) gemäß einem der vorhergehenden Ansprüche, **dadurch gekennzeichnet, dass** R₃ ein Wasserstoffatom darstellt.

8. Verbindung der Formel (I) gemäß einem der vorhergehenden Ansprüche, **dadurch gekennzeichnet, dass** R₄ ein Wasserstoff- oder Halogenatom oder eine Cyano-, Hydroxy-, Hydroxy-alkyl-, -COOR₅- oder -CONH₂-gruppe darstellt.

9. Verbindung der Formel (I) gemäß einem der vorhergehenden Ansprüche, **dadurch gekennzeichnet, dass** R₈ ein Wasserstoffatom, eine Alkylgruppe oder eine Gruppe der Formel -B-Het darstellt, worin B abwesend sein oder eine Bindung, ein Sauerstoffatom oder eine Gruppe -CO- oder -SO₂-(CH₂)ₙ- mit n gleich 0, 1 oder 2 darstellen kann und worin Het eine Pyrrolidinyl-, Piperidinyl-, Piperazinyl-, Tetrahydropyranyl-, Morpholinyl- oder Pyridinylgruppe darstellt, wobei die Gruppe Het gegebenenfalls mit einer Alkyl-, -SO₂-alkyl- oder -COOR₅-gruppe substituiert ist.

10. Verbindung der Formel (I) gemäß Anspruch 1, **dadurch gekennzeichnet, dass**:
- A eine Bindung, ein Sauerstoffatom oder eine -O-CH₂-Gruppe darstellt,
- Ar₁ eine Phenyl- oder Heteroarylgruppe darstellt,
- Ar₂ eine Phenylgruppe darstellt,
- R_{1a,b,c} und R_{2a,b,c}, die gleich oder verschieden sind, jeweils ein Wasserstoff- oder Halogenatom oder eine Alkyl-, Cycloalkyl-, -Alkyl-cycloalkylgruppe, gegebenenfalls mit einem oder mehreren Halogenatom(en) substituiert, -OR₅ (Hydroxy oder Alkoxy), Hydroxy-alkyl, Alkoxy-alkyl, Halogenalkyl, -O-halogenalkyl, Oxo, -CO-alkyl, -CO-alkyl-NR₆R₇, -COOR₅, Alkyl-COOR₅, -SO₂-alkyl, -SO₂-cycloalkyl, -SO₂-alkyl-cycloalkyl, -SO₂-alkyl-OR₅, -SO₂-alkyl-COOR₅, -SO₂-alkyl-NR₆R₇, -SO₂-halogenalkyl, Alkyl-SO₂-alkyl, -SO₂-alkyl-alkoxy-alkoxy, -CONR₆R₇ oder -O-alkyl-NR₆R₇ darstellen oder R₁ₐ, R_{1b}, R_{1c} jeweils an R₂ₐ, R_{2b}, R_{2c} und an das Kohlenstoffatom, das sie trägt, gebunden sind und -O-alkyl-O- darstellen;
- R₃ ein Wasserstoffatom oder ein Alkyl darstellt,
- R₄ ein Wasserstoff- oder Halogenatom oder eine Cyanogruppe, -OR₅, Hydroxy-alkyl, -COOR₅, -CONR₆R₇, -NR₆-COOR₅, -NR₆-COR₅ oder -CO-NR₆-alkyl-OR₅ darstellt;
- R₅, R₆ und R₇, die gleich oder verschieden sind, jeweils ein Wasserstoffatom, eine Alkylgruppe oder eine -Alkyl-phenylgruppe darstellen, und
- R₈ ein Wasserstoffatom, eine Alkylgruppe oder eine Gruppe der Formel -B-Het darstellt, worin B abwesend sein oder eine Bindung, ein Sauerstoffatom oder eine Gruppe -CO- oder -SO₂-(CH₂)ₙ- mit n gleich 0, 1 oder 2 darstellen kann und worin Het eine Pyrrolidinyl-, Piperidinyl-, Piperazinyl-, Tetrahydropyranyl-, Morpholinyl- oder Pyridinylgruppe darstellt, wobei die Gruppe Het gegebenenfalls mit 1 bis 3 Gruppe(n), ausgewählt aus Alkyl-, -SO₂-alkyl- oder -COOR₅-gruppen substituiert ist,
in Form einer Base oder einer Säure oder in Form eines Säure- oder Basenadditionssalzes.

11. Verbindung der Formel (I) gemäß Anspruch 1, **dadurch gekennzeichnet, dass**:
- A eine Bindung, ein Sauerstoffatom oder eine -O-CH₂-Gruppe darstellt,
- Ar₁ eine Phenyl- oder Heteroarylgruppe darstellt,
- Ar₂ eine Heteroarylgruppe darstellt,
- R_{1a,b,c} und R_{2a,b,c}, die gleich oder verschieden sind, jeweils ein Wasserstoff- oder Halogenatom oder eine Alkyl-, Cycloalkyl-, Alkyl-cycloalkylgruppe, gegebenenfalls mit einem oder mehreren Halogenatom(en) substituiert, -OR₅ (Hydroxy oder Alkoxy), Hydroxy-alkyl, Alkoxy-alkyl, Halogenalkyl, -O-halogenalkyl, Oxo, -CO-alkyl, -CO-alkyl-NR₆R₇, -COOR₅, Alkyl-COOR₅, -SO₂-alkyl, -SO₂-cycloalkyl, -SO₂-alkyl-cycloalkyl, -SO₂-alkyl-OR₅, -SO₂-alkyl-COOR₅, -SO₂-alkyl-NR₆R₇, -SO₂-halogenalkyl, Alkyl-SO₂-alkyl-, -SO₂-alkyl-alkoxy-alkoxy, -CONR₆R₇ oder -O-alkyl-NR₆R₇ darstellen oder R₁ₐ, R_{1b}, R_{1c} jeweils an R₂ₐ, R_{2b}, R_{2c} und an das Kohlenstoffatom, das sie trägt, gebunden sind und -O-alkyl-O- darstellen;
- R₃ ein Wasserstoffatom oder ein Alkyl darstellt,
- R₄ ein Wasserstoff- oder Halogenatom oder eine Cyanogruppe, -OR₅, Hydroxy-alkyl, -COOR₅, -CONR₆R₇, -NR₆-COOR₅, -NR₆-COR₅ oder -CO-NR₆-alkyl-OR₅ darstellt;
- R₅, R₆ und R₇, die gleich oder verschieden sind, jeweils ein Wasserstoffatom, eine Alkylgruppe oder eine -Alkyl-phenylgruppe darstellen, und
- R₈ ein Wasserstoffatom, eine Alkylgruppe oder eine Gruppe der Formel -B-Het darstellt, worin B abwesend sein oder eine Bindung, ein Sauerstoffatom oder eine Gruppe -CO- oder -SO₂-(CH₂)ₙ- mit n gleich 0, 1 oder 2 darstellen kann und worin Het eine Pyrrolidinyl-, Piperidinyl-, Piperazinyl-, Tetrahydropyranyl-, Morpholinyl- oder Pyridinylgruppe darstellt, wobei die Gruppe Het gegebenenfalls mit 1 bis 3 Gruppe(n), ausgewählt aus Alkyl-, -SO₂-alkyl- und -COOR₅-gruppen substituiert ist,
in Form einer Base oder einer Säure oder in Form eines Säure- oder Basenadditionssalzes.

12. Verbindung der Formel (I) gemäß Anspruch 1, **dadurch gekennzeichnet, dass**:
- A eine Bindung, ein Sauerstoffatom oder eine -O-CH₂-Gruppe darstellt,
- Ar₁ eine Phenyl- oder Heteroarylgruppe darstellt,
- Ar₂ eine Heterocycloalkylgruppe (wie zum Beispiel eine Piperidinyl-, Piperazinyl-, Homopiperazinyl-, 3,8-Diazabicyclo[3.2.1]octan-, Morpholinyl-, Thiomorpholinyl-, Octahydro-pyrrolo[3,4-c]pyrrol-, 1,2,3,6-Tetrahydro-pyridin- oder 2,5-Diaza-bicyclo[2.2.1]heptangruppe) darstellt,
- R_{1a,b,c} und R_{2a,b,c}, die gleich oder verschieden sind, jeweils ein Wasserstoff- oder Halogenatom oder eine Alkyl-, Cycloalkyl-, -Alkyl-cycloalkylgruppe, gegebenenfalls mit einem oder mehreren Halogenatom(en) substituiert, -ORₛ (Hydroxy oder Alkoxy), Hydroxy-alkyl, Alkoxy-alkyl, Halogenalkyl, -O-halogenalkyl, Oxo, -CO-alkyl, -CO-alkyl-NR₆R₇, -COOR₅, Alkyl-COOR₅, -SO₂-alkyl, -So₂-cycloalkyl, -SO₂-alkyl-cycloalkyl, -SO₂-alkyl-OR₅, -SO₂-alkyl-COOR₅, -So₂-alkyl-NR₆R₇, -SO₂-halogenalkyl, Alkyl-SO₂-alkyl, -So₂-alkyl-alkoxy-alkoxy, -CONR₆R₇ oder -O-alkyl-NR₆R₇ darstellen oder R₁ₐ, R_{1b}, R_{1c} jeweils an R₂ₐ, R_{2b}, R_{2c} und an das Kohlenstoffatom, das sie trägt, gebunden sind und -O-alkyl-O- darstellen;
- R₃ ein Wasserstoffatom oder ein Alkyl darstellt,
- R₄ ein Wasserstoff- oder Halogenatom oder eine Cyanogruppe, -OR₅, Hydroxy-alkyl, -COOR₅, -CONR₆R₇, -NR₆-COOR₅, -NR₆-COR₅ oder -CO-NR₆-alkyl-OR₅ darstellt;
- R₅, R₆ und R₇, die gleich oder verschieden sind, jeweils ein Wasserstoffatom, eine Alkylgruppe oder eine -Alkyl-phenylgruppe darstellen, und
- R₆ ein Wasserstoffatom, eine Alkylgruppe oder eine Gruppe der Formel -B-Het darstellt, worin B abwesend sein oder eine Bindung, ein Sauerstoffatom oder eine Gruppe -CO- oder -SO₂-(CH₂)ₙ- mit n gleich 0, 1 oder 2 darstellen kann und worin Het eine Pyrrolidinyl-, Piperidinyl-, Piperazinyl-, Tetrahydropyranyl-, Morpholinyl- oder Pyridinylgruppe darstellt, wobei die Gruppe Het gegebenenfalls mit 1 bis 3 Gruppe(n), ausgewählt aus Alkyl-, -SO₂-alkyl- oder -COOR₅-gruppen substituiert ist,
in Form einer Base oder einer Säure oder in Form eines Säure- oder Basenadditionssalzes.

13. Verbindung der Formel (I) gemäß Anspruch 1, **dadurch gekennzeichnet, dass** sie ausgewählt ist aus:
. 4-(4-Pyridin-2-yl-phenyl)-3,4-dihydro-2H-chinoxalin-1-carbonsäure-adamantan-2-ylamid,
. 4-{4-[1-(Tetrahydro-pyran-4-carbonyl)-piperidin-4-yloxy]-phenyl}-3,4-dihydro-2H-chinoxalin-1-carbonsäure-adamantan-2-ylamid,
. 4-[4-(Pyrimidin-2-ylmethoxy)-phenyl]-3,4-dihydro-2H-chinoxalin-1-carbonsäure-adamantan-2-ylamid,
. 4-{4-[1-(Morpholin-4-carbonyl)-piperidin-4-yl]-phenyl}-3,4-dihydro-2H-chinoxalin-1-carbonsäure-adamantan-2-ylamid,
. 4-[4-(4-Methoxy-pyrimidin-2-yloxy)-phenyl]-3,4-dihydro-2H-chinoxalin-1-carbonsäure-adamantan-2-ylamid,
. 4-{4-[4-(Morpholin-4-carbonyl)-phenyl]-thiazol-2-yl}-3,4-dihydro-2H-chinoxalin-1-carbonsäure-adamantan-2-ylamid,
. 4-[5-(4-Methoxy-phenyl)-pyrimidin-2-yl]-3,4-dihydro-2H-chinoxalin-1-carbonsäure-adamantan-2-ylamid,
. 4-[5-(4-Methoxy-phenyl)-pyrimidin-2-yl]-3,4-dihydro-2H-chinoxalin-1-carbonsäure-(5-hydroxy-adamantan-2-yl)-amid,
. trans-4-[4-(4-Methansulfonyl-piperazin-1-yl)-phenyl]-3,4-dihydro-2H-chinoxalin-1-carbonsäure-(5-hydroxy-adamantan-2-yl)-amid,
. 4-({4-[4-(4-Methansulfonyl-piperazin-1-yl)-phenyl]-3,4-dihydro-2H-chinoxalin-1-carbonyl}-amino)-adamantan-1-carbonsäure-methylester,
. 4-({4-[4-(4-Methansulfonyl-piperazin-1-yl)-phenyl]-3,4-dihydro-2H-chinoxalin-1-carbonyl}-amino)-adamantan-1-carbonsäure,
. 4-[4-(4-Methansulfonyl-piperazin-1-yl)-phenyl]-3,4-dihydro-2H-chinoxalin-1-carbonsäure-(5-carbamoyl-adamantan-2-yl)-amid,
. trans-4-[4-(Pyridin-2-yloxy)-phenyl]-3,4-dihydro-2H-chinoxalin-1-carbonsäure-(5-hydroxy-adamantan-2-yl)-amid,
. trans-4-[4-(4-Trifluormethyl-pyrimidin-2-yloxy)-phenyl]-3,4-dihydro-2H-chinoxalin-1-carbonsäure-(5-hydroxy-adamantan-2-yl)-amid,
. trans-4-[4-(1-Methansulfonyl-1,2,3,6-tetrahydro-pyridin-4-yl)-phenyl]-3,4-dihydro-2H-chinoxalin-1-carbonsäure-(5-hydroxy-adamantan-2-yl)-amid,
. trans-4-[4-(4-Hydroxy-1-methansulfonyl-piperidin-4-yl)-phenyl]-3,4-dihydro-2H-chinoxalin-1-carbonsäure-(5-hydroxy-adamantan-2-yl)-amid,
. trans-4-[2-(4-Methansulfonyl-piperazin-1-yl)-pyrimidin-5-yl]-3,4-dihydro-2H-chinoxalin-1-carbonsäure-(5-hydroxy-adamantan-2-yl)-amid,
. trans-4-[5-(4-Methansulfonyl-piperazin-1-yl)-pyrimidin-2-yl]-3,4-dihydro-2H-chinoxalin-1-carbonsäure-(5-hydroxy-adamantan-2-yl)-amid,
. trans-4-{4-[1-(Morpholin-4-carbonyl)-piperidin-4-yl]-phenyl}-3,4-dihydro-2H-chinoxalin-1-carbonsäure-(5-hydroxy-adamantan-2-yl)-amid,
. trans-4-{4-[4-(5-Hydroxy-adamantan-2-ylcarbamoyl)-3,4-dihydro-2H-quinoxalin-1-yl]-phenyl}-piperazin-1-carbonsäure-benzylester,
. trans-4-[4-(4-Methansulfonyl-piperazin-1-yl)-phenyl]-3,4-dihydro-2H-chinoxalin-1-carbonsäure-(5-hydroxymethyl-adamantan-2-yl)-amid,
. trans-4-[4-(5-Fluor-pyrimidin-2-yloxy)-phenyl]-3,4-dihydro-2H-chinoxalin-1-carbonsäure-(5-hydroxy-adamantan-2-yl)-amid,
. trans-4-[4-(1-Methansulfonyl-piperidin-4-yloxy)-phenyl]-3,4-dihydro-2H-chinoxalin-1-carbonsäure-(5-hydroxy-adamantan-2-yl)-amid,
. trans-4-(4-Morpholin-4-yl-phenyl)-3,4-dihydro-2H-chinoxalin-1-carbonsäure-(5-hydroxy-adamantan-2-yl)-amid,
. trans-4-[4-(1,1-Dioxo-1lambda6-thiomorpholin-4-yl)-phenyl]-3,4-dihydro-2H-chinoxalin-1-carbonsäure-(5-hydroxy-adamantan-2-yl)-amid,
. trans-4-(4-(5-Isopropoxy-pyridin-2-yl)-phenyl]-3,4-dihydro-2H-chinoxalin-1-carbonsäure-(5-hydroxy-adamantan-2-yl)-amid,
. trans-4-{4-[5-(4-lsopropyl-piperazin-1-carbonyl)-pyridin-2-yl]-phenyl}-3,4-dihydro-2H-chinoxalin-1-carbonsäure-(5-hydroxy-adamantan-2-yl)-amid,
. trans-4-[6-(4-Methansulfonyl-piperazin-1-yl)-pyridin-3-yl]-3,4-dihydro-2H-chinoxalin-1-carbonsäure-(5-hydroxy-adamantan-2-yl)-amid,
. trans-4-(4-Piperazin-1-yl-phenyl)-3,4-dihydro-2H-chinoxalin-1-carbonsäure-(5-hydroxy-adamantan-2-yl)-amid,
. trans-4-[4-(4,4-Difluor-piperidin-1-yl)-phenyl]-3,4-dihydro-2H-chinoxalin-1-carbonsäure-(5-hydroxy-adamantan-2-yl)-amid,
. trans-4-[5-(4-Methansulfonyl-piperazin-1-yl)-pyridin-2-yl]-3,4-dihydro-2H-chinoxalin-1-carbonsäure-(5-hydroxy-adamantan-2-yl)-amid,
. trans-4-[5-(4-Methansulfonyl-piperazin-1-yl)-pyridin-2-yl]-3,4-dihydro-2H-chinoxalin-1-carbonsäure-(5-hydroxy-adamantan-2-yl)-amid,
. trans-4-[2-(1-Methansulfonyl-piperidin-4-yloxy)-pyrimidin-5-yl]-3,4-dihydro-2H-chinoxalin-1-carbonsäure-(5-hydroxy-adamantan-2-yl)-amid,
. trans-4-[5-(5-Isopropoxy-pyridin-2-yl)-pyrimidin-2-yl]-3,4-dihydro-2H-chinoxalin-1-carbonsäure-(5-hydroxy-adamantan-2-yl)-amid,
. trans-4-[4-(4-Ethansulfonyl-piperazin-1-yl)-phenyl]-3,4-dihydro-2H-chinoxalin-1-carbonsäure-(5-hydroxy-adamantan-2-yl)-amid,
. trans-4-{4-[4-(2,2,2-Trifluor-ethansulfonyl)-piperazin-1-yl]-phenyl}-3,4-dihydro-2H-chinoxalin-1-carbonsäure-(5-hydroxy-adamantan-2-yl)-amid,
. trans-4-{4-[4-(Propan-2-sulfonyl)-piperazin-1-yl]-phenyl}-3,4-dihydro-2H-chinoxalin-1-carbonsäure-(4-hydroxy-adamantan-1-yl)-amid,
. trans-4-(4-{4-[4-(5-Hydroxy-adamantan-2-ylcarbamoyl)-3,4-dihydro-2H-quinoxalin-1-yl]-phenyl}-piperazin-1-sulfonyl)-piperidin-1-carbonsäure-benzylester,
. trans-4-{4-[4-(Piperidin-4-sulfonyl)-piperazin-1-y|]-phenyl}-3,4-dihydro-2H-chinoxalin-1-carbonsäure-(5-hydroxy-adamantan-2-yl)-amid,
. trans-4-[4-(4-Cyclopropansulfonyl-piperazin-1-yl)-phenyl]-3,4-dihydro-2H-chinoxalin-1-carbonsäure-(5-hydroxy-adamantan-2-yl)-amid,
. trans-4-[4-(4-Methansulfonyl-piperazin-1-yl)-phenyl]-3,4-dihydro-2H-chinoxalin-1-carbonsäure-(5-fluor-adamantan-2-yl)-amid,
. trans-4-[4-(4-Cyclobutansulfonyl-piperazin-1-yl)-phenyl]-3,4-dihydro-2H-chinoxalin-1-carbonsäure-(5-hydroxy-adamantan-2-yl)-amid,
. trans-4-{4-[4-(Propan-1-sulfonyl)-piperazin-1-yl]-phenyl}-3,4-dihydro-2H-chinoxalin-1-carbonsäure-(5-hydroxy-adamantan-2-yl)-amid,
. trans-4-{4-[4-(Butan-1-sulfonyl)-piperazin-1-yl]-phenyl}-3,4-dihydro-2H-chinoxalin-1-carbonsäure-(5-hydroxy-adamantan-2-yl)-amid,
. trans-4-{4-[4-(Morpholin-4-sulfonyl)-piperazin-1-yl]-phenyl}-3,4-dihydro-2H-chinoxalin-1-carbonsäure-(5-hydroxy-adamantan-2-yl)-amid,
. trans-4-[4-(4-Trifluormethansulfonyl-piperazin-1-yl)-phenyl]-3,4-dihydro-2H-chinoxalin-1-carbonsäure-(5-hydroxy-adamantan-2-yl)-amid,
. trans-4-[4-(1,4-Dioxa-8-aza-spiro[4.5]dec-8-yl)-phenyl]-3,4-dihydro-2H-chinoxalin-1-carbonsäure-(5-hydroxy-adamantan-2-yl)-amid,
. trans-4-[4-(4-Methansulfonyl-3,3-dimethyl-piperazin-1-yl)-phenyl]-3,4-dihydro-2H-chinoxalin-1-carbonsäure-(5-hydroxy-adamantan-2-yl)-amid,
. trans-4-[4-((3R,5S)-4-Methansulfonyl-3,5-dimethyl-piperazin-1-yl)-phenyl]-3,4-dihydro-2H-chinoxalin-1-carbonsäure-(5-hydroxy-adamäntan-2-yl)-amid,
. trans-(4-{4-[4-(5-Hydroxy-adamantan-2-ylcarbamoyl)-3,4-dihydro-2H-quinoxalin-1-yl]-phenyl}-piperazin-1-sulfonyl)-essigsäure,
. trans-(4-{4-[4-(5-Hydroxy-adamantan-2-ylcarbamoyl)-3,4-dihydro-2H-quinoxalin-1-yl]-phenyl}-piperazin-1-sulfonyl)-essigsäure-ethylester,
. trans-4-[4-(4-Hydroxy-piperidin-1-yl)-phenyl]-3,4-dihydro-2H-chinoxalin-1-carbonsäure-(5-hydroxy-adamantan-2-yl)-amid,
. trans-4-[4-(4-Methansulfonyl-[1,4]diazepan-1-yl)-phenyl]-3,4-dihydro-2H-chinoxalin-1-carbonsäure-(5-hydroxy-adamantan-2-yl)-amid,
. trans-2-(4-{4-[4-(5-Hydroxy-adamantan-2-ylcarbamoyl)-3,4-dihydro-2H-quinoxalin-1-yl]-phenyl}-piperazin-1-yl)-2-methyl-propionsäure-ethylester,
. trans-3-(4-{4-[4-(5-Hydroxy-adamantan-2-ylcarbamoyl)-3,4-dihydro-2H-quinoxalin-1-yl]-phenyl}-piperazin-1-sulfonyl)-propionsäure-methylester,
. trans-4-[4-(5-Methoxy-pyrimidin-2-yl)-phenyl]-3,4-dihydro-2H-chinoxalin-1-carbonsäure-(5-hydroxy-adamantan-2-yl)-amid,
. trans-4-{4-[4-(2-Morpholin-4-yl-ethansulfonyl)-piperazin-1-yl]-phenyl}-3,4-dihydro-2H-chinoxalin-1-carbonsäure-(5-hydroxy-adamantan-2-yl)-amid,
. trans-4-[6-(4-Methansulfonyl-piperazin-1-yl)-pyridazin-3-yl]-3,4-dihydro-2H-chinoxalin-1-carbonsäure-(5-hydroxy-adamantan-2-yl)-amid,
. trans-4-[4-(4-Methansulfonyl-piperazin-1-yl)-phenyl]-3,4-dihydro-2H-chinoxalin-1-carbonsäure-(5-hydroxy-adamantan-2-yl)-methyl-amid,
. trans-4-[4-(4-Methansulfonyl-3,5-dimethyl-piperazin-1-yl)-phenyl]-3,4-dihydro-2H-chinoxalin-1-carbonsäure-(5-hydroxy-adamantan-2-yl)-amid,
. trans-4-{4-[4-(2-Diethylamino-ethansulfonyl)-piperazin-1-yl]-phenyl}-3,4-dihydro-2H-chinoxalin-1-carbonsäure-(5-hydroxy-adamantan-2-yl)-amid,
. trans-4-[4-(3-Methansulfonyl-3,8-diaza-bicyclo[3.2.1]oct-8-yl)-phenyl]-3,4-dihydro-2H-chinoxalin-1-carbonsäure-(5-hydroxy-adamantan-2-yl)-amid,
. trans-4-[5-(4-Methoxy-phenyl)-pyrimidin-2-yl]-3,4-dihydro-2H-chinoxalin-1-carbonsäure-(5-carbamoyl-adamantan-2-yl)-amid,
. trans-4-{4-[4-(2-Methoxy-ethansulfonyl)-piperazin-1-yl]-phenyl}-3,4-dihydro-2H-chinoxalin-1-carbonsäure-(5-hydroxy-adamantan-2-yl)-amid,
. trans-4-[4-(4-Isopropoxy-piperidin-1-yl)-phenyl]-3,4-dihydro-2H-chinoxalin-1-carbonsäure-(5-hydroxy-adamantan-2-yl)-amid,
. trans-4-[5-(4-Hydroxy-phenyl)-pyrimidin-2-yl]-3,4-dihydro-2H-chinoxalin-1-carbonsäure-(5-hydroxy-adamantan-2-yl)-amid,
. trans-4-(4-Piperazin-1-yl-phenyl)-3,4-dihydro-2H-chinoxalin-1-carbonsäure-(5-carbamoyl-adamantan-2-yl)-amid,
. trans-4-(4-(5-Brom-pyrimidin-2-yloxy)-phenyl]-3,4-dihydro-2H-chinoxalin-1-carbonsäure-(5-hydroxy-adamantan-2-yl)-amid,
. trans-4-[4-(4-Cyclopropansulfonyl-piperazin-1-yl)-phenyl]-3,4-dihydro-2H-chinoxalin-1-carbonsäure-(5-carbamoyl-adamantan-2-yl)-amid,
. trans-4-[4-(4-Cyclopropansulfonyl-piperazin-1-yl)-phenyl]-3,4-dihydro-2H-chinoxalin-1-carbonsäure-(5-carbamoyl-adamantan-2-yl)-amid,
. trans-6-Chlor-4-[4-(4-methansulfonyl-piperazin-1-yl)-phenyl]-3,4-dihydro-2H-chinoxalin-1-carbonsäure-(5-hydroxy-adamantan-2-yl)-amid,
. trans-4-[4-(5-Methansulfonyl-hexahydro-pyrrolo[3,4-c]pyrrol-2-yl)-phenyl]-3,4-dihydro-2H-chinoxalin-1-carbonsäure-(5-hydroxy-adamantan-2-yl)-amid,
. trans-4-{4-[4-(2-Amino-ethansulfonyl)-piperazin-1-yl]-phenyl}-3,4-dihydro-2H-chinoxalin-1-carbonsäure-(5-hydroxy-adamantan-2-yl)-amid,
. trans-4-(4-Morpholin-4-yl-phenyl)-3,4-dihydro-2H-chinoxalin-1-carbonsäure-(5-carbamoyl-adamantan-2-yl)-amid,
. trans-4-{4-[5-(Piperidin-4-yloxy)-pyridin-2-yl]-phenyl}-3,4-dihydro-2H-chinoxalin-1-carbonsäure-(5-hydroxy-adamantan-2-yl)-amid,
. trans-4-{4-[5-(2-Amino-ethoxy)-pyridin-2-yl]-phenyl}-3,4-dihydro-2H-chinoxalin-1-carbonsäure-(5-hydroxy-adamantan-2-yl)-amid,
. trans-4-{4-[4-(2-Hydroxy-ethansulfonyl)-piperazin-1-yl]-phenyl}-3,4-dihydro-2H-chinoxalin-1-carbonsäure-(5-hydroxy-adamantan-2-yl)-amid,
. trans-4-[4-(8-Methansulfonyl-3,8-diaza-bicyclo[3.2.1]oct-3-yl)-phenyl]-3,4-dihydro-2H-chinoxalin-1-carbonsäure-(5-carbamoyl-adamantan-2-yl)-amid,
. trans-4-[4-(8-Methansulfonyl-3,8-diaza-bicyclo[3.2.1]oct-3-yl)-phenyl]-3,4-dihydro-2H-chinoxalin-1-carbonsäure-(5-hydroxy-adamantan-2-yl)-amid,
. trans-4-[5-(4-Isopropoxy-phenyl)-pyrimidin-2-yl]-3,4-dihydro-2H-chinoxalin-1-carbonsäure-(5-hydroxy-adamantan-2-yl)-amid,
. trans-4-{5-[4-(2,2,2-Trifluor-ethansulfonyl)-piperazin-1-yl]-pyridin-2-yl}-3,4-dihydro-2H-chinoxalin-1-carbonsäure-(5-hydroxy-adamantan-2-yl)-amid,
. trans-4-{5-[4-(2,2,2-Trifluor-ethansulfonyl)-piperazin-1-yl]-pyridin-2-yl}-3,4-dihydro-2H-chinoxalin-1-carbonsäure-(5-carbamoyl-adamantan-2-yl)-amid,
. trans-3-(4-{4-[4-(5-Hydroxy-adamantan-2-ylcarbamoyl)-3,4-dihydro-2H-quinoxalin-1-yl]-phenyl}-piperazin-1-sulfonyl)-propionsäure,
. trans-4-[4-(4-Methansulfonyl-piperazin-1-yl)-phenylj-3,4-dihydro-2H-chinoxalin-1-carbonsäure [5-(2-hydroxy-ethylcarbamoyl)-adamantan-2-yl]-amid,
. trans-4-[4-(5-Isobutoxy-pyridin-2-yl)-phenyl]-3,4-dihydro-2H-chinoxalin-1-carbonsäure-(5-hydroxy-adamantan-2-yl)-amid,
. trans-4-[4-(5-sec-Butoxy-pyridin-2-yl)-phenyl]-3,4-dihydro-2H-chinoxalin-1-carbonsäure-(5-hydroxy-adamantan-2-yl)-amid,
. trans-4-[4-(5-lsopropoxy-pyridin-2-yl)-phenyl]-3,4-dihydro-2H-chinoxalin-1-carbonsäure-(5-carbamoyl-adamantan-2-yl)-amid,
. trans-[4-({4-[4-(4-Methansulfonyl-piperazin-1-yl)-phenyl]-3,4-dihydro-2H-chinoxalin-1-carbonyl}-amino)-adamantan-1-yl]-carbaminsäure-methylester,
. trans-4-[5-(4-Ethansulfonyl-piperazin-1-yl)-pyridin-2-yl]-3,4-dihydro-2H-chinoxalin-1-carbonsäure-(5-hydroxy-adamantan-2-yl)-amid,
. trans-4-[5-(4-Cyclopropansulfonyl-piperazin-1-yl)-pyridin-2-yl]-3,4-dihydro-2H-chinoxalin-1-carbonsäure-(5-hydroxy-adamantan-2-yl)-amid,
. trans-4-[5-(4-Cyclopropansulfonyl-piperazin-1-yl)-pyridin-2-yl]-3,4-dihydro-2H-chinoxalin-1-carbonsäure-(5-carbamoyl-adamantan-2-yl)-amid,
. trans-4-{5-[4-(Propan-2-sulfonyl)-piperazin-1-yl]-pyridin-2-yl}-3,4-dihydro-2H-chinoxalin-1-carbonsäure-(5-hydroxy-adamantan-2-yl)-amid,
. trans-4-{4-[4-(Pyridin-4-yloxy)-piperidin-1-yl]-phenyl}-3,4-dihydro-2H-chinoxalin-1-carbonsäure-(5-hydroxy-adamantan-2-yl)-amid,
. trans-4-{4-[4-(2-Methoxy-ethansulfonyl)-piperazin-1-yl]-phenyl}-3,4-dihydro-2H-chinoxalin-1-carbonsäure-(5-carbamoyl-adamantan-2-yl)-amid,
. trans-4-{4-[4-(2-Amino-ethansulfonyl)-piperazin-1-yl]-phenyl}-3,4-dihydro-2H-chinoxalin-1-carbonsäure-(5-carbamoyl-adamantan-2-yl)-amid,
. trans-4-{4-[4-(2-Diethylamino-ethansulfonyl)-piperazin-1-yl]-phenyl}-3,4-dihydro-2H-chinoxalin-1-carbonsäure-(5-carbamoyl-adamantan-2-yl)-amid,
. trans-4-{4-[4-(Piperidin-4-sulfonyl)-piperazin-1-yl]-phenyl}-3,4-dihydro-2H-chinoxalin-1-carbonsäure-(5-carbamoyl-adamantan-2-yl)-amid,
. trans-4-[4-(4-tert-Butyl-piperazin-1-yl)-phenyl]-3,4-dihydro-2H-chinoxalin-1-carbonsäure-(5-carbamoyl-adamantan-2-yl)-amid,
. trans-4-[5-(4-Methansulfonyl-piperazin-1-yl)-pyridin-2-yl]-3,4-dihydro-2H-chinoxalin-1-carbonsäure-(5-carbamoyl-adamantan-2-yl)-amid,
. trans-4-{5-[4-(Propan-1-sulfonyl)-piperazin-1-yl]-pyridin-2-yl}-3,4-dihydro-2H-chinoxalin-1-carbonsäure-(5-hydroxy-adamantan-2-yl)-amid,
. trans-4-{5-[4-(2-Methyl-propan-1-sulfonyl)-piperazin-1-yl]-pyridin-2-yl}-3,4-dihydro-2H-chinoxalin-1-carbonsäure-(5-hydroxy-adamantan-2-yl)-amid,
. trans-4-(5-Morpholin-4-yl-pyridin-2-yl)-3,4-dihydro-2H-chinoxalin-1-carbonsäure-(5-hydroxy-adamantan-2-yl)-amid,
. trans-4-{4-[4-(2-Hydroxy-ethansulfonyl)-piperazin-1-yl]-phenyl}-3,4-dihydro-2H-chinoxalin-1-carbonsäure-(5-carbamoyl-adamantan-2-yl)-amid,
. trans-4-{4-[4-(Piperidin-4-yloxy)-piperidin-1-yl]-phenyl}-3,4-dihydro-2H-chinoxalin-1-carbonsäure-(5-hydroxy-adamantan-2-yl)-amid,
. trans-4-[4-(4-Dimethylcarbamoyl-piperazin-1-yl)-phenyl]-3,4-dihydro-2H-chinoxalin-1-carbonsäure-(5-hydroxy-adamantan-2-yl)-amid,
. trans-4-[6-(4-Cyclopropansulfonyl-piperazin-1-yl)-pyridin-3-yl]-3,4-dihydro-2H-chinoxalin-1-carbonsäure-(5-carbamoyl-adamantan-2-yl)-amid,
. trans-4-[4-(4-Ethansulfonyl-piperazin-1-yl)-phenyl]-3,4-dihydro-2H-chinoxalin-1-carbonsäure-(5-carbamoyl-adamantan-2-yl)-amid,
. trans-4-{4-[4-(2,2,2-Trifluor-ethansulfonyl)-piperazin-1-yl]-phenyl}-3,4-dihydro-2H-chinoxalin-1-carbonsäure-(5-carbamoyl-adamantan-2-yl)-amid,
. trans-4-{4-[4-(2-Amino-acetyl)-piperazin-1-yl]-phenyl}-3,4-dihydro-2H-chinoxalin-1-carbonsäure-(5-carbamoyl-adamantan-2-yl)-amid,
. trans-4-[4-(5-Hydroxymethyl-pyridin-2-yl)-phenyl]-3,4-dihydro-2H-chinoxalin-1-carbonsäure-(5-carbamoyl-adamantan-2-yl)-amid,
. trans-4-[5-(4-Cyclopentansulfonyl-piperazin-1-yl)-pyridin-2-yl]-3,4-dihydro-2H-chinoxalin-1-carbonsäure-(5-hydroxy-adamantan-2-yl)-amid,
. trans-4-[4-(4-Isopropoxy-piperidin-1-yl)-phenyl]-3,4-dihydro-2H-chinoxalin-1-carbonsäure-(5-carbamoyl-adamantan-2-yl)-amid,
. trans-4-{4-[5-(2,2,2-Trifluor-ethoxy)-pyridin-2-yl]-phenyl}-3,4-dihydro-2H-chinoxalin-1-carbonsäure-(5-hydroxy-adamantan-2-yl)-amid,
. trans-4-{4-[5-(2,2,2-Trifluor-ethoxy)-pyridin-2-yl]-phenyl}-3,4-dihydro-2H-chinoxalin-1-carbonsäure-(5-carbamoyl-adamantan-2-yl)-amid,
. trans-4-(5-(4-Isopropoxy-phenyl)-pyrimidin-2-yl]-3,4-dihydro-2H-chinoxalin-1-carbonsäure-(5-carbamoyl-adamantan-2-yl)-amid,
. trans-4-{5-[4-(Piperidin-4-yloxy)-phenyl]-pyrimidin-2-yl}-3,4-dihydro-2H-chinoxalin-1-carbonsäure-(5-carbamoyl-adamantan-2-yl)-amid,
. trans-4-[5-(4-Trifluormethansulfonyl-piperazin-1-yl)-pyridin-2-yl]-3,4-dihydro-2H-chinoxalin-1-carbonsäure-(5-hydroxy-adamantan-2-yl)-amid,
. trans-4-[5-(4-tert-Butyl-piperazin-1-yl)-pyridin-2-yl]-3,4-dihydro-2H-chinoxalin-1-carbonsäure-(5-hydroxy-adamantan-2-yl)-amid,
. trans-4-[6-(4-Ethansulfonyl-piperazin-1-yl)-pyridin-3-yl]-3,4-dihydro-2H-chinoxalin-1-carbonsäure-(5-carbamoyl-adamantan-2-yl)-amid,
. trans-4-[5-(4-Cyclopropyl-piperazin-1-yl)-pyridin-2-yl]-3,4-dihydro-2H-chinoxalin-1-carbonsäure-(5-hydroxy-adamantan-2-yl)-amid,
. trans-4-[5-(4-Cyclobutansulfonyl-piperazin-1-yl)-pyridin-2-yl]-3,4-dihydro-2H-chinoxalin-1-carbonsäure-(5-hydroxy-adamantan-2-yl)-amid,
. trans-4-[5-(4-Acetyl-piperazin-1-yl)-pyridin-2-yl]-3,4-dihydro-2H-chinoxalin-1-carbonsäure-(5-hydroxy-adamantan-2-yl)-amid,
. trans-4-{4-[4-(1-Ethyl-propyl)-piperazin-1-yl]-phenyl}-3,4-dihydro-2H-chinoxalin-1-carbonsäure-(5-carbamoyl-adamantan-2-yl)-amid,
. trans-4-{5-[4-(2-Methyl-propan-1-sulfonyl)-piperazin-1-yl]-pyridin-2-yl}-3,4-dihydro-2H-chinoxalin-1-carbonsäure-(5-carbamoyl-adamantan-2-yl)-amid,
. trans-4-{5-[4-(3,3,3-Trifluor-propan-1-sulfonyl)-piperazin-1-yl]-pyridin-2-yl}-3,4-dihydro-2H-chinoxalin-1-carbonsäure-(5-hydroxy-adamantan-2-yl)-amid,
. trans-4-{4-[4-(2,2,2-Trifluor-ethyl)-piperazin-1-yl]-phenyl}-3,4-dihydro-2H-chirioxalin-1-carbonsäure-(5-carbamoyl-adamantan-2-yl)-amid,
. trans-4-(4-{4-[2-(2-Methoxy-ethoxy)-ethansulfonyl]-piperazin-1-yl}-phenyl)-3,4-dihydro-2H-chinoxalin-1-carbonsäure-(5-carbamoyl-adamantan-2-yl)-amid,
. trans-4-{5-[4-(2-Isopropoxy-ethansulfonyl)-piperazin-1-yl]-pyridin-2-yl}-3,4-dihydro-2H-chinoxalin-1-carbonsäure-(5-carbamoyl-adamantan-2-yl)-amid,
. 4-[5-(4-Cyclopropylmethansulfonyl-piperazin-1-yl)-pyridin-2-yl]-3,4-dihydro-2H-chinoxalin-1-carbonsäure-(5-hydroxy-adamantan-2-yl)-amid,
. trans-4-[5-(4-tert-Butyl-piperazin-1-yl)-pyridin-2-yl]-3,4-dihydro-2H-chinoxalin-1-carbonsäure-(5-carbamoyl-adamantan-2-yl)-amid,
. trans-4-[5-(4-Cyclopropyl-piperazin-1-yl)-pyridin-2-yl]-3,4-dihydro-2H-chinoxalin-1-carbonsäure-(5-carbamoyl-adamantan-2-yl)-amid,
. trans-4-{4-[4-(Tetrahydro-pyran-4-yl)-piperazin-1-yl]-phenyl}-3,4-dihydro-2H-chinoxalin-1-carbonsäure-(5-carbamoyl-adamantan-2-yl)-amid,
. trans-4-{5-[4-(2-Methoxy-ethansulfonyl)-piperazin-1-yl]-pyridin-2-yl}-3,4-dihydro-2H-chinoxalin-1-carbonsäure-(5-carbamoyl-adamantan-2-yl)-amid,
. trans-4-[4-(7-Methansulfonyl-2,7-diaza-spiro[4.4]non-2-yl)-phenyl]-3,4-dihydro-2H-chinoxalin-1-carbonsäure-(5-hydroxy-adamantan-2-yl)-amid,
. trans-4-{5-[4-(2,2-Dimethyl-propyl)-piperazin-1-yl]-pyridin-2-yl}-3,4-dihydro-2H-chinoxalin-1-carbonsäure-(5-hydroxy-adamantan-2-yl)-amid,
. trans-4-{5-[4-(2-Methoxy-1-methoxymethyl-ethyl)-piperazin-1-yl]-pyridin-2-yl}-3,4-dihydro-2H-chinoxalin-1-carbonsäure-(5-hydroxy-adamantan-2-yl)-amid,
. trans-4-{5-[4-(3-Methoxy-propyl)-piperazin-1-yl]-pyridin-2-yl}-3,4-dihydro-2H-chinoxalin-1-carbonsäure-(5-hydroxy-adamantan-2-yl)-amid,
. trans-4-{5-[4-(3-Methyl-butyryl)-piperazin-1-yl]-pyridin-2-yl}-3,4-dihydro-2H-chinoxalin-1-carbonsäure-(5-hydroxy-adamantan-2-yl)-amid,
. trans-4-[4-(4-Methansulfonyl-piperazin-1-yl)-phenyl]-3,4-dihydro-2H-chinoxalin-1-carbonsäure-(5-acetylamino-adamantan-2-yl)-amid,
. trans-4-{5-[4-(2-Methoxy-ethansulfonyl)-piperazin-1-yl]-pyridin-2-yl}-3,4-dihydro-2H-chinoxalin-1-carbonsäure-(5-hydroxy-adamantan-2-yl)-amid,
. trans-4-{5-[4-(2,2-Dimethyl-propionyl)-piperazin-1-yl]-pyridin-2-yl}-3,4-dihydro-2H-chinoxalin-1-carbonsäure-(5-hydroxy-adamantan-2-yl)-amid,
. trans-4-(4,4-Difluor-3,4,5,6-tetrahydro-2H-[1,3']bipyridinyl-6'-yl)-3,4-dihydro-2H-chinoxalin-1-carbonsäure-(5-hydroxy-adamantan-2-yl)-amid,
. trans-4(4-Pyrrolidin-1-yl-3,4,5,6-tetrahydro-2H-[1,3']bipyridinyl-6'-yl)-3,4-dihydro-2H-chinoxalin-1-carbonsäure-(5-hydroxy-adamantan-2-yl)-amid,
. trans-4-[5-(5-Cyclopropansulfonyl-2,5-diaza-bicyclo[2.2.1]hept-2-yl)-pyridin-2-yl]-3,4-dihydro-2H-chinoxalin-1-carbonsäure-(5-hydroxy-adamantan-2-yl)-amid,
. trans-4-{6-[4-(5-Hydroxy-adamantan-2-ylcarbamoyl)-3,4-dihydro-2H-quinoxalin-1-yl]-pyridin-3-yl}-piperazin-1-carbonsäure-tert-butylester,
. trans-4-{4-[4-(2-Methoxy-ethyl)-piperazin-1-yl]-phenyl}-3,4-dihydro-2H-chinoxalin-1-carbonsäure-(5-carbamoyl-adamantan-2-yl)-amid,
. trans-4-[5-(4-Methansulfonyl-piperazin-1-yl)-3-methyl-pyridin-2-yl]-3,4-dihydro-2H-chinoxalin-1-carbonsäure-(5-carbamoyl-adamantan-2-yl)-amid,
. trans-2-(4-{4-[4-(5-Carbamoyl-adamantan-2-ylcarbamoyl)-3,4-dihydro-2H-quinoxalin-1-yl]-phenyl}-piperazin-1-yl)-2-methyl-propionsäure,
. trans-4-(4-tert-Butyl-3,4,5,6-tetrahydro-2H-[1,3']bipyridinyl-6'-yl)-3,4-dihydro-2H-chinoxalin-1-carbonsäure-(5-carbamoyl-adamantan-2-yl)-amid,
. trans-4-(4-Pyrrolidin-1-yl-3,4,5,6-tetrahydro-2H-[1,3']bipyridinyl-6'-yl)-3,4-dihydro-2H-chinoxalin-1-carbonsäure-(5-carbamoyl-adamantan-2-yl)-amid,
. trans-4-[5-(4-Ethansulfonyl-piperazin-1-yl)-pyridin-2-yl]-3,4-dihydro-2H-chinoxalin-1-carbonsäure-(5-carbamoyl-adamantan-2-yl)-amid,
. trans-4-[5-(4-Methansulfonyl-piperazin-1-yl)-6-methyl-pyridin-2-yl]-3,4-dihydro-2H-chinoxalin-1-carbonsäure-(5-carbamoyl-adamantan-2-yl)-amid,
. trans-4-(4,4-Difluor-3,4,5,6-tetrahydro-2H-[1,3']bipyridinyl-6'-yl)-3,4-dihydro-2H-chinoxalin-1-carbonsäure-(5-carbamoyl-adamantan-2-yl)-amid,
. trans-4-{4-[4-(2-Hydroxy-1,1-dimethyl-ethyl)-piperazin-1-yl]-phenyl}-3,4-dihydro-2H-chinoxalin-1-carbonsäure-(5-carbamoyl-adamantan-2-yl)-amid,
. trans-4-{5-[4-(Propan-2-sulfonyl)-piperazin-1-yl]-pyridin-2-yl}-3,4-dihydro-2H-chinoxalin-1-carbonsäure-(5-carbamoyl-adamantan-2-yl)-amid,
. trans-4-(5-Morpholin-4-yl-pyridin-2-yl)-3,4-dihydro-2H-chinoxalin-1-carbonsäure-(5-carbamoyl-adamantan-2-yl)-amid,
. trans-4-[5-(4-Methansulfonyl-piperazin-1-yl)-4-methyl-pyridin-2-yl]-3,4-dihydro-2H-chinoxalin-1-carbonsäure-(5-carbamoyl-adamantan-2-yl)-amid,
. trans-4-(4-Trifluormethyl-3,4,5,6-tetrahydro-2H-[1,3']bipyridinyl-6'-yl)-3,4-dihydro-2H-chinoxalin-1-carbonsäure-(5-carbamoyl-adamantan-2-yl)-amid,
. trans-4-(4-tert-Butyl-3,4,5,6-tetrahydro-2H-(1,3']bipyridinyl-6'-yl)-3,4-dihydro-2H-chinoxalin-1-carbonsäure-(5-hydroxy-adamantan-2-yl)-amid,
. trans-4-[5-(4-Isobutyl-piperazin-1-yl)-pyridin-2-yl]-3,4-dihydro-2H-chinoxalin-1-carbonsäure-(5-carbamoyl-adamantan-2-yl)-amid,
. trans-4-[5-(4-Cyclopropylmethyl-piperazin-1-yl)-pyridin-2-yl]-3,4-dihydro-2H-chinoxalin-1-carbonsäure-(5-carbamoyl-adamantan-2-yl)-amid,
. trans-4-{5-[4-(2,2,2-Trifluor-ethyl)-piperazin-1-yl]-pyridin-2-yl}-3,4-dihydro-2H-chinoxalin-1-carbonsäure-(5-carbamoyl-adamantan-2-yl)-amid,
. trans-4-[5-(4-tert-Butyl-piperazin-1-yl)-pyridin-2-yl]-3,4-dihydro-2H-chinoxalin-1-carbonsäure-(5-hydroxymethyl-adamantan-2-yl)-amid,
. trans-4-[5-(4-tert-Butyl-piperazin-1-yl)-pyridin-2-yl]-6,7-difluor-3,4-dihydro-2H-chinoxalin-1-carbonsäure-(5-carbamoyl-adamantan-2-yl)-amid,
. trans-4-{5-[4-(2,2-Dimethyl-propionyl)-piperazin-1-yl]-pyridin-2-yl}-3,4-dihydro-2H-chinoxalin-1-carbonsäure-(5-carbamoyl-adamantan-2-yl)-amid,
. trans-4-[5-(4-tert-Butyl-piperazin-1-yl)-pyrimidin-2-yl]-3,4-dihydro-2H-chinoxalin-1-carbonsäure-(5-carbamoyl-adamantan-2-yl)-amid,
. trans-4-[5-(4-Cyclopropansulfonyl-piperazin-1-yl)-pyridin-2-ylj-3,4-dihydro-2H-chinoxalin-1-carbonsäure-(5-cyano-adamantan-2-yl)-amid,
. trans-4-[5-(1-tert-Butyl-piperidin-4-yloxy)-pyridin-2-yl]-3,4-dihydro-2H-chinoxalin-1-carbonsäure-(5-carbamoyl-adamantan-2-yl)-amid,
. trans-{6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-yl}-essigsäure-methylester,
. trans-6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-carbonsäure-ethyl-ester,
. trans-4-[5-(4-lsopropyl-piperazin-1-yl)-pyridin-2-yl]-3,4-dihydro-2H-chinoxalin-1-carbonsäure-(5-carbamoyl-adamantan-2-yl)-amid,
. trans-4-(1'-tert-Butyl-1',2',3',4',5',6'-hexahydro-[3,4']bipyridinyl-6-yl)-3,4-dihydro-2H-chinoxalin-1-carbonsäure-(5-carbamoyl-adamantan-2-yl)-amid,
. trans-{6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-yl}-essigsäure,
. trans-6'-[4-(5-Carbamoyl-adamantan-2-ylcarbamoyl)-3,4-dihydro-2H-quinoxalin-1-yl]-3,4,5,6-tetrahydro-2H-[1,3']bipyridinyl-4-carbonsäure, trans-2-(4-{6-[4-(5-Carbamoyl-adamantan-2-ylcarbamoyl)-3,4-dihydro-2H-quinoxalin-1-yl]-pyridin-3-yl}-piperazin-1-yl)-2-methyl-propionsäure-ethylester,
. trans-4-(4,4-Dimethyl-3,4,5,6-tetrahydro-2H-[1,3']bipyridinyl-6'-yl)-3,4-dihydro-2H-chinoxalin-1-carbonsäure-(5-carbamoyl-adamantan-2-yl)-amid,
. trans-4-{5-[4-(2,2-Difluor-cyclopropylmethyl)-piperazin-1-yl]-pyridin-2-yl}-3,4-dihydro-2H-chinoxalin-1-carbonsäure-(5-carbamoyl-adamantan-2-yl)-amid,
. trans-4-{5-[4-(Difluor-methansulfonyl)-piperazin-1-yl]-pyridin-2-yl}-3,4-dihydro-2H-chinoxalin-1-carbonsäure-(5-carbamoyl-adamantan-2-yl)-amid,
. trans-4-{5-[4-(3,3,3-Trifluor-propyl)-piperazin-1-yl]-pyridin-2-yl}-3,4-dihydro-2H-chinoxalin-1-carbonsäure-(5-carbamoyl-adamantan-2-yl)-amid,
. trans-4-{5-[4-(2- Methansulfonyl-ethyl)-piperazin-1-yl]-pyridin-2-yl}-3,4-dihydro-2H-chinoxalin-1-carbonsäure-(5-carbamoyl-adamantan-2-yl)-amid,
. trans-4-{5-[4-(1-Ethyl-propyl)-piperazin-1-yl]-pyridin-2-yl}-3,4-dihydro-2H-chinoxalin-1-carbonsäure-(5-carbamoyl-adamantan-2-yl)-amid,
. trans-4-[5-(1,1-Dioxo-1lambda6-thiomorpholin-4-yl)-pyridin-2-yl]-3,4-dihydro-2H-chinoxalin-1-carbonsäure-(5-carbamoyl-adamantan-2-yl)-amid,
. trans-4-{5-[4-(Tetrahydro-pyran-4-yl)-piperazin-1-yl]-pyridin-2-yl}-3,4-dihydro-2H-chinoxalin-1-carbonsäure-(5-carbamoyl-adamantan-2-yl)-amid,
. trans-4-[5-(4-Cyclopropansulfonyl-piperazin-1-yl)-3-methyl-pyridin-2-yl]-3,4-dihydro-2H-chinoxalin-1-carbonsäure-(5-carbamoyl-adamantan-2-yl)-amid, trans-4-{5-[4-(2-Hydroxy-1,1-dimethyl-ethyl)-piperazin-1-yl]-pyhdin-2-yl}-3,4-dihydro-2H-chinoxalin-1-carbonsäure-(5-carbamoyl-adamantan-2-yl)-amid,
. trans-4-{5-[4-(2-Fluor-1,1-dimethyl-ethyl)-piperazin-1-yl]-pyridin-2-yl}-3,4-dihydro-2H-chinoxalin-1-carbonsäure-(5-carbamoyl-adamantan-2-yl)-amid,
. trans-4-[5-(4-Trifluormethansulfonyl-piperazin-1-yl)-pyridin-2-yl]-3,4-dihydro-2H-chinoxalin-1-carbonsäure-(5-carbamoyl-adamantan-2-yl)-amid.

14. Verfahren zur Herstellung einer Verbindung der Formel (I) gemäß einem der Ansprüche 1 bis 13, **dadurch gekennzeichnet, dass** eine Verbindung der Formel (IV): 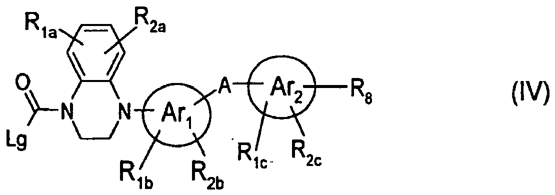 worin A, Ar₁, Ar₂, R_{1a,b,c}, R_{2a,b,c} und R₈ wie in einem der Ansprüche 1 bis 6 und 9 bis 12 definiert sind, und Lg eine Abgangsgruppe darstellt, mit einer Verbindung der Formel (V): 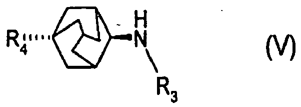 worin R₃ und R₄ wie in einem der Ansprüche 1, 7, 8 und 10 bis 12 definiert sind, umgesetzt wird.

15. Verfahren zur Herstellung gemäß Anspruch 14, **dadurch gekennzeichnet, dass** die Verbindung der Formel (IV) erhalten wird, indem eine Verbindung der Formel (II): 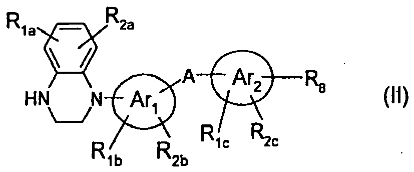 worin A, Ar₁, Ar₂, R_{1a,b,c}, R_{2a,b,c} und R₈ wie in einem der Ansprüche 1 bis 6 und 9 bis 12 definiert sind, mit einer Verbindung der Formel Lg-CO-Lg, worin Lg Abgangsgruppen darstellt, in Anwesenheit einer Base umgesetzt wird.

16. Verfahren zur Herstellung einer Verbindung der Formel (I) gemäß einem der Ansprüche 1 bis 13, worin A eine Bindung darstellt, **dadurch gekennzeichnet, dass** eine Verbindung der Formel (XXVII): 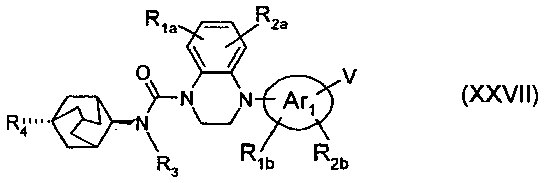 worin Ar₁, R_{1a,b}, R_{2a,b}, R₃ und R₄ wie in einem der Ansprüche 1, 3, 5, 7, 8 und 10 bis 13 definiert sind, und V eine Abgangsgruppe darstellt, mit einer Verbindung der Formel (XVI): 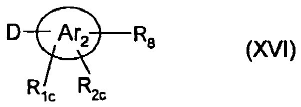 worin Ar₂, R_{1c}, R_{2c} und R₈ wie in einem der Ansprüche 1, 4, 6, 9 und 10 bis 13 definiert sind, und D eine metallorganische Gruppe darstellt, in Anwesenheit eines metallorganischen Katalysators und einer Base umgesetzt wird.

17. Verfahren zur Herstellung einer Verbindung der Formel (I) gemäß einem der Ansprüche 1 bis 13, worin R₄ eine Amidfunktion darstellt, **dadurch gekennzeichnet, dass** eine Verbindung der Formel (XXXIX): 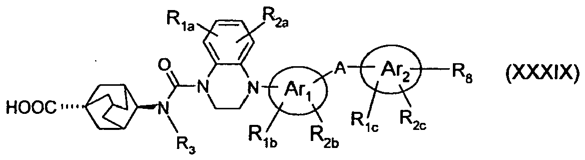 worin A, Ar₁, Ar₂, R_{1a,b,c}, R_{2a,b,c}, R₃ und R₈ wie in einem der Ansprüche 1 bis 7 und 9 bis 13 definiert sind, mit einer Verbindung der Formel (XXXVI), H-NR₆R₇, worin R₆ und R₇ wie in Anspruch 1 definiert sind, in Anwesenheit eines peptidischen Kopplungsmittels und einer Base umgesetzt wird.

18. Verfahren zur Herstellung einer Verbindung der Formel (I) gemäß einem der Ansprüche 1 bis 13, **dadurch gekennzeichnet, dass** eine Verbindung der Formel (XLII): 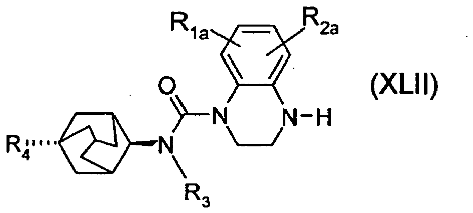 worin R₁ₐ, R₂ₐ, R₃ und R₄ wie in einem der Ansprüche 1, 5, 7, 8 und 10 bis 12 definiert sind, mit einer Verbindung der Formel (VII): 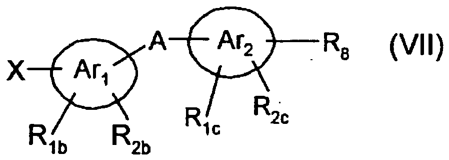 worin A, Ar₁, Ar₂, R_{1b,c}, R_{2b,c} und R₈ wie in einem der Ansprüche 1, 2, 3, 4, 5, 6, 9 und 10 bis 12 definiert sind, und X eine Abgangsgruppe ist, in Anwesenheit eines metallorganischen Katalysators und einer Base umgesetzt wird.

19. Verfahren zur Herstellung einer Verbindung der Formel (I) gemäß einem der Ansprüche 1 bis 13, worin Ar₂ eine Piperazingruppe ist, A eine Einfachbindung ist, die direkt mit einem der beiden Stickstoffatome des Piperazins verbunden ist, R_{1c} mit dem anderen Stickstoffatom des Piperazins verbunden ist; **dadurch gekennzeichnet, dass** eine Verbindung der Formel (LII): 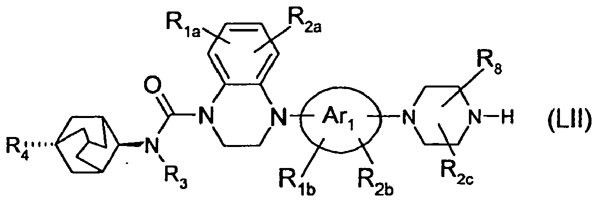 worin Ar₁, R_{1a,b}, R_{2a,b,c}, R₃, R₄ und R₈ wie in einem der Ansprüche 1, 4, 6, 9 und 10 bis 12 definiert sind, mit einer Verbindung der Formel (LIII) Lg-R_{1C} in Anwesenheit einer Base oder mit einer Verbindung (LIV) O=R_{1C} in Anwesenheit eines Reduktionsmittels umgesetzt wird.

20. Verfahren zur Herstellung einer Verbindung der Formel (I) gemäß einem der Ansprüche 1 bis 13, worin R₄ ein Halogenatom ist, **dadurch gekennzeichnet, dass** eine Verbindung der Formel (LVI): 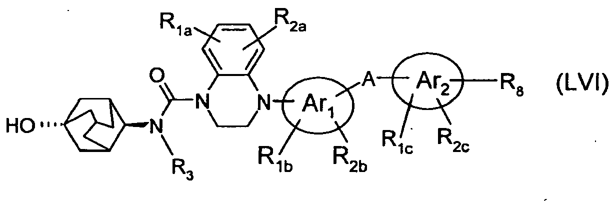 worin A, Ar₁, Ar₂, R_{1a,b,c}, R_{2a,b,c}, R₃ und R₈ wie in einem der Ansprüche 1 bis 7 und 9 bis 12 definiert sind, mit einem Halogenierungsmittel umgesetzt wird.

21. Verbindungen der Formeln (II) und (IV): 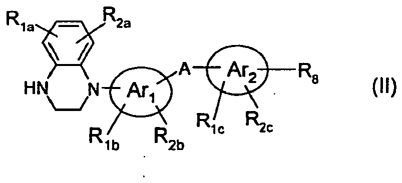 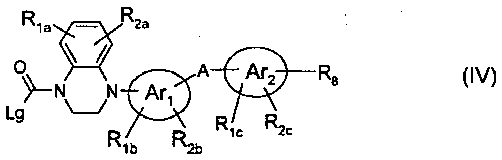 worin A, Ar₁, Ar₂, R_{1a,b,c}, R_{2a,b,c} und R₈ wie in einem der Ansprüche 1 bis 6 und 9 bis 12 definiert sind und Lg eine Abgangsgruppe darstellt.

22. Verbindung der Formel (XXVII): 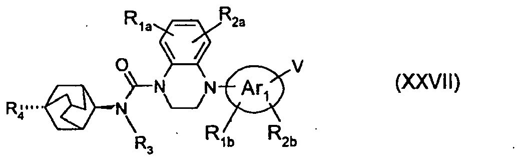 worin Ar₁, R_{1a,b}, R_{2a,b}, R₃ und R₄ wie in einem der Ansprüche 1, 3, 5, 7, 8 und 10 bis 12 definiert sind und V eine Abgangsgruppe darstellt.

23. Medikament, **dadurch gekennzeichnet, dass** es eine Verbindung der Formel (I) gemäß einem der Ansprüche 1 bis 13 oder ein pharmazeutisch verträgliches Säure- oder Basenadditionssalz der Verbindung oder ein Solvat der Verbindung der Formel (I) umfasst.

24. Pharmazeutische Zusammensetzung, **dadurch gekennzeichnet, dass** sie eine Verbindung der Formel (I) gemäß einem der Ansprüche 1 bis 13 oder ein pharmazeutisch verträgliches Salz oder ein Solvat der Verbindung sowie mindestens einen pharmazeutisch verträglichen Hilfsstoff umfasst.

25. Verbindung der Formel (I) gemäß einem der Ansprüche 1 bis 13 zur Verwendung bei der Behandlung und Prävention von Obesitas, Diabetes, Mikrozirkulationsstörungen, Insulinresistenz, metabolischem Syndrom, Cushing-Syndrom, Hypertonie, Arteriösklerose, Kognition und Demenz, Glaukomen, Osteoporose, Lipodystrophie, Herzhypertrophie, Herzinsuffizienz, Lebererkrankungen, bestimmten Infektionskrankheiten durch die Steigerung der Leistungsfähigkeit des Immunsystems oder von Wunden durch Förderung der Wundheilung.

## Claims

1. Compound corresponding to the formula (I) : 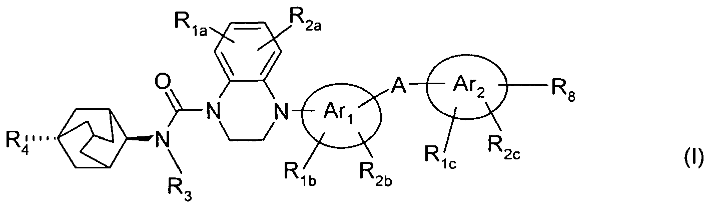 in which:
- A represents a bond, an oxygen atom or an -O-CH₂-group,
- Ar₁ represents a phenyl or heteroaryl group,
- Ar₂ represents a phenyl group, a heteroaryl group or a heterocycloalkyl group,
- R_{1a,b,c} and R_{2a,b,c,} which are identical or different, each represent a hydrogen or halogen atom or an alkyl, cycloalkyl, -alkylcycloalkyl optionally substituted by one or more halogen atoms, -OR₅ (hydroxyl or alkoxy), hydroxyalkyl, alkoxyalkyl, alkoxyalkoxy, haloalkyl, -0-haloalkyl, oxo, -CO-alkyl, -CO-alkyl-NR₆R₇, -CO-haloalkyl, -COOR₅, -alkyl-COOR₅, -O-alkyl-COOR₅, -SO₂-alkyl, -SO₂-cycloalkyl, - SO₂-alkylcycloalkyl, -SO₂-alkyl-OR₅, -SO₂-alkyl-COOR₅, -SO₂-alkyl-NR₆R₇, -SO₂-haloalkyl, -alkyl-SO₂-alkyl, -SO₂-NR₆R₇, -SO₂-alkylalkoxyalkoxy, -CONR₆R₇, -alkyl-CONR₆R₇ or -O-alkyl-NR₆R₇ group, or also R₁ₐ, R_{1b} and R_{1c} are respectively bonded to R₂ₐ, R_{2b} and R_{2c} and to the carbon atom which carries them and represent - O-alkyl-O-;
- R₃ represents a hydrogen atom or an alkyl group,
- R₄ represents a hydrogen or halogen atom or a cyano, -OR₅, hydroxyalkyl, -COOR₅, -NR₆R₇, -CONR₆R₇, -SO₂-alkyl, -SO₂-NR₆R₇, -NR₆-COOR₅, -NR₆-COR₅ or -CO-NR₆-alkyl-OR₅ group;
- R₅, R₆ and R₇, which are identical or different, each represent a hydrogen atom, an alkyl group or an - alkylphenyl group, and
- R₈ represents a hydrogen atom, an alkyl group or a group of formula -B-Het, where B can be absent or can represent a bond, an oxygen atom or a -CO- or - SO₂-(CH₂)ₙ- group with n equal to 0, 1 or 2 and where Het represents a heteroaryl or a heterocycloalkyl optionally substituted by 1 to 3 groups chosen from alkyl, -SO₂-alkyl and -COOR₅ groups,
in the form of the base or acid or of an addition salt with an acid or with a base.

2. Compound of formula (I) according to Claim 1, **characterized in that** A represents a bond.

3. Compound of formula (I) according to Claim 1 or Claim 2, **characterized in that** Ar₁ represents a phenyl, pyridinyl, pyrimidinyl, pyridazinyl or thiazolyl group.

4. Compound of formula (I) according to any one of the preceding claims, **characterized in that** Ar₂ represents a phenyl, pyridinyl, pyrimidinyl, piperidinyl, piperazinyl, homopiperazinyl, 3,8-diazabicyclo[3.2.1]octyl, morpholinyl, thiomorpholinyl, octahydropyrrolo[3,4-c]pyrrolyl, 1,2,3,6-tetrahydropyridinyl or 2,5-diazabicyclo[2.2.1]heptyl group.

5. Compound of formula (I) according to any one of the preceding claims, **characterized in that** R₁ₐ, R₂ₐ, R_{1b} and R_{2b}, which are identical or different, each represent a hydrogen or halogen atom or an alkyl or alkoxy group.

6. Compound of formula (I) according to any one of the preceding claims, **characterized in that** R_{1c} and R_{2c}, which are identical or different, each represent a hydrogen or halogen atom or an alkyl, alkoxy, -SO₂-alkyl or -SO₂-cycloalkyl group.

7. Compound of formula (I) according to any one of the preceding claims, **characterized in that** R₃ represents a hydrogen atom.

8. Compound of formula (I) according to any one of the preceding claims, **characterized in that** R₄ represents a hydrogen or halogen atom or a cyano, hydroxyl, hydroxyalkyl, -COOR₅, or -CONH₂ group.

9. Compound of formula (I) according to any one of the preceding claims, **characterized in that** R₈ represents a hydrogen atom, an alkyl group or a group of formula -B-Het, where B can be absent or can represent a bond, an oxygen atom or a -CO- or -S0₂-(CH₂)ₙ- group with n equal to 0, 1 or 2 and where Het represents a pyrrolidinyl, piperidinyl, piperazinyl, tetrahydropyranyl, morpholinyl or pyridinyl group, the said Het group optionally being substituted by an alkyl, -SO₂-alkyl or -COOR₅ group.

10. Compound of formula (I) according to Claim 1, **characterized in that**:
- A represents a bond, an oxygen atom or an - O-CH₂-group,
- Ar₁ represents a phenyl or heteroaryl group,
- Ar₂ represents a phenyl group,
- R_{1a,b,c} and R_{2a,b,c}, which are identical or different, each represent a hydrogen or halogen atom or an alkyl, cycloalkyl, -alkylcycloalkyl optionally substituted by one or more halogen atoms, -OR₅ (hydroxyl or alkoxy), hydroxyalkyl, alkoxyalkyl, haloalkyl, -0-haloalkyl, oxo, -CO-alkyl, -CO-alkyl-NR₆R₇, -COOR₅, -alkyl-COOR₅, -SO₂-alkyl, -SO₂-cycloalkyl, - SO₂-alkylcycloalkyl, -SO₂-alkyl-OR₅, -SO₂-alkyl-COOR₅, -SO₂-alkyl-NR₆R₇, -SO₂-haloalkyl, -alkyl-SO₂-alkyl, -SO₂-alkylalkoxyalkoxy, -CONR₆R₇ or -O-alkyl-NR₆R₇ group, or also R₁ₐ, R_{1b} and R_{1c} are respectively bonded to R₂ₐ, R_{2b} and R_{2c} and to the carbon atom which carries them and represent -O-alkyl-O-,
- R₃ represents a hydrogen atom or an alkyl group,
- R₄ represents a hydrogen or halogen atom or a cyano, -OR₅, hydroxyalkyl, -COOR₅, -CONR₆R₇, -NR₆-COOR₅, -NR₆-COR₅ or -CO-NR₆-alkyl-OR₅ group,
- R₅, R₆ and R₇, which are identical or different, each represent a hydrogen atom, an alkyl group or an - alkylphenyl group, and
- R₈ represents a hydrogen atom, an alkyl group or a group of formula -B-Het, where B can be absent or can represent a bond, an oxygen atom or a -CO- or - SO₂-(CH₂)ₙ- group with n equal to 0, 1 or 2 and where Het represents a pyrrolidinyl, piperidinyl, piperazinyl, tetrahydropyranyl, morpholinyl or pyridinyl group, the said Het group optionally being substituted by 1 to 3 groups chosen from alkyl, -SO₂-alkyl and -COOR₅ groups,
in the form of the base or acid or of an addition salt with an acid or with a base.

11. Compound of formula (I) according to Claim 1, **characterized in that**:
- A represents a bond, an oxygen atom or an -O-CH₂-group,
- Ar₁ represents a phenyl or heteroaryl group,
- Ar₂ represents a heteroaryl group,
- R_{1a,b,c} and R_{2a,b,c,} which are identical or different, each represent a hydrogen or halogen atom or an alkyl, cycloalkyl, -alkylcycloalkyl optionally substituted by one or more halogen atoms, -OR₅ (hydroxyl or alkoxy), hydroxyalkyl, alkoxyalkyl, haloalkyl, -0-haloalkyl, oxo, -CO-alkyl, -CO-alkyl-NR₆R₇, -COOR₅, -alkyl-COOR₅, -SO₂-alkyl, -SO₂-cycloalkyl, - SO₂-alkylcycloalkyl, -SO₂-alkyl-OR₅, -SO₂-alkyl-COOR₅, -SO₂-alkyl-NR₆R₇, -SO₂-haloalkyl, -alkyl-SO₂-alkyl, -SO₂-alkylalkoxyalkoxy, -CONR₆R₇ or -O-alkyl-NR₆R₇ group, or also R₁ₐ, R_{1b} and R_{1c} are respectively bonded to R₂ₐ, R_{2b} and R_{2c} and to the carbon atom which carries them and represent -O-alkyl-O-,
- R₃ represents a hydrogen atom or an alkyl group,
- R₄ represents a hydrogen or halogen atom or a cyano, -OR₅, hydroxyalkyl, -COOR₅, -CONR₆R₇, -NR₆-COOR₅, - NR₆-COR₅ or -CO-NR₆-alkyl-OR₅ group,
- R₅, R₆ and R₇, which are identical or different, each represent a hydrogen atom, an alkyl group or an - alkylphenyl group, and
- R₈ represents a hydrogen atom, an alkyl group or a group of formula -B-Het, where B can be absent or can represent a bond, an oxygen atom or a -CO- or - SO₂-(CH₂)ₙ- group with n equal to 0, 1 or 2 and where Het represents a pyrrolidinyl, piperidinyl, piperazinyl, tetrahydropyranyl, morpholinyl or pyridinyl group, the said Het group optionally being substituted by 1 to 3 groups chosen from alkyl, -SO₂-alkyl and -COOR₅ groups,
in the form of the base or acid or of an addition salt with an acid or with a base.

12. Compound of formula (I) according to Claim 1, **characterized in that**:
- A represents a bond, an oxygen atom or an -O-CH₂-group,
- Ar₁ represents a phenyl or heteroaryl group,
- Ar₂ represents a heterocycloalkyl group (such as a piperidinyl, piperazinyl, homopiperazinyl, 3,8-diazabicyclo[3.2.1]octyl, morpholinyl, thiomorpholinyl, octahydropyrrolo[3,4-c]pyrrolyl, 1,2,3,6-tetrahydropyridinyl or 2,5-diazabicyclo[2.2.1]heptyl group),
- R_{1a,b,c} and R_{2a,b,c}, which are identical or different, each represent a hydrogen or halogen atom or an alkyl, cycloalkyl, -alkylcycloalkyl optionally substituted by one or more halogen atoms, -OR₅ (hydroxyl or alkoxy), hydroxyalkyl, alkoxyalkyl, haloalkyl, -0-haloalkyl, oxo, -CO-alkyl, -CO-alkyl-NR₆R₇, -COOR₅, -alkyl-COOR₅, -SO₂-alkyl, -SO₂-cycloalkyl, - SO₂-alkylcycloalkyl, -SO₂-alkyl-OR₅, -SO₂-alkyl-COOR₅, -SO₂-alkyl-NR₆R₇, -SO₂-haloalkyl, -alkyl-SO₂-alkyl, -SO₂-alkylalkoxyalkoxy, -CONR₆R₇ or -O-alkyl-NR₆R₇ group, or also R₁ₐ, R_{1b} and R_{1c} are respectively bonded to R₂ₐ, R_{2b} and R_{2c} and to the carbon atom which carries them and represent -O-alkyl-O-,
- R₃ represents a hydrogen atom or an alkyl group,
- R₄ represents a hydrogen or halogen atom or a cyano, -OR₅, hydroxyalkyl, -COOR₅, -CONR₆R₇, -NR₆-COOR₅, - NR₆-COR₅ or -CO-NR₆-alkyl-OR₅ group,
- R₅, R₆ and R₇, which are identical or different, each represent a hydrogen atom, an alkyl group or an - alkylphenyl group, and
- R₈ represents a hydrogen atom, an alkyl group or a group of formula -B-Het, where B can be absent or can represent a bond, an oxygen atom or a -CO- or - SO₂-(CH₂)ₙ- group with n equal to 0, 1 or 2 and where Het represents a pyrrolidinyl, piperidinyl, piperazinyl, tetrahydropyranyl, morpholinyl or pyridinyl group, the said Het group optionally being substituted by 1 to 3 groups chosen from alkyl, -SO₂-alkyl and -COOR₅ groups,
in the form of the base or acid or of an addition salt with an acid or with a base.

13. Compound of formula (I) according to Claim 1, **characterized in that** it is chosen from:
. 4-(4-(Pyridin-2-yl)phenyl)-3-4-dihydro-2H-quinoxaline-1-carboxylic acid adamantan-2-ylamide,
. 4-{4-[1-(Tetrahydropyran-4-carbonyl)piperidin-4-yloxy]phenyl}-3,4-dihydro-2H-quinoxaline-1-carboxylic acid adamantan-2-ylamide,
. 4-[4-(Pyrimidin-2-ylmethoxy)phenyl]-3,4-dihydro-2H-quinoxaline-1-carboxylic acid adamantan-2-ylamide,
. 4-{4-[1-(Morpholine-4-carbonyl)piperidin-4-yl]phenyl}-3,4-dihydro-2H-quinoxaline-1-carboxylic acid adamantan-2-ylamide,
. 4-[4-(4-Methoxypyrimidin-2-yloxy)phenyl]-3,4-dihydro-2H-quinoxaline-1-carboxylic acid adamantan-2-ylamide,
. 4-{4-[4-(Morpholine-4-carbonyl)phenyl]thiazol-2-yl}-3,4-dihydro-2H-quinoxaline-1-carboxylic acid adamantan-2-ylamide,
. 4-[5-(4-Methoxyphenyl)pyrimidin-2-yl]-3,4-dihydro-2H-quinoxaline-1-carboxylic acid adamantan-2-ylamide,
. 4-[5-(4-Methoxyphenyl)pyrimidin-2-yl]-3,4-dihydro-2H-quinoxaline-1-carboxylic acid (5-hydroxyadamantan-2-yl)amide,
. trans-4-[4-(4-(Methanesulphonyl)piperazin-1-yl)phenyl]-3,4-dihydro-2H-quinoxaline-1-carboxylic acid (5-hydroxyadamantan-2-yl)amide,
. Methyl ester of 4-({4-[4-(4-(Methanesulphonyl)piperazin-1-yl)phenyl]-3,4-dihydro-2H-quinoxaline-1-carbonyl}amino)adamantane-1-carboxylic acid
. 4-({4-[4-(4-(Methanesulphonyl)piperazin-1-yl)phenyl]-3,4-dihydro-2H-quinoxaline-1-carbonyl}amino)adamantane-1-carboxylic acid
. 4-[4-(4-(Methanesulphonyl)piperazin-1-yl)phenyl]-3,4-dihydro-2H-quinoxaline-1-carboxylic acid (5-carbamoyladamantan-2-yl)amide,
. trans-4-[4-(Pyridin-2-yloxy)phenyl]-3,4-dihydro-2H-quinoxaline-1-carboxylic acid (5-hydroxyadamantan-2-yl)amide,
. trans-4-[4-(4-(Trifluoromethyl)pyrimidin-2-yloxy)phenyl]-3,4-dihydro-2H-quinoxaline-1-carboxylic acid (5-hydroxyadamantan-2-yl)amide,
. trans-4-[4-(1-(Methanesulphonyl)-1,2,3,6-tetrahydropyridin-4-yl)phenyl]-3,4-dihydro-2H-quinoxaline-1-carboxylic acid (5-hydroxyadamantan-2-yl)amide,
. trans-4-[4-(4-Hydroxy-1-(Methanesulphonyl)piperidin-4-yl)phenyl]-3,4-dihydro-2H-quinoxaline-1-carboxylic acid (5-hydroxyadamantan-2-yl)amide,
. trans-4-[2-(4-(Methanesulphonyl)piperazin-1-yl)pyridimin-5-yl]-3,4-dihydro-2H-quinoxaline-1-carboxylic acid (5-hydroxyadamantan-2-yl)amide,
. trans-4-[5-(4-(Methanesulphonyl)piperazin-1-yl)pyrimidin-2-yl]-3,4-dihydro-2H-quinoxaline-1-carboxylic acid (5-hydroxyadamantan-2-yl)amide,
. trans-4-{4-[1-(Morpholine-4-carbonyl)piperidin-4-yl]phenyl}-3,4-dihydro-2H-quinoxaline-1-carboxylic acid (5-hydroxyadamantan-2-yl)amide,
. trans-4-{4-[4-(5-Hydroxyadamantan-2-ylcarbamoyl)-3,4-dihydro-2H-quinoxalin-1-yl]phenyl}piperazine-1-carboxylic acid benzyl ester,
. trans-4-[4-(4-(Methanesulphonyl)piperazin-1-yl)phenyl]-3,4-dihydro-2H-quinoxaline-1-carboxylic acid (5-(hydroxymethyl)adamantan-2-yl)amide,
. trans-4-[4-(5-Fluoropyrimidin-2-yloxy)phenyl]-3,4-dihydro-2H-quinoxaline-1-carboxylic acid (5-hydroxyadamantan-2-yl)amide,
. trans-4-[4-(1-(Methanesulphonyl)piperidin-4-yloxy)phenyl]-3,4-dihydro-2H-quinoxaline-1-carboxylic acid (5-hydroxyadamantan-2-yl)amide,
. trans-4-(4-(Morpholin-4-yl)phenyl)-3,4-dihydro-2H-quinoxaline-1-carboxylic acid (5-hydroxyadamantan-2-yl)amide,
. trans-4-[4-(1,1-Dioxo-1λ⁶-thiomorpholin-4-yl)phenyl]-3,4-dihydro-2H-quinoxaline-1-carboxylic acid (5-hydroxyadamantan-2-yl)amide,
. trans-4-[4-(5-Isopropoxypyridin-2-yl)phenyl]-3,4-dihydro-2H-quinoxaline-1-carboxylic acid (5-hydroxyadamantan-2-yl)amide,
. trans-4-{4-[5-(4-Isopropylpiperazine-1-carbonyl)pyridin-2-yl]phenyl}-3,4-dihydro-2H-quinoxaline-1-carboxylic acid (5-hydroxyadamantan-2-yl)amide,
. trans-4-[6-(4-(Methanesulphonyl)piperazin-1-yl)pyridin-3-yl]-3,4-dihydro-2H-quinoxaline-1-carboxylic acid (5-hydroxyadamantan-2-yl)amide,
. trans-4-(4-(Piperazin-1-yl)phenyl)-3,4-dihydro-2H-quinoxaline-1-carboxylic acid (5-hydroxyadamantan-2-yl)amide,
. trans-4-[4-(4,4-Difluoropiperidin-1-yl)phenyl]-3,4-dihydro-2H-quinoxaline-1-carboxylic acid (5-hydroxyadamantan-2-yl)amide,
. trans-4-[5-(4-(Methanesulphonyl)piperazin-1-yl)pyridin-2-yl]-3,4-dihydro-2H-quinoxaline-1-carboxylic acid (5-hydroxyadamantan-2-yl)amide,
. trans-4-[5-(4-(Methanesulphonyl)piperazin-1-yl)pyridin-2-yl]-3,4-dihydro-2H-quinoxaline-1-carboxylic acid (5-hydroxyadamantan-2-yl)amide,
. trans-4-[2-(1-(Methanesulphonyl)piperidin-4-yloxy)pyrimidin-5-yl]-3,4-dihydro-2H-quinoxaline-1-carboxylic acid (5-hydroxyadamantan-2-yl)amide,
. trans-4-[5-(5-Isopropoxypyridin-2-yl)pyrimidin-2-yl]-3,4-dihydro-2H-quinoxaline-1-carboxylic acid (5-hydroxyadamantan-2-yl)amide,
. trans-4-[4-(4-(Ethanesulphonyl)piperazin-1-yl)phenyl]-3,4-dihydro-2H-quinoxaline-1-carboxylic acid (5-hydroxyadamantan-2-yl)amide,
. trans-4-{4-[4-(2,2,2-Trifluoroethanesulphonyl)piperazin-1-yl]phenyl}-3,4-dihydro-2H-quinoxaline-1-carboxylic acid (5-hydroxyadamantan-2-yl)amide,
. trans-4-{4-[4-(Propane-2-sulphonyl)piperazin-1-yl]phenyl}-3,4-dihydro-2H-quinoxaline-1-carboxylic acid (4-hydroxyadamantan-1-yl)amide,
. trans-4-(4-{4-[4-(5-Hydroxyadamantan-2-ylcarbamoyl)-3,4-dihydro-2H-quinoxalin-1-yl]phenyl}piperazine-1-sulphonyl)piperidine-1-carboxylic acid benzyl ester,
. trans-4-{4-[4-(Piperidine-4-sulphonyl)piperazin-1-yl]phenyl}-3,4-dihydro-2H-quinoxaline-1-carboxylic acid (5-hydroxyadamantan-2-yl)amide,
. trans-4-[4-(4-(Cyclopropanesulphonyl)piperazin-1-yl)phenyl]-3,4-dihydro-2H-quinoxaline-1-carboxylic acid (5-hydroxyadamantan-2-yl)amide,
. trans-4-[4-(4-(Methanesulphonyl)piperazin-1-yl)phenyl]-3,4-dihydro-2H-quinoxaline-1-carboxylic acid (5-fluoroadamantan-2-yl)amide,
. trans-4-[4-(4-(Cyclobutanesulphonyl)piperazin-1-yl)phenyl]-3,4-dihydro-2H-quinoxaline-1-carboxylic acid (5-hydroxyadamantan-2-yl)amide,
. trans-4-{4-[4-(Propane-1-sulphonyl)piperazin-1-yl]phenyl}-3,4-dihydro-2H-quinoxaline-1-carboxylic acid (5-hydroxyadamantan-2-yl)amide,
. trans-4-{4-[4-(Butane-1-sulphonyl)piperazin-1-yl]phenyl}-3,4-dihydro-2H-quinoxaline-1-carboxylic acid (5-hydroxyadamantan-2-yl)amide,
. trans-4-{4-[4-(Morpholine-4-sulphonyl)piperazin-1-yl]phenyl}-3,4-dihydro-2H-quinoxaline-1-carboxylic acid (5-hydroxyadamantan-2-yl)amide,
. trans-4-[4-(4-(Trifluoromethanesulphonyl)piperazin-1-yl)phenyl]-3,4-dihydro-2H-quinoxaline-1-carboxylic acid (5-hydroxyadamantan-2-yl)amide,
. trans-4-[4-(1,4-Dioxa-8-azaspiro[4.5]dec-8-yl)phenyl]-3,4-dihydro-2H-quinoxaline-1-carboxylic acid (5-hydroxyadamantan-2-yl)amide,
. trans-4-[4-(4-Methanesulphonyl-3,3-dimethylpiperazin-1-yl)phenyl]-3,4-dihydro-2H-quinoxaline-1-carboxylic acid (5-hydroxyadamantan-2-yl)amide,
. trans-4-[4-((3R,5S)-4-Methanesulphonyl-3,5-dimethylpiperazin-1-yl)phenyl]-3,4-dihydro-2H-quinoxaline-1-carboxylic acid (5-hydroxyadamantan-2-yl)amide,
. trans-(4-{4-[4-(5-Hydroxyadamantan-2-ylcarbamoyl)-3,4-dihydro-2H-quinoxalin-1-yl]phenyl}piperazine-1-sulphonyl)acetic acid,
. trans-(4-{4-[4-(5-Hydroxyadamantan-2-ylcarbamoyl)-3,4-dihydro-2H-quinoxalin-1-yl]phenyl}piperazine-1-sulphonyl)acetic acid ethyl ester,
. trans-4-[4-(4-Hydroxypiperidin-1-yl)phenyl]-3,4-dihydro-2H-quinoxaline-1-carboxylic acid (5-hydroxyadamantan-2-yl)amide,
. trans-4-[4-(4-Methanesulphonyl-1,4-diazepan-1-yl)phenyl]-3,4-dihydro-2H-quinoxaline-1-carboxylic acid (5-hydroxyadamantan-2-yl)amide,
. trans-2-(4-{4-[4-(5-Hydroxyadamantan-2-ylcarbamoyl)-3,4-dihydro-2H-quinoxalin-1-yl]phenyl}piperazin-1-yl)-2-methylpropionic acid ethyl ester,
. trans-3-(4-{4-[4-(5-Hydroxyadamantan-2-ylcarbamoyl)-3,4-dihydro-2H-quinoxalin-1-yl]phenyl}piperazine-1-sulphonyl)propionic acid methyl ester,
. trans-4-[4-(5-Methoxypyrimidin-2-yl)phenyl]-3,4-dihydro-2H-quinoxaline-1-carboxylic acid (5-hydroxyadamantan-2-yl)amide,
. trans-4-{4-[4-(2-(Morpholin-4-yl)ethanesulphonyl)piperazin-1-yl]phenyl}-3,4-dihydro-2H-quinoxaline-1-carboxylic acid (5-hydroxyadamantan-2-yl)amide,
. trans-4-[6-(4-(Methanesulphonyl)piperazin-1-yl)pyridazin-3-yl]-3,4-dihydro-2H-quinoxaline-1-carboxylic acid (5-hydroxyadamantan-2-yl)amide,
. trans-4-[4-(4-(Methanesulphonyl)piperazin-1-yl)phenyl]-3,4-dihydro-2H-quinoxaline-1-carboxylic acid (5-hydroxyadamantan-2-yl)(methyl)amide,
. trans-4-[4-(4-Methanesulphonyl-3,5-dimethylpiperazin-1-yl)phenyl]-3,4-dihydro-2H-quinoxaline-1-carboxylic acid (5-hydroxyadamantan-2-yl)amide,
. trans-4-{4-[4-(2-(Diethylamino)ethanesulphonyl)piperazin-1-yl]phenyl}-3,4-dihydro-2H-quinoxaline-1-carboxylic acid (5-hydroxyadamantan-2-yl)amide,
. trans-4-[4-(3-Methanesulphonyl-3,8-diazabicyclo[3.2.1]oct-8-yl)phenyl]-3,4-dihydro-2H-quinoxaline-1-carboxylic acid (5-hydroxyadamantan-2-yl)amide,
. trans-4-[5-(4-Methoxyphenyl)pyrimidin-2-yl]-3,4-dihydro-2H-quinoxaline-1-carboxylic acid (5-carbamoyladamantan-2-yl)amide,
. trans-4-{4-[4-(2-Methoxyethanesulphonyl)piperazin-1-yl]phenyl}-3,4-dihydro-2H-quinoxaline-1-carboxylic acid (5-hydroxyadamantan-2-yl)amide,
. trans-4-[4-(4-Isopropoxypiperidin-1-yl)phenyl]-3,4-dihydro-2H-quinoxaline-1-carboxylic acid (5-hydroxyadamantan-2-yl)amide,
. trans-4-[5-(4-Hydroxyphenyl)pyrimidin-2-yl]-3,4-dihydro-2H-quinoxaline-1-carboxylic acid (5-hydroxyadamantan-2-yl)amide,
. trans-4-(4-(Piperazin-1-yl)phenyl)-3,4-dihydro-2H-quinoxaline-1-carboxylic acid (5-carbamoyladamantan-2-yl)amide,
. trans-4-[4-(5-Bromopyrimidin-2-yloxy)phenyl]-3,9-dihydro-2H-quinoxaline-1-carboxylic acid (5-hydroxyadamantan-2-yl)amide,
. trans-4-[4-(4-(Cyclopropanesulphonyl)piperazin-1-yl)phenyl)-3,4-dihydro-2H-quinoxaline-1-carboxylic acid (5-carbamoyladamantan-2-yl)amide,
. trans-4-[4-(4-(Cyclopropanesulphonyl)piperazin-1-yl)phenyl]-3,4-dihydro-2H-quinoxaline-1-carboxylic acid (5-carbamoyladamantan-2-yl)amide,
. trans-6-Chloro-4-[4-(4-(Methanesulphonyl)piperazin-1-yl)phenyl]-3,4-dihydro-2H-quinoxaline-1-carboxylic acid (5-hydroxyadamantan-2-yl)amide,
. trans-4-[4-(5-(Methanesulphonyl)hexahydropyrrolo[3,4-c]pyrrol-2-yl)phenyl]-3,4-dihydro-2H-quinoxaline-1-carboxylic acid (5-hydroxyadamantan-2-yl)amide,
. trans-4-{4-[4-(2-Aminoethanesulphonyl)piperazin-1-yl]phenyl}-3,4-dihydro-2H-quinoxaline-1-carboxylic acid (5-hydroxyadamantan-2-yl)amide,
. trans-4-(4-(Morpholin-4-yl)phenyl)-3,4-dihydro-2H-quinoxaline-1-carboxylic acid (5-carbamoyladamantan-2-yl)amide,
. trans-4-{4-[5-(Piperidin-4-yloxy)pyridin-2-yl]phenyl}-3,4-dihydro-2H-quinoxaline-1-carboxylic acid (5-hydroxyadamantan-2-yl)amide,
. trans-4-{4-[5-(2-Aminoethoxy)pyridin-2-yl]phenyl}-3,4-dihydro-2H-quinoxaline-1-carboxylic acid (5-hydroxyadamantan-2-yl)amide,
. trans-4-{4-[4-(2-Hydroxyethanesulphonyl)piperazin-1-yl]phenyl}-3,4-dihydro-2H-quinoxaline-1-carboxylic acid (5-hydroxyadamantan-2-yl)amide,
. trans-4-[4-(8-Methanesulphonyl-3,8-diazabicyclo[3.2.1]oct-3-yl)phenyl]-3,4-dihydro-2H-quinoxaline-1-carboxylic acid (5-carbamoyladamantan-2-yl)amide,
. trans-4-[4-(8-Methanesulphonyl-3,8-diazabicyclo[3.2.1]oct-3-yl)phenyl]-3,4-dihydro-2H-quinoxaline-1-carboxylic acid (5-hydroxyadamantan-2-yl)amide,
. trans-4-[5-(4-Isopropoxyphenyl)pyrimidin-2-yl]-3,4-dihydro-2H-quinoxaline-1-carboxylic acid (5-hydroxyadamantan-2-yl)amide,
. trans-4-{5-[4-(2,2,2-Trifluoroethanesulphonyl)piperazin-1-yl]pyridin-2-yl}-3,4-dihydro-2H-quinoxaline-1-carboxylic acid (5-hydroxyadamantan-2-yl)amide,
. trans-4-{5-[4-(2,2,2-Trifluoroethanesulphonyl)piperazin-1-yl]pyridin-2-yl}-3,4-dihydro-2H-quinoxaline-1-carboxylic acid (5-carbamoyladamantan-2-yl)amide,
. trans-3-(4-{4-[4-(5-Hydroxyadamantan-2-ylcarbamoyl)-3,4-dihydro-2H-quinoxalin-1-yl]phenyl}piperazine-1-sulphonyl)propionic acid,
. trans-4-[4-(4-(Methanesulphonyl)piperazin-1-yl)phenyl]-3,4-dihydro-2H-quinoxaline-1-carboxylic acid [5-(2-hydroxyethylcarbamoyl)adamantan-2-yl]amide,
. trans-4-[4-(5-Isobutoxypyridin-2-yl)phenyl]-3,4-dihydro-2H-quinoxaline-1-carboxylic acid (5-hydroxyadamantan-2-yl)amide,
. trans-4-[4-(5-(sec-Butoxy)pyridin-2-yl)phenyl]-3,4-dihydro-2H-quinoxaline-1-carboxylic acid (5-hydroxyadamantan-2-yl)amide,
. trans-4-[4-(5-Isopropoxypyridin-2-yl)phenyl]-3,4-dihydro-2H-quinoxaline-1-carboxylic acid (5-carbamoyladamantan-2-yl)amide,
. trans-[4-({4-[4-(4-(Methanesulphonyl)piperazin-1-yl)phenyl]-3,4-dihydro-2H-quinoxaline-1-carbonyl}amino)adamantan-1-yl]carbamic acid methyl ester,
. trans-4-[5-(4-(Ethanesulphonyl)piperazin-1-yl)pyridin-2-yl]-3,4-dihydro-2H-quinoxaline-1-carboxylic acid (5-hydroxyadamantan-2-yl)amide,
. trans-4-[5-(4-(Cyclopropanesulphonyl)piperazin-1-yl)pyridin-2-yl]-3,4-dihydro-2H-quinoxaline-1-carboxylic acid (5-hydroxyadamantan-2-yl)amide,
. trans-4-[5-(4-(Cyclopropanesulphonyl)piperazin-1-yl)pyridin-2-yl]-3,4-dihydro-2H-quinoxaline-1-carboxylic acid (5-carbamoyladamantan-2-yl)amide,
. trans-4-{5-[4-(Propane-2-sulphonyl)piperazin-1-yl]pyridin-2-yl}-3,4-dihydro-2H-quinoxaline-1-carboxylic acid (5-hydroxyadamantan-2-yl)amide,
. trans-4-{4-[4-(Pyridin-4-yloxy)piperidin-1-yl]phenyl}-3,4-dihydro-2H-quinoxaline-1-carboxylic acid (5-hydroxyadamantan-2-yl)amide,
. trans-4-{4-[4-(2-Methoxyethanesulphonyl)piperazin-1-yl]phenyl}-3,4-dihydro-2H-quinoxaline-1-carboxylic acid (5-carbamoyladamantan-2-yl)amide,
. trans-4-{4-[4-(2-Aminoethanesulphonyl)piperazin-1-yl]phenyl}-3,4-dihydro-2H-quinoxaline-1-carboxylic acid (5-carbamoyladamantan-2-yl)amide,
. trans-4-{4-[4-(2-(Diethylamino)ethanesulphonyl)piperazin-1-yl]phenyl}-3,4-dihydro-2H-quinoxaline-1-carboxylic acid (5-carbamoyladamantan-2-yl)amide,
. trans-4-{4-[4-(Piperidine-4-sulphonyl)piperazin-1-yl]phenyl}-3,4-dihydro-2H-quinoxaline-1-carboxylic acid (5-carbamoyladamantan-2-yl)amide,
. trans-4-[4-(4-(tert-Butyl)piperazin-1-yl)phenyl]-3,4-dihydro-2H-quinoxaline-1-carboxylic acid (5-carbamoyladamantan-2-yl)amide,
. trans-4-[5-(4-(Methanesulphonyl)piperazin-1-yl)pyridin-2-yl]-3,4-dihydro-2H-quinoxaline-1-carboxylic acid (5-carbamoyladamantan-2-yl)amide,
. trans-4-{5-[4-(Propane-1-sulphonyl)piperazin-1-yl]pyridin-2-yl}-3,4-dihydro-2H-quinoxaline-1-carboxylic acid (5-hydroxyadamantan-2-yl)amide,
. trans-4-{5-[4-(2-Methylpropane-1-sulphonyl)piperazin-1-yl]pyridin-2-yl}-3,4-dihydro-2H-quinoxaline-1-carboxylic acid (5-hydroxyadamantan-2-yl)amide,
. trans-4-(5-(Morpholin-4-yl)pyridin-2-yl)-3,4-dihydro-2H-quinoxaline-1-carboxylic acid (5-hydroxyadamantan-2-yl)amide,
. trans-4-{4-[4-(2-Hydroxyethanesulphonyl)piperazin-1-yl]phenyl}-3,4-dihydro-2H-quinoxaline-1-carboxylic acid (5-carbamoyladamantan-2-yl)amide,
. trans-4-{4-[4-(Piperidin-4-yloxy)piperidin-1-yl]phenyl}-3,4-dihydro-2H-quinoxaline-1-carboxylic acid (5-hydroxyadamantan-2-yl)amide,
. trans-4-[4-(4-(Dimethylcarbamoyl)piperazin-1-yl)phenyl]-3,4-dihydro-2H-quinoxaline-1-carboxylic acid (5-hydroxyadamantan-2-yl)amide,
. trans-4-[6-(4-(Cyclopropanesulphonyl)piperazin-1-yl)pyridin-3-yl]-3,4-dihydro-2H-quinoxaline-1-carboxylic acid (5-carbamoyladamantan-2-yl)amide,
. trans-4-[4-(4-(Ethanesulphonyl)piperazin-1-yl)phenyl]-3,4-dihydro-2H-quinoxaline-1-carboxylic acid (5-carbamoyladamantan-2-yl)amide,
. trans-4-{4-[4-(2,2,2-Trifluoroethanesulphonyl)piperazin-1-yl]phenyl}-3,4-dihydro-2H-quinoxaline-1-carboxylic acid (5-carbamoyladamantan-2-yl)amide,
. trans-4-{4-[4-(2-Aminoacetyl)piperazin-1-yl]phenyl}-3,4-dihydro-2H-quinoxaline-1-carboxylic acid (5-carbamoyladamantan-2-yl)amide,
. trans-4-[4-(5-(Hydroxymethyl)pyridin-2-yl)phenyl]-3,4-dihydro-2H-quinoxaline-1-carboxylic acid (5-carbamoyladamantan-2-yl)amide,
. trans-4-[5-(4-(Cyclopentanesulphonyl)piperazin-1-yl)pyridin-2-yl]-3,4-dihydro-2H-quinoxaline-1-carboxylic acid (5-hydroxyadamantan-2-yl)amide,
. trans-4-[4-(4-Isopropoxypiperidin-1-yl)phenyl]-3,4-dihydro-2H-quinoxaline-1-carboxylic acid (5-carbamoyladamantan-2-yl)amide,
. trans-4-{4-[5-(2,2,2-Trifluoroethoxy)pyridin-2-yl]phenyl}-3,4-dihydro-2H-quinoxaline-1-carboxylic acid (5-hydroxyadamantan-2-yl)amide,
. trans-4-{4-[5-(2,2,2-Trifluoroethoxy)pyridin-2-yl]phenyl}-3,4-dihydro-2H-quinoxaline-1-carboxylic acid (5-carbamoyladamantan-2-yl)amide,
. trans-4-[5-(4-Isopropoxyphenyl)pyrimidin-2-yl]-3,4-dihydro-2H-quinoxaline-1-carboxylic acid (5-carbamoyladamantan-2-yl)amide,
. trans-4-{5-[4-(Piperidin-4-yloxy)phenyl]pyrimidin-2-yl}-3,4-dihydro-2H-quinoxaline-1-carboxylic acid (5-carbamoyladamantan-2-yl)amide,
. trans-4-[5-(4-(Trifluoromethanesulphonyl)piperazin-1-yl)pyridin-2-yl]-3,4-dihydro-2H-quinoxaline-1-carboxylic acid (5-hydroxyadamantan-2-yl)amide,
. trans-4-[5-(4-(tert-Butyl)piperazin-1-yl)pyridin-2-yl]-3,4-dihydro-2H-quinoxaline-1-carboxylic acid (5-hydroxyadamantan-2-yl)amide,
. trans-4-[6-(4-(Ethanesulphonyl)piperazin-1-yl)pyridin-3-yl]-3,4-dihydro-2H-quinoxaline-1-carboxylic acid (5-carbamoyladamantan-2-yl)amide,
. trans-4-[5-(4-Cyclopropylpiperazin-1-yl)pyridin-2-yl]-3,4-dihydro-2H-quinoxaline-1-carboxylic acid (5-hydroxyadamantan-2-yl)amide,
. trans-4-[5-(4-(Cyclobutanesulphonyl)piperazin-1-yl)pyridin-2-yl]-3,4-dihydro-2H-quinoxaline-1-carboxylic acid (5-hydroxyadamantan-2-yl)amide,
. trans-4-[5-(4-Acetylpiperazin-1-yl)pyridin-2-yl]-3,4-dihydro-2H-quinoxaline-1-carboxylic acid (5-hydroxyadamantan-2-yl)amide,
. trans-4-{4-[4-(1-Ethylpropyl)piperazin-1-yl]phenyl}-3,4-dihydro-2H-quinoxaline-1-carboxylic acid (5-carbamoyladamantan-2-yl)amide,
. trans-4-{5-[4-(2-Methylpropane-1-sulphonyl)piperazin-1-yl]pyridin-2-yl}-3,4-dihydro-2H-quinoxaline-1-carboxylic acid (5-carbamoyladamantan-2-yl)amide,
. trans-4-{5-[4-(3,3,3-Trifluoropropane-1-sulphonyl)piperazin-1-yl]pyridin-2-yl}-3,4-dihydro-2H-quinoxaline-1-carboxylic acid (5-hydroxyadamantan-2-yl)amide,
. trans-4-{4-[4-(2,2,2-Trifluoroethyl)piperazin-1-yl]phenyl}-3,4-dihydro-2H-quinoxaline-1-carboxylic acid (5-carbamoyladamantan-2-yl)amide,
. trans-4-(4-{4-[2-(2-Methoxyethoxy)ethanesulphonyl]piperazin-1-yl}phenyl)-3,4-dihydro-2H-quinoxaline-1-carboxylic acid (5-carbamoyladamantan-2-yl)amide,
. trans-4-{5-[4-(2-Isopropoxyethanesulphonyl)piperazin-1-yl]pyridin-2-yl}-3,4-dihydro-2H-quinoxaline-1-carboxylic acid (5-carbamoyladamantan-2-yl)amide;
. 4-[5-(4-(Cyclopropylmethanesulphonyl)piperazin-1-yl)pyridin-2-yl]-3,4-dihydro-2H-quinoxaline-1-carboxylic acid (5-hydroxyadamantan-2-yl)amide,
. trans-4-[5-(4-(tert-Butyl)piperazin-1-yl)pyridin-2-yl]-3,4-dihydro-2H-quinoxaline-1-carboxylic acid (5-carbamoyladamantan-2-yl)amide,
. trans-4-[5-(4-Cyclopropylpiperazin-1-yl)pyridin-2-yl]-3,4-dihydro-2H-quinoxaline-1-carboxylic acid (5-carbamoyladamantan-2-yl)amide,
. trans-4-{4-[4-(Tetrahydropyran-4-yl)piperazin-1-yl]phenyl}-3,4-dihydro-2H-quinoxaline-1-carboxylic acid (5-carbamoyladamantan-2-yl)amide,
. trans-4-{5-[4-(2-Methoxyethanesulphonyl)piperazin-1-yl]pyridin-2-yl}-3,4-dihydro-2H-quinoxaline-1-carboxylic acid (5-carbamoyladamantan-2-yl)amide,
. trans-4-[4-(7-Methanesulphonyl-2,7-diazaspiro[4.4]non-2-yl)phenyl]-3,4-dihydro-2H-quinoxaline-1-carboxylic acid (5-hydroxyadamantan-2-yl)amide,
. trans-4-{5-[4-(2,2-Dimethylpropyl)piperazin-1-yl]pyridin-2-yl}-3,4-dihydro-2H-quinoxaline-1-carboxylic acid (5-hydroxyadamantan-2-yl)amide,
. trans-4-{5-[4-(2-Methoxy-1-(methoxymethyl)ethyl)piperazin-1-yl]pyridin-2-yl}-3,4-dihydro-2H-quinoxaline-1-carboxylic acid (5-hydroxyadamantan-2-yl)amide,
. trans-4-{5-[4-(3-Methoxypropyl)piperazin-1-yl]pyridin-2-yl}-3,4-dihydro-2H-quinoxaline-1-carboxylic acid (5-hydroxyadamantan-2-yl)amide,
. trans-4-{5-[4-(3-Methylbutyryl)piperazin-1-yl]pyridin-2-yl}-3,4-dihydro-2H-quinoxaline-1-carboxylic acid (5-hydroxyadamantan-2-yl)amide,
. trans-4-[4-(4-(Methanesulphonyl)piperazin-1-yl)phenyl]-3,4-dihydro-2H-quinoxaline-1-carboxylic acid (5-(acetylamino)adamantan-2-yl)amide,
. trans-4-{5-[4-(2-Methoxyethanesulphonyl)piperazin-1-yl]pyridin-2-yl}-3,4-dihydro-2H-quinoxaline-1-carboxylic acid (5-hydroxyadamantan-2-yl)amide,
. trans-4-{5-[4-(2,2-Dimethylpropionyl)piperazin-1-yl]pyridin-2-yl}-3,4-dihydro-2H-quinoxaline-1-carboxylic acid (5-hydroxyadamantan-2-yl)amide,
. trans-4-(4,4-Difluoro-3,4,5,6-tetrahydro-2H-1,3'-bipyridinyl-6'-yl)-3,4-dihydro-2H-quinoxaline-1-carboxylic acid (5-hydroxyadamantan-2-yl)amide,
. trans-4-(4-(Pyrrolidin-1-yl)-3,4,5,6-tetrahydro-2H-1,3'-bipyridinyl-6'-yl)-3,4-dihydro-2H-quinoxaline-1-carboxylic acid (5-hydroxyadamantan-2-yl)amide,
. trans-4-[5-(5-Cyclopropanesulphonyl-2,5-diazabicyclo[2.2.1]hept-2-yl)pyridin-2-yl]-3,4-dihydro-2H-quinoxaline-1-carboxylic acid (5-hydroxyadamantan-2-yl)amide,
. trans-4-{6-[4-(5-Hydroxyadamantan-2-ylcarbamoyl)-3,4-dihydro-2H-quinoxalin-1-yl]-pyridin-3-yl}piperazine-1-carboxylic acid tert-butyl ester,
. trans-4-{4-[4-(2-Methoxyethyl)piperazin-1-yl]phenyl}-3,4-dihydro-2H-quinoxaline-1-carboxylic acid (5-carbamoyladamantan-2-yl)amide,
. trans-4-[5-(4-(Methanesulphonyl)piperazin-1-yl)-3-methylpyridin-2-yl]-3,4-dihydro-2H-quinoxaline-1-carboxylic acid (5-carbamoyladamantan-2-yl)amide,
. trans-2-(4-{4-[4-(5-Carbamoyladamantan-2-ylcarbamoyl)-3,4-dihydro-2H-quinoxalin-1-yl]-phenyl}piperazin-1-yl)-2-methylpropionic acid,
. trans-4-(4-(tert-Butyl)-3,4,5,6-tetrahydro-2H-1,3'-bipyridinyl-6'-yl)-3,4-dihydro-2H-quinoxaline-1-carboxylic acid (5-carbamoyladamantan-2-yl)amide,
. trans-4-(4-(Pyrrolidin-1-yl)-3,4,5,6-tetrahydro-2H-1,3'-bipyridinyl-6'-yl)-3,4-dihydro-2H-quinoxaline-1-carboxylic acid (5-carbamoyladamantan-2-yl)amide,
. trans-4-[5-(4-(Ethanesulphonyl)piperazin-1-yl)pyridin-2-yl]-3,4-dihydro-2H-quinoxaline-1-carboxylic acid (5-carbamoyladamantan-2-yl)amide,
. trans-4-[5-(4-(Methanesulphonyl)piperazin-1-yl)-6-methylpyridin-2-yl]-3,4-dihydro-2H-quinoxaline-1-carboxylic acid (5-carbamoyladamantan-2-yl)amide,
. trans-4-(4,4-Difluoro-3,4,5,6-tetrahydro-2H-1,3'-bipyridinyl-6'-yl)-3,4-dihydro-2H-quinoxaline-1-carboxylic acid (5-carbamoyladamantan-2-yl)amide,
. trans-4-{4-[4-(2-Hydroxy-1,1-dimethylethyl)piperazin-1-yl]phenyl}-3,4-dihydro-2H-quinoxaline-1-carboxylic acid (5-carbamoyladamantan-2-yl)amide,
. trans-4-{5-[4-(Propane-2-sulphonyl)piperazin-1-yl]-pyridin-2-yl}-3,4-dihydro-2H-quinoxaline-1-carboxylic acid (5-carbamoyladamantan-2-yl)amide,
. trans-4-(5-(Morpholin-4-yl)-pyridin-2-yl)-3,4-dihydro-2H-quinoxaline-1-carboxylic acid (5-carbamoyladamantan-2-yl)amide,
. trans-4-[5-(4-(Methanesulphonyl)piperazin-1-yl)-4-methylpyridin-2-yl]-3,4-dihydro-2H-quinoxaline-1-carboxylic acid (5-carbamoyladamantan-2-yl)amide,
. trans-4-(4-Trifluoromethyl-3,4,5,6-tetrahydro-2H-1,3'-bipyridinyl-6'-yl)-3,4-dihydro-2H-quinoxaline-1-carboxylic acid (5-carbamoyladamantan-2-yl)amide,
. trans-4-(4-(tert-Butyl)-3,4,5,6-tetrahydro-2H-1,3'-bipyridinyl-6'-yl)-3,4-dihydro-2H-quinoxaline-1-carboxylic acid (5-hydroxyadamantan-2-yl)amide,
. trans-4-[5-(4-Isobutylpiperazin-1-yl)pyridin-2-yl]-3,4-dihydro-2H-quinoxaline-1-carboxylic acid (5-carbamoyladamantan-2-yl)amide,
. trans-4-[5-(4-(Cyclopropylmethyl)piperazin-1-yl)pyridin-2-yl]-3,4-dihydro-2H-quinoxaline-1-carboxylic acid (5-carbamoyladamantan-2-yl)amide,
. trans-4-{5-[4-(2,2,2-Trifluoroethyl)piperazin-1-yl]pyridin-2-yl}-3,4-dihydro-2H-quinoxaline-1-carboxylic acid (5-carbamoyladamantan-2-yl)amide,
. trans-4-[5-(4-(tert-Butyl)piperazin-1-yl)pyridin-2-yl]-3,4-dihydro-2H-quinoxaline-1-carboxylic acid (5-(hydroxymethyl)adamantan-2-yl)amide,
. trans-4-[5-(4-(tert-Butyl)piperazin-1-yl)pyridin-2-yl]-6,7-difluoro-3,4-dihydro-2H-quinoxaline-1-carboxylic acid (5-carbamoyladamantan-2-yl)amide,
. trans-4-{5-[4-(2,2-Dimethylpropionyl)piperazin-1-yl]pyridin-2-yl}-3,4-dihydro-2H-quinoxaline-1-carboxylic acid (5-carbamoyladamantan-2-yl)amide,
. trans-4-[5-(4-(tert-Butyl)piperazin-1-yl)pyrimidin-2-yl]-3,4-dihydro-2H-quinoxaline-1-carboxylic acid (5-carbamoyladamantan-2-yl)amide,
. trans-4-[5-(4-(Cyclopropanesulphonyl)piperazin-1-yl)pyridin-2-yl]-3,4-dihydro-2H-quinoxaline-1-carboxylic acid (5-cyanoadamantan-2-yl)amide,
. trans-4-[5-(1-(tert-Butyl)piperidin-4-yloxy)pyridin-2-yl]-3,4-dihydro-2H-quinoxaline-1-carboxylic acid (5-carbamoyladamantan-2-yl)amide,
. trans-{6'-[4-(5-Carbamoyladamantan-2-ylcarbamoyl)-3,4-dihydro-2H-quinoxalin-1-yl]-3,4,5,6-tetrahydro-2H-1,3'-bipyridinyl-4-yl}-acetic acid methyl ester,
. trans-6'-[4-(5-Carbamoyladamantan-2-ylcarbamoyl)-3,4-dihydro-2H-quinoxalin-1-yl]-3,4,5,6-tetrahydro-2H-1,3'-bipyridinyl-4-carboxylic acid ethyl ester,
. trans-4-[5-(4-Isopropylpiperazin-1-yl)pyridin-2-yl]-3,4-dihydro-2H-quinoxaline-1-carboxylic acid (5-carbamoyladamantan-2-yl)amide,
. trans-4-(1'-(tert-Butyl)-1',2',3',4',5',6'-hexahydro-3,4'-bipyridinyl-6-yl)-3,4-dihydro-2H-quinoxaline-1-carboxylic acid (5-carbamoyladamantan-2-yl)amide,
. trans-{6'-[4-(5-Carbamoyladamantan-2-ylcarbamoyl)-3,4-dihydro-2H-quinoxalin-1-yl]-3,4,5,6-tetrahydro-2H-1,3'-bipyridinyl-4-yl}acetic acid,
. trans-6'-[4-(5-Carbamoyladamantan-2-ylcarbamoyl)-3,4-dihydro-2H-quinoxalin-1-yl]-3,4,5,6-tetrahydro-2H-1,3'-bipyridinyl-4-carboxylic acid,
. trans-2-(4-{6-[4-(5-Carbamoyladamantan-2-ylcarbamoyl)-3,4-dihydro-2H-quinoxalin-1-yl]-pyridin-3-yl}-piperazin-1-yl)-2-methylpropionic acid ethyl ester,
. trans-4-(4,4-Dimethyl-3,4,5,6-tetrahydro-2H-1,3'-bipyridinyl-6'-yl)-3,4-dihydro-2H-quinoxaline-1-carboxylic acid (5-carbamoyladamantan-2-yl)amide,
. trans-4-{5-[4-(2,2-Difluorocyclopropylmethyl)piperazin-1-yl]pyridin-2-yl}-3,4-dihydro-2H-quinoxaline-1-carboxylic acid (5-carbamoyladamantan-2-yl)amide,
. trans-4-{5-[4-(Difluoromethanesulphonyl)piperazin-1-yl]pyridin-2-yl}-3,4-dihydro-2H-quinoxaline-1-carboxylic acid (5-carbamoyladamantan-2-yl)amide,
. trans-4-{5-[4-(3,3,3-Trifluoropropyl)piperazin-1-yl]pyridin-2-yl}-3,4-dihydro-2H-quinoxaline-1-carboxylic acid (5-carbamoyladamantan-2-yl)amide,
. trans-4-{5-[4-(2-(Methanesulphonyl)ethyl)piperazin-1-yl]pyridin-2-yl}-3,4-dihydro-2H-quinoxaline-1-carboxylic acid (5-carbamoyladamantan-2-yl)amide,
. trans-4-{5-[4-(1-Ethylpropyl)piperazin-1-yl]pyridin-2-yl}-3,4-dihydro-2H-quinoxaline-1-carboxylic acid (5-carbamoyladamantan-2-yl)amide,
. trans-4-[5-(1,1-Dioxo-1λ⁶-thiomorpholin-4-yl)pyridin-2-yl]-3,4-dihydro-2H-quinoxaline-1-carboxylic acid (5-carbamoyladamantan-2-yl)amide,
. trans-4-{5-[4-(Tetrahydropyran-4-yl)piperazin-1-yl]pyridin-2-yl}-3,4-dihydro-2H-quinoxaline-1-carboxylic acid (5-carbamoyladamantan-2-yl)amide,
. trans-4-[5-(4-(Cyclopropanesulphonyl)piperazin-1-yl)-3-methylpyridin-2-yl]-3,4-dihydro-2H-quinoxaline-1-carboxylic acid (5-carbamoyladamantan-2-yl)amide,
. trans-4-{5-[4-(2-Hydroxy-1,1-dimethylethyl)-piperazin-1-yl]pyridin-2-yl}-3,4-dihydro-2H-quinoxaline-1-carboxylic acid (5-carbamoyladamantan-2-yl)amide,
. trans-4-{5-[4-(2-Fluoro-1,1-dimethylethyl)piperazin-1-yl]pyridin-2-yl}-3,4-dihydro-2H-quinoxaline-1-carboxylic acid (5-carbamoyladamantan-2-yl)amide,
. trans-4-[5-(4-(Trifluoromethanesulphonyl)piperazin-1-yl)pyridin-2-yl]-3,4-dihydro-2H-quinoxaline-1-carboxylic acid (5-carbamoyladamantan-2-yl)amide.

14. Process for the preparation of a compound of formula (I) according to any one of Claims 1 to 13, **characterized in that** a compound of formula (IV): 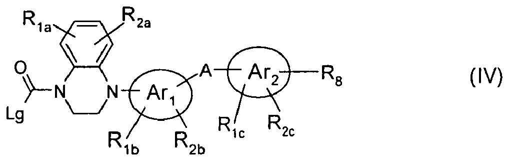 in which A, Ar₁, Ar₂, R_{1a,b,c}, R_{2a,b,c} and R₈ are as defined in any one of Claims 1 to 6 and 9 to 12 and Lg represents a leaving group, is reacted with a compound of formula (V): 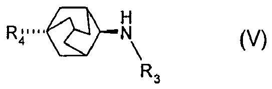 in which R₃ and R₄ are as defined in any one of Claims 1, 7, 8 and 10 to 12.

15. Preparation process according to Claim 14, **characterized in that** the compound of formula (IV) is obtained by reacting a compound of formula (II): 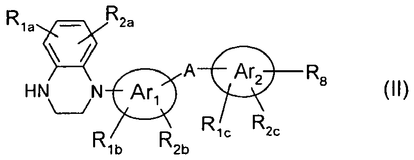 in which A, Ar₁, Ar₂, R_{1a,b,c}, R_{2a,b,c} and R₈ are as defined in any one of Claims 1 to 6 and 9 to 12, with a compound of formula Lg-CO-Lg, where Lg represents leaving groups, in the presence of a base.

16. Process for the preparation of a compound of formula (I) according to any one of Claims 1 to 13, in which A represents a bond, **characterized in that** a compound of formula (XXVII): 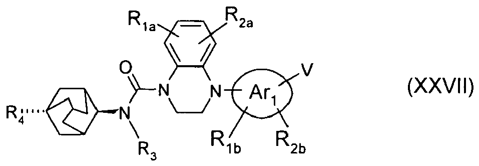 in which Ar₁, R_{1a,b}, R_{2a,b}, R₃ and R₄ are as defined in any one of Claims 1, 3, 5, 7, 8 and 10 to 13 and V represents a leaving group, is reacted with a compound of formula (XVI): 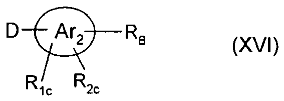 in which Ar₂, R_{1c}, R_{2c} and R₈ are as defined in any one of Claims 1, 4, 6, 9 and 10 to 13 and D represents an organometallic group, in the presence of an organometallic catalyst and of a base.

17. Process for the preparation of a compound of formula (I) according to any one of Claims 1 to 13, in which R₄ represents an amide functional group, **characterized in that** a compound of formula (XXXIX): 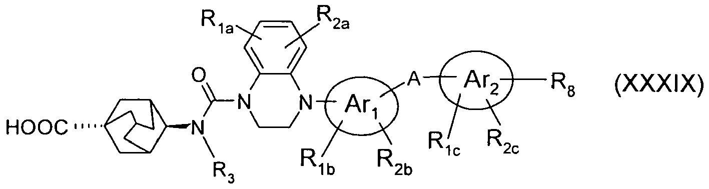 in which A, Ar₁, Ar₂, R_{1a,b,c}, R_{2a,b,c}, R₃ and R₈ are as defined in any one of Claims 1 to 7 and 9 to 13, is reacted with a compound of formula (XXXVI), H-NR₆R₇, where R₆ and R₇ are as defined in Claim 1, in the presence of a peptide coupling agent and of a base.

18. Process for the preparation of a compound of formula (I) according to any one of Claims 1 to 13, **characterized in that** a compound of formula (XLII): 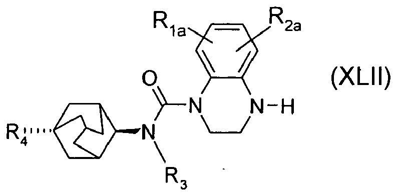 in which R₁ₐ, R₂ₐ, R₃ and R₄ are as defined in any one of Claims 1, 5, 7, 8 and 10 to 13, is reacted with a compound of formula (VII): 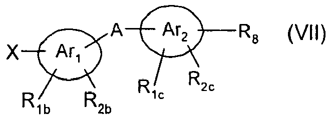 in which A, Ar₁, Ar₂, R_{1b,c}, R_{2b,c} and R₈ are as defined in any one of Claims 1, 2, 3, 4, 5, 6, 9 and 10 to 12 and X is a leaving group, in the presence of an organometallic catalyst and of a base.

19. Process for the preparation of a compound of formula (I) according to any one of Claims 1 to 13, in which Ar₂ is a piperazine group, A is a single bond directly connected to one of the two nitrogens of the piperazine and R_{1c} is connected to the other nitrogen atom of the piperazine, **characterized in that** a compound of formula (LII): 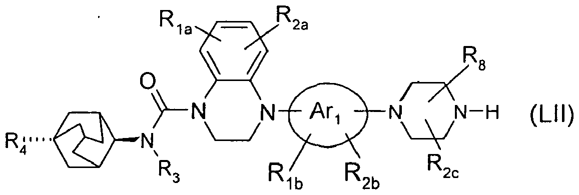 in which Ar₁, R_{1a,b}, R_{2a,b,c}, R₃, R₄ and R₈ are as defined in any one of Claims 1, 4, 6, 9 and 10 to 12, is reacted with a compound of formula (LIII), L_{g}-R_{1c}, in the presence of a base, or with a compound (LIV), O=R_{1c}, in the presence of a reducing agent.

20. Process for the preparation of a compound of formula (I) according to any one of Claims 1 to 13, in which R₄ is a halogen atom, **characterized in that** a compound of formula (LVI): 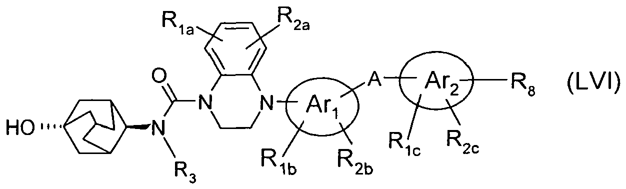 in which A, Ar₁, Ar₂, R_{1a,b,c}, R_{2a,b,c}, R₃ and R₈ are as defined in any one of Claims 1 to 7 and 9 to 12, is reacted with a halogenating reagent.

21. Compounds of formulae (II) and (IV): 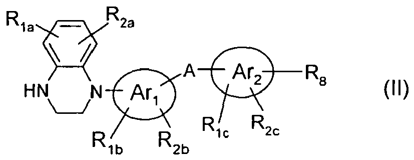 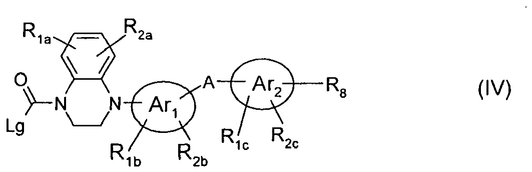 in which A, Ar₁, Ar₂, R_{1a,b,c} R_{2a,b,c} and R₈ are as defined in any one of Claims 1 to 6 and 9 to 12 and Lg represents a leaving group.

22. Compound of formula (XXVII): 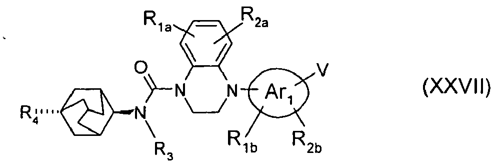 in which Ar₁, R_{1a,b}, R_{2a,b}. R₃ and R₄ are as defined in any one of Claims 1, 3, 5, 7, 8 and 10 to 12 and V represents a leaving group.

23. Medicament, **characterized in that** it comprises a compound of formula (I) according to any one of Claims 1 to 13 or an addition salt of this compound with a pharmaceutically acceptable acid or base or also a solvate of the compound of formula (I).

24. Pharmaceutical composition, **characterized in that** it comprises a compound of formula (I) according to any one of Claims 1 to 13 or a pharmaceutically acceptable salt or a solvate of this compound, and at least one pharmaceutically acceptable excipient.

25. Compound of formula (I) according to any one of Claims 1 to 13 for use thereof in the treatment and prevention of obesity, diabetes, microcirculatory disorders, insulin resistance, the metabolic syndrome, Cushing's syndrome, hypertension, atherosclerosis, cognition and dementia, glaucoma, osteoporosis, lipodystrophy, cardiac hypertrophy, cardiac insufficiency, liver diseases and some infectious diseases, by enhancing the effectiveness of the immune system, or of wounds, by promoting healing.
